(19) Europäisches Patentamt
European Patent Office
Office européen des brevets

(11) EP 4 674 859 A1

(12) **EUROPEAN PATENT APPLICATION**
published in accordance with Art. 153(4) EPC

(43) Date of publication:
**07.01.2026 Bulletin 2026/02**

(21) Application number: **24788847.2**

(22) Date of filing: **12.04.2024**

(51) International Patent Classification (IPC):
***C07K 1/14*** (2006.01) ***C07K 19/00*** (2006.01)
***C12N 15/12*** (2006.01) ***C12Q 1/6844*** (2018.01)
***G01N 33/50*** (2006.01) ***G01N 33/68*** (2006.01)

(52) Cooperative Patent Classification (CPC):
**C07K 1/14; C12Q 1/6844; G01N 33/50; G01N 33/68;** C07K 14/435; C07K 19/00

(86) International application number:
**PCT/JP2024/014861**

(87) International publication number:
**WO 2024/214825 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.04.2023 PCT/JP2023/015233**
**14.12.2023 PCT/JP2023/044862**

(71) Applicant: **CHUGAI SEIYAKU KABUSHIKI KAISHA**
**Tokyo 115-8543 (JP)**

(72) Inventors:
- **KOJIMA, Tetsuo**
**Yokohama City, Kanagawa 244-8602 (JP)**
- **KURIKI, Yugo**
**Yokohama City, Kanagawa 244-8602 (JP)**
- **YAMAGISHI, Yusuke**
**Yokohama City, Kanagawa 244-8602 (JP)**
- **OHARA, Kazuhiro**
**Yokohama City, Kanagawa 244-8602 (JP)**
- **OHTA, Atsushi**
**Kamakura-shi, Kanagawa 247-8530 (JP)**
- **YOSHII, Sakiko**
**Yokohama City, Kanagawa 244-8602 (JP)**
- **BUNNO, Reito**
**Yokohama City, Kanagawa 244-8602 (JP)**

(74) Representative: **Vossius & Partner**
**Patentanwälte Rechtsanwälte mbB**
**Siebertstrasse 3**
**81675 München (DE)**

Remarks:
The complete document including Reference Table(s) and the Sequence Listing(s) can be downloaded from the EPO website

(54) **METHOD FOR SCREENING MOLECULES OF INTEREST CAPABLE OF FORMING COMPLEXES WITH PLURALITY OF TYPES OF TARGET MOLECULES, AND METHOD FOR PRODUCING MOLECULES OF INTEREST INCLUDING SCREENING METHOD**

(57) The present invention relates to a method of screening for a molecule of interest, comprising: (1) a step of preparing a linked construct by linking a plurality of kinds of target molecules to each other, or linking one or more kinds of target molecules and a candidate molecule-nucleic acid conjugate (linking step); and (2) a step of contacting the linked construct in which the plurality of kinds of target molecules are linked to each other with a candidate molecule-nucleic acid conjugate, or contacting the linked construct in which the one or more kinds of target molecules and the candidate molecule-nucleic acid conjugate are linked with a target molecule that is not used in the linking step (contacting step).



Processed by Luminess, 75001 PARIS (FR)

## Description

### Technical Field

The present invention relates to a method of screening for a molecule of interest capable of forming a complex with a plurality of kinds of target molecules, and a method of producing a molecule of interest, comprising the screening method.

### Background Art

In recent years, with progress of searching for drug targets, not only molecules capable of binding to a single target molecule as conventionally, but also molecules capable of forming a complex with a plurality of kinds of target molecules have been sought.

As a method of obtaining such a molecule, for example, Non Patent Literature 2 proposes a method of confirming binding of FRB and FKBP12 with rapamycin by fluorescence with Split-EGFP or by luminescence with Split-luciferase. Non Patent Literatures 3 and 4 further disclose a method of screening under a plurality of conditions including changes of the kind of target molecules used and with or without immobilization to a solid phase support. In addition, various methods for obtaining a molecule of interest capable of forming a complex with a plurality of kinds of target molecules have also been proposed in the art of antibodies. For example, Patent Literature 1 discloses a method of obtaining an antibody that recognizes the interface between PD-1 and PD-L1 by immunizing an animal with a fusion protein of the two proteins, and Non Patent Literature 1 discloses a method of obtaining an antibody that recognizes the interface between IL6 and gp80 (IL6R) by immunizing an animal with a fusion protein of the two proteins.

### Citation List

#### Patent Literature

Patent Literature 1: International Publication No. WO2021/235509

#### Non Patent Literature

Non Patent Literature 1: Scientific Reports, 7: 37716
Non Patent Literature 2: Cancer Res., 2005 August, 15; 65 (16): 7413-7420
Non Patent Literature 3: ACS Chem. Biol., 2023, 18, 1, 25-33
Non Patent Literature 4: https://doi.org/10.1101/2022.10.13.512184

### Summary of Invention

#### Technical Problem

The Patent Literature 1 and the Non Patent Literature 1 merely disclose a method of obtaining an antibody of interest by an immune reaction using a fusion protein, and do not disclose or suggest an application to a screening using a fusion protein.

The Non Patent Literature 2 only discloses a method of confirming binding of FRB and FKBP12 by emission by luciferase or fluorescence by EGFP. This method is difficult to apply to highly diverse libraries and has a possibility that molecules that can associate with Split-luciferase or Split-EGFP rather than a molecule capable of binding a target molecule such as FRB and FKBP12 are obtained because the luminescence or fluorescence is generated by the association of the labeled molecule Split-luciferase or Split-EGFP binding thereto.

The Non Patent Literatures 3 and 4 neither disclose nor suggest that a plurality of kinds of target molecules in proximity be contacted with the display library.

When it is sought to obtain a molecule capable of forming a complex with a plurality of kinds of target molecules, known methods include (i) a method including screening for candidate molecules for each target molecule, and linking the obtained candidate molecules with each other, and (ii) a method including obtaining a candidate molecule capable of forming a complex with one target molecule, and then contacting the complex of the target molecule and the candidate molecule with another target molecule.

For example, when increasing oral absorption or targeting an intracellular target, one potent method is to reduce

the molecular weight of a molecule of interest to some extent. Thus, when aiming to obtain a molecule of interest for such purposes, the method (i) including obtaining candidate molecules capable of forming complexes with each target molecule and then linking the obtained candidate molecules with each other will impose a significant limitation on the upper limit of the molecular weight of each candidate molecule. Contrarily, when the molecular weight of the candidate molecule is too small, the structure of the candidate molecule and the binding site of the target molecule that can be used for binding are limited, thus the applicable target molecules are limited. Meanwhile, the method (ii) is a method including screening a library for a molecule of interest capable of forming a complex with a plurality of kinds of target molecules, without linking two candidate molecules with each other, and thus the first step is a screening based on mono-specificity for a single target molecule. In this step, candidate molecules having a predominant binding mode to the target (first target) molecule are concentrated, and thus, in the next step, a population of candidate molecules that are predominated in the binding mode to the first target molecule must be screened for the molecule of interest. The predominant binding mode to the first target molecule does not necessarily match the binding mode to form a complex with the plurality of kinds of target molecules, thus the diversity of the candidate molecules to be screened for is limited. Thus, a screening method that is capable of directly selecting a molecule of interest from a library which may include a variety of binding modes to each target molecule is desired.

In addition, as seeking more diverse properties of the molecule of interest besides the binding activity to a single target molecule, the need for screening a library containing a variety of candidate molecules increases. Furthermore, to form a complex with a plurality of target molecules, the molecule of interest is required to provide a complicated binding and reaction mode, which makes a prediction of the optimal molecular structure difficult. Thus, a screening method that is applicable to as diverse libraries as possible, easily selects and identifies a molecule of interest, and has a high throughput performance is required.

The present invention has been made considering such circumstances, and an object of the present invention is to provide a screening method that can efficiently obtain a molecule of interest capable of forming a complex with a plurality of kinds of target molecules. Another object of the present invention is to provide a method of efficiently producing a molecule of interest, comprising the screening method.

Solution to Problem

As a result of intensive studies to solve the above problems, the present inventors have found that a molecule of interest can be obtained by contacting a candidate molecule-nucleic acid conjugate library with a plurality of kinds of target molecules in proximity to each other, recovering a candidate molecule-nucleic acid conjugate capable of forming a complex with the plurality of kinds of target molecules from the library, and identifying a molecule of interest that is a molecule capable of forming a complex with the plurality of kinds of target molecules from the recovered candidate molecule-nucleic acid conjugate.

The present inventors have also found that a molecule of interest capable of forming a complex with the plurality of kinds of target molecules can be efficiently obtained by the method of the present disclosure, comprising a step of linking at least two of the components used in the screening (linking a plurality of kinds of target molecules to each other, or linking one or more kinds of target molecules and a candidate molecule-nucleic acid conjugate).

The present inventors have also found that a molecule of interest can be obtained by a screening comprising: (1) a step of preparing a linked construct by linking a plurality of kinds of target molecules to each other, or linking one or more kinds of target molecules and a candidate molecule-nucleic acid conjugate (linking step); and (2) a step of contacting the linked construct in which the plurality of kinds of target molecules are linked to each other with a candidate molecule-nucleic acid conjugate, or contacting the linked construct in which the one or more kinds of target molecules and the candidate molecule-nucleic acid conjugate are linked with a target molecule that is not used in the linking step (contacting step).

The present inventors have also found that a molecule of interest can be obtained by a screening comprising contacting a linked construct in which a plurality of kinds of target molecules are linked to each other with a candidate molecule-nucleic acid conjugate, or contacting a linked construct in which one or more kinds of target molecules and a candidate molecule-nucleic acid conjugate are linked with a target molecule that is not contained in the linked construct (contacting step).

The screening method provided by the present invention enables efficient selection of a molecule of interest capable of forming a complex with the plurality of kinds (e.g., two or more kinds) of target molecules from candidate molecules included in a library. Without intention to be bound by a particular theory, it is believed that this is because, in the screening method provided by the present invention, the complex formed by the molecule of interest and the plurality of kinds of target molecules is more stable when the plurality of kinds of target molecules are in proximity.

The use of a display library including (i) the plurality of kinds of target molecules in proximity to each other and (ii) a candidate molecule-nucleic acid conjugate in the screening method provided by the present invention also contributes to efficiently obtaining the molecule of interest.

Initially, it had been predicted that even when a candidate molecule-nucleic acid conjugate library is contacted with a plurality of kinds of target molecules in proximity to each other, a molecule capable of forming a complex with only one kind of target molecule that constitutes the plurality of kinds of target molecules would be predominantly obtained. However, it has been found surprisingly that a molecule capable of forming a complex with the plurality of kinds of target molecules is predominantly obtained.

The present invention can obtain a molecule of interest capable of forming a complex with a plurality of kinds of target molecules by applying the property, that is a property of predominantly obtaining a molecule capable of forming a complex with a plurality of kinds of target molecules by contacting the plurality of kinds of target molecules in proximity to each other, to not only panning but also identification of the molecule of interest.

As shown in Examples, the plurality of kinds of target molecules are not required to have the property of binding to each other to make the plurality of kinds of target molecules be in proximity (for example, the dissociation constant of the plurality of kinds of target molecules to each other may be more than 10 μM), and, for example, the plurality of kinds of target molecules can be brought in proximity at a desired distance (e.g., 60 nm or less) by a fusion moiety, a solid phase support, or the like.

Without intention to be bound by a particular theory, it is believed that the molecule of interest obtained by the method of the present invention forms a complex with the plurality of kinds of target molecules by (i) binding to a binding site formed only when the plurality of kinds of target molecules are in proximity to each other, (ii) having two or more different sites that bind to each of the plurality of kinds of target molecules, (iii) binding to one or more kinds of target molecules and promoting binding to other kinds of target molecules, and the like.

The present invention is based on such findings, and encompasses, for example, the following.

[1] A method of screening for a molecule of interest, comprising: (1) a step of preparing a linked construct by linking a plurality of kinds of target molecules to each other, or linking one or more kinds of target molecules and a candidate molecule-nucleic acid conjugate (linking step); and (2) a step of contacting the linked construct in which the plurality of kinds of target molecules are linked to each other with a candidate molecule-nucleic acid conjugate, or contacting the linked construct in which the one or more kinds of target molecules and the candidate molecule-nucleic acid conjugate are linked with a target molecule that is not used in the linking step (contacting step).

[2] The method according to [1], wherein the method comprises: (1) a step of preparing a linked construct by linking a plurality of kinds of target molecules to each other (linking step); and (2) a step of contacting the linked construct prepared in the linking step with a candidate molecule-nucleic acid conjugate (contacting step).

[3] The method according to [1], wherein the method comprises: (1) a step of preparing a linked construct by linking one or more kinds of target molecules to a candidate molecule-nucleic acid conjugate (linking step); and (2) a step of contacting the linked construct prepared in the linking step with a target molecule that is not used in the linking step (contacting step).

[4] A method of screening for a molecule of interest, comprising: (1) a step of preparing a linked construct by linking a plurality of kinds of target molecules to each other (linking step); and (2) a step of contacting the linked construct prepared in the linking step with a candidate molecule-nucleic acid conjugate (contacting step).

[5] A method of screening for a molecule of interest, comprising: (1) a step of preparing a linked construct by linking one or more kinds of target molecules to a candidate molecule-nucleic acid conjugate (linking step); and (2) a step of contacting the linked construct prepared in the linking step with a target molecule that is not used in the linking step (contacting step).

[6] A method of screening for a molecule of interest, comprising a step of contacting a linked construct in which a plurality of kinds of target molecules are linked to each other with a candidate molecule-nucleic acid conjugate, or contacting a linked construct in which one or more kinds of target molecules and a candidate molecule-nucleic acid conjugate are linked with a target molecule that is not contained in the linked construct (contacting step).

[7] The method according to [6], wherein the method comprises a step of contacting a linked construct in which a plurality of kinds of target molecules are linked to each other with a candidate molecule-nucleic acid conjugate.

[8] The method according to [6], wherein the method comprises a step of contacting a linked construct in which one or more kinds of target molecules and a candidate molecule-nucleic acid conjugate are linked with a target molecule that is not contained in the linked construct.

[9] A method of screening for a molecule of interest, comprising a step of contacting a linked construct in which a plurality of kinds of target molecules are linked to each other with a candidate molecule-nucleic acid conjugate.

[10] A method of screening for a molecule of interest, comprising a step of contacting a linked construct in which one or more kinds of target molecules and a candidate molecule-nucleic acid conjugate are linked with a target molecule that is not contained in the linked construct.

[11] The method according to [1], [3], [5], [6], [8], or [10], wherein the one or more kinds of target molecules linked to the candidate molecule-nucleic acid conjugate are one kind of target molecule.

[12] A method of screening for a molecule of interest, comprising a step of contacting a plurality of kinds of target

molecules in proximity to each other with a candidate molecule-nucleic acid conjugate library (contacting step).
[13] The method according to [1], [2], [4], [6], [7], [9], or [12], wherein the plurality of kinds of target molecules are fused to each other to form a target fusion molecule.
[14] A method of screening for a molecule of interest, comprising a step of contacting a target fusion molecule in which a plurality of kinds of target molecules are fused to each other with a candidate molecule-nucleic acid conjugate library (contacting step).
[15] The method according to [1], [2], [4], [6], [7], [9], [12], [13], or [14], wherein the plurality of kinds of target molecules form a target fusion molecule via a fusion moiety.
[16] The method according to [15], wherein the fusion moiety is a peptide consisting of 150 or less amino acid residues.
[17] The method according to [15] or [16], wherein N-termini of the plurality of kinds of target molecules are conjugated via the fusion moiety.
[18] The method according to [15] or [16], wherein C-termini of the plurality of kinds of target molecules are conjugated via the fusion moiety.
[19] The method according to [15] or [16], wherein an N-terminus of a first target molecule in the plurality of kinds of target molecules and a C-terminus of a second target molecule in the plurality of kinds of target molecules are conjugated via the fusion moiety.
[20] The method according to any of [13] to [19], wherein the target fusion molecule is immobilized on a solid phase support.
[21] The method according to any of [1] to [20], wherein the candidate molecule-nucleic acid conjugate is immobilized on a solid phase support.
[22] The method according to [20] or [21], wherein the solid phase support has on its surface one or more selected from the group consisting of avidin, neutravidin, and streptavidin.
[23] The method according to [12], wherein the plurality of kinds of target molecules in proximity to each other are immobilized on a solid phase support.
[24] The method according to any of [1] to [23], wherein the plurality of kinds of target molecules are immobilized on the same solid phase support.
[25] A method of screening for a molecule of interest, comprising a step of contacting a plurality of kinds of target molecules immobilized on the same solid phase support with a candidate molecule-nucleic acid conjugate library (contacting step).
[26] The method according to [24] or [25], wherein $1 \times 10^{13}$ or more target molecules are immobilized per $m^2$ of a surface of the solid phase support.
[27] The method according to [26], comprising a step of contacting $1 x 10^{13}$ or more target molecules per $m^2$ of the surface of the solid phase support with the solid phase support.
[28] The method according to any of [23] to [27], wherein the solid phase support has on its surface one or more selected from the group consisting of avidin, neutravidin, and streptavidin.
[29] The method according to any of [1] to [28], wherein the contacting step includes a step of mixing the candidate molecule-nucleic acid conjugate library with the target molecule or the target fusion molecule.
[30] The method according to [1], [3], [5], [6], [8], [10], [11], or [12], wherein the contacting step is a step of contacting a first target molecule, which is one kind selected from the plurality of kinds of target molecules, with a linked construct library including a plurality of kinds of linked constructs in which each candidate molecule-nucleic acid conjugate and a target molecule different from the first target molecule are linked.
[31] A method of screening for a molecule of interest, comprising a step of contacting a first target molecule, which is one kind selected from a plurality of kinds of target molecules, with a linked construct library including a plurality of kinds of linked constructs in which each candidate molecule-nucleic acid conjugate and a target molecule different from the first target molecule are linked.
[32] The method according to [30] or [31], wherein each of the candidate molecule-nucleic acid conjugates and a target molecule different from the first target molecule form a linked construct via a linkage moiety.
[33] The method according to [32], wherein the linkage moiety contains a His tag and Ni-NTA.
[34] The method according to [33], wherein the Ni-NTA is one or more selected from the group consisting of mono-Ni-NTA, di-Ni-NTA, tetrakis-Ni-NTA, and Tris-Ni-NTA.
[35] The method according to any of [30] to [34], wherein the target molecule contains a His tag.
[36] The method according to any of [30] to [35], wherein the candidate molecule-nucleic acid conjugate contains Ni-NTA.
[37] The method according to any of [30] to [36], wherein the linked construct is immobilized on a solid phase support.
[38] The method according to any of [30] to [36], wherein the first target molecule is immobilized on a solid phase support.
[39] The method according to [37] or [38], wherein the solid phase support has on its surface one or more selected from the group consisting of avidin, neutravidin, and streptavidin.

[40] The method according to any of [30] to [39], wherein the contacting step includes a step of mixing the linked construct library with the first target molecule.

[41] The method according to any of [1] to [40], wherein, in the contacting step, the linked construct and a target molecule other than the linked construct are contacted with the candidate molecule-nucleic acid conjugate.

[42] The method according to [41], wherein the target molecule other than the linked construct is the same kind of target molecule as one of target molecules contained in the linked construct.

[43] The method according to [41] or [42], wherein the target molecule other than the linked construct is one kind of target molecule.

[44] The method according to any of [41] to [43], wherein the target molecule other than the linked construct is not immobilized on a solid phase support.

[45] The method according to any of [41] to [44], wherein, in the contacting step, a ratio of a concentration of the target molecule other than the linked construct to a concentration of the linked construct is 1 or more.

[45-1]

The method according to any of [41] to [45], wherein the contacting step is performed under conditions in which there are no molecules that are not included in the candidate molecule-nucleic acid conjugate library and that may selectively form a complex with the plurality of kinds of target molecules in proximity.

[46] The method according to any of [1] to [45-1], further comprising a step of recovering a complex of at least one kind of target molecule selected from the plurality of kinds of target molecules and the candidate molecule-nucleic acid conjugate (recovery step).

[47] The method according to [46], wherein the recovery in the recovery step is carried out by a method utilizing an affinity.

[48] The method according to [47], wherein the method utilizing an affinity is a pull-down method.

[49] The method according to [47] or [48], wherein the method utilizing an affinity is a method utilizing an affinity between biotin and a biotin-binding protein, an affinity between a His tag and Ni-NTA, or an affinity between an antigen and an antibody.

[50] The method according to any of [47] to [49], wherein the method utilizing an affinity is a method utilizing an affinity between biotin and a biotin-binding protein.

[51] The method according to any of [46] to [50], further comprising a step of eluting a nucleic acid in the candidate molecule-nucleic acid conjugate recovered by the recovery step (elution step).

[52] The method according to [51], wherein the elution in the elution step is carried out by one or more selected from the group consisting of a method using an enzyme, a method using light, and a method using heat.

[53] The method according to [51] or [52], wherein the elution in the elution step is carried out by a method using an enzyme.

[54] The method according to [52] or [53], wherein the enzyme is a TEV protease.

[55] The method according to any of [51] to [54], wherein the binding of the solid phase support and the target molecule is cleaved in the elution step.

[56] The method according to any of [51] to [55], wherein the binding of the target molecule and the candidate molecule-nucleic acid conjugate is cleaved in the elution step.

[57] The method according to any of [51] to [56], wherein the binding of the candidate molecule and the nucleic acid is cleaved in the elution step.

[58] The method according to any of [46] to [57], further comprising a step of amplifying a nucleic acid in a candidate molecule-nucleic acid conjugate forming the complex (amplification step).

[59] The method according to [58], wherein the amplification step is carried out after the elution step.

[60] The method according to [58] or [59], wherein a candidate molecule-nucleic acid conjugate library is newly generated from the nucleic acid amplified in the amplification step, and the newly generated candidate molecule-nucleic acid conjugate library is used in multiple repeating of the contacting step, the recovery step, and the amplification step.

[61] The method according to [59], wherein a candidate molecule-nucleic acid conjugate library is newly generated from the nucleic acid amplified in the amplification step, and the newly generated candidate molecule-nucleic acid conjugate library is used in multiple repeating of the contacting step, the recovery step, the elution step, and the amplification step.

[62] The method according to any of [46] to [61], further comprising a step of identifying a molecule of interest from a candidate molecule-nucleic acid conjugate forming the complex (identification step).

[63] The method according to [62], wherein the identification step is performed after the elution step and/or the amplification step.

[64] The method according to [62] or [63], wherein the identification step includes determining a sequence of the nucleic acid eluted in the elution step or the nucleic acid amplified in the amplification step.

[65] The method according to any of [62] to [64], wherein the identification step includes comparing a recovery rate of

the candidate molecule-nucleic acid conjugate when the plurality of kinds of target molecules in proximity to each other is contacted with the candidate molecule-nucleic acid conjugate library (recovery rate A), with a recovery rate of the candidate molecule-nucleic acid conjugate when only one kind of the target molecule selected from the plurality of kinds of target molecules is contacted with the candidate molecule-nucleic acid conjugate library (recovery rate B).

[66] The method according to [65], wherein the recovery rate B includes a recovery rate B1 of the case when only a first target molecule, which is one kind of the target molecule selected from the plurality of kinds of target molecules, is contacted with the candidate molecule-nucleic acid conjugate library as a target molecule, and a recovery rate B2 of the case when only a second target molecule different from the first target molecule is contacted with the candidate molecule-nucleic acid conjugate library, and wherein the method includes comparing the recovery rate A and the recovery rate B1 and comparing the recovery rate A and the recovery rate B2.

[67] The method according to [65], wherein the candidate molecule is identified as the molecule of interest when the recovery rate A is 5-fold or more compared to the recovery rate B.

[68] The method according to [66], wherein the candidate molecule is identified as the molecule of interest when the recovery rate A is 5-fold or more compared to both the recovery rate B1 and the recovery rate B2.

[69] The method according to any of [62] to [68], wherein the identification step includes comparing a recovery rate of the candidate molecule-nucleic acid conjugate when the plurality of kinds of target molecules in proximity to each other is contacted with the candidate molecule-nucleic acid conjugate library (recovery rate A), and a recovery rate of the candidate molecule-nucleic acid conjugate when the target molecule is absent (recovery rate C).

[70] The method according to [69], wherein the candidate molecule is identified as the molecule of interest when the recovery rate A is 5-fold or more compared to the recovery rate C.

[71] The method according to any of [62] to [70], wherein the identification step includes comparing (1) a recovery rate of the candidate molecule-nucleic acid conjugate when the plurality of kinds of target molecules in proximity to each other is contacted with the candidate molecule-nucleic acid conjugate library (recovery rate A), with (2) a recovery rate of the candidate molecule-nucleic acid conjugate when a first target molecule that is one kind of the target molecule selected from the plurality of kinds of target molecules and immobilized on a solid phase support and a target molecule that is different from the first target molecule and not immobilized on the solid phase support are contacted with the candidate molecule-nucleic acid conjugate library (recovery rate D).

[72] The method according to [71], wherein the recovery rate D includes a recovery rate D1 of the candidate molecule-nucleic acid conjugate when the first target molecule that is one kind of the target molecule selected from the plurality of kinds of target molecules and immobilized on a solid phase support and the target molecule that is different from the first target molecule and not immobilized on the solid phase support are contacted with the candidate molecule-nucleic acid conjugate library, and a recovery rate D2 of the case when replacing the first target molecule with a second target molecule, and wherein the method includes comparing the recovery rate A with the recovery rate D1 and comparing the recovery rate A with the recovery rate D2.

[73] The method according to [71], wherein the candidate molecule is identified as the molecule of interest when the recovery rate A is 5-fold or more compared to the recovery rate D.

[74] The method according to [72], wherein the candidate molecule is identified as the molecule of interest when the recovery rate A is 5-fold or more compared to both the recovery rate D1 and the recovery rate D2.

[75] The method according to any of [62] to [74], wherein the identification step includes all comparing the recovery rates A, B, C, and D to each other.

[76] The method according to any of [62] to [75], wherein the identification step includes all comparing each of the recovery rates B, C, and D to the recovery rate A.

[77] The method according to [76], wherein the candidate molecule is identified as the molecule of interest when the recovery rate A is 5-fold or more compared to any of the recovery rate B, the recovery rate C, or the recovery rate D.

[78] The method according to any of [62] to [77], wherein the identification step includes comparing the recovery rate D to the recovery rate B.

[79] The method according to [78], wherein the candidate molecule is identified as the molecule of interest when the recovery rate D is 5-fold or more compared to the recovery rate B.

[80] The method according to any of [1] to [79], wherein the method is carried out in vitro.

[81] The method according to any of [1] to [80], wherein the method is performed without using fluorescence by GFP or luminescence by luciferase.

[82] The method according to any of [1] to [81], wherein the library is a display library.

[83] The method according to any of [1] to [82], wherein the library is an mRNA display library.

[84] The method according to any of [1] to [81], wherein the library is a DNA encoded library.

[85] The method according to any of [1] to [84], wherein candidate molecules included in the library has a diversity of $1 \times 10^3$ or more.

[86] The method according to any of [1] to [85], wherein candidate molecules included in the library has a diversity of $1 \times 10^5$ or more.

[87] The method according to any of [1] to [86], wherein candidate molecules included in the library has a diversity of 1 $\times 10^7$ or more.

[88] The method according to any of [1] to [87], wherein candidate molecules included in the library has a diversity of 1 $\times 10^9$ or more.

[89] The method according to any of [1] to [88], wherein the nucleic acid is a nucleic acid encoding a candidate molecule forming a candidate molecule-nucleic acid conjugate with the nucleic acid.

[90] The method according to any of [1] to [89], wherein the nucleic acid is DNA.

[91] The method according to any of [1] to [90], wherein the nucleic acid is mRNA.

[92] The method according to any of [1] to [91], wherein, in 1 $\times 10^3$ or more candidate molecules among all candidate molecules included in the candidate molecule-nucleic acid conjugate library, a molecular weight of the candidate molecule is 300 g/mol or more and 5,000 g/mol or less.

[93] The method according to any of [1] to [92], wherein the candidate molecule is a peptide.

[94] The method according to [93], wherein, in 1 $\times 10^3$ or more candidate molecules among all candidate molecules included in the candidate molecule-nucleic acid conjugate library, the number of amino acid residues contained in the candidate molecule is 5 or more and 30 or less.

[95] The method according to [93] or [94], wherein, in 1 $\times 10^3$ or more candidate molecules among all candidate molecules included in the candidate molecule-nucleic acid conjugate library, the number of non-natural amino acid residues contained in the candidate molecule is 1 or more.

[96] The method according to any of [93] to [95], wherein, in 1 $\times 10^3$ or more candidate molecules among all candidate molecules included in the candidate molecule-nucleic acid conjugate library, the number of N-substituted amino acid residues contained in the candidate molecule is 1 or more.

[97] The method according to [96], wherein a substituent on the nitrogen atom of the N-substituted amino acid residue is a $C_1$-$C_6$ alkyl group.

[98] The method according to any of [93] to [97], wherein the candidate molecule has a cyclic portion.

[99] The method according to [98], wherein, in 1 $\times 10^3$ or more candidate molecules among all candidate molecules included in the candidate molecule-nucleic acid conjugate library, the number of amino acid residues constituting the cyclic portion is 5 or more and 15 or less.

[100] The method according to any of [93] to [99], wherein, in 1 $\times 10^3$ or more candidate molecules among all candidate molecules included in the candidate molecule-nucleic acid conjugate library, ClogP of the candidate molecule is 4 or more and 25 or less.

[101] The method according to any of [93] to [100], wherein, in 1 $\times 10^3$ or more candidate molecules among all candidate molecules included in the candidate molecule-nucleic acid conjugate library, ClogP/number of amino acid residues of the candidate molecule is 1.0 or more.

[102] The method according to any of [1] to [101], wherein the plurality of kinds of target molecules are two kinds or more and five kinds or less of target molecules.

[103] The method according to any of [1] to [102], wherein the plurality of kinds of target molecules are two kinds of target molecules.

[104] The method according to any of [1] to [103], wherein a dissociation constant of at least two target molecules to each other selected from the plurality of kinds of target molecules is more than 10 μM.

[105] The method according to any of [1] to [104], wherein all dissociation constants of the plurality of kinds of target molecules to each other are more than 10 μM.

[106] The method according to any of [1] to [105], wherein the target molecule is a protein.

[106-1]

The method according to any of [1] to [106], wherein the target molecule is a molecule present in a human body.

[106-2]

The method according to any of [1] to [106-1], wherein the target molecule is a molecule that does not contain a split protein.

[106-3]

The method according to any of [1] to [106-2], wherein the target molecule is a molecule that does not contain any of GFP and a fragment thereof.

[106-4]

The method according to any of [1] to [106-3], wherein the target molecule is a molecule that does not contain any of luciferase and a fragment thereof.

[106-5]

The method according to any of [1] to [106-4], wherein the target molecule is a molecule that does not contain any of a SNAP tag and a fragment thereof.

[106-6]

The method according to any of [1] to [106-5], wherein the target molecule is a molecule that does not contain any of ubiquitin and a fragment thereof.

[107] The method according to any of [1] to [106-5], wherein the target molecule includes one or more selected from the group consisting of an FK506 binding protein (FKBP), an FKBP-rapamycin binding protein (FRB), cyclophilin A (CyA), and calcineurin (Cn).
[108] The method according to any of [1] to [107], wherein FKBP is included as the target molecule.
[109] The method according to any of [1] to [107], wherein CyA is included as the target molecule.
[110] The method according to any of [1] to [107], wherein FKBP and FRB are included as the target molecule.
[111] The method according to any of [1] to [107], wherein CyA and Cn are included as the target molecule.
[112] The method according to any of [1] to [107], wherein the target molecule is FKBP and FRB.
[113] The method according to any of [1] to [107], wherein the target molecule is CyA and Cn.
[114] The method according to any of [1] to [107], wherein the target molecule is FKBP and Cn.
[115] The method according to any of [1] to [114], wherein the candidate molecule and the nucleic acid form a conjugate via a conjugation moiety.
[116] The method according to [115], wherein the conjugation moiety contains puromycin.
[117] The method according to any of [1] to [116], wherein a dissociation constant of at least two target molecules to each other selected from the plurality of kinds of target molecules in the presence of the molecule of interest of 10 μM is 10 μM or less.
[118] The method according to any of [1] to [117], wherein all dissociation constants of the plurality of kinds of target molecules to each other in the presence of the molecule of interest of 10 μM are 10 μM or less.
[119] The method according to any of [1] to [118], wherein the molecule of interest is a candidate molecule when the recovery rate A is 5-fold or more compared to the recovery rate B.
[120] The method according to any of [1] to [119], wherein the molecule of interest is a candidate molecule when the recovery rate A is 5-fold or more compared to the recovery rate C.
[121] The method according to any of [1] to [120], wherein the molecule of interest is a candidate molecule when the recovery rate A is 5-fold or more compared to the recovery rate D.
[122] The method according to any of [1] to [121], wherein the molecule of interest is a molecule capable of forming a complex with the plurality of kinds of target molecules.
[123] The method according to any of [1] to [122], wherein a value of a dissociation constant of the plurality of kinds of target molecules to each other in the presence of the molecule of interest of 10 μM is small compared to a value of a dissociation constant of the plurality of kinds of target molecules to each other in the absence of the molecule of interest.
[124] The method according to any of [1] to [123], wherein a value of a dissociation constant of the plurality of kinds of target molecules to each other in the presence of the molecule of interest of 10 μM is less than one-fifth compared to a value of a dissociation constant of the plurality of kinds of target molecules to each other in the absence of the molecule of interest.
[125] The method according to any of [1] to [124], wherein a value of a dissociation constant of the plurality of kinds of target molecules to each other in the presence of the molecule of interest of 10 μM is 30 μM or less.
[126] The method according to any of [1] to [125], wherein a value of a dissociation constant of the plurality of kinds of target molecules to each other in the presence of the molecule of interest of 10 μM is 30 μM or less and less than one-fifth compared to a value of a dissociation constant of the plurality of kinds of target molecules to each other in the absence of the molecule of interest.
[127] A method of producing a peptide, comprising the method according to any of [1] to [126].
[128] A method of producing a peptide, comprising the steps of:

(i) screening for the molecule of interest by the method according to any of [1] to [127]; and
(ii) producing a peptide based on an amino acid sequence of the molecule of interest selected in (i).

The present invention also includes, for example, the following.

[2-1]
A target molecule-candidate molecule linked construct in which the target molecule and the candidate molecule are linked via a linkage moiety.
[2-2]
A target molecule-candidate molecule-nucleic acid linked construct in which the target molecule and the candidate molecule are linked via a linkage moiety and the candidate molecule and the nucleic acid are conjugated via a conjugation moiety.

[2-3]
The linked construct according to [2-1] or [2-2], wherein the linkage moiety contains a His tag and Ni-NTA.
[2-4]
The linked construct according to [2-3], wherein the Ni-NTA is one or more selected from the group consisting of mono-Ni-NTA, di-Ni-NTA, tetrakis-Ni-NTA, and Tris-Ni-NTA.
[2-5]
The linked construct according to [2-3] or [2-4], wherein the target molecule includes a His tag.
[2-6]
The linked construct according to any of [2-3] to [2-5], wherein the candidate molecule-nucleic acid conjugate contains Ni-NTA.
[2-7]
The linked construct according to any of [2-1] to [2-6], wherein the conjugation moiety contains puromycin.
[2-8]
A method of producing a target molecule-candidate molecule linked construct by linking the target molecule and the candidate molecule via a linkage moiety.
[2-9]
A method of producing a target molecule-candidate molecule-nucleic acid linked construct by linking the target molecule and the candidate molecule via a linkage moiety and conjugating the candidate molecule and the nucleic acid via a conjugation moiety.
[2-10]
The production method according to [2-8] or [2-9], wherein the linkage moiety contains a His tag and Ni-NTA.
[2-11]
The production method according to [2-10], wherein the Ni-NTA is one or more selected from the group consisting of mono-Ni-NTA, di-Ni-NTA, tetrakis-Ni-NTA, and Tris-Ni-NTA.
[2-12]
The production method according to [2-10] or [2-11], wherein the target molecule contains a His tag.
[2-13]
The production method according to any of [2-10] to [2-12], wherein the candidate molecule-nucleic acid conjugate contains Ni-NTA.
[2-14]
The production method according to any of [2-8] to [2-13], wherein the conjugation moiety contains puromycin.

The present invention further includes, for example, the following.

[3-1]
A peptide having a structure represented by formula (I) or (II):

[Formula 1]

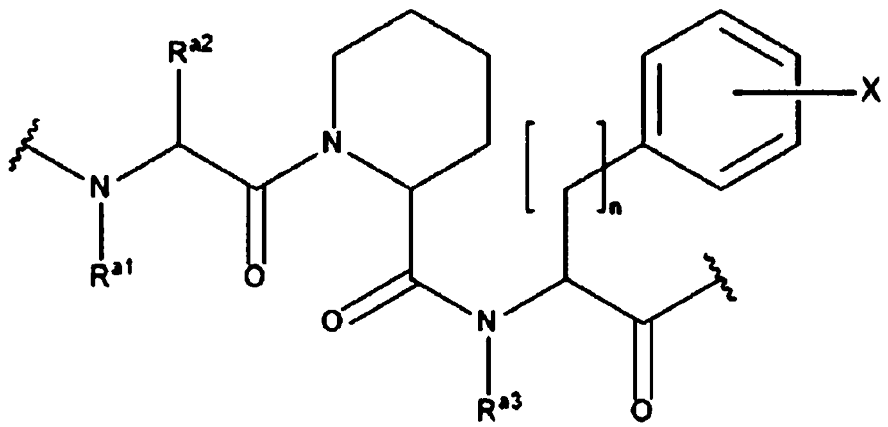

・・・(I)

[Formula 2]

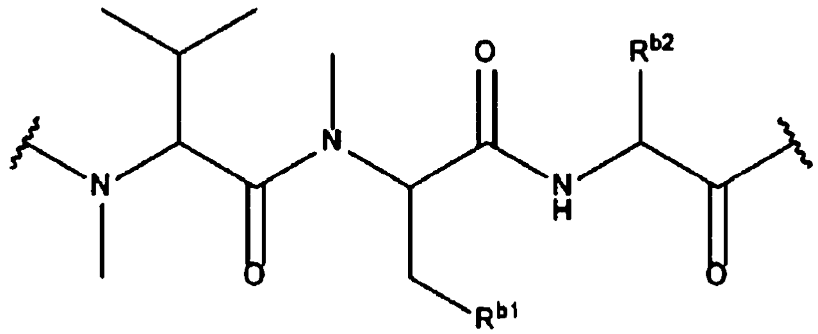

・・・(II)

wherein

[Formula 3]

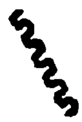

represents a bond,

in the formula (I), $R^{a1}$ is a hydrogen atom or $C_1$-$C_6$ alkyl, $R^{a2}$ is a hydrogen atom or $C_1$-$C_3$ alkyl optionally substituted with hydroxy, $R^{a3}$ is a hydrogen atom or methyl, X is halogen, and n is 1 or 2, and
in the formula (II), $R^{b1}$ is $C_1$-$C_6$ alkyl optionally substituted with any one selected from the group consisting of halogen and aryl, or -O-$R^{b3}$ wherein $R^{b3}$ is $C_1$-$C_3$ alkyl, and $R^{b2}$ is $C_1$-$C_4$ alkyl optionally substituted with a hydroxy.

[3-2]
The peptide according to [3-1], wherein the peptide has a structure represented by formula (I'):

[Formula 4]

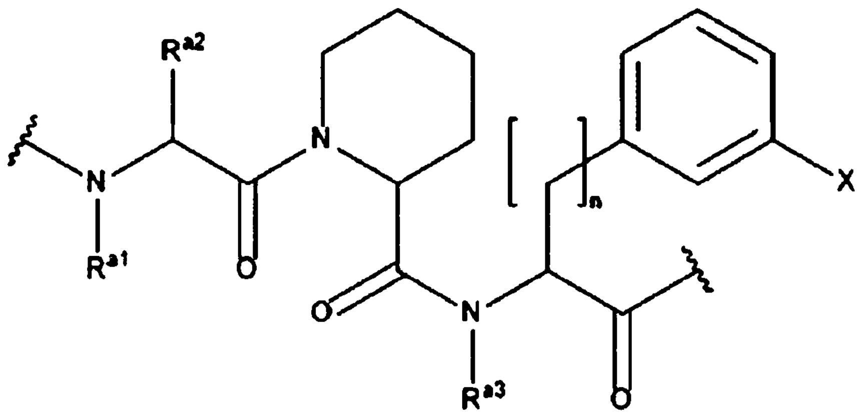

・・・(I')

wherein $R^{a1}$, $R^{a2}$, $R^{a3}$, n, and X are the same as $R^{a1}$, $R^{a2}$, $R^{a3}$, n, and X in the formula (I).
[3-3]
The peptide according to [3-1] or [3-2], wherein the peptide has a structure represented by the formula (I) or (I').
[3-4]
The peptide according to any of [3-1] to [3-3], wherein $R^{a1}$ is a hydrogen atom or $C_1$-$C_4$ alkyl.
[3-5]

The peptide according to any of [3-1] to [3-4], wherein $R^{a1}$ is $C_1$-$C_4$ alkyl.

[3-6]

The peptide according to any of [3-1] to [3-5], wherein $R^{a1}$ is methyl or n-butyl.

[3-7]

The peptide according to any of [3-1] to [3-6], wherein $R^{a2}$ is a hydrogen atom or hydroxymethyl.

[3-8]

The peptide according to any of [3-1] to [3-7], wherein $R^{a3}$ is a hydrogen atom.

[3-9]

The peptide according to any of [3-1] to [3-8], wherein X is a chlorine atom.

[3-10]

The peptide according to any of [3-1] to [3-9], wherein the peptide has a structure represented by formula (I-A1).

[Formula 5]

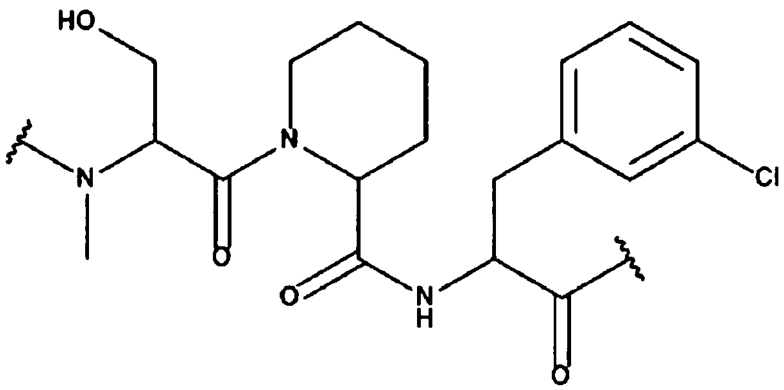

・・・(I-A1)

[3-11]

The peptide according to any of [3-1] to [3-10], wherein the peptide has a structure represented by formula (I-A2).

[Formula 6]

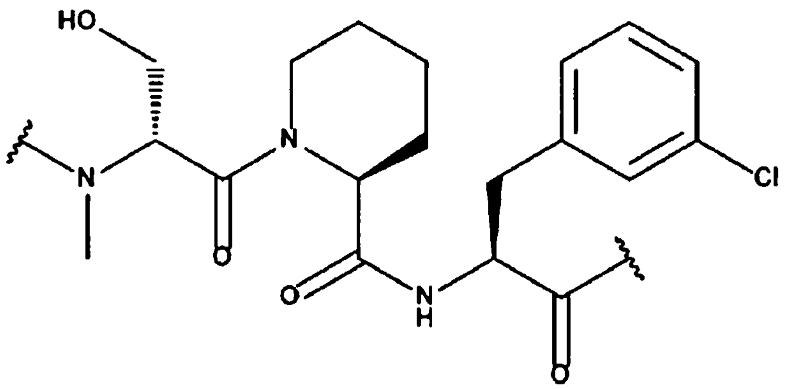

・・・(I-A2)

[3-12]

The peptide according to any of [3-1] to [3-9], wherein the peptide has a structure represented by formula (I-B1).

[Formula 7]

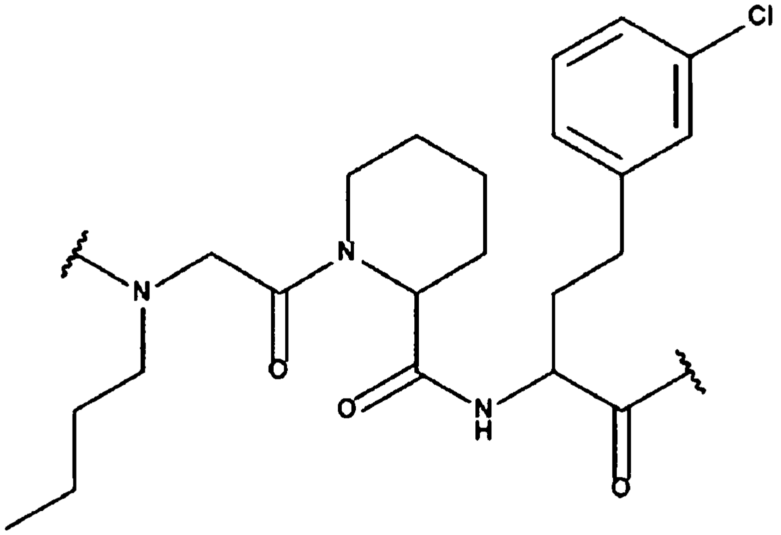

・・・(I－B1)

[3-13]
The peptide according to any of [3-1] to [3-9] and [3-12], wherein the peptide has a structure represented by formula (I-B2).

[Formula 8]

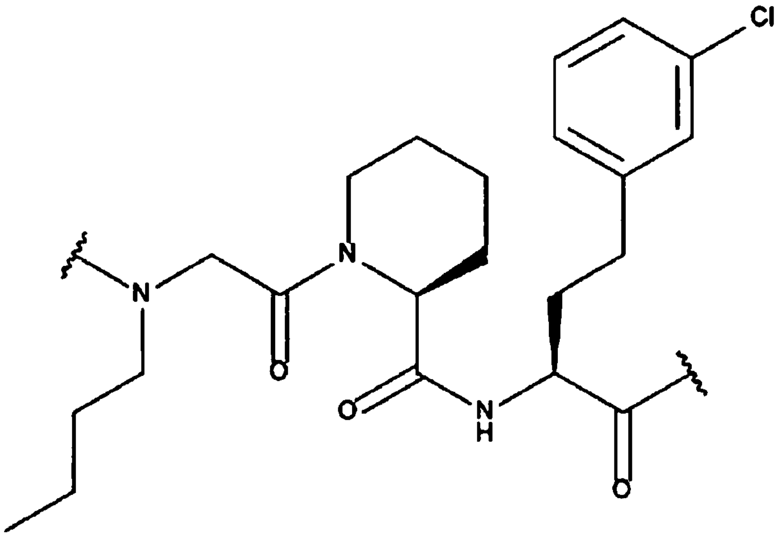

・・・(I－B2)

[3-14]
The peptide according to [3-1], wherein the peptide has a structure represented by formula (II).
[3-15]
The peptide according to [3-1] or [3-14], wherein $R^{b1}$ is $C_1$-$C_5$ alkyl optionally substituted with any one selected from the group consisting of halogen and aryl, or -O-$R^{b3}$ where $R^{b3}$ is n-propyl.
[3-16]
The peptide according to any of [3-1] and [3-14] to [3-15], wherein $R^{b1}$ is any one of the following formula (II-a), (II-b) or (II-c).

[Formula 9]

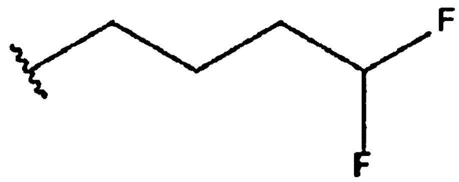

・・・(II-a)

[Formula 10]

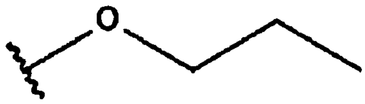

・・・(II-b)

[Formula 11]

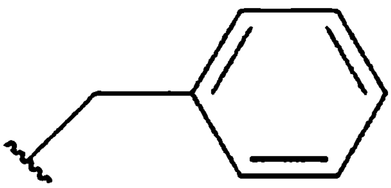

・・・(II-c)

[3-17]

The peptide according to any of [3-1] and [3-14] to [3-16], wherein $R^{b2}$ is 1-hydroxyethyl or 1-methylpropyl.

[3-18]

The peptide according to any of [3-1] and [3-14] to [3-17], wherein a structure represented by formula (II) is a structure represented by the following formula (II-A1).

[Formula 12]

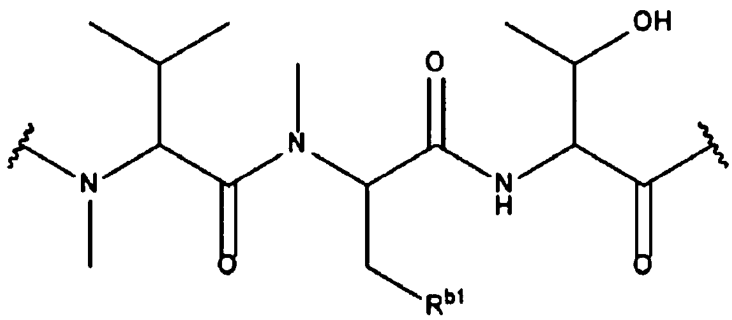

・・・(II-A1)

[3-19]

The peptide according to any of [3-1] and [3-14] to [3-18], wherein a structure represented by formula (II) is a structure represented by the following formula (II-A2).

[Formula 13]

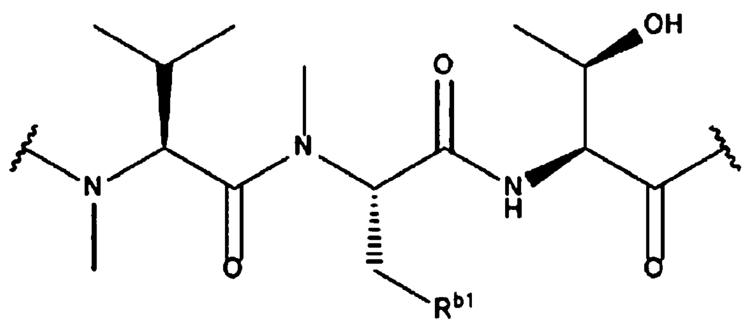

・・・(II-A2)

[3-20]
The peptide according to any of [3-1] and [3-14] to [3-18], wherein a structure represented by formula (II) is a structure represented by the following formula (II-Aa1).

[Formula 14]

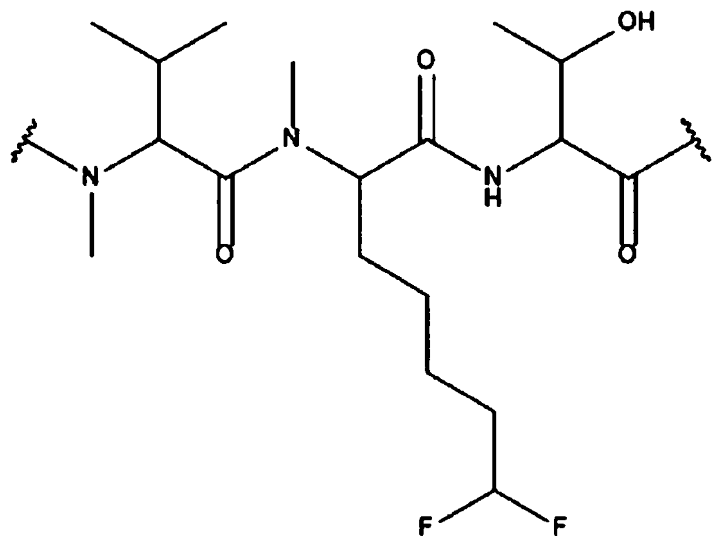

・・・(II-Aa1)

[3-21]
The peptide according to any of [3-1] and [3-14] to [3-20], wherein a structure represented by formula (II) is a structure represented by the following formula (II-Aa2).

[Formula 15]

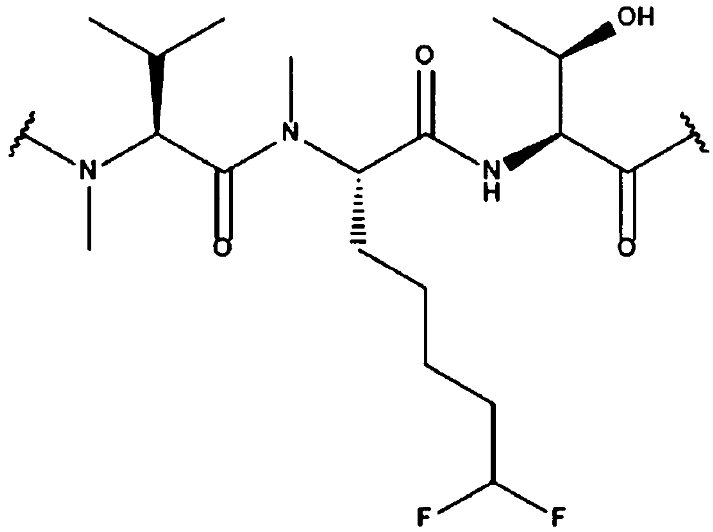

・・・(I I－A a 2)

[3-22]
The peptide according to any of [3-1] and [3-14] to [3-18], wherein a structure represented by formula (II) is a structure represented by the following formula (II-Ab1).

[Formula 16]

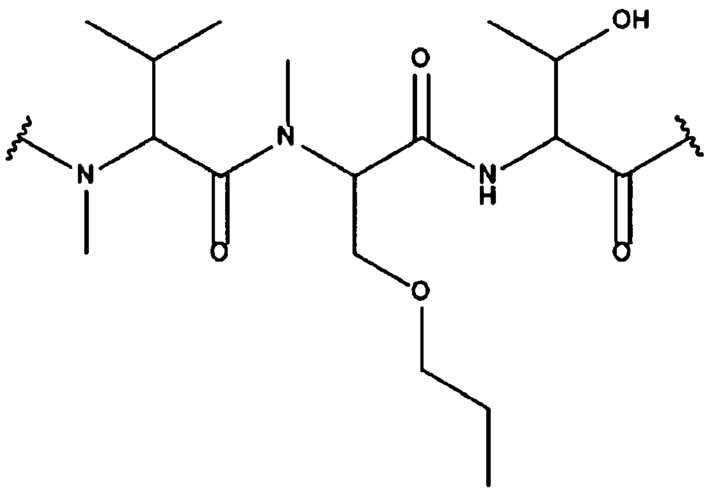

・・・(I I－A b 1)

[3-23]
The peptide according to any of [3-1], [3-14] to [3-19] and [3-22], wherein a structure represented by formula (II) is a structure represented by the following formula (II-Ab2).

[Formula 17]

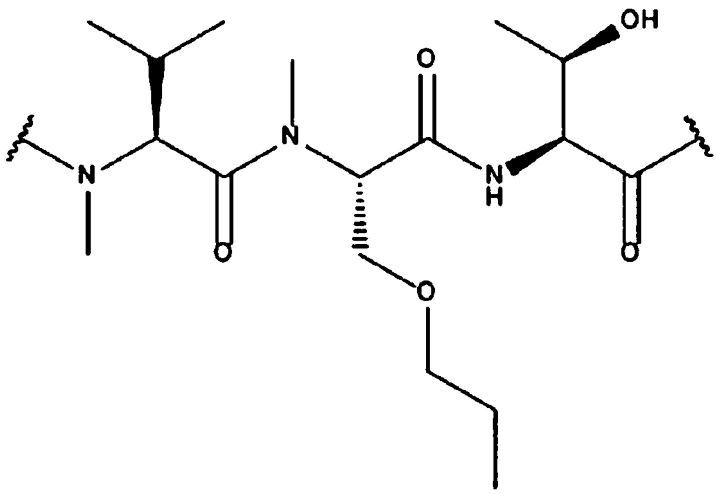

・・・(II−Ab2)

[3-24]
The peptide according to any of [3-1] and [3-14] to [3-18], wherein a structure represented by formula (II) is a structure represented by the following formula (II-Ac1).

[Formula 18]

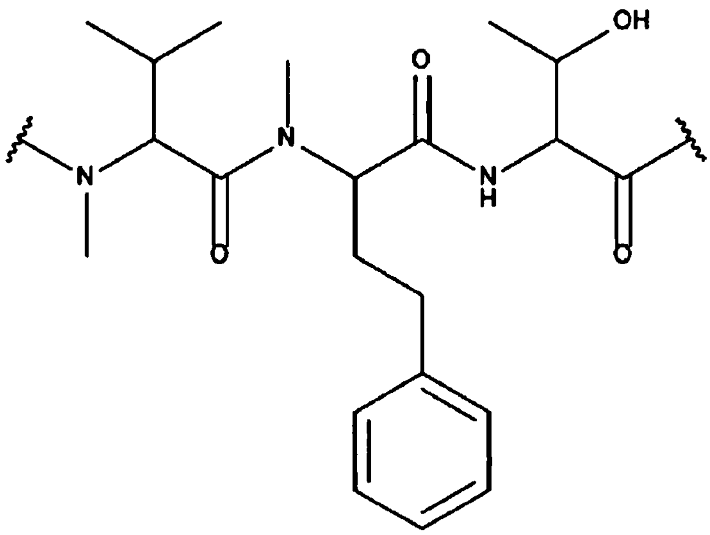

・・・(II−Ac1)

[3-25]
The peptide according to any of [3-1], [3-14] to [3-19] and [3-24], wherein a structure represented by formula (II) is a structure represented by the following formula (II-Ac2).

[Formula 19]

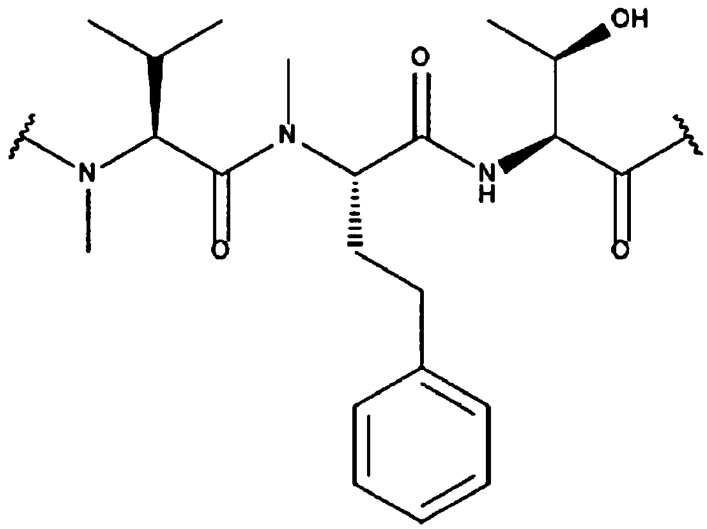

・・・(II-Ac2)

[3-26]

The peptide according to any of [3-1] and [3-14] to [3-17], wherein a structure represented by formula (II) is a structure represented by the following formula (II-B1).

[Formula 20]

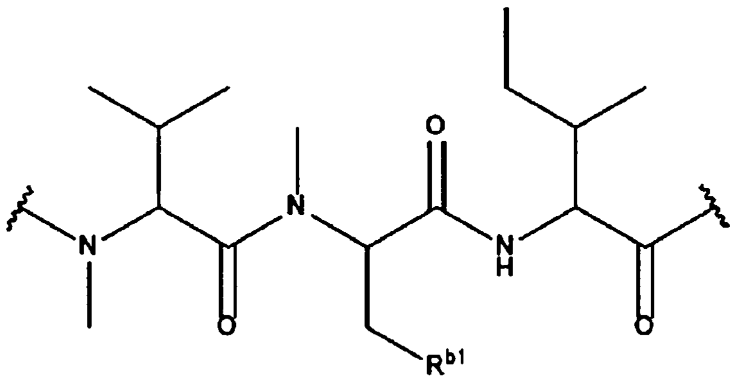

・・・(II-B1)

[3-27]

The peptide according to any of [3-1], [3-14] to [3-17] and [3-26], wherein a structure represented by formula (II) is a structure represented by the following formula (II-B2).

[Formula 21]

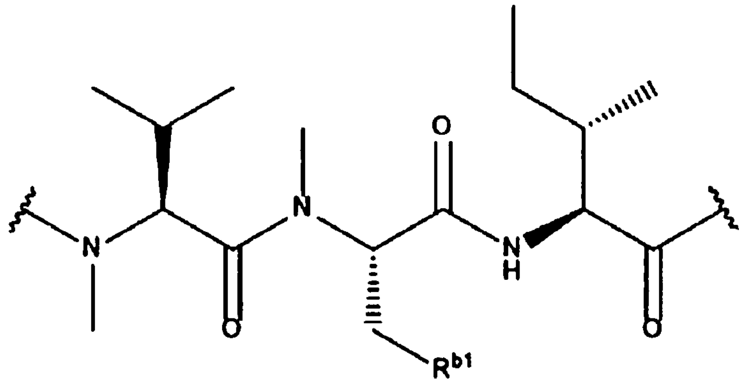

・・・(II-B2)

[3-28]
The peptide according to any of [3-1], [3-14] to [3-17] and [3-26], wherein a structure represented by formula (II) is a structure represented by the following formula (II-Ba1).

[Formula 22]

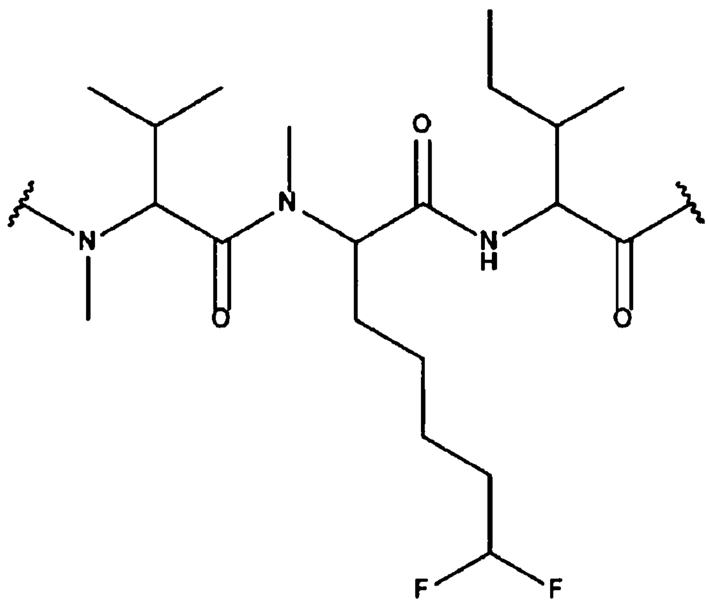

・・・(II-Ba1)

[3-29]
The peptide according to any of [3-1], [3-14] to [3-17] and [3-26] to [3-27], wherein a structure represented by formula (II) is a structure represented by the following formula (II-Ba2).

[Formula 23]

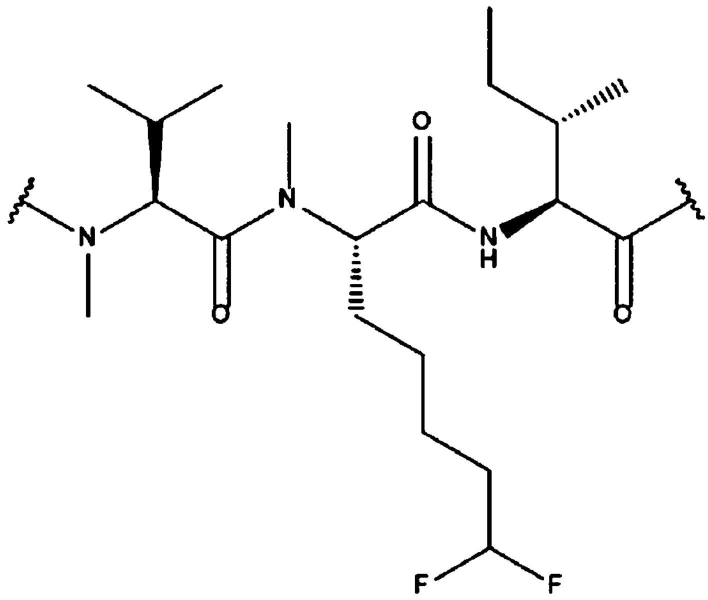

・・・(II-Ba2)

[3-30]
The peptide according to any of [3-1], [3-14] to [3-17] and [3-26], wherein a structure represented by formula (II) is a structure represented by the following formula (II-Bb1).

[Formula 24]

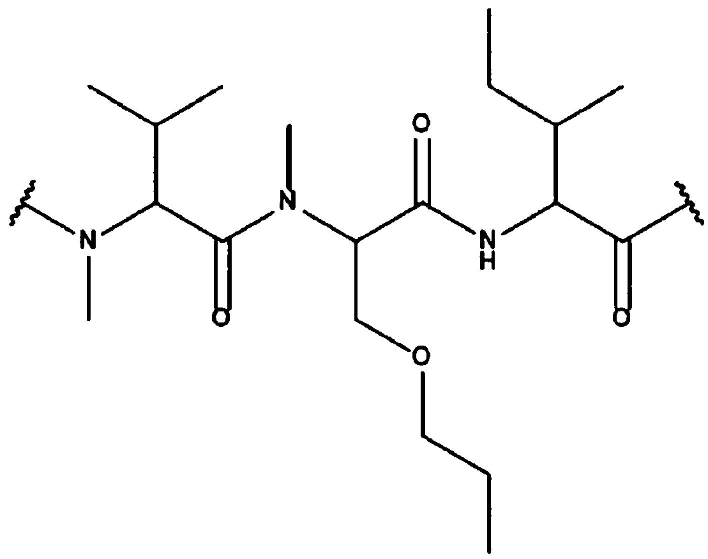

・・・(II-Bb1)

[3-31]
The peptide according to any of [3-1], [3-14] to [3-17] and [3-26] to [3-27], wherein a structure represented by formula (II) is a structure represented by the following formula (II-Bb2).

[Formula 25]

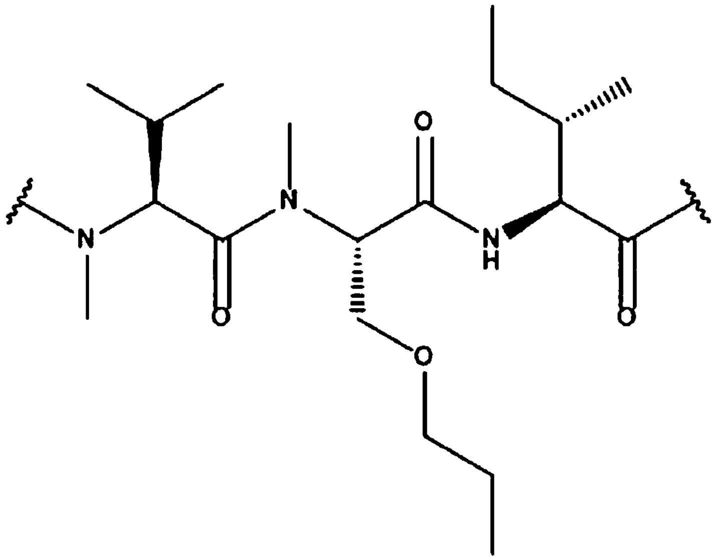

・・・(II-Bb2)

[3-32]
The peptide according to any of [3-1], [3-14] to [3-17] and [3-26], wherein a structure represented by formula (II) is a structure represented by the following formula (II-Bc1).

[Formula 26]

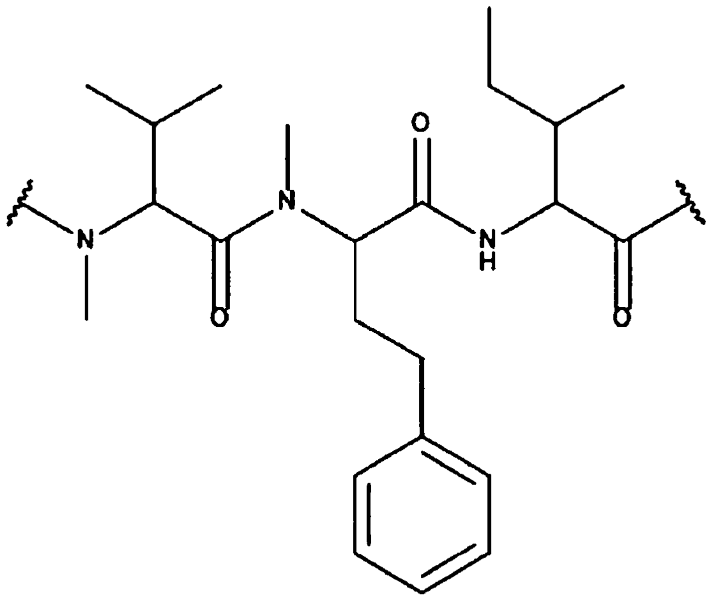

・・・(II-Bc1)

[3-33]
The peptide according to any of [3-1], [3-14] to [3-17] and [3-26] to [3-27], wherein a structure represented by formula (II) is a structure represented by the following formula (II-Bc2).

[Formula 27]

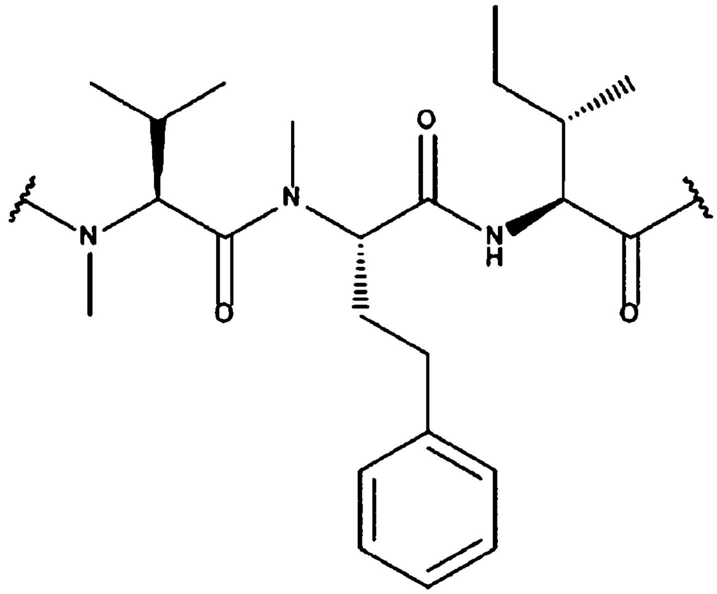

・・・(II−Bc2)

Advantageous Effects of Invention

The present invention can provide a screening method that enables efficiently obtaining a molecule of interest capable of forming a complex with a plurality of kinds of target molecules. The present invention can also provide a method of efficiently producing a molecule of interest, comprising the screening method.

Description of Embodiments

Hereinafter, the embodiments for carrying out the present invention are described in detail. However, the present invention is not limited by the embodiments given below.

The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.

As used herein, the term "to" that indicates a range includes values of both ends thereof. For example, "A to B" means the range of A or more and B or less.

The term "about" as used herein, when used in combination with a numeric value, means the value range of +10% and -10% of the numeric value.

In the present invention, the meaning of the term "and/or" includes any combination in which "and" and "or" are appropriately combined. Specifically, for example, the term "A, B, and/or C" includes the following seven variations: (i) A, (ii) B, (iii) C, (iv) A and B, (v) A and C, (vi) B and C, and (vii) A, B, and C.

[Screening method]

The screening method according to the present invention relates to a method of screening for a molecule of interest. The molecule of interest may be a molecule capable of forming a complex with a plurality of kinds of target molecules.

The screening method includes a step of contacting the plurality of kinds of target molecules in proximity to each other with a candidate molecule-nucleic acid conjugate library (contacting step).

Examples of specific aspects of the contacting step can include, but are not limited to, the following aspects.

In a first aspect, the contacting step may be a step of contacting (i) a target fusion molecule in which the plurality of kinds of target molecules are fused to each other with (ii) a candidate molecule-nucleic acid conjugate library.

In a second aspect, the contacting step may be a step of contacting (i) the plurality of kinds of target molecules immobilized on the same solid phase support with (ii) a candidate molecule-nucleic acid conjugate library.

In a third aspect, the contacting step may be a step of contacting (i) a first target molecule, which is one kind selected from a plurality of kinds of target molecules, with (ii) a linked construct library including a plurality of kinds of linked constructs in which each candidate molecule-nucleic acid conjugate and a target molecule different from the first target molecule are linked.

In a fourth aspect, the contacting step may be a step of contacting (i) a linked construct prepared through a step of preparing a linked construct by linking a plurality of kinds of target molecules to each other (linking step) with (ii) a candidate molecule-nucleic acid conjugate, or a step of contacting (i) a linked construct prepared through a step of preparing a linked construct by linking one or more kinds of target molecules and a candidate molecule-nucleic acid conjugate (linking step) with (ii) a target molecule that is not used in the linking step.

In a fifth aspect, the contacting step may be a step of contacting (i) a linked construct in which a plurality of kinds of target molecules are linked to each other with (ii) a candidate molecule-nucleic acid conjugate, or contacting (i) a linked construct in which one or more kinds of target molecules and a candidate molecule-nucleic acid conjugate are linked with (ii) a target molecule that is not contained in the linked construct.

The screening method may further include a recovery step, an elution step, an amplification step, and/or an identification step, in addition to the contacting step.

The recovery step is a step of recovering a complex of at least one kind of target molecule selected from the plurality of kinds of target molecules and the candidate molecule-nucleic acid conjugate.

The elution step is a step of eluting a nucleic acid in the candidate molecule-nucleic acid conjugate recovered by the recovery step.

The amplification step is a step of amplifying a nucleic acid in a candidate molecule-nucleic acid conjugate forming the complex.

The identification step is a step of identifying a molecule of interest from the candidate molecule-nucleic acid conjugate forming the complex.

[Contacting step]

In the contacting step, the plurality of kinds of target molecules in proximity to each other is contacted with the candidate molecule-nucleic acid conjugate library.

The means for contacting a plurality of kinds of target molecules in proximity to each other with a candidate molecule-nucleic acid conjugate library is not particularly limited, but may include, for example:

(1) a method of contacting (i) a target fusion molecule in which a plurality of kinds (e.g., two or more kinds) of target molecules are fused to each other (e.g., by a fusion moiety) with (ii) a candidate molecule-nucleic acid conjugate library,
(2) a method of contacting (i) a plurality of kinds of target molecules immobilized on the same solid phase support with (ii) a candidate molecule-nucleic acid conjugate library,
(3) a method of contacting (i) a first target molecule, which is one kind selected from a plurality of kinds of target molecules, with (ii) a linked construct library including a plurality of kinds of linked constructs in which each candidate molecule-nucleic acid conjugate and a target molecule different from the first target molecule are linked,
(4) a method of contacting (i) a plurality of kinds of target molecules which are made in proximity to each other by embedding each of the target molecules into a lipid bilayer such as a liposome or nanodisc, with (ii) a candidate molecule-nucleic acid conjugate library, or
(5) a method of binding all candidate molecule-nucleic acid conjugates included in the library to a target fusion molecule and cleaving (i) a fusion moiety in the target fusion molecule and/or (ii) a conjugation moiety between the candidate molecule-nucleic acid conjugate and the target fusion molecule.

In the case of (5), candidate molecules that do not form a complex with a plurality of kinds of target molecules can be excluded by confirming whether a complex of the plurality of kinds of target molecules contained in the target fusion molecule and the candidate molecule-nucleic acid conjugate is maintained after cleaving of (i) the fusion moiety in the target fusion molecule and/or (ii) the conjugation moiety between the candidate molecule-nucleic acid conjugate and the target fusion molecule. In particular, among these, the method of (1) is most preferred. By employing the method of (1), it is easier to control the distance between the plurality of kinds of target molecules. In other words, the distance (degree of proximity) between the plurality of kinds of target molecules can be directly controlled by using a method of fusing (e.g., via a fusion moiety) the plurality of kinds of target molecules described herein and/or found in the prior art.

The contacting step is preferably performed under conditions in which there are no molecules that are not included in the candidate molecule-nucleic acid conjugate library and that may selectively form a complex with the plurality of kinds of target molecules in proximity.

(Target molecule)

In one aspect, the plurality of kinds of target molecules are any plurality of kinds of target molecules and the number and characteristics thereof are not limited, but may be, for example, two kinds or more, three kinds or more, four kinds or more, five kinds or more, six kinds or more, seven kinds or more, eight kinds or more, nine kinds or more, or ten kinds or more, and ten kinds or less, nine kinds or less, eight kinds or less, seven kinds or less, six kinds or less, five kinds or less, four kinds or less, three kinds or less, or two kinds or less of target molecules. The plurality of kinds of target molecules may be, for example, two kinds or more and five kinds or less, preferably two kinds or more and four kinds or less, more preferably two kinds or more and three kinds or less, and most preferably two kinds of target molecules. When the kinds of the target molecules are in the above range, it is easier to obtain a molecule of interest.
In one aspect, the dissociation constant of at least two (two kinds) target molecules to each other selected from the plurality of kinds of target molecules may be more than 10 μM, and preferably all the dissociation constants of the plurality of kinds of target molecules to each other are more than 10 μM. The screening method according to the present invention includes a step of contacting a plurality of kinds of target molecules in proximity to each other with a candidate molecule-nucleic acid conjugate library, so that a molecule of interest capable of forming a complex with a plurality of kinds of target molecules having a large dissociation constant can also be selected.
The dissociation constant of at least two (two kinds) target molecules to each other selected from the plurality of kinds of target molecules may be, for example, more than 10 μM, 15 μM or more, 20 μM or more, 25 μM or more, 30 μM or more, 35 μM or more, 40 μM or more, 45 μM or more, or 50 μM or more. The dissociation constant of at least two (two kinds) target molecules to each other selected from the plurality of kinds of target molecules may be, for example, more than 10 μM, and is preferably 20 μM or more, more preferably 30 μM or more, and most preferably 50 μM or more. By using the technique of the present invention, it is possible to more efficiently obtain a molecule of interest capable of forming a complex with a plurality of kinds of target molecules in which the dissociation constant is in the above range.
The target molecule is not particularly limited, and examples thereof include a protein, a sugar (an oligosaccharide, a polysaccharide, or the like), a lipid, and a nucleic acid (DNA, RNA, or the like). The target molecule is preferably a protein.
The target molecule is preferably a molecule present in a human body.
The target molecule is preferably a molecule that does not contain a split protein.
The target molecule is preferably a molecule that does not contain any of GFP and a fragment thereof.
The target molecule is preferably a molecule that does not contain any of luciferase and a fragment thereof.
The target molecule is preferably a molecule that does not contain any of a SNAP tag and a fragment thereof.
The target molecule is preferably a molecule that does not contain any of ubiquitin and a fragment thereof.
The target molecule may include one or more selected from the group consisting of an FK506 binding protein (FKBP), an FKBP-rapamycin binding protein (FRB), cyclophilin, and calcineurin (Cn).
The target molecule may include FKBP. Examples of FKBP preferably include FKBP 12, FKBP 12.6, FKBP 13, FKBP 19, FKBP 22, FKBP 23, FKBP 25, FKBP 36, FKBP 38, FKBP 51, FKBP 52, FKBP 60, and FKBP 65.
The target molecule may include cyclophilin. Examples of cyclophilin preferably include cyclophilin A (CyA), cyclophilin B, cyclophilin C, cyclophilin D, cyclophilin E, cyclophilin F, cyclophilin G, cyclophilin H, cyclophilin I, cyclophilin J, cyclophilin 40, NKTR, PPWD1, PPIL1, PPIL2, PPIL4, PPIL5, RANBP2, and SDCCAG-10.
The target molecule, for example, may include or may be FKBP and FRB.
The target molecule, for example, may include or may be cyclophilin and Cn.
The target molecule, for example, may include or may be FKPB and Cn.
The cyclophilin may be, for example, CyA.

(Library)

The candidate molecule-nucleic acid conjugate library is a collection (library) of conjugates (candidate molecule-nucleic acid conjugates) in which the candidate molecule and the nucleic acid as a tag are associated. The conjugate (candidate molecule-nucleic acid conjugate) included in the candidate molecule-nucleic acid conjugate library may be a conjugate (candidate molecule-nucleic acid conjugate) itself, and may be a linked construct in which the conjugate (candidate molecule-nucleic acid conjugate) and a target molecule are linked. The assembly of such linked constructs is also referred to as, in particular, a linked construct library.
In the candidate molecule-nucleic acid conjugate, the candidate molecule and the nucleic acid may be in a one-to-one association between a kind of the candidate molecule and a kind of the nucleic acid, and the candidate molecule and the nucleic acid may form a conjugate by conjugating directly or via a conjugation moiety, or the candidate molecule and the nucleic acid may form a conjugate without conjugating directly or via a conjugation moiety. In one nonlimiting aspect, the candidate molecule and the nucleic acid form a conjugate, preferably, via a conjugation moiety.
The conjugation moiety is not limited as long as the candidate molecule can be linked to a nucleic acid, but may

contain, for example, an antibiotic puromycin, which is an analogue of an aminoacyl tRNA. The conjugation moiety can further contain a spacer well known to those skilled in the art.

The nucleic acid may be a nucleic acid encoding a candidate molecule forming a candidate molecule-nucleic acid conjugate with the nucleic acid. This makes it possible to easily reproduce the candidate molecule-nucleic acid conjugate by amplifying the nucleic acid from the recovered candidate molecule-nucleic acid conjugate and then synthesizing (translating) a protein from the amplified nucleic acid.

The nucleic acid may be DNA or mRNA. The nucleic acid may also have a sequence for use in amplification (such as a primer binding sequence) in addition to a tag sequence for identifying a candidate molecule contained in the conjugate. The sequence for use in amplification may be a sequence common in all the nucleic acids.

The library may be a display library, such as a phage display library, a ribosomal display library, a bacterial display library, a yeast display library, an mRNA display library, and a DNA display library, or a DNA encoded library. The library is preferably an mRNA display library, a DNA encoded library, and more preferably an mRNA display library.

The candidate molecules contained in the library may have a diversity of $1 \times 10^3$ or greater. By this, the efficiency of screening for the molecule of interest can be improved. The diversity of the candidate molecules included in the library may be $5 \times 10^3$ or more, $1 \times 10^4$ or more, $5 \times 10^4$ or more, $1 \times 10^5$ or more, $5 \times 10^5$ or more, $1 \times 10^6$ or more, $5 \times 10^6$ or more, $1 \times 10^7$ or more, $5 \times 10^7$ or more, $1 \times 10^8$ or more, $5 \times 10^8$ or more, $1 \times 10^9$ or more, $5 \times 10^9$ or more, $1 \times 10^{10}$ or more, $5 \times 10^{10}$ or more, or $1 \times 10^{11}$ or more. The diversity of the candidate molecules included in the library may be, for example, $1 \times 10^3$ or more, preferably $1 \times 10^5$ or more, more preferably $1 \times 10^7$ or more, most preferably $1 \times 10^9$ or more. The diversity of the candidate molecules has the same meaning as the number of kinds of candidate molecules. When the diversity of candidate molecules included in the library is in the above range, the efficiency to obtain a molecule of interest is more increased.

The library can be prepared in a manner well known to those skilled in the art. For example, an mRNA display library can be produced, but is not limited to, as follows. First, a library of DNA in which a desired nucleotide sequence is placed downstream of a promoter is chemically synthesized, then used as a template to obtain a double-stranded DNA by a primer extension reaction. Next, the double-stranded DNA as a template is transcribed to mRNA using an RNA polymerase. To the 3' end of this mRNA, a linker (spacer) to which an antibiotic puromycin, that is an analogue of aminoacyl tRNA, or the like is attached is linked. This linked product is added to a known cell-free translation system, and the system is kept warm to translate mRNA, and to link the mRNA and the peptide encoded thereby via a linker containing puromycin or the like. In this way, an mRNA display library composed of a complex of mRNA and its products in which the mRNA and its products are associated can be constructed. In addition, the complex binding to the target molecule can be concentrated by contacting a desired immobilized target molecule with the mRNA display library, and washing away the complex not binding to the target molecule (panning). It is possible to identify the amino acid sequence of the bounded peptide by synthesizing cDNA from mRNA contained in such a selected complex, subjecting it to PCR amplification, and analyzing the nucleotide sequence.

The candidate molecule corresponds to the moiety excluding the nucleic acid and the conjugation moiety in the candidate molecule-nucleic acid conjugate. For example, in Examples of the present application, the candidate molecule is a peptide, and the candidate molecule in the candidate molecule-nucleic acid conjugate is a moiety in which, after excluding the conjugation moiety containing a peptide linker amino acid sequence (PTGTGTGKK: SEQ ID NO: 45) and puromycin and the nucleic acid moiety, pyrrolidine is attached to the conjugation moiety of the carboxy to which the peptide linker had been attached. In one nonlimiting aspect, the candidate molecule may be a molecule in which the carboxy group generated when the nucleic acid and the conjugation moiety are excluded from the candidate molecule-nucleic acid conjugate as such is capped. The candidate molecule is not particularly limited, and any compounds including small molecular compounds, medium molecular compounds, and macromolecular compounds can be used as the candidate molecule. The candidate molecule is preferably a medium molecular compound such as a peptide, and more preferably a cyclic peptide.

In $1 \times 10^3$ or more candidate molecules among all candidate molecules included in the candidate molecule-nucleic acid conjugate library, a molecular weight of the candidate molecule may be, for example, 300 g/mol or more, 350 g/mol or more, 400 g/mol or more, 450 g/mol or more, or 500 g/mol or more, and may be 5,000 g/mol or less, 4,500 g/mol or less, 4,000 g/mol or less, 3,500 g/mol or less, 3,000 g/mol or less, 2,500 g/mol or less, or 2,000 g/mol or less. In $1 \times 10^3$ or more candidate molecules among all candidate molecules included in the candidate molecule-nucleic acid conjugate library, a molecular weight of the candidate molecule may be, for example, 300 g/mol or more and 5,000 g/mol or less, preferably 350 g/mol or more and 4,500 g/mol or less, more preferably 400 g/mol or more and 4,000 g/mol or less, most preferably 450 g/mol or more and 3,500 g/mol or less. The molecular weight herein means the sum of the atomic weights of the atoms constituting the compound molecule (unit: "g/mol"), and is obtained by calculating the sum of the atomic weights of the atoms contained in the molecular structure formula (unit: "g/mol"). When the molecular weight of candidate molecules is in the above range, the efficiency to obtain a molecule of interest is more increased.

As used herein, the peptide is not particularly limited as long as amino acid residues in the peptide are linked by an amide bond or an ester bond. As the peptide, it is preferred that two or more amino acid residues are linked by an amide

bond. In this case, the peptide may have an ester bond in part of the main chain, like a depth peptide. In 1 × $10^3$ or more peptides among all peptides included in the candidate molecule-nucleic acid conjugate library, the number of amino acid residues of the peptide is not particularly limited, but may be, for example, 5 or more, 7 or more, 8 or more, or 9 or more. In 1 × $10^3$ or more peptides among all peptides included in the candidate molecule-nucleic acid conjugate library, the number of amino acid residues of the peptide may be, for example, 30 or less, 25 or less, 15 or less, or 13 or less. In 1 × $10^3$ or more peptides among all peptides included in the candidate molecule-nucleic acid conjugate library, the number of amino acid residues of the peptide may be, for example, 5 or more and 30 or less, preferably 7 or more and 25 or less, more preferably 8 or more and 15 or less, most preferably 9 or more and 13 or less. When the number of amino acid residues of the peptide is in the above range, the efficiency to obtain a molecule of interest with high membrane permeability and metabolic stability is more increased. The peptide may have a branched structure.

The term "amino acid" as used herein includes a natural amino acid and a non-natural amino acid. Also, as used herein, the term "amino acid residue" includes a natural amino acid residue and a non-natural amino acid residue.

Natural amino acids refer to glycine (Gly), L-alanine (Ala), L-serine (Ser), L-threonine (Thr), L-valine (Val), L-leucine (Leu), L-isoleucine (Ile), L-phenylalanine (Phe), L-tyrosine (Tyr), L-tryptophan (Trp), L-histidine (His), L-glutamic acid (Glu), L-aspartic acid (Asp), L-glutamine (Gln), L-asparagine (Asn), L-cysteine (Cys), L-methionine (Met), L-lysine (Lys), L-arginine (Arg), and L-proline (Pro).

The non-natural amino acid means amino acids other than natural amino acids. Examples of the non-natural amino acid include a β-amino acid, a D-type amino acid, an N-substituted amino acid (excluding Pro), an α,α-disubstituted amino acid, an amino acid having a side chain different from that of natural amino acids, and a hydroxycarboxylic acid. As used herein, the non-natural N-substituted amino acid means an N-substituted amino acid other than Pro.

As the amino acid as used herein, any steric configuration is acceptable. The selection of a side chain of the amino acid is not particularly restricted, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, and a spiro-bonded cycloalkyl group. Each of the side chains may have a substituent. The substituent is also not limited, and one or two or more substituents may be freely selected independently from any substituents including, for example, a halogen atom, an O atom, an S atom, an N atom, a B atom, a Si atom, or a P atom. That is, examples of the side chain include an alkyl group, an alkoxy group, an alkenyl group, an alkynyl group, an aryl group, a heteroaryl group, an aralkyl group, or a cycloalkyl group, which may be substituted, oxo, aminocarbonyl, and a halogen atom. The amino acid according to one embodiment may be a compound having a carboxy group and an amino group in the same molecule (even in this case, proline, hydroxyproline, azetidine-2-carboxylic acid, and the like in which a nitrogen atom of the amino group and any atom of the side chain together forms a ring are also included in the amino acid).

Examples of halogen-derived substituents include fluoro (-F), chloro (-Cl), bromo (-Br), and iodo (-I).

Examples of O-atom-derived substituents include hydroxy (-OH), oxy (-OR), carbonyl (-C(=O)-R), carboxy ($-CO_2H$), oxycarbonyl(-C(=O)-OR), carbonyloxy (-O-C(=O)-R), thiocarbonyl (-C(=O)-SR), a carbonylthio group (-S-C(=O)-R), aminocarbonyl (-C(=O)-NHR), carbonylamino (-NH-C(=O)-R), oxycarbonylamino (-NH-C(=O)-OR), sulfonylamino ($-NH-SO_2-R$), aminosulfonyl ($-SO_2-NHR$), sulfamoylamino ($-NH-SO_2-NHR$), thiocarboxy (-C(=O)-SH), and carboxycarbonyl ($-C(=O)-CO_2H$).

Examples of oxy (-OR) include alkoxy, cycloalkoxy, alkenyloxy, alkynyloxy, aryloxy, heteroaryloxy, and aralkyloxy.

Examples of carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C(=O)-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. In addition to these, examples include compounds in which an H atom bonded to the N atom in -C(=O)-NHR is further replaced with alkyl, cycloalkyl, alkenyl, alkynyl, aryl, heteroaryl, or aralkyl.

Examples of carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. In addition to these, examples include compounds in which an H atom bonded to the N atom in -NH-C(=O)-R is further replaced with alkyl, cycloalkyl, alkenyl, alkynyl, aryl, heteroaryl, or aralkyl.

Examples of oxycarbonylamino (-NH-C(=O)-OR) include alkoxycarbonylamino, cycloalkoxycarbonylamino,

alkenyloxycarbonylamino, alkynyloxycarbonylamino, aryloxycarbonylamino, heteroaryloxycarbonylamino, and aralkyloxycarbonylamino. In addition to these, examples include compounds in which an H atom bonded to the N atom in - NH-C(=O)-OR is further replaced with alkyl, cycloalkyl, alkenyl, alkynyl, aryl, heteroaryl, or aralkyl.

Examples of sulfonylamino ($-NH-SO_2-R$) include alkylsulfonylamino, cycloalkylsulfonylamino, alkenylsulfonylamino, alkynylsulfonylamino, arylsulfonylamino, heteroarylsulfonylamino, and aralkylsulfonylamino. In addition to these, examples include compounds in which an H atom bonded to the N atom in $-NH-SO_2-R$ is further replaced with alkyl, cycloalkyl, alkenyl, alkynyl, aryl, heteroaryl, or aralkyl.

Examples of aminosulfonyl ($-SO_2-NHR$) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. In addition to these, examples include compounds in which an H atom bonded to the N atom in $-SO_2-NHR$ is further replaced with alkyl, cycloalkyl, alkenyl, alkynyl, aryl, heteroaryl, or aralkyl.

Examples of sulfamoylamino ($-NH-SO_2-NHR$) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Further, the two H atoms bonded to the N atom in $-NH-SO_2-NHR$ may be replaced with substituents independently selected from the group consisting of alkyl, cycloalkyl, alkenyl, alkynyl, aryl, heteroaryl, and aralkyl, and the two substituents may form a ring.

Examples of S atom-derived substituents include thiol (-SH), thio (-S-R), sulfinyl (-S(=O)-R), sulfonyl ($-S(O)_2-R$), sulfo ($-SO_3H$), and pentafluorosulfanyl ($-SF_5$).

Examples of thio (-S-R) include alkylthio, cycloalkylthio, alkenylthio, alkynylthio, arylthio, heteroarylthio, and aralkylthio.

Examples of sulfinyl (-S(=O)-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl ($-S(O)_2-R$) include alkylsulfonyl, cycloalkylsulfonyl, alkenylsulfonyl, alkynylsulfonyl, arylsulfonyl, heteroarylsulfonyl, and aralkylsulfonyl.

Examples of N atom-derived substituents include azido ($-N_3$, also referred to as "azide group"), cyano (-CN), primary amino ($-NH_2$), secondary amino (-NH-R), tertiary amino (-NR(R')), amidino ($-C(=NH)-NH_2$), substituted amidino (-C(=NR)-NR'R"), guanidino ($-NH-C(=NH)-NH_2$), substituted guanidino (-NR-C(=NR'")-NR'R"), and aminocarbonylamino (-NR-CO-NR'R").

Examples of secondary amino (-NH-R) include alkylamino, cycloalkylamino, alkenylamino, alkynylamino, arylamino, heteroarylamino, and aralkylamino.

Examples of tertiary amino (-NR(R')) include amino groups having any two substituents each independently selected from alkyl, cycloalkyl, alkenyl, alkynyl, aryl, heteroaryl, aralkyl, and the like, such as alkyl(aralkyl)amino, and the any two substituents may form a ring.

Examples of substituted amidino (-C(=NR)-NR'R") include groups in which the three substituents R, R', and R" on the N atom are each independently selected from alkyl, cycloalkyl, alkenyl, alkynyl, aryl, heteroaryl, and aralkyl, such as alkyl(aralkyl)(aryl)amidino.

Examples of substituted guanidino (-NR-C(=NR'")-NR'R") include groups in which R, R', R", and R'" are each independently selected from alkyl, cycloalkyl, alkenyl, alkynyl, aryl, heteroaryl, and aralkyl, and groups in which these substituents form a ring.

Examples of aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

Examples of B atom-derived substituents include boryl (-BR(R')) and dioxyboryl (-B(OR)(OR')). The two substituents R and R' may be groups each independently selected from alkyl, cycloalkyl, alkenyl, alkynyl, aryl, heteroaryl, aralkyl, or the like, or may be a group in which they form a ring. Specific examples include a cyclic boryl group, and more specifically include a pinacolate boryl group, a neopentanediolate boryl group, and a catecholate boryl group.

The amino group of the main chain of the amino acid may be unsubstituted ($-NH_2$) or substituted (that is, -NHR). R represents, for example, an alkyl group, an alkenyl group, an alkynyl group, an aryl group, a heteroaryl group, an aralkyl group, or a cycloalkyl group, which is optionally substituted, or a carbon chain attached to the N atom and a carbon atom at position $\alpha$ may form a ring, like proline.

As used herein, an amino acid residue in which the amino group of the main chain is substituted is referred to as an "N-substituted amino acid residue". Examples of the "N-substituted amino acid residue" as used herein include an N-alkylamino acid residue, an N-$C_1$-$C_6$ alkylamino acid residue, an N-$C_1$-$C_5$ alkylamino acid residue, an N-$C_1$-$C_4$ alkylamino acid residue, an N-$C_1$-$C_3$ alkylamino acid residue, an N-ethylamino acid residue, an N-methylamino acid residue, an N-$C_7$-$C_{14}$ aralkylamino acid residue, an N-benzylamino acid residue, and an N-phenethylamino acid residue.

Specific examples of the substituent on the nitrogen atom of the N-substituted amino acid residue (R of -NHR described above) herein include an alkyl group (preferably a $C_1$-$C_6$ alkyl group, more preferably a $C_1$-$C_4$ alkyl group, further preferably a $C_1$-$C_3$ alkyl group, still more preferably an ethyl group or a methyl group), a $C_7$-$C_{14}$ aralkyl group, a

benzyl group, and a phenethyl group. The substituent on the nitrogen atom of the N-substituted amino acid may be, for example, a $C_1$-$C_6$ alkyl group, preferably a $C_1$-$C_3$ alkyl group, more preferably an ethyl group or a methyl group, most preferably a methyl group (i.e., N-methylamino acid is most preferred as the N-substituted amino acid).

The "amino acid" as used herein includes all isotopes corresponding to each. The isotope of the "amino acid" is a form in which at least one atom is replaced with an atom having the same atomic number (proton number) and a different mass number (total number of protons and neutrons). Examples of the isotope included in the "amino acid" as used herein include a hydrogen atom, a carbon atom, a nitrogen atom, an oxygen atom, a phosphorus atom, a sulfur atom, a fluorine atom, and a chlorine atom, which respectively include $^{2}H$, $^{3}H$, $^{13}C$, $^{14}C$, $^{15}N$, $^{17}O$, $^{18}O$, $^{32}P$, $^{35}S$, $^{18}F$, and $^{36}Cl$.

In $1 \times 10^3$ or more peptides among all peptides included in the candidate molecule-nucleic acid conjugate library, the number of non-natural amino acid residues contained in the peptide may be, for example, 1 or more, 2 or more, 3 or more, 4 or more, 5 or more, 6 or more, 7 or more, 8 or more, 9 or more, or 10 or more. In $1 \times 10^3$ or more peptides among all peptides included in the candidate molecule-nucleic acid conjugate library, the number of non-natural amino acid residues contained in the peptide may be, for example, 30 or less, 25 or less, 20 or less, 15 or less, 14 or less, 13 or less, or 12 or less. In $1 \times 10^3$ or more peptides among all peptides included in the candidate molecule-nucleic acid conjugate library, the range of the number of non-natural amino acid residues contained in the peptide may be, for example, 1 or more and 30 or less, 2 or more and 25 or less, 3 or more and 20 or less, 4 or more and 15 or less, 5 or more and 14 or less, 6 or more and 13 or less, 7 or more and 12 or less, or 11. In $1 \times 10^3$ or more peptides among all peptides included in the candidate molecule-nucleic acid conjugate library, the range of the number of non-natural amino acid residues contained in the peptide may be, for example, 1 or more and 13 or less, preferably 3 or more and 12 or less, more preferably 4 or more and 11 or less, most preferably 5 or more and 10 or less.

In $1 \times 10^3$ or more peptides among all peptides included in the candidate molecule-nucleic acid conjugate library, the number of N-substituted amino acid residues contained in the peptide may be, for example, 1 or more, 2 or more, 3 or more, 4 or more, or 5 or more. In $1 \times 10^3$ or more peptides among all peptides included in the candidate molecule-nucleic acid conjugate library, the number of N-substituted amino acid residues contained in the peptide may be, for example, 10 or less, 9 or less, preferably 8 or less, more preferably 7 or less, and most preferably 6 or less. In $1 \times 10^3$ or more peptides among all peptides included in the candidate molecule-nucleic acid conjugate library, the range of the number of N-substituted amino acid residues contained in the peptide may be, for example, 1 or more and 10 or less, 1 or more and 8 or less, 1 or more and 7 or less, or 2 or more and 6 or less. In $1 \times 10^3$ or more peptides among all peptides included in the candidate molecule-nucleic acid conjugate library, the number of N-substituted amino acid residues contained in the peptide may be, for example, 1 or more and 13 or less, preferably 3 or more and 12 or less, more preferably 4 or more and 11 or less, most preferably 5 or more and 10 or less.

The peptide may be a cyclic peptide. As used herein, the term "cyclic peptide" is not particularly limited as long as it is a peptide having a cyclic portion constituted of 5 or more amino acid residues. In $1 \times 10^3$ or more peptides among all peptides included in the candidate molecule-nucleic acid conjugate library, the number of amino acid residues constituting the cyclic portion of the cyclic peptide may be, for example, 5 or more and 15 or less, 6 or more and 15 or less, 6 or more and 14 or less, 7 or more and 14 or less, 8 or more and 14 or less, 7 or more and 13 or less, 7 or more and 12 or less, 8 or more and 12 or less, 8 or more and 11 or less, 9 or more and 11 or less, or 10 or 11. In $1 \times 10^3$ or more peptides among all peptides included in the candidate molecule-nucleic acid conjugate library, the number of amino acid residues constituting the cyclic portion may be, for example, 5 or more and 15 or less, preferably 9 or more and 15 or less, more preferably 10 or more and 14 or less, most preferably 11 or more and 13 or less. When the number of amino acid residues constituting the cyclic portion is in the above range, the efficiency to obtain a molecule of interest with high membrane permeability and metabolic stability is more increased. The cyclic portion is preferably formed via a covalent bond such as an amide bond, a carbon-carbon bond formation reaction, an S-S bond, a thioether bond, and a triazole bond. The cyclization may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization through a covalent bond such as an amide bond and a carbon-carbon bond is preferred, and cyclization through an amide bond by a carboxy group of the side chain and an amino group of the main chain is more preferred. The positions of the carboxy group, the amino group, and the like used for cyclization may be on the main chain or the side chain, and are not particularly restricted as long as the positions allow the groups to be cyclized.

The cyclic peptide may have a linear portion in addition to the cyclic portion. The specific aspects of the number of amino acid residues of the cyclic peptide are the same as the specific aspects of the number of amino acid residues of the peptide described above. When the cyclic peptide has a linear portion, the sum of the numbers of amino acid residues of the cyclic portion and the linear portion preferably falls within the range of the number of amino acid residues of the peptide described above. When the cyclic peptide has a linear portion, in $1 \times 10^3$ or more peptides among all peptides included in the candidate molecule-nucleic acid conjugate library, the number of amino acid residues constituting the cyclic portion may be, for example, 5 or more and 15 or less, 6 or more and 15 or less, 6 or more and 14 or less, 7 or more and 14 or less, 8 or more and 14 or less, 7 or more and 13 or less, 7 or more and 12 or less, 8 or more and 11 or less, 9 or more and 11 or less,

or 10 or 11, and the number of amino acid residues constituting the linear portion may be, for example, 1 or more and 8 or less, 1 or more and 7 or less, 1 or more and 6 or less, 1 or more and 5 or less, 1 or more and 4 or less, or 1 or more and 3 or less. When the cyclic peptide has a linear portion, in $1 \times 10^3$ or more peptides among all peptides included in the candidate molecule-nucleic acid conjugate library, the number of amino acid residues constituting the cyclic portion may be, for example, 5 or more and 15 or less, preferably 9 or more and 15 or less, more preferably 10 or more and 14 or less, most preferably 11 or more and 13 or less, and the number of amino acid residues constituting the linear portion may be, for example, 1 or more and 8 or less, preferably 1 or more and 6 or less, more preferably 1 or more and 4 or less, most preferably 1 or more and 3 or less. When the number of amino acid residues constituting the linear portion is in the above range, the efficiency to obtain a molecule of interest with high membrane permeability and metabolic stability is more increased.

In $1 \times 10^3$ or more peptides among all peptides included in the candidate molecule-nucleic acid conjugate library, ClogP of the peptide may be, for example, 4 or more and 25 or less. ClogP is a partition coefficient calculated by a computer, and can be determined according to the principle described in "CLOGP Reference Manual Daylight Version 4.9 (release date: August 1, 2011, https://www.daylight.com/dayhtml/doc/clogp/)". Examples of the method of calculating ClogP include calculating using Daylight Version 4.95 (release date: August 1, 2011, ClogP algorithm version 5.4, database version 28, https://www.daylight.com/dayhtml/doc/release_notes/index.html) of Daylight Chemical Information Systems, Inc.

The principle described in "CLOGP Reference Manual Daylight Version 4.9 (release date: August 1, 2011, https://www.daylight.com/dayhtml/doc/clogp/)" is as follows.

1. Introduction

The CLOGP Reference Manual will help the user understand why the CLOGP program calculates logP(ow) the way it does. Although the procedure cannot be derived from first principles, we have tried to make the rules consistent with solvation theory, if for no other reason than they are more easily remembered. The method simply adds together values for structural parts of a solute molecule and correction factors dependent upon the particular way the parts are put together.

The CLOGP EXAMPLES section contains example CLOGP calculations for a variety of chemicals and is designed as a companion to the CLOGP Reference Manual. An asterisk (*) appears in the CLOGP Reference Manual when one or more examples are provided in CLOGP Example Calculations to illustrate aspects of the CLOGP computation. Examples also demonstrate DEPICT (chemical depiction).

Funding for the development of CLOGP was provided by the U.S. Environmental Protection Agency through Cooperative Agreement No. 809295, and we wish to acknowledge the encouragement and support of the project officer, Dr. Gilman Veith of ERL-Duluth.

1.1 Measurement and Past Uses of Partition Coefficients

The partition coefficient is the equilibrium concentration of solute in a non-polar solvent divided by the concentration of the same species in a polar solvent. In this and most other applications, the polar solvent is water. The logarithm of the partition coefficient, log P, has been successfully used as a hydrophobic parameter in 'extrathermodynamic' Hammett methodology. 1-octanol has much to recommend it as the choice for the non-polar phase (1) and logP(ow) has been used successfully in Quantitative Structure Activity Relationships (QSAR) in the following special fields: drug and pesticide design (2,3); pharmacokinetics (4); anaesthesiology (5); environmental transport and soil binding (6,7); toxicology (8); bioaccumulation (9); protein folding (10); enzyme binding (11,12); enzymic reactions in non-aqueous solvents (13); and hostguest complexation (14a,b).

In principle, the measurement of the equilibrium concentration of solute in the octanol and water phases, after shaking in a separatory funnel, is very simple, and since good measured values are always to be preferred over calculated ones, it would seem that there should be little need for a procedure to calculate them. As it turns out, reliable shake-flask measurements are timeconsuming and often difficult to make. The criteria for high reliability are: measurements over a 10-fold concentration range (with upper concentration no more than 75% of solubility or CMC, or no more than 5% of the aqueous phase, whichever is lower) and standard deviation of 0.03 or less in log terms. This often requires working at sub-micromolar concentrations, and so, with either UV spectrophotometry or gas chromatography, it means that the standard curves must be established with utmost care. Radiotracer methods seem well-suited for analyses at these low concentrations, but impurities as well as adsorption at phase boundaries (including container walls) can introduce significant errors.

HPLC procedures provide a way around this bottleneck(15, 16) and can save time if there is a limited variety of structural types, and the log P values fall in the range of 0.5 to 4.0. Most HPLC procedures which are used to develop log P(ow) values do no use octanol and thus have to be referred to that system by standard curves which can be different depending on whether the solutes do or do not contain certain basic fragments, such as pyridine nitrogen. If the solutes do

not absorb well in the UV, difficulties in detecting the elution time eliminates any advantage HPLC may have over the shake-flask method.

Procedures which employ filter probes (17) or solubility columns(18) speed up partition coefficient measurements by eliminating centrifugation as the means of phase separation. However, each has its own set of disadvantages and limit its acceptance as a method for establishing the standard values for a calculation procedure.

More efficient methods of measurement of octanol/water partition coefficients are certain to be developed in the future, but no conceivable 'breakthrough' is likely to eliminate the need for logP calculation. To put the problem in proper prospective, one need only imagine some dedicated synthetic chemist making all possible tri-substituted benzoic acids with the methods commonly available today. When finished, there would be five million analogs for which partition coefficients could be determined. And of course only by calculation is one going to have an estimate of hydrophobicity before synthesis.

The Pomona MedChem Project saw these and other arguments as reason enough to develop a method to calculate log P(ow) from structure by an additive-constitutive procedure. As it turns out, the 'constitutive' portion of the procedure was, by the very nature of the two competing solvation equilibria, very complex, and the manual method required considerable effort before it could be applied with confidence. It is the aim of the second-generation program, CLOGP, to take most of the routine calculation burden from the user but still encourage him to study the interplay of hydrophobic and polar solvation forces which can be so crucial to the design of bioactive chemicals.

1.2 How to Understand CLOGP Calculations

The first published method for calculating log P(ow) from structure (19) was based on a 'substitution' procedure and was developed with substituent pi constants for aromatic rings in mind. Of course this method was limited to deriving a new log P from a 'parent' structure whose log P was already known. Rekker(20) was the first to publish a procedure which was more general in that it assigned 'fragmental constants' to a variety of structural pieces, and the calculated log P was the sum of the values appropriate for the molecule in question. The original pi system can be expressed as:

[Expression 1]

$$\pi_x = \log P_{C_6H_5X} - \log P_{C_6H_6}$$

while the expression for Rekker's fragment system is:

[Expression 2]

$$\mathrm{LogP} = \sum_{i=1,N} a_i f_i + \sum_{j=1,M} b_j F_j$$

The method developed by Pomona MedChem (21) follows Rekker's general formulation, but there are some important differences in the approach used to derive the actual working constants. Rekker used a 'reductionist' approach - deriving the constants for carbon and hydrogen as well as those for polar fragments from a statistical treatment of a large body of log P data which contained numerous interaction factors. Both the fragment values (f) and interaction factors (F) had to be identified and evaluated concurrently. Also, Rekker neglected to clearly define just what constitutes a fragment. Instead he provides a table in which the known constants can be found (see footnote). Rekker also treats all correction factors as some multiple of a 'Magic Number' (+0.28), but the selection of multiples was not made clear in his published work. Although his method gained some acceptance for manual calculation, we considered it too seriously flawed to serve as the basis for a computer method.

In order to construct a dependable, verifiable algorithm suitable for log P calculations used in developing QSAR at Pomona College, we first elected to clearly define what constitutes a fragment. Next we chose a 'constructionist' approach to evaluate them; that is, we accepted as axiomatic that the hydrophobic portions of solutes were those most 'hydrocarbon-like', and defined these carbons and hydrogen fragment values as being truly constant. We gave very heavy emphasis to the carefully-measured values for three solutes; molecular hydrogen, methane and ethane, because from these we could derive fragment constants for carbon and hydrogen, which would be free of obscuring interactions. For all hydrocarbon structures more complex than these, whose measured values were NOT the sum of fragment values, we attempted to define the difference in terms of universally-applicable correction factors. It appears that this approach has led not only to a workable algorithm, but has highlighted the importance of certain types of polar solvation forces which have received insufficient attention in the past.

The first attempt to reduce the 'Pomona Method' of log P calculation to computer algorithm was made in collaboration with Dr. Jack Chou and Dr. Peter Jurs of Pennsylvania State University (22). It was called CLOGP. A great

deal was learned in the process of developing this first version, and it certainly established the real need for a 'stand-alone' program to make these calculations. Nevertheless, CLOGP was difficult to install and modify, and many well-known correction factors could not be implemented due to programming difficulties. In light of this experience, we deemed it essential to incorporate, in the second generation program, design features which would encourage its continuing evolution. To achieve that objective, the program had to be conceived as a 'modeling system' which could operate from one or more easily-revised 'value files'. As an example of the ease of updating, the largest fragment encountered to date is:

[Formula 28]

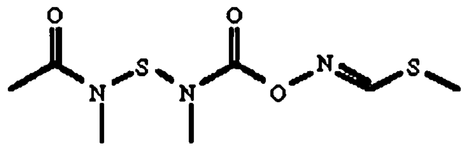

It took less than two minutes to enter it into the database and begin to use it in calculations.

A number of significant improvements have been made to CLOGP over the past few years.

The most significant improvement is the ability to estimate a polar fragment value which has not appeared in a solute having a measure log P (oct). This type of estimation is designated as 'calculated'. If the fragment in question has appeared in a measured solute, but in a different bonding environment (e.g., aromatic attached when aliphatic attached is needed), the new 4.0 version allows for all extrapolations and designates the value as 'derived'. The methodology for the 'No Missing Fragments' algorithm is explained in more detail in ref. 23.

Earlier versions of CLOGP were able to assign corrections to polar fragments interacting over two Isolating Carbons (see Section 2. following). In the latest version, this distance has been extended to three I.C.s. A significant improvement in steroid calculations recognizes the unique contribution of polar groups at the 11 position as well as some long-range intramolecular hydrogen bonds. These and a few other minor improvements will be noted in the changed values in the CLOGP Example Calculations, 6.3.

2. Fundamental Fragments

In view of the decision to make alkane carbons and hydrogens the most fundamental fragments in the system, it is necessary to define these very carefully before defining the polar, more hydrophilic fragments.

2.1. Isolating Carbons

An 'Isolating Carbon' (I.C.) atom is carbon which is NOT doubly- or triply- bonded to a hetero atom. An I.C. may be bonded to a hetero atom by a single or an aromatic bond. This definition can be made clearer from the following two examples:

[Formula 29]

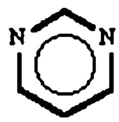

1. pyrimidine

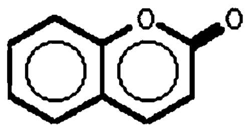

2. coumarin

In an earlier version of the manual calculation procedure (Ref. 21 p. 34) the Kekule structures for pyrimidine was considered; the earlier rule is now superceded. In coumarin, both rings are designated as aromatic, and the only carbon which is not isolating is the one in the carbonyl group, because it is doubly bonded to a hetero outside the ring.

Although the hydrophobic value of an I.C. is constant, several types must still be identified; the degree to which they delocalize electrons in any polar fragments attached to them has a great influence on overall log P. The types of I.C.s presently identified are listed below with appropriate symbols:

[Table 1]

| Symbol | Type |
|---|---|
| A | Aliphatic |
| Z | Benzyl |
| V | Vinyl |
| Y | Styryl |
| a | Aromatic |

To be completely characterized, a polar fragment must have each of its 'valence bonds' designated with one of the above symbols (see section "Fragment Valence Types"). The numerical value of the fragment will increase roughly in the order 'A' to 'a', but must be experimentally determined for high reliability.

All hydrogens bonded to I.C.s are fragments. These two kinds of fundamental fragments are the most important members of the non-polar class. A comparison of their relative values (C = 0.2; H = 0.225) is a reminder that the measure of effective cavity size may not be as simple as using van der Waals radii or CPK models.

2.2. Polar Fragments

A fragment is any atom or group of atoms bounded by Isolating Carbon atoms, and all except hydrogen are considered polar. A fragment may have many internal bonds but those connecting it to I.C.s are called 'valence bonds'. Valence bonds are most often single, but can be aromatic, as in the case of the N fragments in pyrimidine shown above. Each hydrogen in methane is a fragment, but the hydrogens in formaldehyde are not because the carbon to which they are bonded is not isolating. This is very important to remember, for one frequently sees published calculations in which one fragment value is obtained from another by the replacement of a fragment hydrogen with another fragment of known value. At the present time a good rule to follow is: "Never break up a Fragment; estimations can be made from values measured for different bond environments (see below) but a Fragment cannot be constructed from parts." Examples of fragments which cannot be "broken down" further are:

[Table 2]

| | |
|---|---|
| **Monovalent:** | **-Cl;-CN** |
| **Divalent** | **-OC(=O)NH-** |
| **Trivalent:** | **-OC(=O)N<** |
| **Tetravalent:** | **>NC(=O)N<** |

As will become evident in the following sections, polar fragments can interact in various ways. To quantitate this interaction it is necessary to define several types of polar fragments:
(A) X = any halogen, but for one type of interaction fluorine must be assigned to a special subclass,'F'. (B) Y = all non-X fragments; these are further subdivided according to: sensitivity to halogen interaction as 'Y-1', 'Y-2', and 'Y-3' containing '-OH' or not.

2.3. Intrinsic Values

Here the term, 'intrinsic' fragment values, means those which would, if summed, yield the correct log P without any correction factors. It is worthwhile to examine some of the accepted hypotheses as to what solvation forces or other phenomena determine these intrinsic values.

It takes more energy to form a cavity in water than in octanol. One would predict, therefore, that increasing the size of a solute would increase its log P. Other factors being equal, this appears to be the case. However, other features of the solute can partly or completely override the effect of its size.

Water is much more capable than is octanol of accommodating localized dipoles, and it contains, on a molar basis, more hydrogen bond accepting and donating groups. So it is these three factors -size, localized dipole strength, and H-bonding ability - which largely determine the sign and magnitude of any fragment value.

2.3.1 Halogens

Halogens form an intense localized dipole when bonded to an aliphatic carbon atom.* This intensity is somewhat lessened if the I.C. is benzyl and greatly lessened if it is vinyl, styryl or aromatic.* Fluorine has a negative fragment value

when attached to an aliphatic carbon, because the dipole effect outweighs the effect of size. Size is of greater importance with chlorine and bromine, but even bromine is less hydrophobic than a hydrogen in an aliphatic setting. As will become evident in the following section, much of this hydrophilic polar effect can be lost through 'shielding' by other halogens, or by electronic interaction with 'Y' type polar groups.

2.3.2 H-Polar Fragments

H-Polar Fragments ('Y') almost universally form some sort of hydrogen bonds with the donor (H) or acceptor (O) of the aqueous phase. This is thought to interrupt the peculiar 'ice-like' water shell which forms around the non-polar, hydrocarbon-like portions of each solute molecule, and thereby effectively reduces cavity size. As noted above, this should reduce log P.

2.3.3 Ions

Octanol can accept some larger solutes containing a full formal charge in sufficient concentration for measurement. However, one must be careful that the species measured is the same, because water easily supports complete ionization while ion-pairing is the usual condition in octanol except at the very lowest concentrations. Consistent values can be obtained if 'standard conditions' are adhered to: 0.1 M small counter-ion (Na+ or Cl-) and extrapolation to infinite dilution. Measured in this way a carboxylate ion is about 4.1 log units lower than the undissociated acid. No single value can be given to the positive charge on a protonatedamine or quaternary ammonium, because the charge is delocalized along the hydrocarbon chain and thus the effect is dependent on chain-length. It should be emphasized at this point that, except for zwitterions, CLOGP calculates values for the neutral solute only.*

2.3.4 Unsaturations

Double bonds in isolation have a slightly negative effect on log P.* This effect may arise from the polarity of the pi electrons or else it may be due to the shorter bond length reducing cavity size. At any rate, it disappears if the double bonds are conjugated.* Triple bonds are decidedly hydrophilic and require a large negative correction factor.

3. Correction Factors

3.1 Structural Factors

3.1.1 Bonds

To properly perform its calculations, CLOGP needs to know the number and types of certain bonds in the solute structure. There is some reason to believe that, for the bonds in question, factors other than bond length affect the size of the solvent cavity needed to contain the hydrophobic portions of the solute molecule.

The effect of all bonds within any fragment is taken care of by the fragment value, and so it is NOT necessary to keep track of them, NOR of any bonds to hydrogen. And, as explained below, it is convenient to allow for the bond effect in aromatic rings by including it in a special aromatic I.C. type, 'aromatic carbon'; therefore, aromatic bonds also are NOT given special attention.

Bonds which DO need to be identified are the following:

3.1.1.1 Chain bonds
Chain bonds are non-ring bonds between I.C.s plus any valence bonds to fragments*.

3.1.1.2 Ring Bonds
Ring bonds are non-aromatic ring bonds between I.C.s plus any valence bonds to fragments*.

3.1.1.3 Branch Bonds
Branch bonds are chain bonds emanating from 'Branched Fragments' (a fragment type presently limited to phosphate esters) and counted to the last I.C. preceding any polar fragment. *

A separate count of each of these bonds types must be made, and a negative correction applied. For chain bonds only, this correction applies to bonds AFTER the first in each chain. For example, there is no net bond correction for ethane but there is one for propane. This suggests that the correction accounts for flexing of the chain which is not possible in methane or ethane. 1,2-diethylbenzene gets only a net of two bond corrections, because each chain is counted separately.

Also compatible with a 'flexing' hypothesis is the fact that the correction is greater for chains than for aliphatic rings.

As noted above, an isolated double bond is assigned a negative correction factor (-0.09). This factor actually becomes slightly positive if the double bonds are conjugated in a ring such as benzene. Since it is much more convenient to assign all bonds in large fused ring systems as aromatic type, rather than using the Kekule system of alternating doubles and singles, it is worthwhile to assign a special fragment value to an aromatic carbon and include all the necessary bonding effects therein. The value of aliphatic carbon is +0.20;the value for aromatic carbon which includes all bond effects associated with the aromatic ring system is +0.13.*

3.1.2 Branching at Isolating Carbons

3.1.2.1 Chain Branch

It is well-known that iso-alkanes are more water-soluble than their n-isomers.* This branching evidently does not produce a corresponding solubility increase in the octanol phase in the partitioning process, because the correction required in CLOGP is negative in sign.

In CLOGP, the concept of branching was expanded, and now replaces the earlier use of the 'ring' cluster' correction(21). Fusion carbons in non-aromatic rings are considered as branched and given the same correction factor as chains; i.e., -0.13.* They are designated cluster branches.

3.1.2.2 Group Branch

If an H-Polar group branches from an I.C. the increase in water solubility, compared to the n-isomer, is even greater than with chain branching. Again this carries over to partitioning equilibrium; H-Polar group branching requires a larger correction than does chain branching. For this reason isopropyl alcohol is given one group branch correction and no chain branch is considered.* Tertiary butyl alcohol gets one of each type.*

If a fragment has more than two external(valence) bonds, it could be considered a branching point. However, in all cases except the 'Branched Fragments' noted above (t-amines and phosphate esters), the entire negative branching effect is included in the fragment value itself. Only in the case of the 'Branched Fragments' is the effect chain-length dependent.

3.2 Interaction Factors

3.2.1 Aliphatic Proximity (Measured Topologically)

3.2.1.1 Halogen vs. Halogen (X vs. X)

The positive correction to log P for this interaction is thought to result from dipole shielding and is limited to halogens on the same (geminal) or adjacent (vicinal) I.C.s. The geminal interaction is designated 'X-C-X', and the corrections can be thought to arise as follows: adding a second halogen to an I.C. which already has one creates the first X-C-X pair, and the correction required is +0.60.* Adding the third halogen to the same I.C. creates two more such paintings, each of which requires a correction of +0.5.* If the fourth halogen is added, the dipole is almost completely shielded, and the three additional pairings require corrections of +0.40 each.* For carbon tetrachloride the total geminal halogen correction would be:

EQ on pg. 12

For the vicinal halogen correction, X-C-C-X, the bond between carbons must not be double.* The correction is evaluated by subtracting one from the number of halogens meeting the structural requirement and multiplying by the factor 0.28. (Again Rekker's Magic Constant pops up!)

3.2.1.2 H-Polar vs. H-Polar (Y vs. Y)

As noted in the section on 'Intrinsic Values', the negative sign on the 'Y' fragments is thought to result from their 'structure-breaking' (and thus cavity-reducing) ability in the water phase. 'Y' fragments appear to eliminate the cavity requirement for two or more I.C.s to which they are attached. Obviously if two 'Y' fragments are located on the same or adjacent I.C.s to which they are attached. Obviously if two 'Y' fragments are located on the same or adjacent I.C.s some of this cavity reduction is going to be counted twice. Thus a positive correction factor is called for when the topological separation is less than three I.C.s. The CLOGP algorithm is an improvement over the original Rekker procedure(20) in that, in place of the same correction for every Y-C-Y or Y-C-C-Y, it makes the correction proportional to how much hydrophilic character (negative fragment value) is involved. This proportionality appears to apply even if one of the fragments is

charged and has a highly negative value, but as previously noted, the CLOGP algorithm currently does not treat ions.

If one of the 'Y' fragments in a Y-C-Y interaction contains an -OH moiety (e.g. -NHOH, - COOH, or -OH itself), a greater proportion of the hydrophilic character of the pair is lost. The coefficient by which the fragment sum is multiplied increases from 0.32 to 0.42.*

If both the 'Y' fragments and the carbons of Y-C-C-Y are in a ring, the hydrophilicity loss is not as great as if they are all in a chain (coefficient 0.26 vs. 0.20).* If one 'Y' is a substituent on the ring while the other is in the ring, the correction coefficients are averaged (0.23).* If one of the carbons has two 'Y' fragments, the geminal correction is applied first; then, for the (Y-C-C,Y'Y") correction, both pairings are calculated and averaged.* If both I.C.s have geminal 'Y' fragments, then the vicinal correction is not applicable.* (See penicillin in EXAMPLES).

3.2.1.3 Halogen vs. H-Polar (X vs. Y)

The interaction being considered at this point is limited to that which takes place across single bonds. It is therefore, probably due to an inductive or field effect. (The electronic interaction between fragments on or in aromatic rings is discussed in the following section.) In evaluating the X-C-Y correction factor, all halogens can be treated alike. However, there are at least three levels of sensitivity shown by 'Y' type fragments. In CLOGP the most sensitive class, 'Y-3', is restricted to the structural type: -SO2-R.* 'Y-2' consists of the types: -CONH-R, -O-R, -S-R, and -NH-R; and 'Y-1' of all other, H-polar fragments.* The correction factor for the first alpha-halogen (i.e. X-C-Y) is the same for all three Y-types (+0.9). For 'Y-3' fragments, this correction factor is doubled when there are two alpha halogens (X{2}-C-Y3) and tripled when there are three (X{3}-C-Y3). For 'Y-2' fragments, the second and third alpha-halogens need much less correction, and for 'Y-1', virtually none. In the case of multiple halogenation, the X-C-X and the X-C-Y corrections are additive.

The CLOGP algorithm makes no separation of 'Y' types to make the X-C-C-Y correction, but needs to distinguish fluorine from the other 'X' halogens.*

3.2.2 Electronic (through Pi-bonds)

3.2.2.1 Fragment Valence Type

As previously noted, all fragments (X or Y type) are assigned the most negative values when bonded to aliphatic I.C.s (designated as 'A'). This can be considered as the 'base' or 'intrinsic' level. If the fragment value when attached to a vinyl I.C. (V) has not been measured it can be estimated as the average of the base and aromatic-bonded (a) values.* Likewise the value for the styryl-attached fragment can be estimated as two-thirds the way from the base to the aromatic value.* CLOGP will only make these estimations when measured values have not been entered in the database.

3.2.2.2 Extension of Aromaticity

The extension of the aromatic ring system through fusion (as in naphthalene or direct substitution (as in biphenyl)) appears to increase log P, especially if the heteroaromatic atom is next to the juncture.* If the ringjoining carbons are attached only to other aromatic carbons, electron delocalization is minimal and so is the correction: +0.10 for each I.C. If the I.C.s are also attached to a polar (fused-in) fragment, such as in quinoline or 2-phenylpyrimidine, the correction is greater, +0.31. *

3.2.2.3 Sigma/Rho Fragment Interaction

When two or more X and/or Y type fragments are attached to an aromatic ring system, the correction factors can be calculated by a method very similar to that used by Hammett(23) to calculate the electronic effects in other equilibria, such as acid ionization.* This requires the assignment of a measure of electronic 'strength' (sigma) and 'susceptibility' (rho). In dealing with electronic effects on partitioning equilibria, a few fragments appear to act 'bidirectionally' and require both sigma and rho values, although they cannot, of course, act upon themselves. Most of the details of sigma and rho assignment to X and Y type fragments can be found elsewhere (24), but it should be pointed out that the latest version of the program (CLOGP) follows a newer procedure for 'fused-in' fragments. Fragments fused in aromatic rings(e.g. -N= or >C=O) may also be assigned both rho and sigma constants and treated together with 'on-ring' fragments instead of requiring a separate treatment.*

Fragments on different rings in an aromatic ring system interact with one another but the effect is attenuated.* If the two rings in the system are fused, as in 5-acetyl-1-naphthylamine, the 'intrinsic' effect is only half the sigma rho product just as it is in the biphenyl system such as in 4-(m-chlorophenyl)aniline.

One frequently encounters aromatic ring systems containing several fragments with rho and sigma values assigned, and the potential correction from all cross products of sigma/rho could be very large. Since these multiple effects

are NOT additive, some scaling down procedure was indicated. The one chosen for CLOGP takes the following steps:

a) The full potential sigma/rho product for each possible interaction is calculated and placed in descending value order, AFTER considering if the fragment pair are on the same or separate rings.
b) Except for the sigma for a pyridine type nitrogen, each use of sigma or rho causes it to 'age'. The first interaction at the top of the list is entered at full potential because the current age of its sigma and rho components is 'zero' for each. Each use reduces the effective sigma or rho value to 1/2 its previous value, and so if each were at 'age 1', the increment to the correction would only be 1/4 as much as a 'fresh' interaction. The mechanics of this computation are best understood by looking at the detailed output of some complex structures. Two such examples are provided in the example section.*

3.2.3 Special Ortho

As noted in the previous section, aromatic substituent (fragment) pairs, if they have sigma and rho values assigned to them, are given the same correction factor regardless of their relative position on the ring. It is important to keep in mind that if the fragment pair are on adjacent positions (i.e. ortho), an additional correction may be required.

3.2.3.1 Crowding

'Crowding' of certain fragment types can effectively lower their aromatic-attached values. This is most apparent in the case of fragments attached to the aromatic ring through a hetero atom which possesses an electron pair, such as -NHCOCH3.* A reasonable explanation of this observation is that the lone pair can no longer remain in the plane of the ring, making the fragment attachment resemble aliphatic (A) rather than true aromatic (a). The magnitude of the correction appears to depend on both steric and electronic (field) effects(25).

If this explanation is valid, one would expect the correction to vary continuously up to a maximum characteristic of each fragment type. It was surprising, therefore, to find that the rather large data set used in the original evaluation of the 'negative ortho' effect (24) seemed to fit multiples of Rekker's Magic Constant(20). This is handled in CLOGP by assigning integers to a matrix which has generalized fragment types for coordinates.

More recent data provides many examples which do not support this 'quantized' correction. Nevertheless, it is being retained for the present because of simplicity and because its maximum is only 0.14.

3.2.3.2 Intra-Molecular Hydrogen Bonding

Hydrogen bonding is known to occur intramolecularly between two ortho subsituents if one is a donor and the other an acceptor. A classical example of such an H-bond is that in o-nitrophenol. As might be expected, an intramolecular H-bond reduces water's ability to accommodate that solute, and the log P of o-nitrophenol is over two log units higher than the m- and p-isomers in the heptane and carbon tetrachloride solvent systems. One must always keep in mind, however, that the octanol phase possesses both H-donor and H-acceptor capability, not only because it is an alcohol, but because of the 2M water present at saturation. In actuality, the presence of the intramolecular H-bond in o-nitrophenol penalizes solvation in octanol slightly more than it does solvation in water, and its log P is 0.09 log units lower than the m- and p-isomers.

In terms of intramolecular H-bonding between aromatic ortho substituents, the octanol/water system appears to be sensitive to a very restricted class. The only clear-cut cases seem to result from a carbonyl group directly attached to the ring acting as acceptor, and a directly-attached - OH or -NH- acting as donor.* In all of the cases observed so far, the correction is very close to +0.63, and is stored in the same matrix used for the 'negative ortho' corrections. Thus the 'crowding' and H-bonding ortho effects never are applied simultaneously, but a sigma/rho correction cannot be added to either.

4. Summary

The fragment method of calculating log P(ow) has been proved valuable in many fields, including drug design and hazard assessment. However, manual calculations require a great deal of instructions and become very lengthy for complex structures and thus are error-prone. The computer program, CLOGP, enables the method to be applied by non-experts and includes an estimate of error, which is not possible in the future. Regular users can avail themselves of an annual update which will bring them current with all newly measured fragment values and improved correction factors. Versions with the Unified Driver compare the calculation from structure with a measured value from log P(ow) for neutral solutes. Starlist is also included in the annual updating service. We plan to make available in the near future a searching program, GENIE, which will extend the search of Starlist to close analogs.

The current literature contains many examples of QSAR accompanied by calculations of hydrophobicity which have not been made according to a consistent application of the rules they purport to follow. This has caused some confusion and cast doubt upon the entire approach. Perhaps, if the use of CLOGP becomes more widespread, published calculations will become more comparable, especially if reference is made to the program version.

Any prediction of the future is risky, but, judging from the recent past, we can expect an increasing demand for logP(ow) values. It is inconceivable that CLOGP will be perfected to such an extent that it supplants partition coefficient measurement. The two methods should remain as they are now: mutually complimentary.

With the STARLIST module in CLOGP, the user will be able to check the calculated value against an acceptable measured value if the solute structure entered is one of over 4,000 contained in that special file which is limited to non-tautomeric structures measured at a pH where the neutral form predominates.

5. Bibliography

1) Smith, R., Hansch, C. Ames, M., J. Pharm. Sci., 64, 599 (1975).
2) Martin, Y., Quantitative Drug Design, Medicinal Research Series No. 8, Marcel Dekker, New York, 1978.
3) Magee, P., Chemtech, 11, 378 (1981).
4) Smyth, R., Pfeffer, M., Van Harken, D., Cohen, A. and Hottendorf, G. Antimicrob. Agents Chemother., 10, 1004, (1981).
5) Koblin, D., Eger II, E.,Johnson, B.,Collins, P., Terrell, R., and Spears, L., Anesth. Analg., 60, 464 (1981).
6) Brown, D. and Flagg, E., J. Environ. Qual., 10, 382 (1981).
7) Ellinghausen, H.,Guth, J. and Eser, H., Ecotox. Environ. Safety, 4, 26 (1980).
8) Levitan, H., Proc. Nat. Acad. Sci. USA, 74, 2914 (1977).
9) Neeley, W., Branson, D. and Blau, G., Environ. Sci. Technol., 8, 1113 (1974).
10) Tanford, C., The Hydrophobic Effect, Wiley, New York, 1980, 2nd Ed., Ch. 13.
11) Coats, E., Genther, G., Dietrich, S., Guo, Z., and Hansch, C., J. Med. Chem., 24, 1422 (1981).
12) Smith, R., Hansch, C. and Langridge, R., Arch. Giochem. Biophys., 215, 319 (1982).
13) Martinek, K., and Semenov, A.,J. Appl. Biochem., 3, 93 (1981).
14a) Newcomb, M., Moore, S. and Cram, D., J. Am. Chem. Soc.,99, 6405 (1977).
14b) Hansch, C., J. Org. Chem., 43, 4889 (1978).
15) Unger, S. and Feuerman, T.,J. Chromatog., 176, 426 (1979).
16) Mirrlees, R. and Taylor, P., J. Med. Chem., 19, 615 (1976).
17) Tomlinson, E., Proceedings of A.Ph.A., Symposium on Partition Coefficients, San Diego, CA, (1982).
18) Tewari, Y., Miller, M., Wasik, S. and Martire, D., J. Chem. Eng. Data, 27, 451 (1982).
19) Fujita, T., Iwasa, J. and Hansch, C.,J. Am. Chem. Soc., 86, 5157(1964).
20) Rekker, R., The Hydrophobic Fragmental Constant, Elsevier, Amsterdam (1977).
21) Hansch, C. and Leo, A., Substituent Constants for Correlation Analysis in Chemistry and Biology, Wiley Interscience New York (1979).
21) Leo., A. and Hoekman, D., Perspect. in Drug Discov. & Design, 18, 19 (2000).
22) Chou, J. and Jurs, P., J. Chem. Inf. Comput. Sci., 19, 172 (1979).
23) Hammett, L., Physical Organic Chemistry: Reaction Rates, Equilibria and Mechanism, McGrawHill, New York, 1970, 2nd Ed.
24) Leo, A., J. Chem. Soc., Perkin Trans. II, 825 (1983).
25) Ogino, A., Matsumura, S. and Fujita, T., J. Med. Chem., 23, 437 (1980).
26) Leo, A. and Hansch, C.,J. Org. Chem., 36, 1539, (1971).


In $1 \times 10^3$ or more peptides among all peptides included in the candidate molecule-nucleic acid conjugate library, ClogP of the peptide may be, for example, 24 or less, 23 or less, 22 or less, 21 or less, or 20 or less. In $1 \times 10^3$ or more peptides among all peptides included in the candidate molecule-nucleic acid conjugate library, the lower limit of ClogP of the peptide may be, for example, 5 or more, 6 or more, 7 or more, 8 or more, or 10 or more. In $1 \times 10^3$ or more peptides among all peptides included in the candidate molecule-nucleic acid conjugate library, the range of ClogP of the peptide may be, for example, 5 or more and 24 or less, 6 or more and 23 or less, 7 or more and 22 or less, 8 or more and 21 or less, 9 or more and 20 or less, and 10 or more and 20 or less, 11 or more and 18 or less. In $1 \times 10^3$ or more peptides among all peptides included in the candidate molecule-nucleic acid conjugate library, the range of ClogP of the peptide may be, for example, 4 or more and 25 or less, preferably 6 or more and 23 or less, more preferably 8 or more and 21 or less, most preferably 9 or more and 20 or less.

In $1 \times 10^3$ or more peptides among all peptides included in the candidate molecule-nucleic acid conjugate library,

ClogP/number of amino acid residues of the peptide may be, for example, 1.0 or more. The "number of amino acid residues" means the total number of amino acid residues constituting the peptide. For example, the number of amino acid residues of the cyclic peptide in which the cyclic portion is constituted of 10 amino acid residues and the linear portion is constituted of 1 amino acid residue is 11. The ClogP/number of amino acid residues is a value calculated by dividing ClogP of a peptide by the number of amino acid residues contained in the peptide. For example, when ClogP of a peptide is 14.0 and the number of amino acid residues contained in the peptide is 7, ClogP/number of amino acid residues of the peptide is calculated to be 2.0.

In $1 \times 10^3$ or more peptides among all peptides included in the candidate molecule-nucleic acid conjugate library, ClogP/number of amino acid residues of the peptide may be, for example, 1.1 or more or 1.2 or more. In $1 \times 10^3$ or more peptides among all peptides included in the candidate molecule-nucleic acid conjugate library, the upper limit of ClogP/number of amino acid residues of the peptide may be, for example, 1.8 or less, 1.7 or less, 1.6 or less, or 1.5 or less. In $1 \times 10^3$ or more peptides among all peptides included in the candidate molecule-nucleic acid conjugate library, the range of ClogP/number of amino acid residues of the peptide may be, for example, 1.0 or more and 1.8 or less, preferably 1.0 or more and 1.7 or less, more preferably 1.1 or more and 1.6 or less, most preferably 1.1 or more and 1.5 or less.

Herein, whether $1 \times 10^3$ or more candidate molecules among all candidate molecules included in the candidate molecule-nucleic acid conjugate library are in a range of a specific value or not can be determined by seeking the value of each of all candidate molecules actually included in the candidate molecule-nucleic acid conjugate library and checking whether the number is $1 \times 10^3$ or more or not.

However, when the number of all candidate molecules included in the candidate molecule-nucleic acid conjugate library is $1 \times 10^3$ or more, all kinds of the candidate molecules that may theoretically be included in the candidate molecule-nucleic acid conjugate library are assumed, then $1 \times 10^3$ kinds or more candidate molecules among all candidate molecules included in the candidate molecule-nucleic acid conjugate library are considered to be included in a range of a specific value when $1 \times 10^3$ kinds or more candidate molecules among those all kinds of the candidate molecules are included in a range of a specific value.

(Method of carrying out contacting step)

In the contacting step, the plurality of kinds of target molecules in proximity to each other is contacted with the candidate molecule-nucleic acid conjugate library. Since the plurality of kinds of target molecules are in proximity, a molecule of interest capable of forming a complex with the plurality of kinds of target molecules can be efficiently obtained.

In one non-limiting aspect, the plurality of kinds of target molecules "in proximity to each other" means that the distance between the target molecules is 60 nm or less. The distance between the target molecules may be, for example, 55 nm or less, 50 nm or less, 45 nm or less, 40 nm or less, 35 nm or less, or 30 nm or less. The distance between the target molecules is preferably 50 nm or less, more preferably 40 nm or less, and most preferably 30 nm or less. Also, the distance between the target molecules is, for example, 1 nm or more, preferably 5 nm or more, more preferably 10 nm or more, and most preferably 15 nm or more. The distance between the target molecules is, for example, 1 nm or more and 60 nm or less, preferably 5 nm or more and 50 nm or less, more preferably 10 nm or more and 40 nm or less, and most preferably 15 nm or more and 30 nm or less. When the distance between the target molecules is in the above range, the efficiency to obtain a molecule of interest is more increased. The distance between the target molecules is the distance of a part having the shortest linear distance of the gaps between the target molecules. The distance between the target molecules may be 0 nm, that is the target molecules may be in contact or fused with each other. The distance between the target molecules can be controlled according to the means of bringing the target molecules in proximity to each other, for example, by adjusting a length of a linker when the plurality of kinds of target molecules are linked to each other by the linker, and by adjusting a density of the plurality of kinds of target molecules when they are placed in proximity to each other on a solid phase support. For example, when a plurality of kinds of target molecules forms a target fusion molecule via a fusion moiety, the maximum value of the distance between the target molecules is determined by the length of the fusion moiety. When the fusion moiety is a peptide of 15 alpha-amino acids which is known that length is approximately 5.7 nm (Advanced Drug Delivery Reviews, 2013, vol. 65, pp. 1357-1369), the distance between the target molecules in this case is 5.7 nm at the maximum that is the length assuming that the peptide has fully extended in a linear manner. As such, the plurality of kinds of target molecules forming the target fusion molecules are in proximity to each other.

In a non-limiting first aspect, the plurality of kinds of target molecules may be fused to each other to form a target fusion molecule. A plurality of kinds of target molecules can be brought in proximity to each other by forming a target fusion molecule.

That is, in the first aspect, the contacting step may be a step of contacting a target fusion molecule in which the plurality of kinds of target molecules are fused to each other with a candidate molecule-nucleic acid conjugate library. The target fusion molecule is, for example, a fused protein (also referred to herein as a fusion protein).

The target fusion molecule may be one in which a plurality of kinds of target molecules form a target fusion

molecule via a fusion moiety. The fusion moiety may be, for example, a peptide consisting of 150 or less amino acid residues, a peptide consisting of 125 or less amino acid residues, a peptide consisting of 100 or less amino acid residues, a peptide consisting of 75 or less amino acid residues, a peptide consisting of 55 or less amino acid residues, a peptide consisting of 35 or less amino acid residues, a peptide consisting of 25 or less amino acid residues, or a peptide consisting of 15 or less amino acid residues. Also, the fusion moiety may be, for example, a peptide consisting of 150 or less amino acid residues, preferably a peptide consisting of 125 or less amino acid residues, more preferably a peptide consisting of 100 or less amino acid residues, or most preferably a peptide consisting of 75 or less amino acid residues. This increases the frequency of the plurality of kinds of target molecules being in proximity to each other. The fusion moiety may also include, for example, a linker by a covalent bond other than a peptide, or a compound (such as a peptide) that photochemically degrades, and may be an antibody, a coiled-coil peptide tag (PNAS, 2018, vol. 115, no. 51, pp. 12961-12966), a nucleic acid, or a non-covalent linker such as a linker using Ni-NTA and a His tag.

When the plurality of kinds of target molecules form a target fusion molecule via a fusion moiety, the N-termini of the plurality of kinds of target molecules may be conjugated via the fusion moiety, the C-termini of the plurality of kinds of target molecules may be conjugated via the fusion moiety, or the N-terminus of a first target molecule in the plurality of kinds of target molecules and the C-terminus of a second target molecule in the plurality of kinds of target molecules may be conjugated via the fusion moiety.

In a non-limiting second aspect, the plurality of kinds of target molecules may be immobilized on the same solid phase support. By immobilizing on the same solid phase support, the plurality of kinds of target molecules can be in proximity to each other with higher frequency.

That is, in a second aspect, the contacting step may be a step of contacting (1) the plurality of kinds of target molecules immobilized on the same solid phase support with (2) a candidate molecule-nucleic acid conjugate library.

The shape of the solid phase support is not particularly limited, but may be, for example, a shape of a bead, a plate, or a chip. The solid phase support may have on the surface a molecule that may be utilized for affinity binding. Specifically, for example, the solid phase support has on its surface one or more selected from the group consisting of avidin, neutravidin, and streptavidin.

For example, $1 \times 10^{13}$ or more, $5 \times 10^{13}$ or more, $1 \times 10^{14}$ or more, or $5 \times 10^{14}$ or more target molecules may be immobilized per $m^2$ of a surface of the solid phase support. For example, $1 \times 10^{13}$ or more, preferably $5 \times 10^{13}$ or more, more preferably $1 \times 10^{14}$ or more, and most preferably $5 \times 10^{14}$ or more target molecules may be immobilized. This increases the frequency of the plurality of kinds of target molecules being in proximity to each other.

For example, a step of contacting $1 \times 10^{13}$ or more, $5 \times 10^{13}$ or more, $1 \times 10^{14}$ or more, or $5 \times 10^{14}$ or more target molecules per $m^2$ of the surface of a solid phase support with the solid phase support may be included. Also, for example, a step of contacting $1 \times 10^{13}$ or more, preferably $5 \times 10^{13}$ or more, more preferably $1 \times 10^{14}$ or more, and most preferably $5 \times 10^{14}$ or more target molecules with the solid phase support may be included. This increases the frequency of the plurality of kinds of target molecules being in proximity to each other.

In the second aspect, each of the target fusion molecules, the candidate molecule-nucleic acid conjugates, and/or the plurality of kinds of target molecules in proximity to each other may be immobilized on a solid phase support. Specific aspects of the solid phase support are as described above.

The contacting step may be a step including a step of mixing the candidate molecule-nucleic acid conjugate library with the target molecule or the target fusion molecule.

Specific examples of the contacting step include a step of contacting by a method of adding a candidate molecule-nucleic acid conjugate library to a liquid containing a plurality of kinds of target molecules or a target fusion molecule, mixing them, and then incubating the mixture. The conditions (time of incubation, temperature at the time of incubation, or the like) for contacting the plurality of kinds of target molecules or the target fusion molecule with the candidate molecule-nucleic acid conjugate library can be appropriately selected depending on the kind of the target molecule or target fusion molecule, the kind of the candidate molecule-nucleic acid conjugate library, and the like.

In a non-limiting third aspect, the contacting step may be a step of contacting a first target molecule, which is one kind selected from the plurality of kinds of target molecules, with a linked construct library including a plurality of kinds of linked constructs in which each candidate molecule-nucleic acid conjugate and a target molecule different from the first target molecule are linked.

In the third aspect, the candidate molecule-nucleic acid conjugate is bound to a target molecule that is different from the first target molecule. Thus, when the candidate molecule-nucleic acid conjugate and the first target molecule are close together, the frequency of the first target molecule and the target molecule bound to the candidate molecule-nucleic acid conjugate, which is a target molecule different from the first target molecule, to be in proximity can be increased.

In the third aspect, each of the candidate molecule-nucleic acid conjugates and a target molecule different from the first target molecule may form a linked construct via a linkage moiety.

The linkage moiety is not particularly limited as long as the candidate molecule-nucleic acid conjugate and the target molecule can be linked, for example, the linkage moiety may be one containing a His tag and Ni-NTA. Examples of the Ni-NTA include one or more selected from the group consisting of mono-Ni-NTA, di-Ni-NTA, tetrakis-Ni-NTA, and Tris-Ni-NTA (J. Am. Chem. Soc., 2005, vol. 127, pp. 10205-10215). Specifically, for example, a linked construct can be obtained by attaching Ni-NTA to a candidate molecule, attaching His-tag to a target molecule, and mixing the two.

In the third aspect, the linked construct and/or the first target molecule may be immobilized on a solid phase support. Specific aspects of the solid phase support are as described above.

The contacting step may include a step of mixing the linked construct library and the first target molecule.

Specific examples of the contacting step include a step of contacting by a method of adding a linked construct library to a liquid containing a first target molecule, mixing them, and then incubating the mixture. The conditions (incubation time, incubation temperature, etc.) under which the first target molecule is contacted with the linked construct library can be appropriately selected according to the kind of first target molecule, the kind of linked construct library, and the like.

Examples of variations of the third aspect include a target molecule-candidate molecule linked construct in which the target molecule and the candidate molecule are linked via a linkage moiety, and a method of producing a target-molecule-candidate molecule linked construct by linking the target molecule and the candidate molecule via a linkage moiety. Further examples of variations of the third aspect include a target molecule-candidate molecule-nucleic acid linked construct in which the target molecule and the candidate molecule are linked via a linkage moiety, and in which the candidate molecule and the nucleic acid are conjugated via a conjugation moiety, and a method of producing a target molecule-candidate molecule-nucleic acid linked construct by linking the target molecule and the candidate molecule via a linkage moiety and conjugating the candidate molecule and the nucleic acid via a conjugation moiety. Specific aspects of the linkage moiety and the conjugation moiety are as described above.

In a fourth aspect, the contacting step may be a step of contacting a linked construct prepared through a step of preparing a linked construct by linking a plurality of kinds of target molecules to each other (linking step) with a candidate molecule-nucleic acid conjugate, or a step of contacting a linked construct prepared through a step of preparing a linked construct by linking one or more kinds of target molecules and a candidate molecule-nucleic acid conjugate (linking step) with a target molecule that is not used in the linking step.

In one non-limiting aspect, the linked construct and a target molecule other than the linked construct are contacted with the candidate molecule-nucleic acid conjugate in the contacting step.

The target molecule other than the linked construct may be the same kind of target molecule as one of target molecules contained in the linked construct.

The target molecule other than the linked construct may be one kind of target molecule.

The target molecule other than the linked construct may be not immobilized on a solid phase support.

In the contacting step, the ratio of the concentration of the target molecule other than the linked construct to the concentration of the linked construct is, for example, 1 or more, preferably 3 or more, more preferably 5 or more, and most preferably 10 or more.

Also, in the contacting step, the ratio of the concentration of the target molecule other than the linked construct to the concentration of the linked construct is, for example, 1,000 or less, preferably 500 or less, more preferably 300 or less, and most preferably 100 or less.

Also, in the contacting step, the ratio of the concentration of the target molecule other than the linked construct to the concentration of the linked construct is, for example, 1 or more and 1,000 or less, preferably 3 or more and 500 or less, more preferably 5 or more and 300 or less, and most preferably 10 or more and 100 or less.

(Linking step)

In the linking step, a plurality of kinds of target molecules are linked to each other, or a plurality of kinds of target molecules and a candidate molecule-nucleic acid conjugate are linked. By the linking step, a linked construct in which a target molecule and a target molecule are linked, or a linked construct in which a target molecule and a candidate molecule-nucleic acid conjugate are linked is obtained. The target molecules contained in the linked construct in which a target molecule and a target molecule are linked may be the same to each other or may be different, but are preferably different from each other.

In a fifth aspect, the contacting step may be a step of contacting a linked construct in which a plurality of kinds of target molecules are linked to each other with a candidate molecule-nucleic acid conjugate, or contacting a linked construct in which one or more kinds of target molecules and a candidate molecule-nucleic acid conjugate are linked with a target molecule that is not contained in the linked construct.

In the fourth and fifth aspects, examples of the means for preparing a linked construct in which a target molecule and a target molecule are linked include the same aspects as the aspects described for the means for contacting a plurality of kinds of target molecules in proximity with a candidate molecule-nucleic acid conjugate library. That is, the means for preparing a linked construct in which a target molecule and a target molecule are linked may be, but is not particularly limited to, (1) preparing a target fusion molecule in which a plurality of kinds of target molecules are fused to each other, (2) immobilizing a plurality of kinds of target molecules on the same solid phase support, or (3) linking by embedding each of

the target molecules into a lipid bilayer such as a liposome or nanodisc. In particular, among these, the method of (1) is most preferred. In addition, for (1), the linked construct may be a linked construct (target fusion molecule) in which target molecules are linked to each other via a fusion moiety. The linked construct of a target molecule and a candidate molecule-nucleic acid conjugate may be a linked construct in which the target molecule and the candidate molecule-nucleic acid conjugate are linked via a fusion moiety. Examples of the fusion moiety include the same aspects as the aspects described for the target fusion molecule. That is, the fusion moiety may be, for example, a peptide consisting of 150 or less amino acid residues, a peptide consisting of 125 or less amino acid residues, a peptide consisting of 100 or less amino acid residues, or a peptide consisting of 75 or less amino acid residues. Also, the fusion moiety may be, for example, a peptide consisting of 150 or less amino acid residues, preferably a peptide consisting of 125 or less amino acid residues, more preferably a peptide consisting of 100 or less amino acid residues, or most preferably a peptide consisting of 75 or less amino acid residues. This increases the frequency of the plurality of kinds of target molecules being in proximity to each other. The fusion moiety may also include, for example, a linker by a covalent bond other than a peptide, or a compound (such as a peptide) that photochemically degrades, and may be an antibody, a coiled-coil peptide tag (PNAS, 2018, vol. 115, no. 51, pp. 12961-12966), a nucleic acid, or a non-covalent linker such as a linker using Ni-NTA and a His tag.

The fusion moiety between the target molecules may include, for example, a linker by a covalent bond other than a peptide, or a compound (such as a peptide) that photochemically degrades, and may be an antibody, a coiled-coil peptide tag (PNAS, 2018, vol. 115, no. 51, pp. 12961-12966), a nucleic acid, or a non-covalent linker such as a linker using Ni-NTA and a His tag.

The fusion moiety between the target molecule and the candidate molecule-nucleic acid conjugate may include, for example, a linker by a covalent bond other than a peptide, or a compound (such as a peptide) that photochemically degrades, and may be an antibody, a coiled-coil peptide tag (PNAS, 2018, vol. 115, no. 51, pp. 12961-12966), a nucleic acid, or a non-covalent linker such as a linker using Ni-NTA and a His tag.

[Recovery step]

In the recovery step, a complex of at least one kind of target molecule selected from the plurality of kinds of target molecules and the candidate molecule-nucleic acid conjugate is recovered.

The recovery of the complex can be performed in a manner well known to those skilled in the art. For example, a complex can be recovered by a method utilizing affinity such as a pull-down method.

Examples of the method utilizing an affinity include a method utilizing an affinity between biotin and a biotin-binding protein, an affinity between a His tag and Ni-NTA, or an affinity between an antigen and an antibody, and preferably a method utilizing an affinity between biotin and a biotin-binding protein.

In one non-limiting aspect, the candidate molecule-nucleic acid conjugate that forms a complex with a target molecule can be recovered by, for example, modifying the target molecule with biotin, mixing the modified target molecule with a solid phase support (e.g., a bead) on which biotin-binding proteins are bound at the surface, thereby affinity binding the biotin to the biotin binding protein, and recovering the bead by centrifugation or the like.

Examples of the biotin-binding protein herein include avidin, neutravidin, and streptavidin.

[Elution step]

In the elution step, a nucleic acid in the candidate molecule-nucleic acid conjugate recovered by the recovery step is eluted.

The nucleic acid may be eluted in the form of the nucleic acid itself, of a candidate molecule-nucleic acid conjugate, or of a complex of the target molecule with the candidate molecule-nucleic acid conjugate.

That is, in the elution step, the binding of the candidate molecule and the nucleic acid may be cleaved, the binding of the target molecule and the candidate molecule-nucleic acid conjugate may be cleaved, and/or the binding of the solid phase support and the target molecule may be cleaved. By cleaving the binding of the candidate molecule and the nucleic acid, the nucleic acid can be eluted in the form of the nucleic acid itself. By cleaving the binding of the target molecule and the candidate molecule-nucleic acid conjugate, the nucleic acid can be eluted in the form of the candidate molecule-nucleic acid conjugate. By cleaving the binding of the solid phase support and the target molecule, the nucleic acid can be eluted in the form of a complex between the target molecule and the candidate molecule-nucleic acid conjugate.

The cleaving of the bond can be carried out, for example, by one or more selected from the group consisting of a method using an enzyme, a method using light, or a method using heat. The cleaving of the bond is preferably carried out by a method using an enzyme.

The cleaving of the bond by the method using an enzyme can be carried out, for example, by making a substrate that is specifically recognized and cleaved by an enzyme be contained in the conjugation moiety of the candidate molecule and the nucleic acid, the conjugation moiety of the target molecule and the candidate molecule-nucleic acid conjugate, or the conjugation moiety of the solid phase support and the target molecule. By making the enzyme react with the complex

containing the substrate, the bond is cleaved and the nucleic acid is eluted. Specific examples of the combination of an enzyme and a substrate can include a combination of a proteolytic enzyme such as a TEV protease or a 3C protease, and a peptide containing an amino acid sequence specifically recognized and cleaved by the proteolytic enzyme, and a combination of a DNA degradation enzyme such as a restriction enzyme and a DNA containing a nucleotide sequence specifically recognized and cleaved by the DNA degradation enzyme.

The cleaving of the bond by the method using light can be carried out, for example, by making a structure that degrades photochemically be contained in the conjugation moiety of the candidate molecule and the nucleic acid, the conjugation moiety of the target molecule and the candidate molecule-nucleic acid conjugate, or the conjugation moiety of the solid phase support and the target molecule. By performing light irradiation at an appropriate wavelength on the complex containing the structure, the bond is cleaved and the nucleic acid is eluted. Specific examples of the structure that degrades photochemically include a 6-nitroveratryloxycarbonyl (NVOC) structure and a coumarin structure.

The cleaving of the bond by the method using heat can be carried out, for example, by using a binding utilizing an affinity, such as a binding between biotin and a biotin-binding protein, in the binding of the candidate molecule and the nucleic acid, the binding of the target molecule and the candidate molecule-nucleic acid conjugate, or the binding of the solid phase support and the target molecule. By applying heat, for example, at 95°C for 10 minutes, to the complex having such a bond, the bond is cleaved and the nucleic acid is eluted.

[Amplification step]

In the amplification step, a nucleic acid in a candidate molecule-nucleic acid conjugate forming the complex is amplified. The amplification step may be performed after the recovery step, but the step is preferably performed after the elution step, and more preferably after the elution step and before the identification step.

Amplification of a nucleic acid can be carried out, for example, by a PCR method with a recovered complex or eluted nucleic acid as a template, using a primer that binds to a sequence for use in amplification in the nucleic acid.

[Repeating method]

The screening method according to the present embodiment may be a method in which a candidate molecule-nucleic acid conjugate library is newly generated from the nucleic acid amplified in the amplification step, and the newly generated candidate molecule-nucleic acid conjugate library is used in multiple repeating of the contacting step, the recovery step, and the amplification step.

By repeating the contacting step, the recovery step, and the amplification step, a library in which the molecule of interest is concentrated can be obtained. The number of repeating may be one or more, two or more, three or more, four or more, or five or more, specifically, for example, one, two, three, four, or five. In one non-limiting aspect, when the number of repeating is four or more, the molecule of interest is usually highly concentrated, and thus the molecule of interest can be obtained without performing an identification step.

The screening method according to another embodiment may be a method in which a candidate molecule-nucleic acid conjugate library is newly generated from the nucleic acid amplified in the amplification step, and the newly generated candidate molecule-nucleic acid conjugate library is used in multiple repeating of the contacting step, the recovery step, the elution step, and the amplification step.

By repeating the contacting step, the recovery step, the elution step and the amplification step, a library in which the molecule of interest is concentrated can be obtained. The number of repeating may be two or more, three or more, four or more, or five or more, specifically, for example, two, three, four, or five. In one non-limiting aspect, when the number of repeating is four or more, the molecule of interest is usually highly concentrated, and thus the molecule of interest can be obtained without performing an identification step.

[Identification step]

In the identification step, a molecule of interest from a candidate molecule-nucleic acid conjugate forming the complex is identified. The identification step may be performed after the recovery step, but the step is preferably performed after the elution step or after the amplification step, and more preferably after the elution step and after the amplification step.

The identification step may include determining a sequence of the nucleic acid eluted in the elution step or the nucleic acid amplified in the amplification step. By sequencing the nucleic acid, the kind of candidate molecule forming a conjugate with the nucleic acid can be identified.

The identification step may include comparing a recovery rate of the candidate molecule-nucleic acid conjugate when the plurality of kinds of target molecules in proximity to each other (recovery rate A) is contacted with the candidate molecule-nucleic acid conjugate library, with a recovery rate of the candidate molecule-nucleic acid conjugate when only

one kind of the target molecule selected from the plurality of kinds of target molecules is contacted with the candidate molecule-nucleic acid conjugate library (recovery rate B).

In this case, when the recovery rate A is 5-fold or more, preferably 10-fold or more, more preferably 20-fold or more, and most preferably 30-fold or more compared to the recovery rate B, the candidate molecule can be identified as the molecule of interest. When the fold ratio of the recovery rate A compared to the recovery rate B is in the above range, it can be said that the nature of the identified molecule of interest to form a complex with the plurality of kinds of target molecules is stronger.

Here, the term "recovery rate" is the proportion (percentage: %) of the number of molecules of the candidate molecule-nucleic acid conjugate recovered by forming a complex with the target molecule (number of output molecules) relative to the number of molecules of the candidate molecule-nucleic acid conjugate in the library contacted with the target molecule (number of input molecules).

The recovery rate B also includes the recovery rate when only each one of the plurality of kinds of target molecules is contacted with the candidate molecule-nucleic acid conjugate library. For example, when the target molecules are two kinds of target molecules, the recovery rate B includes the recovery rate B1 of the case when only a first target molecule is contacted with the candidate molecule-nucleic acid conjugate library, and the recovery rate B2 of the case when only a second target molecule is contacted with the candidate molecule-nucleic acid conjugate library. In this case, when the recovery rate A is 5-fold or more, preferably 10-fold or more, more preferably 20-fold or more, and most preferably 30-fold or more compared to both the recovery rate B1 and the recovery rate B2, the candidate molecule can be identified as the molecule of interest. When the fold ratio of the recovery rate A compared to both the recovery rate B1 and the recovery rate B2 is in the above range, it can be said that the nature of the identified molecule of interest to form a complex with the plurality of kinds of target molecules is stronger.

The identification step may include comparing a recovery rate of the candidate molecule-nucleic acid conjugate when the plurality of kinds of target molecules in proximity to each other is contacted with the candidate molecule-nucleic acid conjugate library (recovery rate A), with a recovery rate of the candidate molecule-nucleic acid conjugate when the target molecule is absent (recovery rate C).

In this case, when the recovery rate A is 5-fold or more, preferably 10-fold or more, more preferably 20-fold or more, and most preferably 30-fold or more compared to the recovery rate C, the candidate molecule can be identified as the molecule of interest. When the fold ratio of the recovery rate A compared to the recovery rate C is in the above range, it can be said that the nature of the identified molecule of interest to form a complex with the plurality of kinds of target molecules is stronger.

The identification step may include comparing (1) a recovery rate of the candidate molecule-nucleic acid conjugate when the plurality of kinds of target molecules in proximity to each other is contacted with the candidate molecule-nucleic acid conjugate library (recovery rate A), and (2) a recovery rate of the candidate molecule-nucleic acid conjugate when a first target molecule that is one kind of the target molecule selected from the plurality of kinds of target molecules and immobilized on a solid phase support and a target molecule that is different from the first target molecule and not immobilized on the solid phase support are contacted with the candidate molecule-nucleic acid conjugate library (recovery rate D).

In this case, when the recovery rate A is 5-fold or more, preferably 10-fold or more, more preferably 20-fold or more, and most preferably 30-fold or more compared to the recovery rate D, the candidate molecule can be identified as the molecule of interest. When the fold ratio of the recovery rate A compared to the recovery rate D is in the above range, it can be said that the nature of the identified molecule of interest to form a complex with the plurality of kinds of target molecules is stronger. The recovery rate D also includes a recovery rate in each of the case when any one target molecule of the plurality of kinds of target molecules is selected as the first target molecule. For example, when the target molecules are two kinds of target molecules of target molecule X and target molecule Y, the recovery rate D includes a recovery rate D1 when the target molecule X is selected as the first target molecule and a recovery rate D2 when the target molecule Y is selected as the first target molecule. In this case, when the recovery rate A is 5-fold or more, preferably 10-fold or more, more preferably 20-fold or more, and most preferably 30-fold or more compared to both the recovery rate D1 and the recovery rate D2, the candidate molecule can be identified as the molecule of interest. When the fold ratio of the recovery rate A compared to both the recovery rate D1 and the recovery rate D2 is in the above range, it can be said that the nature of the identified molecule of interest to form a complex with the plurality of kinds of target molecules is stronger.

The identification step may include all comparing the recovery rates A, B, C, and D to each other.

The identification step may also include all comparing each of the recovery rates B, C, and D to the recovery rate A.

In this case, when the recovery rate A is 5-fold or more, preferably 10-fold or more, more preferably 20-fold or more, and most preferably 30-fold or more compared to any of the recovery rate B, the recovery rate C, and the recovery rate D, the candidate molecule can be identified as the molecule of interest. When the fold ratio of the recovery rate A compared to any of the recovery rate B, the recovery rate C, and the recovery rate D is in the above range, it can be said that the nature of the identified molecule of interest to form a complex with the plurality of kinds of target molecules is stronger.

The identification step may also include comparing the recovery rate D to the recovery rate B.

In this case, when the recovery rate D is 5-fold or more, preferably 10-fold or more, more preferably 20-fold or more, and most preferably 30-fold or more compared to the recovery rate B, the candidate molecule can be identified as the molecule of interest. When the fold ratio of the recovery rate D compared to the recovery rate B is in the above range, it can be said that the nature of the identified molecule of interest to form a complex with the plurality of kinds of target molecules is stronger.

The screening method described above may be performed in vitro.

The screening method described above may be performed without using fluorescence by Split-GFP or luminescence by luciferase.

The molecule of interest selected by the screening method according to the present invention is a molecule capable of forming a complex with a plurality of kinds of target molecules.

When the molecule of interest is a molecule capable of forming a complex with a plurality of kinds of target molecules, a value of a dissociation constant of the plurality of kinds of target molecules to each other in the presence of the molecule of interest of 10 μM is small compared to a value of a dissociation constant of the plurality of kinds of target molecules to each other in the absence of the molecule of interest. That is, in the present invention, the state in which the molecule of interest forms a complex with a plurality of kinds of target molecules means a state in which the value of the dissociation constant of the plurality of kinds of target molecules to each other in the presence of the molecule of interest of 10 μM is small compared to the value of the dissociation constant of the plurality of kinds of target molecules to each other in the absence of the molecule of interest.

It can be said that the smaller the value of the dissociation constant of the plurality of kinds of target molecules to each other in the presence of the molecule of interest of 10 μM compared to the value of the dissociation constant of the plurality of kinds of target molecules to each other in the absence of the molecule of interest, the stronger the nature of the molecule of interest to form a complex with the plurality of kinds of target molecules and the more stable the complex formed by the molecule of interest with the plurality of kinds of target molecules.

Here, the value of the dissociation constant of the plurality of kinds of target molecules to each other in the presence of the molecule of interest of 10 μM may be, for example, less than one-fifth, preferably less than one-tenth, more preferably less than one-twentieth, and most preferably less than one-thirtieth compared to the value of the dissociation constant of the plurality of kinds of target molecules to each other in the absence of the molecule of interest. It can be said that, when the value of the dissociation constant of the plurality of kinds of target molecules to each other in the presence of the molecule of interest of 10 μM is in the above range compared to the value of the dissociation constant of the plurality of kinds of target molecules to each other in the absence of the molecule of interest, the nature of the molecule of interest to form a complex with the plurality of kinds of target molecules is stronger and the complex formed by the molecule of interest with the plurality of kinds of target molecules is more stable.

The value of the dissociation constant of the plurality of kinds of target molecules to each other in the presence of the molecule of interest of 10 μM may be, for example, 30 μM or less, and is preferably 20 μM or less, more preferably 10 μM or less, and most preferably 5 μM or less. It can be said that, when the value of the dissociation constant of the plurality of kinds of target molecules to each other in the presence of the molecule of interest of 10 μM is in the above range, the nature of the molecule of interest to form a complex with the plurality of kinds of target molecules is stronger and the complex formed by the molecule of interest with the plurality of kinds of target molecules is more stable.

The value of the dissociation constant of the plurality of kinds of target molecules to each other in the presence of the molecule of interest of 10 μM may be, for example, 30 μM or less and less than one-fifth compared to the value of the dissociation constant of the plurality of kinds of target molecules to each other in the absence of the molecule of interest, and is preferably 20 μM or less and less than one-fifth compared to the value of the dissociation constant of the plurality of kinds of target molecules to each other in the absence of the molecule of interest, more preferably 10 μM or less and less than one-fifth compared to the value of the dissociation constant of the plurality of kinds of target molecules to each other in the absence of the molecule of interest, and most preferably 5 μM or less and less than one-fifth compared to the value of the dissociation constant of the plurality of kinds of target molecules to each other in the absence of the molecule of interest. It can be said that, when the value of the dissociation constant of the plurality of kinds of target molecules to each other in the presence of the molecule of interest of 10 μM is in the above range, the nature of the molecule of interest to form a complex with the plurality of kinds of target molecules is stronger and the complex formed by the molecule of interest with the plurality of kinds of target molecules is more stable.

The value of the dissociation constant of the plurality of kinds of target molecules to each other in the presence of the molecule of interest of 10 μM may be, for example, 30 μM or less and less than one-fifth compared to the value of the dissociation constant of the plurality of kinds of target molecules to each other in the absence of the molecule of interest, and is preferably 20 μM or less and less than one-tenth compared to the value of the dissociation constant of the plurality of kinds of target molecules to each other in the absence of the molecule of interest, more preferably 10 μM or less and less than one-twentieth compared to the value of the dissociation constant of the plurality of kinds of target molecules to each other in the absence of the molecule of interest, and most preferably 5 μM or less and less than one-thirtieth compared to

the value of the dissociation constant of the plurality of kinds of target molecules to each other in the absence of the molecule of interest. It can be said that, when the value of the dissociation constant of the plurality of kinds of target molecules to each other in the presence of the molecule of interest of 10 μM is in the above range, the nature of the molecule of interest to form a complex with the plurality of kinds of target molecules is stronger and the complex formed by the molecule of interest with the plurality of kinds of target molecules is more stable.

The molecule of interest selected by the screening method according to the present invention may have a dissociation constant of at least two target molecules to each other selected from the plurality of kinds of target molecules in the presence of the molecule of interest of 10 μM of 10 μM or less, preferably 5 μM or less, more preferably 3 μM or less, most preferably 1 μM or less. It can be said that the smaller the value of the dissociation constant, the stronger the nature of the molecule of interest to form a complex with at least two target molecules selected from the plurality of kinds of target molecules.

The molecule of interest selected by the screening method according to the present invention may have a dissociation constant of 10 μM or less, preferably 5 μM or less, more preferably 3 μM or less, most preferably 1 μM or less in all of the plurality of kinds of target molecules to each other in the presence of the molecule of interest of 10 μM.

The molecule of interest selected by the screening method according to the present invention may have a value of the dissociation constant of the plurality of kinds of target molecules to each other in the absence of the molecule of interest of 5-fold or more, preferably 7-fold or more, more preferably 10-fold or more, and most preferably 20-fold or more compared to the value of the dissociation constant of the plurality of kinds of target molecules to each other in the presence of the molecule of interest of 10 μM. It can be said that the smaller the value of the dissociation constant, the stronger the nature of the molecule of interest to form a complex with the plurality of kinds of target molecules.

In the present invention, the state in which the molecule of interest forms a complex with a plurality of kinds of target molecules means a state in which the dissociation constant of the plurality of kinds of target molecules to each other in the presence of the molecule of interest of 10 μM is 10 μM or less and the value of the dissociation constant of the plurality of kinds of target molecules to each other in the absence of the molecule of interest is 5-fold or more compared to the value of the dissociation constant of the plurality of kinds of target molecules to each other in the presence of the molecule of interest of 10 μM.

As another aspect in the present invention, the state in which the molecule of interest forms a complex with a plurality of kinds of target molecules means, for example, a state in which the dissociation constant of the plurality of kinds of target molecules to each other in the presence of the molecule of interest of 10 μM is 30 μM or less and the value of the dissociation constant of the plurality of kinds of target molecules to each other in the absence of the molecule of interest is 5-fold or more compared to the value of the dissociation constant of the plurality of kinds of target molecules to each other in the presence of the molecule of interest of 10 μM.

As another aspect in the present invention, the state in which the molecule of interest forms a complex with a plurality of kinds of target molecules means preferably a state in which the dissociation constant of the plurality of kinds of target molecules to each other in the presence of the molecule of interest of 10 μM is 20 μM or less and the value of the dissociation constant of the plurality of kinds of target molecules to each other in the absence of the molecule of interest is 5-fold or more compared to the value of the dissociation constant of the plurality of kinds of target molecules to each other in the presence of the molecule of interest of 10 μM.

As another aspect in the present invention, the state in which the molecule of interest forms a complex with a plurality of kinds of target molecules means more preferably a state in which the dissociation constant of the plurality of kinds of target molecules to each other in the presence of the molecule of interest of 10 μM is 15 μM or less and the value of the dissociation constant of the plurality of kinds of target molecules to each other in the absence of the molecule of interest is 5-fold or more compared to the value of the dissociation constant of the plurality of kinds of target molecules to each other in the presence of the molecule of interest of 10 μM.

As another aspect in the present invention, the state in which the molecule of interest forms a complex with a plurality of kinds of target molecules means most preferably a state in which the dissociation constant of the plurality of kinds of target molecules to each other in the presence of the molecule of interest of 10 μM is most preferably 10 μM or less and the value of the dissociation constant of the plurality of kinds of target molecules to each other in the absence of the molecule of interest is 5-fold or more compared to the value of the dissociation constant of the plurality of kinds of target molecules to each other in the presence of the molecule of interest of 10 μM.

It should be noted that the dissociation constant of the plurality of kinds of target molecules to each other can be measured by surface plasmon resonance (SPR). For example, the dissociation constant of the two kinds of target molecules to each other in the presence of the molecule of interest of 10 μM can be determined by the following method. First, the Biotin CAPture reagent is immobilized on all flow cells on the sensor chip, and then a first kind of target molecule is immobilized on some of the flow cells. Next, as an analyte, a mixed solution of a second kind of target molecule in a dilution series with the highest concentration of 10 μM or 20 μM and a molecule of interest of 10 μM is prepared, and added to all flow cells to obtain a sensorgram of the binding.

As a running buffer, HBS, 1 mM dithiothreitol, 0.01% Tween 20, and 4% dimethylsulfoxide (DMSO) can be used.

In the running buffer, 5 mM $CaCl_2$ can be added additionally.

Analysis of the resulting sensorgram can be performed, for example, with T200 evaluation software (Cytiva). From a curve fitting using a 1 : 1 binding model for a sensorgram subjected to the processing in which the sensorgram obtained with the flow cell where the first kind of target molecule is not immobilized and the sensorgram obtained when the running buffer is added are subtracted as double references and DMSO solvent correction is performed, the binding rate constant ($k_a$), the dissociation rate constant ($k_d$), and the dissociation constant $K_D$ can be determined as the apparent target molecule interaction parameters for the analyte in the presence of the molecule of interest of 10 $\mu$M.

In one non-limiting aspect, the molecule of interest selected by the screening method according to the present invention may be a candidate molecule when the recovery rate A is 5-fold or more compared to the recovery rate B.

In one non-limiting aspect, the molecule of interest selected by the screening method according to the present invention may be a candidate molecule when the recovery rate A is 5-fold or more compared to the recovery rate C.

In one non-limiting aspect, the molecule of interest selected by the screening method according to the present invention may be a candidate molecule when the recovery rate A is 5-fold or more compared to the recovery rate D.

[Method of producing peptide]

The method of producing a peptide according to the present invention includes carrying out the screening method according to the present invention described above. Thereby, a peptide capable of forming a complex with a plurality of kinds of target molecules can be produced.

The method of producing a peptide may include a contacting step, a recovery step, optionally an elution step, an amplification step, and optionally an identification step in this order, and further include a step of transcribing and translating a nucleic acid amplified in an amplification step as a template to obtain a peptide of interest (translation step). The translation step can be performed by a method well known to those skilled in the art (e.g., a method using a cell-free translation system).

The method of producing a peptide may also include a contacting step, a recovery step, optionally an elution step, an amplification step, and optionally an identification step in this order, and further include a step of decoding the nucleotide sequence of the nucleic acid amplified in the amplification step to identify the amino acid sequence of the peptide of interest (decoding step) and a step of synthesizing the peptide of interest based on the identified amino acid sequence (synthesis step). The decoding step and the synthesis step can be carried out by a method well known to those skilled in the art.

[Peptide having specific motif]

The present inventors have analyzed the amino acid sequence of a molecule of interest (peptide) selected by the screening method according to the present invention, and have found that a molecule of interest (peptide) capable of forming a complex with a particular plurality of kinds of target molecules (CyA and Cn, or FKBP and FRP) has a particular amino acid sequence structure (motif). Specifically, the following findings (a) to (h) have been obtained.

(a) A peptide having a structure represented by formula (I-A2) (amino acid sequence: * - (D-MeSer)-(Pic(2))-(Phe(3-Cl))- *, wherein * indicates a bond with another amino acid residue) forms a complex with FKBP and FRP.

[Formula 30]

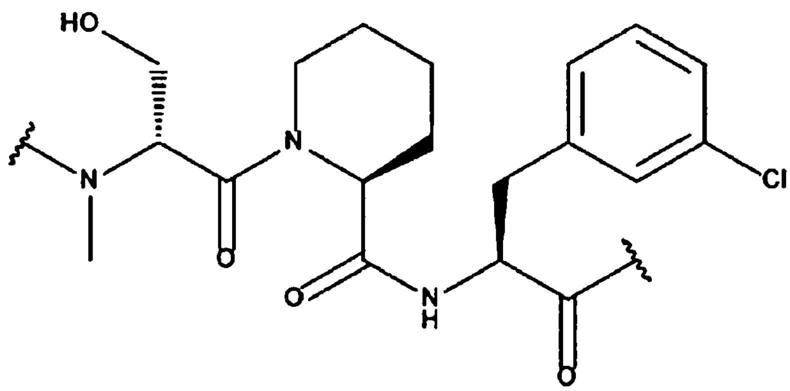

・・・(I－A2)

(b) A peptide having a structure represented by formula (I-B2) (amino acid sequence: * - (nBuGly)-(Pic(2))-(Hph(3-Cl))- *, wherein * indicates a bond with another amino acid residue) forms a complex with FKBP and FRP.

[Formula 31]

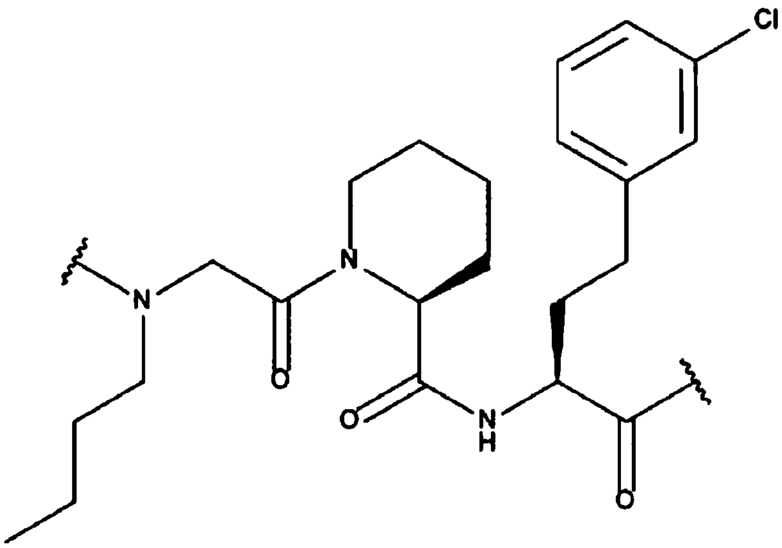

・・・(I－B2)

(c) A peptide having a structure represented by formula (II-Aa2) (amino acid sequence: * -(MeV)-(MeHnl(7-F2))-(Thr)-*, wherein * indicates a bond with another amino acid residue) forms a complex with Cy-A and Cn.

[Formula 32]

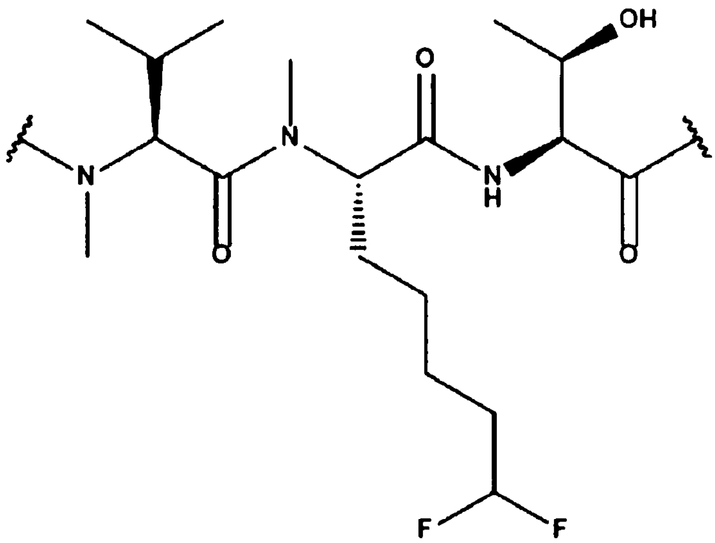

・・・(II－Aa2)

(d) A peptide having a structure represented by formula (II-Ab2) (amino acid sequence: * -(MeV)-(MeSer(nPr))-(Thr)-*, wherein * indicates a bond with another amino acid residue) forms a complex with Cy-A and Cn.

[Formula 33]

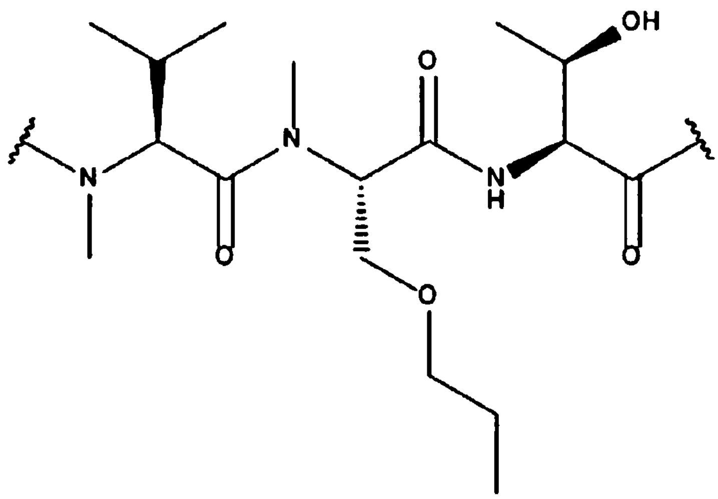

・・・(II-Ab2)

(e) A peptide having a structure represented by formula (II-Ac2) (amino acid sequence: * -(MeV)-(MeHph)-(Thr)- *, wherein * indicates a bond with another amino acid residue) forms a complex with Cy-A and Cn.

[Formula 34]

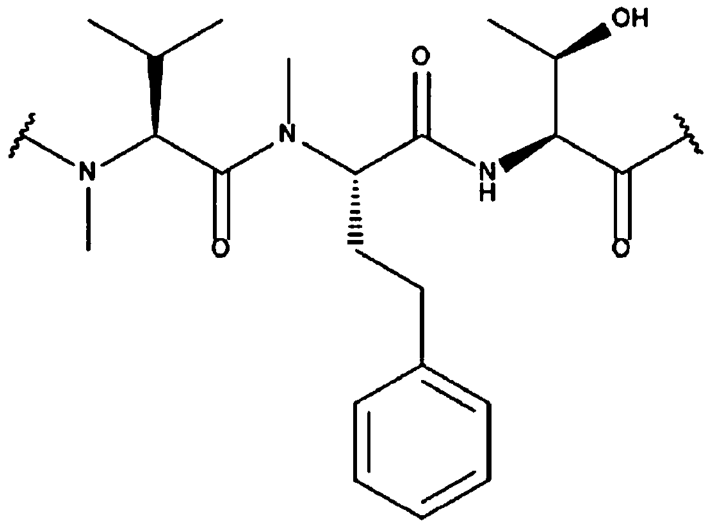

・・・(II-Ac2)

(f) A peptide having a structure represented by formula (II-Ba2) (amino acid sequence: * -(MeV)-(MeHnl(7-F2))-(Ile)- *, wherein * indicates a bond with another amino acid residue) forms a complex with Cy-A and Cn.

[Formula 35]

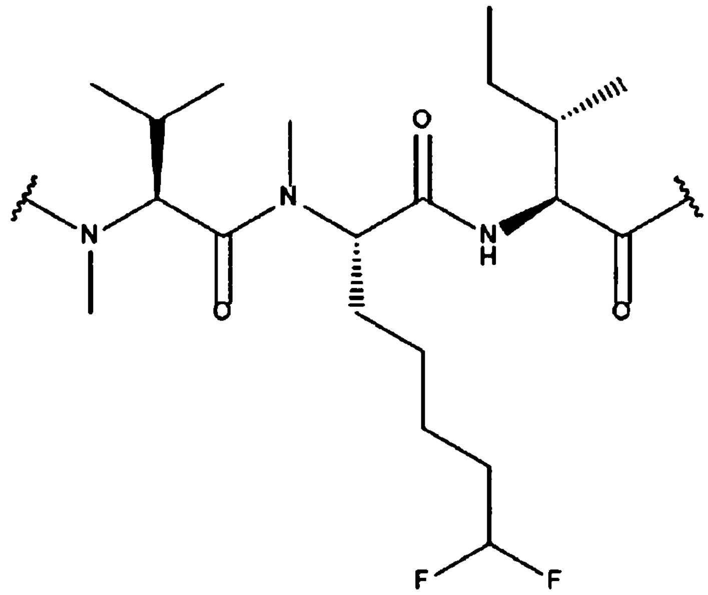

・・・(II-Ba2)

(g) A peptide having a structure represented by formula (II-Bb2) (amino acid sequence: * -(MeV)-(MeSer(nPr))-(Ile)- *, wherein * indicates a bond with another amino acid residue) forms a complex with Cy-A and Cn.

[Formula 36]

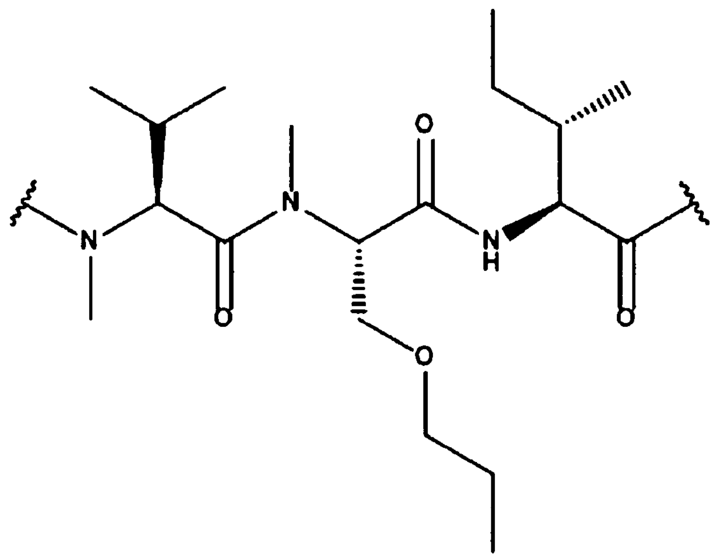

・・・(II-Bb2)

(h) A peptide having a structure represented by formula (II-Bc2) (amino acid sequence: * -(MeV)-(MeHph)-(Ile)- *, wherein * indicates a bond with another amino acid residue) forms a complex with Cy-A and Cn.

[Formula 37]

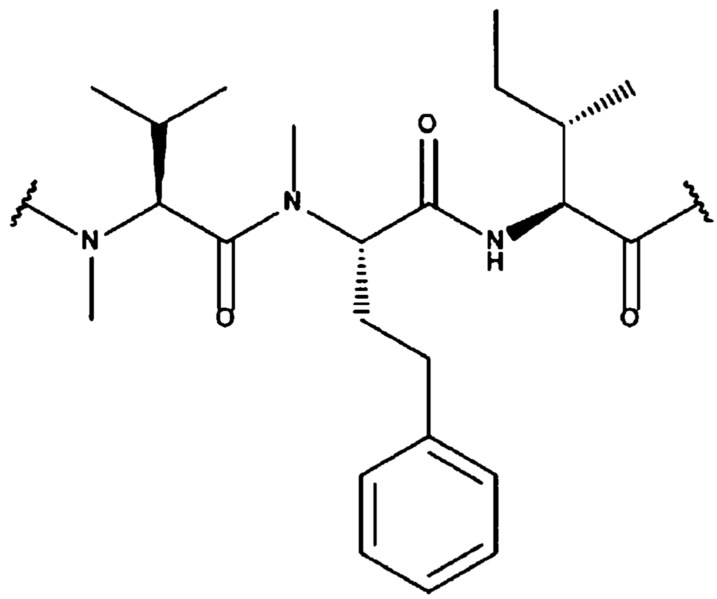

・・・(II-Bc2)

Based on these findings of (a) to (h), the present invention encompasses a peptide having a structure represented by formula (I) or (II).

[Formula 38]

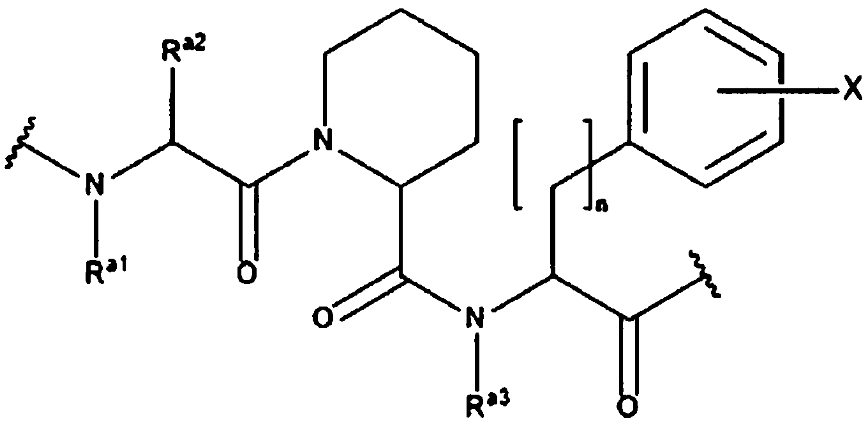

・・・(I)

[Formula 39]

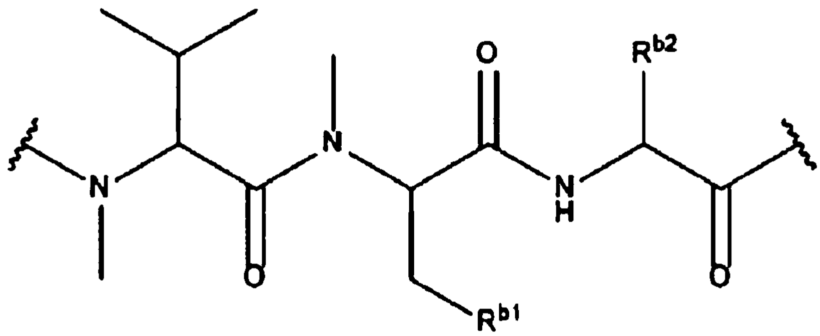

・・・(II)

In the formulas (I) and (II),

[Formula 40]

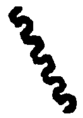

represents a bond,

in the formula (I), $R^{a1}$ is a hydrogen atom or $C_1$-$C_6$ alkyl, $R^{a2}$ is a hydrogen atom or $C_1$-$C_3$ alkyl optionally substituted with hydroxy, $R^{a3}$ is a hydrogen atom or methyl, X is halogen, and n is 1 or 2, and
in the formula (II), $R^{b1}$ is $C_1$-$C_6$ alkyl optionally substituted with any one selected from the group consisting of halogen and aryl, or -O-$R^{b3}$, in which $R^{b3}$ is $C_1$-$C_3$ alkyl, and $R^{b2}$ is $C_1$-$C_4$ alkyl optionally substituted with hydroxy.

A peptide having a structure represented by the formula (I) can be expected to form a complex with FKBP and FRP. A peptide having a structure represented by the formula (II) can be expected to form a complex with Cy-A and Cn.
In the formula (I), $R^{a1}$ is a hydrogen atom or $C_1$-$C_6$ alkyl.
As used herein, the term "alkyl" is a monovalent group induced by the removal of any one hydrogen atom from aliphatic hydrocarbon, and has a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl includes not only a linear form but a branched form. Specifically, the alkyl is an alkyl having 1 to 20 carbon atoms ($C_1$-$C_{20}$), preferably $C_1$-$C_{10}$ alkyl, more preferably $C_1$-$C_6$ alkyl. Hereinafter, "$C_p$-$C_q$" means that it has p to q carbon atoms. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.
$R^{a1}$ is preferably a hydrogen atom or $C_1$-$C_4$ alkyl, more preferably $C_1$-$C_4$ alkyl, and further preferably methyl or n-butyl.
In the formula (I), $R^{a2}$ is a hydrogen atom or $C_1$-$C_3$ alkyl which is optionally substituted with hydroxy. Preferably, $R^{a2}$ is a hydrogen atom or $C_1$-$C_3$ alkyl substituted with hydroxy, and more preferably a hydrogen atom or hydroxymethyl.
In the formula (I), $R^{a3}$ is a hydrogen atom or methyl. Preferably, $R^{a3}$ is a hydrogen atom.
In the formula (I), X is halogen. Specific examples of X include F, Cl, Br, and I. Preferably, X is Cl.
The peptide having a structure represented by the formula (I) is preferably a peptide having a structure represented by formula (I'),

[Formula 41]

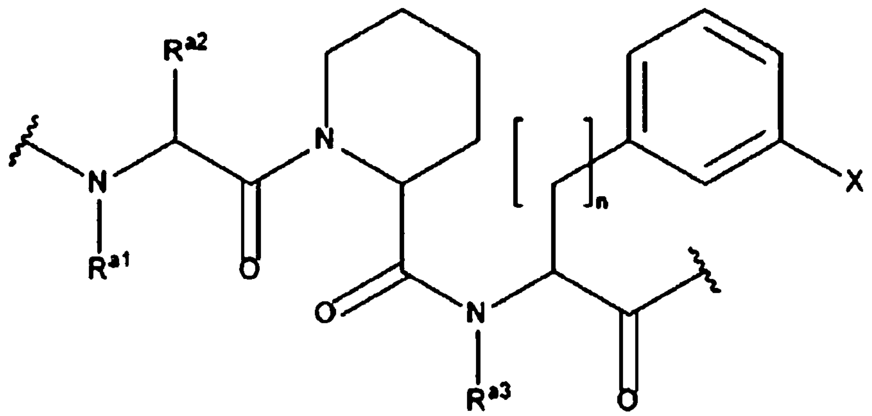

・・・(I')

in the formula (I'), $R^{a1}$, $R^{a2}$, $R^{a3}$, n, and X are the same as $R^{a1}$, $R^{a2}$, $R^{a3}$, n, and X in the formula (I),

more preferably a peptide having a structure represented by formula (I-A1), or a peptide having a structure

represented by formula (I-B1),

[Formula 42]

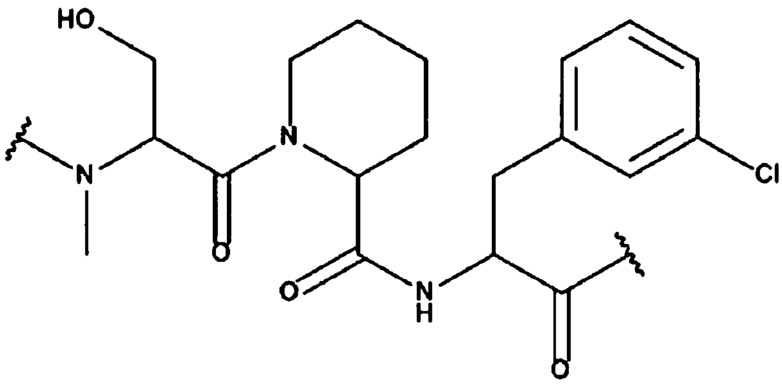

・・・(I－A1)

[Formula 43]

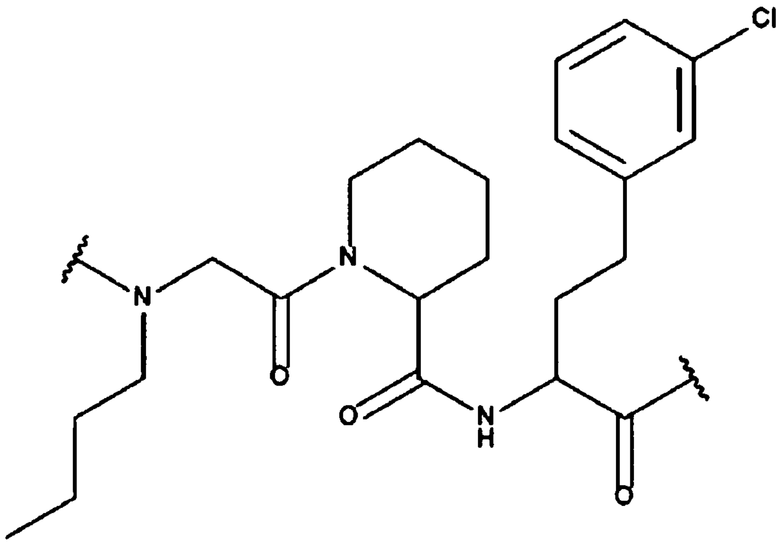

・・・(I－B1)

further preferably a peptide having a structure represented by formula (I-A2), or a peptide having a structure represented by formula (I-B2).

[Formula 44]

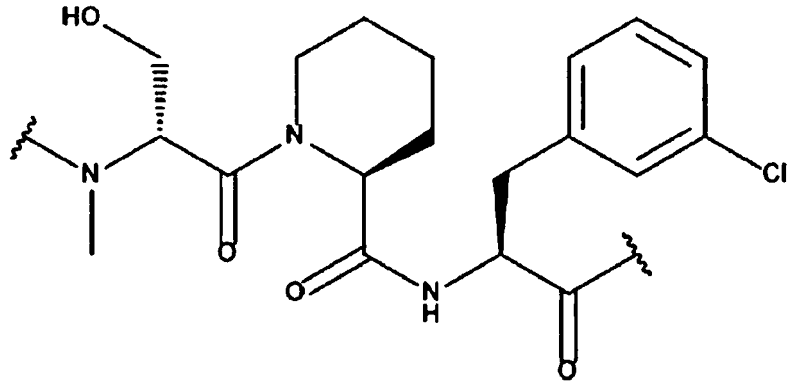

・・・(I－A2)

[Formula 45]

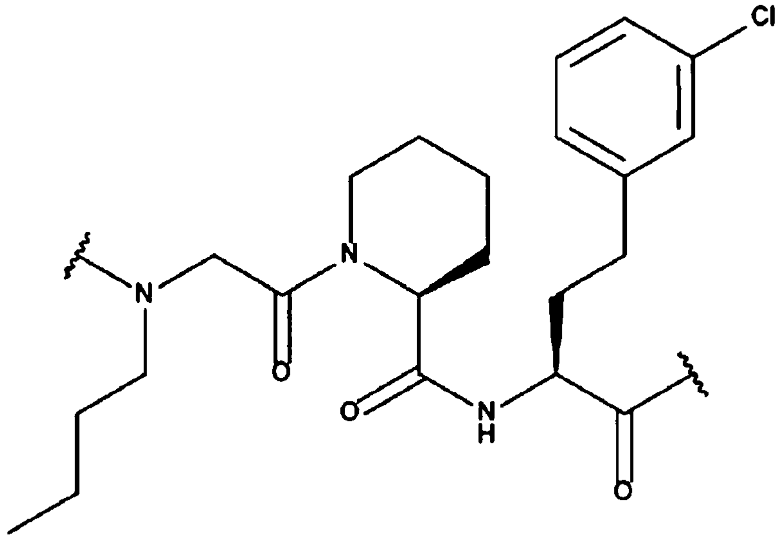

・・・(I－B2)

In the formula (II), $R^{b1}$ is $C_1$-$C_6$ alkyl optionally substituted with any one selected from the group consisting of halogen and aryl, or -O-$R^{b3}$. $R^{b3}$ is $C_1$-$C_3$ alkyl.

The term "aryl" as used herein means a monovalent aromatic hydrocarbon ring and is preferably $C_6$-$C_{10}$ aryl. Examples of the aryl specifically include phenyl and naphthyl (e.g., 1-naphthyl and 2-naphthyl). As used herein, the aryl includes a bicyclic aryl in which the aromatic hydrocarbon ring is condensed with another saturated or unsaturated ring. For example, the aryl includes an aryl of a condensed ring structure in which the aromatic hydrocarbon ring is a benzene ring and the saturated ring is a 5-membered, 6-membered, or 7-membered saturated hydrocarbon ring or a saturated heterocyclic ring. Specific examples include indanyl, 1,2,3,4-tetrahydronaphthyl, and 2,3-dihydrobenzofuran.

Preferably, $R^{b1}$ is $C_1$-$C_5$ alkyl which may be substituted with any one selected from the group consisting of halogen and aryl, or -O-$R^{b3}$, and more preferably any one of the following formulas (II-a), (II-b), or (II-c).

[Formula 46]

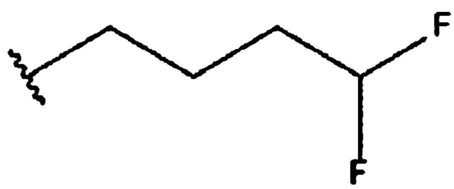

・・・(II－a)

[Formula 47]

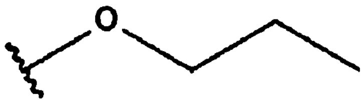

・・・(II-b)

[Formula 48]

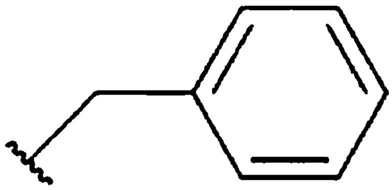

・・・(II-c)

$R^{b3}$ is preferably $C_3$ alkyl, more preferably n-propyl.

In the formula (II), $R^{b2}$ is $C_1$-$C_4$ alkyl optionally substituted with hydroxy. $R^{b2}$ is preferably 1-hydroxyethyl or 1-methylpropyl.

The peptide having a structure represented by the formula (II) is preferably a peptide having a structure represented by formula (II-A1) or a peptide having a structure represented by formula (II-B1), more preferably a peptide having a structure represented by formula (II-A2) or a peptide having a structure represented by formula (II-B2).

[Formula 49]

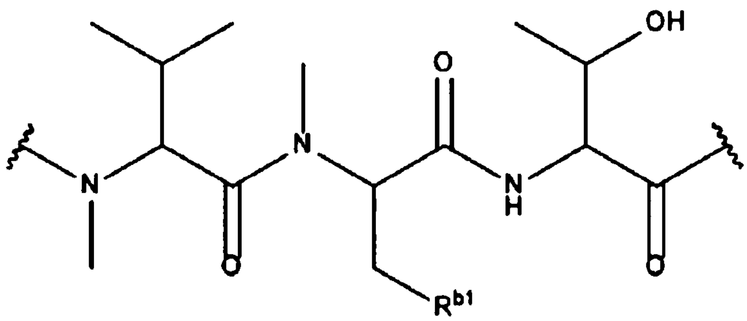

・・・(II-A1)

[Formula 50]

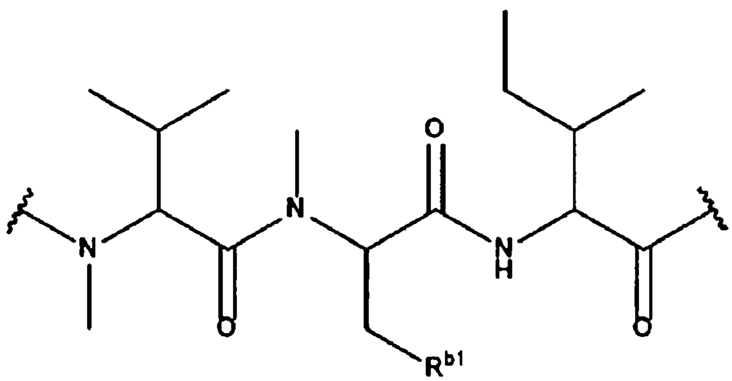

・・・(II-B1)

[Formula 51]

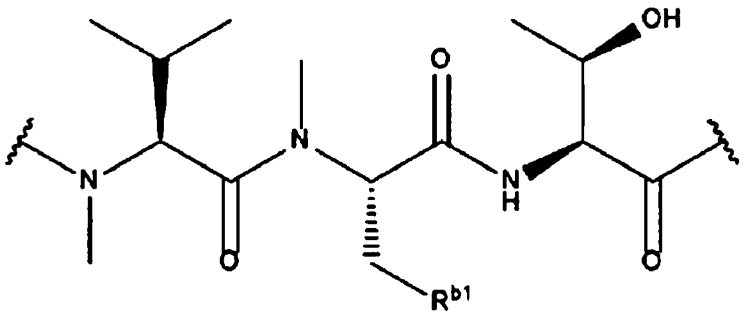

・・・(II-A2)

[Formula 52]

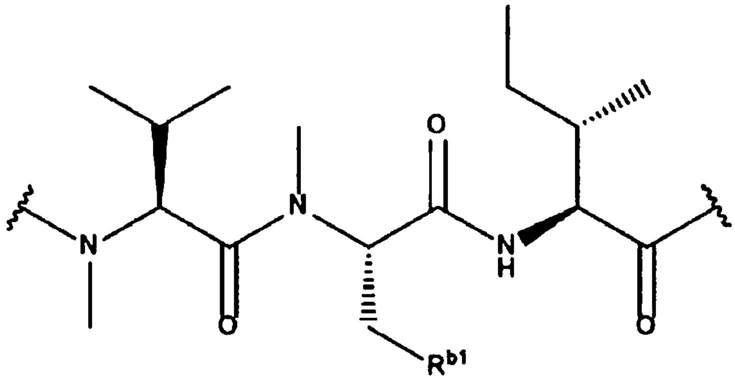

・・・(II-B2)

In the formulas (II-A1), (II-B1), (II-A2), and (II-B2), $R^{b1}$ is the same as $R^{b1}$ in the formula (II).

The peptide having a structure represented by the formula (II-A1) is preferably a peptide having a structure represented by formula (II-Aal), a peptide having a structure represented by formula (II-Ab1), or a peptide having a structure represented by formula (II-Ac1).

[Formula 53]

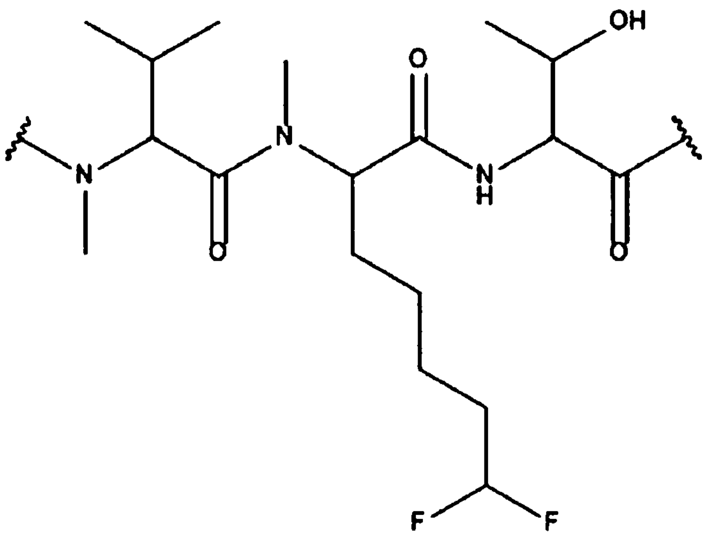

・・・(II-Aa1)

[Formula 54]

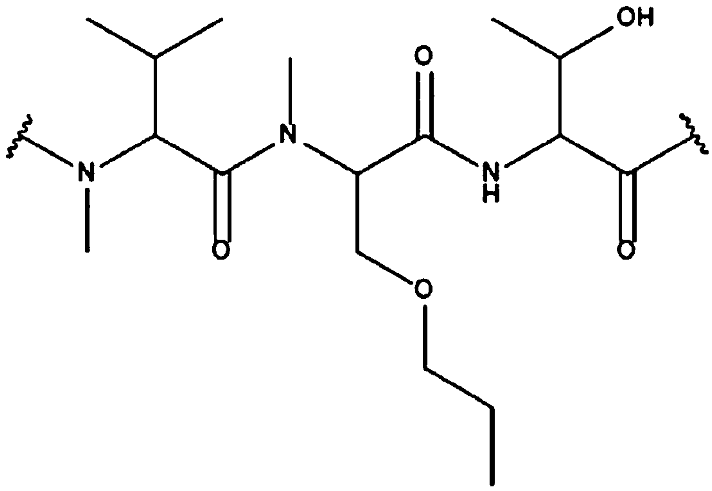

・・・(II-Ab1)

[Formula 55]

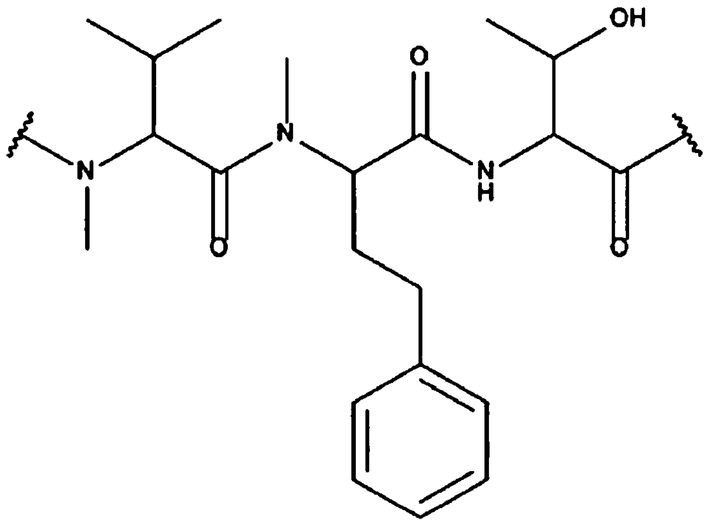

・・・(II-Ac1)

The peptide having a structure represented by the formula (II-B1) is preferably a peptide having a structure represented by formula (II-Ba1), a peptide having a structure represented by formula (II-Bb1), or a peptide having a structure represented by formula (II-Bc1).

[Formula 56]

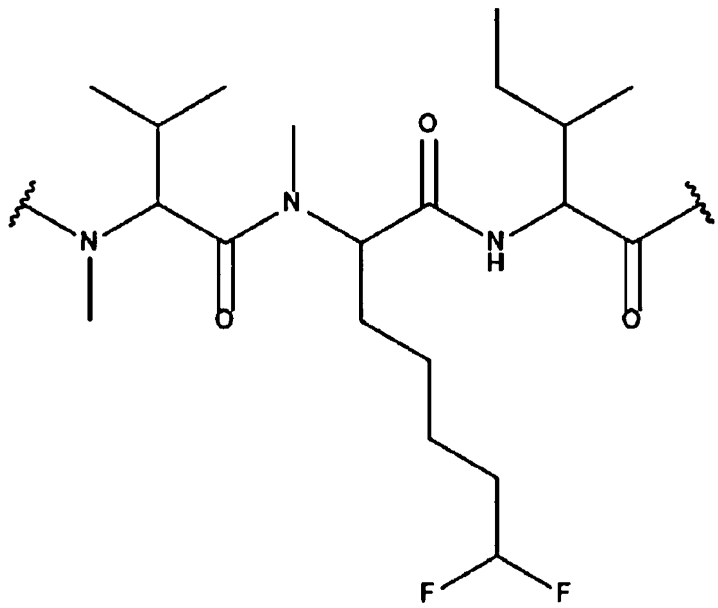

・・・(II-Ba1)

[Formula 57]

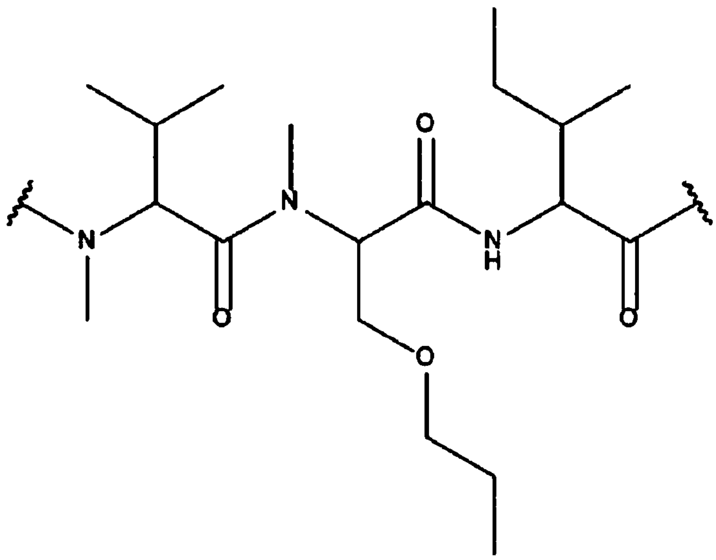

・・・(II-Bb1)

[Formula 58]

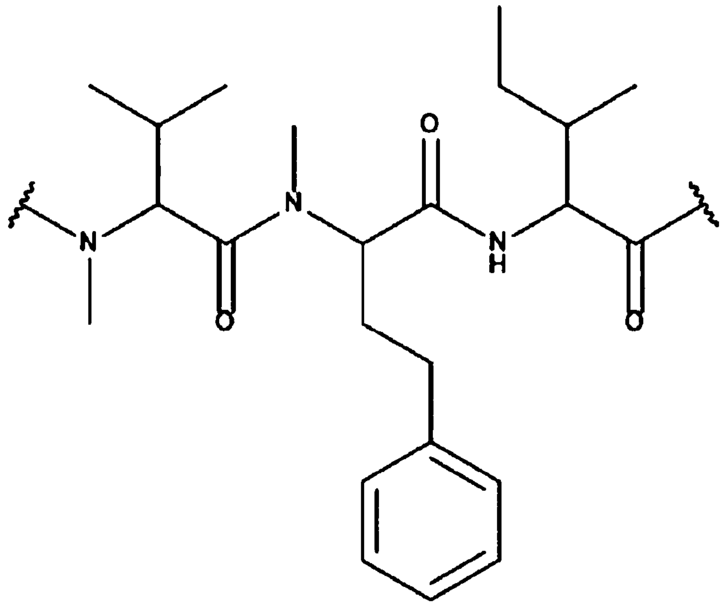

・・・(II-Bc1)

The peptide having a structure represented by the formula (II-A2) is preferably a peptide having a structure represented by formula (II-Aa2), a peptide having a structure represented by formula (II-Ab2), or a peptide having a structure represented by formula (II-Ac2).

[Formula 59]

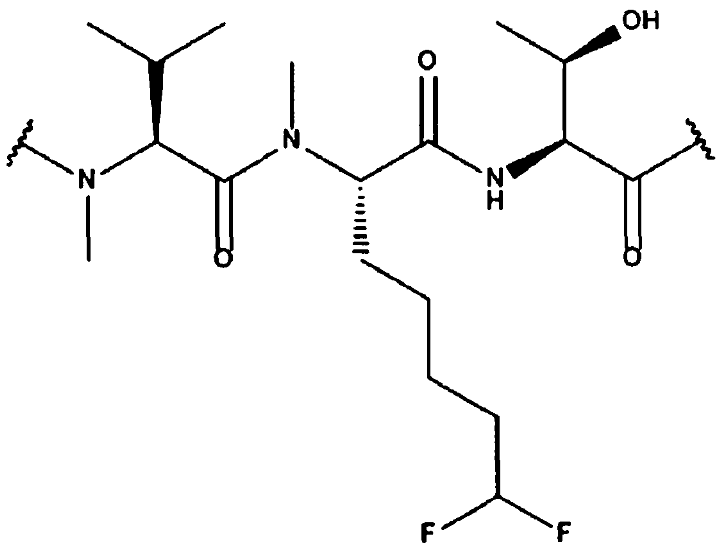

・・・(II-Aa2)

[Formula 60]

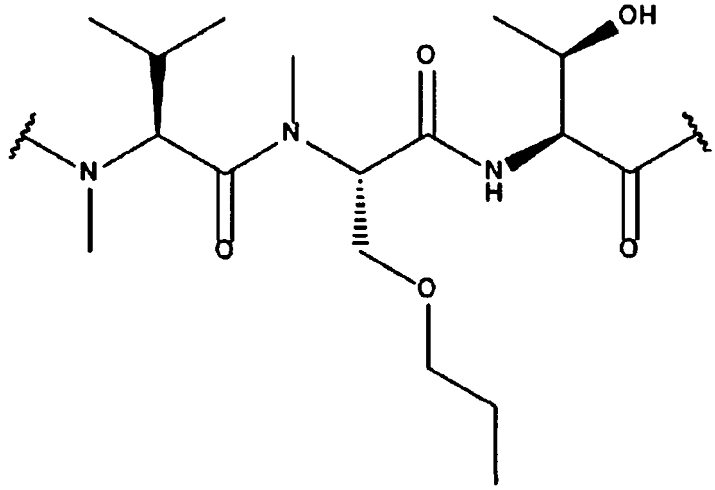

・・・(II-Ab2)

[Formula 61]

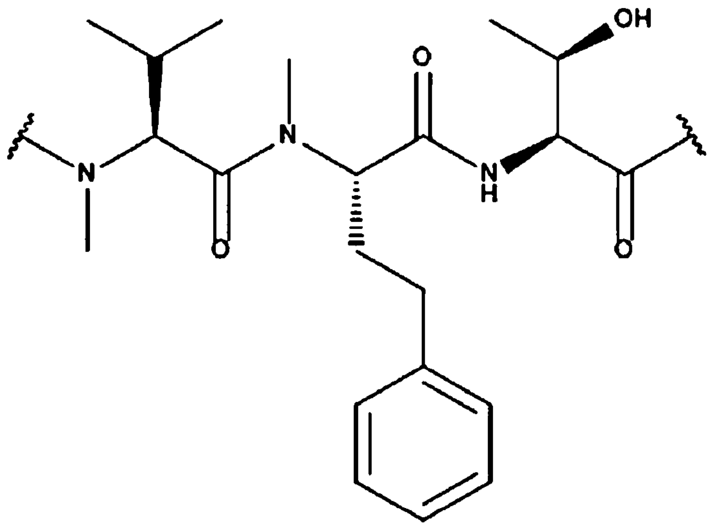

・・・(II-Ac2)

The peptide having a structure represented by the formula (II-B2) is preferably a peptide having a structure represented by formula (II-Ba2), a peptide having a structure represented by formula (II-Bb2), or a peptide having a structure represented by formula (II-Bc2).

[Formula 62]

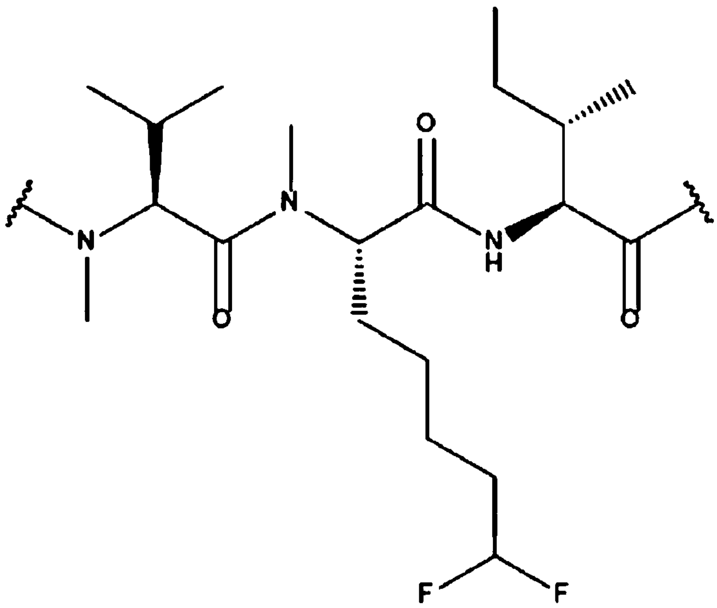

・・・(I I－B a 2)

[Formula 63]

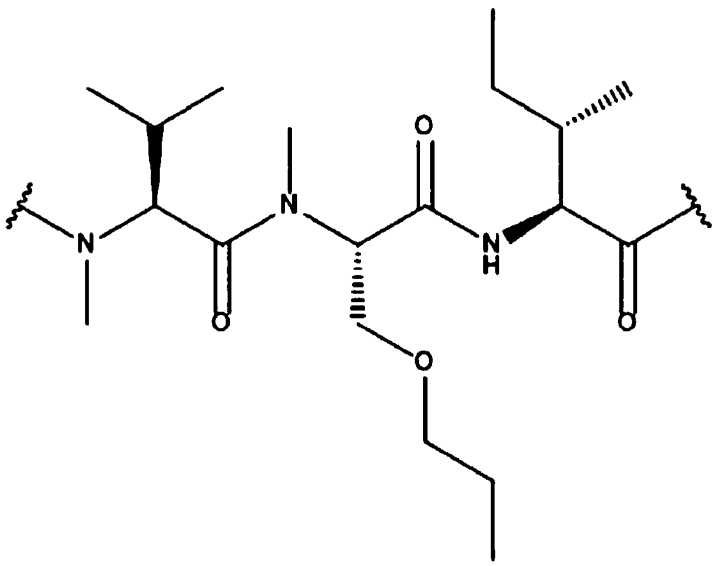

・・・(I I－B b 2)

[Formula 64]

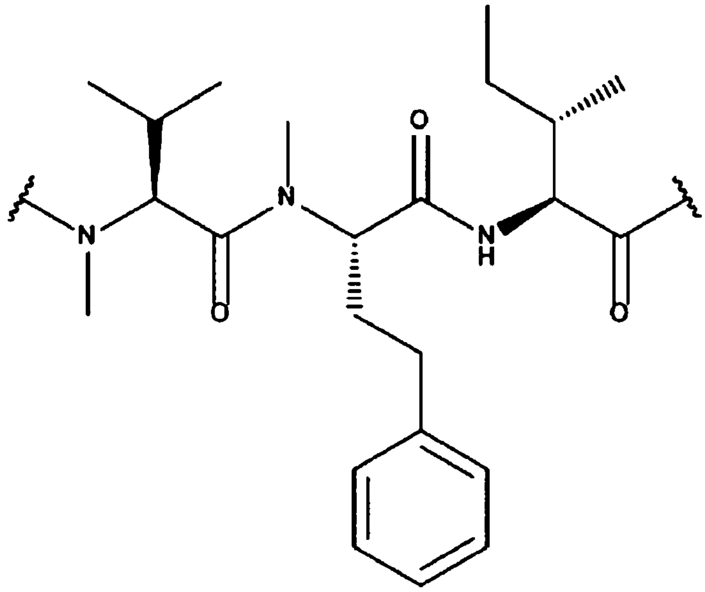

・・・(I I - B c 2)

In each of the formulas described above,

[Formula 65]

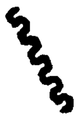

represents a bond.

The peptide according to the present embodiments can be applied without limitation to aspects of the peptides already described as the candidate molecule and the molecule of interest, except for having the specific structure (motif) described above. For example, in one non-limiting aspect, the peptide according to the present embodiment may be a cyclic peptide.

Examples

Hereinafter, preferred specific aspects of the present invention are described by way of Examples, but the present invention is not limited thereto. In Examples, the following abbreviations were used.

DIPEA: N,N-diisopropylethylamine
DMF: N,N-dimethylformamide
DMSO: dimethylsulfoxide
FA: formic acid
HATU: (1-[bis(dimethylamino)methylene]-1H-1,2,3-triazolo[4,5-b]pyridinium 3-oxide hexafluorophosphate

[Experimental Example 1: obtaining of peptide forming complex with plurality of kinds of target proteins by panning using fusion protein]

[Preparation Example 1: preparation of FKBP12, FRB and fusion protein thereof]

<Preparation Example 1-1: preparation of FKBP-FLAG-TEV-Avi-bio>

FKBP12 (FKBP), which was expressed in E. coli and purified, was used as a target protein for the study. A purification tag, a TEV protease cleavage tag, and a biotinylation enzyme recognition tag were added to the C-terminus to prepare FKBP-FLAG-TEV-Avi-bio. "K*" represents biotinylated Lys.

Amino acid sequence of FKBP-FLAG-TEV-Avi-bio (SEQ ID NO: 1)

MGVQVETISPGDGRTFPKRGQTCVVHYTGMLEDGKKFDSSRDRNKPFKFMLGKQEVIR
GWEEGVAQMSVGQRAKLTISPDYAYGATGHPGIIPPHATLVFDVELLKLEGGGSSDYKD
DDDKSSGENLYFQSSGGGLNDIFEAQK*IEWHE

Specifically, an expression vector prepared by cloning genes of FKBP-FLAG-TEV-Avi-bio and biotin ligase (BirA) in pCDFDuet-1 was used to transform E. coli BL21 (DE3) to obtain an expression strain. The expression strain was cultured in LB medium until $OD_{600}$ was about 0.4 to 0.6. IPTG and biotin were then added to be the final concentrations of 1 mM and 100 μM, respectively to cause induction, and after cultured at 37°C for 3 hours, the sample was centrifuged and the cultured bacteria cells were recovered.

The recovered cultured bacteria cells were suspended with a disruption buffer (50 mM Tris-HCl pH 7.0, 200 mM NaCl, 2 mM DTT, 0.1% CHAPS, 0.1 mg/mL lysozyme, 1/1000 (v/v) Benzonase, Protease inhibitor cocktail). The suspension was stirred at room temperature for 20 minutes, then the cells were ultrasonically disrupted. This sample was centrifuged at 15000 × g, 30 minutes, at 4°C, and the supernatant was recovered. The supernatant was then further centrifuged at 15000 × g, 15 minutes, at 4°C, and then filtered through a 0.22 μm filter to obtain a lysate sample.

The lysate sample was purified with Streptactin XT Superflow HC (IBA, #2-4030-010) 5 mL resin and Econo-Pac Chromatography Columns (bio-rad, #7321010). For purification, a binding buffer (50 mM Tris-HCl pH 7.5, 200 mM NaCl, 1 mM TCEP) and an elution buffer (50 mM Tris-HCl pH 7.5, 200 mM NaCl, 1 mM TCEP, 50 mM biotin) were used. A fraction containing the protein of interest was recovered and concentrated with Amicon Ultra-15 10K (MERCK, #UFC901024).

The concentrated sample was purified by gel filtration using HiLoad 26/600 Superdex 75 pg (Cytiva, #28-9893-34). For purification, a running buffer (50 mM Tris-HCl pH 7.5, 150 mM NaCl, 1 mM TCEP) was used. A fraction containing the protein of interest was recovered and concentrated with Amicon Ultra-15 10K (MERCK, #UFC901024), then filtered with a 0.22 μm filter to obtain the protein of interest FKBP-FLAG-TEV-Avi-bio. The sample was stored at -80°C.

<Preparation Example 1-2: preparation of FRB-FLAG-TEV-Avi-bio>

FRB, which was expressed in E. coli and purified, was used as a target protein for the study. A purification tag, a TEV protease cleavage tag, and a biotinylation enzyme recognition tag were added to the C-terminus to prepare FRB-FLAG-TEV-Avi-bio. "K*" represents biotinylated Lys.

Amino acid sequence of FRB-FLAG-TEV-Avi-bio (SEQ ID NO: 2)

MELIRVAILWHEMWHEGLEEASRLYFGERNVKGMFEVLEPLHAMMERGPQTLKETSFN
QAYGRDLMEAQEWCRKYMKSGNVKDLTQAWDLYYHVFRRISKQGGGSSDYKDDDD
KSSGENLYFQSSGGGLNDIFEAQK*IEWHE

Specifically, an expression vector prepared by cloning genes of FRB-FLAG-TEV-Avi-bio and biotin ligase (BirA) in pCDFDuet-1 was used to transform E. coli BL21 (DE3) to obtain an expression strain. The expression strain was cultured in LB medium until $OD_{600}$ was about 0.4 to 0.6. IPTG and biotin were then added to be the final concentrations of 1 mM and 100 μM, respectively to cause induction, and after cultured at 18°C for 18 hours, the sample was centrifuged and the cultured bacteria cells were recovered.

The recovered cultured bacteria cells were suspended with a disruption buffer (50 mM Tris-HCl pH 7.0, 200 mM NaCl, 2 mM DTT, 0.1% CHAPS, 0.1 mg/mL lysozyme, 1/1000 (v/v) Benzonase, Protease inhibitor cocktail). The suspension was stirred at room temperature for 20 minutes, then the cells were ultrasonically disrupted. This sample was centrifuged at 15000 × g, 30 minutes, at 4°C, and the supernatant was recovered. The supernatant was then further filtered through a 0.22 μm filter to obtain a lysate sample.

The lysate sample was purified with Streptactin XT Superflow HC (IBA, #2-4030-010) 5 mL resin and Econo-Pac Chromatography Columns (bio-rad, #7321010). For purification, a binding buffer (50 mM Tris-HCl pH 7.5, 200 mM NaCl, 1 mM TCEP) and an elution buffer (50 mM Tris-HCl pH 7.5, 200 mM NaCl, 1 mM TCEP, 50 mM biotin) were used. A fraction containing the protein of interest was recovered and concentrated with Amicon Ultra-15 10K (MERCK, #UFC901024).

The concentrated sample was purified by gel filtration using HiLoad 26/600 Superdex 75 pg (Cytiva, #28-9893-34). For purification, a running buffer (50 mM Tris-HCl pH 7.5, 300 mM NaCl, 1 mM TCEP) was used. A fraction containing the protein of interest was recovered and concentrated with Amicon Ultra-15 10K (MERCK, #UFC901024), then filtered with a 0.22 μm filter to obtain the protein of interest FRB-FLAG-TEV-Avi-bio. The sample

was stored at - 80°C.

<Preparation Example 1-3: preparation of FKBP-FLAG-Sor-His>

FKBP, which was expressed in E. coli and purified, was used as a target protein for the study. A purification tag and a sortase recognition sequence were added to the C-terminus to prepare FKBP-FLAG-Sor-His.
Amino acid sequence of FKBP-FLAG-Sor-His (SEQ ID NO: 3)

MGVQVETISPGDGRTFPKRGQTCVVHYTGMLEDGKKFDSSRDRNKPFKFMLGKQEVIR
GWEEGVAQMSVGQRAKLTISPDYAYGATGHPGIIPPHATLVFDVELLKLEGGGSSDYKD
DDDKGGSSLPMTGGGGSSHHHHHH

Specifically, an expression vector prepared by cloning a gene sequence of FKBP-FLAG-Sor-His in pET11a was used to transform E. coli BL21 (DE3) to obtain an expression strain. The expression strain was cultured in an LB medium until $OD_{600}$ was about 0.5. IPTG was then added to be the final concentration of 1 mM to cause induction, and after cultured at 18°C for 18 hours, the sample was centrifuged and the cultured bacteria cells were recovered.
The recovered cultured bacteria cells were suspended with a disruption buffer (50 mM Tris-HCl pH 7.0, 200 mM NaCl, 2 mM DTT, 0.1% CHAPS, 0.1 mg/mL lysozyme, 1/1000 (v/v) Benzonase, Protease inhibitor cocktail). The suspension was stirred at room temperature for 20 minutes, then the cells were ultrasonically disrupted. This sample was centrifuged at 15000 × g, 30 minutes, at 4°C, and the supernatant was recovered. The supernatant was then further centrifuged at 15000 × g, 15 minutes, at 4°C, and then filtered through a 0.22 μm filter to obtain a lysate sample.
The lysate sample was purified with His Trap FF (Cytiva, 17531901). For purification, a binding buffer (100 mM Tris-HCl pH 8.0, 150 mM NaCl, 10% Glycerol) and an elution buffer (100 mM Tris-HCl pH 8.0, 150 mM NaCl, 10% Glycerol, 500 mM imidazole) were used. A fraction containing the protein of interest was recovered and concentrated with Amicon Ultra-15 10K (MERCK, #UFC901024).
The concentrated sample was purified by gel filtration using HiLoad 26/600 Superdex 75 pg (Cytiva, #28-9893-34). For purification, a running buffer (50 mM Tris-HCl pH 7.5, 150 mM NaCl, 1 mM TCEP) was used. A fraction containing the protein of interest was recovered and concentrated with Amicon Ultra-15 10K (MERCK, #UFC901024), then filtered with a 0.22 μm filter to obtain the protein of interest FKBP-FLAG-Sor-His. The sample was stored at - 80°C.

<Preparation Example 1-4: preparation of FRB-FLAG-Sor-His>

FRB, which was expressed in E. coli and purified, was used as a target protein for the study. A purification tag and a sortase recognition sequence were added to the C-terminus to prepare FRB-FLAG-Sor-His.
Amino acid sequence of FRB-FLAG-Sor-His (SEQ ID NO: 4)

MELIRVAILWHEMWHEGLEEASRLYFGERNVKGMFEVLEPLHAMMERGPQTLKETSFN
QAYGRDLMEAQEWCRKYMKSGNVKDLTQAWDLYYHVFRRISKQGGGSSDYKDDDD
KGGSSLPMTGGGGSSHHHHHH

Specifically, an expression vector prepared by cloning a gene sequence of FRB-FLAG-Sor-His in pET11a was used to transform E. coli BL21 (DE3) to obtain an expression strain. The expression strain was cultured in LB medium until $OD_{600}$ was 0.6. IPTG was then added to be the final concentration of 0.1 mM to cause induction, and after cultured at 18°C for 18 hours, the sample was centrifuged and the cultured bacteria cells were recovered.
The recovered cultured bacteria cells were suspended with a disruption buffer (50 mM Tris-HCl pH 7.0, 200 mM NaCl, 2 mM DTT, 0.1% CHAPS, 0.1 mg/mL lysozyme, 1/1000 (v/v) Benzonase, Protease inhibitor cocktail). The suspension was stirred at room temperature for 20 minutes, then the cells were ultrasonically disrupted. This sample was centrifuged at 15000 × g, 30 minutes, at 4°C, and the supernatant was recovered. The supernatant was then further centrifuged at 15000 × g, 15 minutes, at 4°C, and then filtered through a 0.22 μm filter to obtain a lysate sample.
The lysate sample was purified with His Trap FF (Cytiva, 17531901). For purification, a binding buffer (100 mM Tris-HCl pH 8.0, 150 mM NaCl, 10% Glycerol) and an elution buffer (100 mM Tris-HCl pH 8.0, 150 mM NaCl, 10% Glycerol, 500 mM imidazole) were used. A fraction containing the protein of interest was recovered and concentrated with Amicon Ultra-15 10K (MERCK, #UFC901024).
The concentrated sample was purified by gel filtration using HiLoad 26/600 Superdex 75 pg (Cytiva,

#28-9893-34). For purification, a running buffer (50 mM Tris-HCl pH 7.5, 300 mM NaCl, 1 mM TCEP) was used. A fraction containing the protein of interest was recovered and concentrated with Amicon Ultra-15 10K (MERCK, #UFC901024), then filtered with a 0.22 μm filter to obtain the protein of interest FRB-FLAG-Sor-His. The sample was stored at -80°C.

<Preparation Example 1-5: preparation of bio-Avi-TEV-FLAG-FKBP-FRB-His>

As a target protein for the study, a fusion protein in which FKBP and FRB were linked with a linker was expressed in E. coli and purified, and used as the target protein used for panning. A purification tag, a TEV protease cleavage tag, and a biotinylation enzyme recognition tag were added to the N-terminus and a purification tag was added to the C-terminus to prepare bio-Avi-TEV-FLAG-FKBP-FRB-His. "K*" represents biotinylated Lys.
Amino acid sequence of bio-Avi-TEV-FLAG-FKBP-FRB-His (SEQ ID NO: 5)

MGLNDIFEAQK*IEWHESSGENLYFQSGGGSSDYKDDDDKSGGVQVETISPGDGRTFPK
RGQTCVVHYTGMLEDGKKFDSSRDRNKPFKFMLGKQEVIRGWEEGVAQMSVGQRAK
LTISPDYAYGATGHPGIIPPHATLVFDVELLKLEGSGSSGLEVLFQGPSSGSSGTELIRVAI
LWHEMWHEGLEEASRLYFGERNVKGMFEVLEPLHAMMERGPQTLKETSFNQAYGRDL
MEAQEWCRKYMKSGNVKDLTQAWDLYYHVFRRISKQGGGSSHHHHHH

Specifically, an expression vector prepared by cloning gene sequences of bio-Avi-TEV-FLAG-FKBP-FRB-His and biotin ligase (BirA) in pCDFDuet-1 was used to transform E. coli BL21 (DE3) to obtain an expression strain. The expression strain was cultured in LB medium until $OD_{600}$ was about 0.4 to 0.6. IPTG at the final concentration of 1 mM and 100 μM biotin were then added to cause induction, and after cultured at 18°C for 18 hours, the sample was centrifuged and the cultured bacteria cells were recovered.
The recovered cultured bacteria cells were suspended with a disruption buffer (50 mM Tris-HCl pH 7.0, 200 mM NaCl, 2 mM DTT, 0.1% CHAPS, 0.1 mg/mL lysozyme, 1/1000 (v/v) Benzonase, Protease inhibitor cocktail). The suspension was stirred at room temperature for 20 minutes, then the cells were ultrasonically disrupted. This sample was centrifuged at 15000 × g, 30 minutes, at 4°C, and the supernatant was recovered. The supernatant was then further centrifuged at 15000 × g, 15 minutes, at 4°C, and then filtered through a 0.22 μm filter to obtain a lysate sample.
The lysate sample was purified with Streptactin XT Superflow HC (IBA, #2-4030-010) 5 mL resin and Econo-Pac Chromatography Columns (bio-rad, #7321010). For purification, a binding buffer (50 mM Tris-HCl pH 7.5, 200 mM NaCl, 1 mM TCEP) and an elution buffer (50 mM Tris-HCl pH 7.5, 200 mM NaCl, 1 mM TCEP, 50 mM biotin) were used. A fraction containing the protein of interest was recovered and concentrated with Amicon Ultra-15 10K (MERCK, #UFC901024).
The concentrated sample was purified by gel filtration using HiLoad 26/600 Superdex 75 pg (Cytiva, #28-9893-34). For purification, a running buffer (50 mM Tris-HCl pH 7.5, 300 mM NaCl, 1 mM TCEP) was used. A fraction containing the protein of interest was recovered and concentrated with Amicon Ultra-15 10K (MERCK, #UFC901024), then filtered with a 0.22 μm filter to obtain the protein of interest bio-Avi-TEV-FLAG-FKBP-FRB-His. The sample was stored at -80°C.

[Preparation Example 2: preparation of cyclophilin A (CyA), calcineurin (Cn) and fusion protein thereof]

<Preparation Example 2-1: preparation of CyA-FLAG-TEV-Avi-bio>

Cyclophilin A, which was expressed in a mammalian cell and purified, was used as a target protein for the study. A purification tag, a TEV protease cleavage tag, and a biotinylation enzyme recognition tag were added to the C-terminus to prepare CyA-FLAG-TEV-Avi-bio. "K*" represents biotinylated Lys.
Amino acid sequence of CyA-FLAG-TEV-Avi-bio (SEQ ID NO: 6)

GPMVNPTVFFDIAVDGEPLGRVSFELFADKVPKTAENFRALSTGEKGFGYKGSCFHRIIP
GFMCQGGDFTRHNGTGGKSIYGEKFEDENFILKHTGPGILSMANAGPNTNGSQFFICTA
KTEWLDGKHVVFGKVKEGMNIVEAMERFGSRNGKTSKKITIADCGQLEGSSDYKDDD
DKSSGENLYFQSSGGGLNDIFEAQK*IEWHE

Specifically, an expression vector prepared by cloning a gene sequence of CyA-FLAG-TEV-Avi-bio, in which a

His tag and a 3C protease cleavage site were attached to the N-terminus, into a protein expression vector for mammalian cells was transfected into Expi293F cells with ExpiFectamine293 Transfection Kit (Thermo Fisher Scientific Inc., #A14525) according to the basic protocol attached to the manufacturing product and the gene was expressed. The cells were cultured at 37°C for 3 days in Expi293 Expression medium (Thermo Fisher Scientific Inc., #A1435102).

The culture solution was centrifuged, and the supernatant was removed to recover the cells. The cells were stored at -80°C until purification. The obtained cells were suspended with a lysis buffer (20 mM Tris-HCl, 150 mM NaCl, pH 7.5) containing a protease inhibitor cocktail, and the cells were disrupted by an ultrasonic homogenizer. To the cell solution after disruption, 1 mM $MgCl_2$ and 1/5000 (v/v) Benzonase were added, and the mixture was incubated on ice for 20 minutes. The resultant solution was centrifuged and the supernatant was recovered. The recovered supernatant was filtered with a 0.45 μm filter to obtain a sample.

The recovered supernatant was purified with an equilibration buffer (20 mM Tris-HCl, 150 mM NaCl, pH 7.5), a wash buffer (20 mM Tris-HCl, 150 mM NaCl, 2 mM ATP, 5 mM $MgCl_2$, pH 7.5), and an elution buffer (20 mM Tris-HCl, 150 mM NaCl, 0.1 mg/mL FLAG peptide, pH 7.5) using ANTI-FLAG (R) M2 Affinity Gel (sigma). Purification was performed according to the basic protocol attached to the manufacturing product. The obtained product was treated with BirA in the presence of Biotin to be biotinylated. The biotinylated protein of interest was also confirmed by a gel shift assay utilizing the interaction between the biotinylated protein and streptavidin.

Tag cleavage was performed with Turbo 3C Protease (Accelagen), and purification was performed with an equilibration buffer (20 mM Tris-HCl, 150 mM NaCl, 1 mM DTT, pH 7.5) and an elution buffer (20 mM Tris-HCl, 150 mM NaCl, 1 mM DTT, 50 mM Biotin, pH 7.5) using Strep-Tactin XT Superflow High Capacity (IBA Lifesciences, GmbH). Purification and tag cleavage was performed according to the basic protocol attached to each of the manufacturing products.

The prepared sample was subjected to size exclusion chromatography using Superdex 200 Increase 10/300 GL (GE) with a running buffer (20 mM Tris-HCl, 150 mM NaCl, 1 mM DTT, pH 7.5) to obtain the protein of interest CyA-FLAG-TEV-Avi-bio. The sample was stored at - 80°C.

<Preparation Example 2-2: preparation of CnB-CnA-FLAG-TEV-Avi-bio>

A fusion protein in which calcineurin subunit B (CnB) and calcineurin subunit A (CnA) were linked with a linker was expressed in a mammalian cell and purified, and used as a target protein for the study. A purification tag, a TEV protease cleavage tag, and a biotinylation enzyme recognition tag were added to the C-terminus to prepare CnB-CnA-FLAG-TEV-Avi-bio. "K*" represents biotinylated Lys.

Amino acid sequence of CnB-CnA-FLAG-TEV-Avi-bio (SEQ ID NO: 7)

GPMGNEASYPLEMCSHFDADEIKRLGKRFKKLDLDNSGSLSVEEFMSLPELQQNPLVQR
VIDIFDTDGNGEVDFKEFIEGVSQFSVKGDKEQKLRFAFRIYDMDKDGYISNGELFQVLK
MMVGNNLKDTQLQQIVDKTIINADKDGDGRISFEEFCAVVGGLDIHKKMVVDVGGGG
GGMSEPKAIDPKLSTTDRVVKAVPFPPSHRLTAKEVFDNDGKPRVDILKAHLMKEGRLE
ESVALRIITEGASILRQEKNLLDIDAPVTVCGDIHGQFFDLMKLFEVGGSPANTRYLFLGD
YVDRGYFSIECVLYLWALKILYPKTLFLLRGNHECRHLTEYFTFKQECKIKYSERVYDA
CMDAFDCLPLAALMNQQFLCVHGGLSPEINTLDDIRKLDRFKEPPAYGPMCDILWSDPL
EDFGNEKTQEHFTHNTVRGCSYFYSYPAVCEFLQHNNLLSILRAHEAQDAGYRMYRKS
QTTGFPSLITIFSAPNYLDVYNNKAAVLKYENNVMNIRQFNCSPHPYWLPNFMDVFTWS
LPFVGEKVTEMLVNVLNICSDDELGSEEDGFDGATAAARKEVIRNKIRAIGKMARVFSV

LREESESVLTLKGLTPTGMLPSGVLSGGKQTLQSATVEAIEADEAIKGFSPQHKITSFEEA
KGLDRINERMPPRRDAMPSDANLNSINKALTSETNGTDSNGSNSSNIQGSSDYKDDDDK
SSGENLYFQSSGGGLNDIFEAQK*IEWHE

Specifically, an expression vector prepared by cloning a gene sequence of CnB-CnA-FLAG-TEV-Avi-bio, in which a His tag and a 3C protease cleavage site were attached to the N-terminus, into a protein expression vector for mammalian cells, and an expression vector prepared by cloning a gene sequence of biotin ligase (BirA) to a protein

expression vector for mammalian cells, were transfected into Expi293F cells with ExpiFectamine293 Transfection Kit (Thermo Fisher Scientific Inc., #A14525) according to the basic protocol attached to the manufacturing product and the genes were expressed. The cells were cultured at 37°C for 3 days in Expi293 Expression medium (Thermo Fisher Scientific Inc., #A1435102) to which biotin was added to be the final concentration of 0.1 mM.

The culture solution was centrifuged, and the supernatant was removed to recover the cells. The cells were stored at -80°C until purification. The obtained cells were suspended with a lysis buffer (20 mM Tris-HCl, 150 mM NaCl, 5 mM $CaCl_2$, pH 7.5) containing a protease inhibitor cocktail, and the cells were disrupted by an ultrasonic homogenizer. To the cell solution after disruption, 1 mM $MgCl_2$ and 12.5 U/mL Benzonase were added, and the mixture was incubated on ice for 20 minutes. The resultant solution was centrifuged and the supernatant was recovered. The recovered supernatant was filtered with a 0.45 μm filter to obtain a sample.

The recovered supernatant was purified with an equilibration buffer (20 mM Tris-HCl, 150 mM NaCl, 5 mM $CaCl_2$, pH 7.5), a wash buffer (20 mM Tris-HCl, 150 mM NaCl, 5 mM $CaCl_2$, 2 mM ATP, 5 mM $MgCl_2$, pH 7.5), and an elution buffer (20 mM Tris-HCl, 150 mM NaCl, 5 mM $CaCl_2$, 0.1 mg/mL FLAG peptide, pH 7.5) using ANTI-FLAG (R) M2 Affinity Gel (sigma). Purification was performed according to the basic protocol attached to the manufacturing product.

The purified sample was subjected to tag cleavage with Turbo 3C Protease (Accelagen). The sample thus obtained was purified with an equilibration buffer (20 mM Tris-HCl, 150 mM NaCl, 5 mM $CaCl_2$, pH 7.5) and an elution buffer (20 mM Tris-HCl, 150 mM NaCl, 5 mM $CaCl_2$, 50 mM biotin, pH 7.5) using Strep-Tactin XT Superflow high capacity (IBA Lifesciences, GmbH). Purification and tag cleavage were performed according to the basic protocol attached to each of the manufacturing products.

The prepared sample was subjected to size exclusion chromatography using HiLoad 16/600 Superdex 200 pg (GE) with a running buffer (20 mM Tris-HCl, 150 mM NaCl, 1 mM DTT, 5 mM $CaCl_2$, pH 7.5). The sample containing the fraction of interest was concentrated with Amicon Ultra-4 10K (Merck) to obtain the protein of interest CnB-CnA-FLAG-TEV-Avi-bio. The sample was stored at -80°C.

<Preparation Example 2-3: preparation of CyA-FLAG-Sor-His>

Cyclophilin A, which was expressed in a mammalian cell and purified, was used as a target protein for the study. A purification tag and a sortase recognition sequence were added to the C-terminus to prepare CyA-FLAG-Sor-His.

Amino acid sequence of CyA-FLAG-Sor-His (SEQ ID NO: 8)

MVNPTVFFDIAVDGEPLGRVSFELFADKVPKTAENFRALSTGEKGFGYKGSCFHRIIPGF
MCQGGDFTRHNGTGGKSIYGEKFEDENFILKHTGPGILSMANAGPNTNGSQFFICTAKTE
WLDGKHVVFGKVKEGMNIVEAMERFGSRNGKTSKKITIADCGQLEGSSDYKDDDDKG
GSSLPMTGGGGSSHHHHHH

Specifically, an expression vector prepared by cloning a gene sequence of CyA-FLAG-Sor-His into a protein expression vector for mammalian cells was transfected into Expi293F cells with ExpiFectamine293 Transfection Kit (Thermo Fisher Scientific Inc., #A14525) according to the basic protocol attached to the manufacturing product and the gene was expressed. The cells were cultured at 37°C for 3 days in Expi293 Expression medium (Thermo Fisher Scientific Inc., #A1435102).

The culture solution was centrifuged, and the supernatant was removed to recover the cells. The cells were stored at -80°C until purification. The obtained cells were suspended with a lysis buffer (50 mM Na-Phosphate, 500 mM NaCl, pH 7.5) containing a protease inhibitor cocktail, and the cells were disrupted by an ultrasonic homogenizer. To the cell solution after disruption, 1 mM $MgCl_2$ and 12.5 U/mL Benzonase were added, and the mixture was incubated on ice for 20 minutes. The resultant solution was centrifuged and the supernatant was recovered. The recovered supernatant was filtered with a 0.45 μm filter to obtain a sample.

The recovered supernatant was purified with a wash buffer (50 mM Na-Phosphate, 500 mM NaCl, 5 mM Imidazole, pH 7.5) and an elution buffer (50 mM Na-Phosphate, 500 mM NaCl, 150 mM Imidazole, pH 7.5) using TALON Metal Affinity Resin (Clontech). The prepared sample was further purified with an equilibration buffer (50 mM Na-Phosphate, 500 mM NaCl, pH 7.5) and an elution buffer (50 mM Na-Phosphate, 500 mM NaCl, 0.1 mg/mL flag peptide, pH 7.5) using DDDDK-tagged Protein PURIFICATION GEL (MBL, #3329R). Purification was performed according to the basic protocol attached to each of the manufacturing products.

The prepared sample was subjected to size exclusion chromatography using Superdex 200 Increase 10/300 GL (GE) with a running buffer (20 mM Tris-HCl, 150 mM NaCl, 1 mM DTT, pH 7.5). The sample containing the fraction of interest was recovered to obtain the protein of interest CyA-FLAG-Sor-His. The sample was stored at -80°C.

<Preparation Example 2-4: preparation of CnB-CnA-FLAG-Sor-His>

A fusion protein in which calcineurin subunits B and A were linked with a linker was expressed in a mammalian cell and purified, and used as a target protein for the study. A purification tag and a sortase recognition sequence were added to the C-terminus to prepare CnB-CnA-FLAG-Sor-His.

Amino acid sequence of CnB-CnA-FLAG-Sor-His (SEQ ID NO: 9)

MGNEASYPLEMCSHFDADEIKRLGKRFKKLDLDNSGSLSVEEFMSLPELQQNPLVQRVI
DIFDTDGNGEVDFKEFIEGVSQFSVKGDKEQKLRFAFRIYDMDKDGYISNGELFQVLKM
MVGNNLKDTQLQQIVDKTIINADKDGDGRISFEEFCAVVGGLDIHKKMVVDVGGGGGG
MSEPKAIDPKLSTTDRVVKAVPFPPSHRLTAKEVFDNDGKPRVDILKAHLMKEGRLEES
VALRIITEGASILRQEKNLLDIDAPVTVCGDIHGQFFDLMKLFEVGGSPANTRYLFLGDY
VDRGYFSIECVLYLWALKILYPKTLFLLRGNHECRHLTEYFTFKQECKIKYSERVYDAC
MDAFDCLPLAALMNQQFLCVHGGLSPEINTLDDIRKLDRFKEPPAYGPMCDILWSDPLE
DFGNEKTQEHFTHNTVRGCSYFYSYPAVCEFLQHNNLLSILRAHEAQDAGYRMYRKSQ
TTGFPSLITIFSAPNYLDVYNNKAAVLKYENNVMNIRQFNCSPHPYWLPNFMDVFTWSL
PFVGEKVTEMLVNVLNICSDDELGSEEDGFDGATAAARKEVIRNKIRAIGKMARVFSVL
REESESVLTLKGLTPTGMLPSGVLSGGKQTLQSATVEAIEADEAIKGFSPQHKITSFEEAK
GLDRINERMPPRRDAMPSDANLNSINKALTSETNGTDSNGSNSSNIQGSSDYKDDDDKG
GSSLPMTGGGGSSHHHHHH

Specifically, an expression vector prepared by cloning a gene sequence of CnB-CnA-FLAG-Sor-His into a protein expression vector for mammalian cells was transfected into Expi293F cells with ExpiFectamine293 Transfection Kit (Thermo Fisher Scientific Inc., #A 14525) according to the basic protocol attached to the manufacturing product and the gene was expressed. The cells were cultured at 37°C for 3 days in Expi293 Expression medium (Thermo Fisher Scientific Inc., #A1435102).

The culture solution was centrifuged, and the supernatant was removed to recover the cells. The cells were stored at -80°C until purification. The obtained cells were suspended with a lysis buffer (20 mM Tris-HCl, 150 mM NaCl, 5 mM $CaCl_2$, pH 7.5) containing a protease inhibitor cocktail, and the cells were disrupted by an ultrasonic homogenizer. To the cell solution after disruption, 1 mM $MgCl_2$ and 12.5 U/mL Benzonase were added, and the mixture was incubated on ice for 20 minutes. The resultant solution was centrifuged and the supernatant was recovered. The recovered supernatant was filtered with a 0.45 μm filter to obtain a sample.

The recovered supernatant was purified with an equilibration buffer (20 mM Tris-HCl, 150 mM NaCl, 5 mM $CaCl_2$, pH 7.5), a wash buffer (20 mM Tris-HCl, 150 mM NaCl, 5 mM $CaCl_2$, 2 mM ATP, 5 mM $MgCl_2$, pH 7.5), and an elution buffer (20 mM Tris-HCl, 150 mM NaCl, 5 mM $CaCl_2$, 0.1 mg/mL FLAG peptide, pH 7.5) using ANTI-FLAG (R) M2 Affinity Gel (sigma). Purification was performed according to the basic protocol attached to the manufacturing product.

The prepared sample was subjected to size exclusion chromatography using HiLoad 16/600 Superdex 200 pg (GE) with a running buffer (20 mM Tris-HCl pH 7.5, 150 mM NaCl, 1 mM DTT, 5 mM $CaCl_2$). The sample containing the fraction of interest was concentrated with Amicon Ultra-4 10K (Merck) to obtain the protein of interest CnB-CnA-FLAG-Sor-His. The sample was stored at -80°C.

<Preparation Example 2-5: preparation of His-CnB-CnA-CyA-FLAG-TEV-Avi-bio>

A fusion protein in which calcineurin subunit B, calcineurin subunit A, and cyclophilin A were linked with a linker was expressed in a mammalian cell and purified, and used as a target protein for the study. A purification tag was added to the N-terminus, and a purification tag, a TEV protease cleavage tag, and a biotinylation enzyme recognition tag were added to the C-terminus to prepare His-CnB-CnA-CyA-FLAG-TEV-Avi-bio. "K*" represents biotinylated Lys.

Amino acid sequence of His-CnB-CnA-CyA-FLAG-TEV-Avi-bio (SEQ ID NO: 10)

MHHHHHHGNEASYPLEMCSHFDADEIKRLGKRFKKLDLDNSGSLSVEEFMSLPELQQN
PLVQRVIDIFDTDGNGEVDFKEFIEGVSQFSVKGDKEQKLRFAFRIYDMDKDGYISNGEL
FQVLKMMVGNNLKDTQLQQIVDKTIINADKDGDGRISFEEFCAVVGGLDIHKKMVVDV
GGGGGGSEPKAIDPKLSTTDRVVKAVPFPPSHRLTAKEVFDNDGKPRVDILKAHLMKEG
RLEESVALRIITEGASILRQEKNLLDIDAPVTVCGDIHGQFFDLMKLFEVGGSPANTRYLF
LGDYVDRGYFSIECVLYLWALKILYPKTLFLLRGNHECRHLTEYFTFKQECKIKYSERVY
DACMDAFDCLPLAALMNQQFLCVHGGLSPEINTLDDIRKLDRFKEPPAYGPMCDILWSD
PLEDFGNEKTQEHFTHNTVRGCSYFYSYPAVCEFLQHNNLLSILRAHEAQDAGYRMYR
KSQTTGFPSLITIFSAPNYLDVYNNKAAVLKYENNVMNIRQFNCSPHPYWLPNFMDVFT
WSLPFVGEKVTEMLVNVLNICSDDELGSEEDGFDGATAARAIGKMARVFSVLREESESV
LTLKGLTPTGMLPSGVLSGGKQTLQSATVEAIEADEAIKGFSPQHKITSEEAKGLDRINER
MPPRRDAMPSDANLNSINKALTSETNGTDSNGSNSSNIDGGGGSGLEVLFQGPGGGSGG
GGSGGGSTVNPTVFFDIAVDGEPLGRVSFELFADKVPKTAENFRALSTGEKGFGYKGSC
FHRIIPGFMCQGGDFTRHNGTGGKSIYGEKFEDENFILKHTGPGILSMANAGPNTNGSQF
FICTAKTEWLDGKHVVFGKVKEGMNIVEAMERFGSRNGKTSKKITIADCGQLEDYKDD
DDKGSGGENLYFQSSGGGLNDIFEAQK*IEWHE

Specifically, an expression vector prepared by cloning a gene sequence of His-CnB-CnA-FLAG-TEV-Avi-bio into a protein expression vector for mammalian cells, and an expression vector prepared by cloning a gene sequence of biotin ligase (BirA) to a protein expression vector for mammalian cells, were transfected into Expi293F cells with ExpiFectamine293 Transfection Kit (Thermo Fisher Scientific Inc., #A14525) according to the basic protocol attached to the manufacturing product and the genes were expressed. The cells were cultured at 37°C for 3 days in Expi293 Expression medium (Thermo Fisher Scientific Inc., #A1435102) to which biotin was added to be the final concentration of 0.1 mM.

The culture solution was centrifuged, and the supernatant was removed to recover the cells. The cells were stored at -80°C until purification. The obtained cells were suspended with a lysis buffer (20 mM Bis-Tris, 100 mM NaCl, 1 mM DTT, pH 6.0) containing a protease inhibitor cocktail, and the cells were disrupted by an ultrasonic homogenizer. To the cell solution after disruption, 1 mM $MgCl_2$ and 12.5 U/mL Benzonase were added, and the mixture was incubated on ice for 20 minutes. The resultant solution was centrifuged and the supernatant was recovered. The recovered supernatant was filtered with a 0.22 μm filter to obtain a sample.

The recovered supernatant was purified with an equilibration buffer (20 mM Bis-Tris, 100 mM NaCl, 1 mM DTT, pH 6.0), a wash buffer (20 mM Bis-Tris, 100 mM NaCl, 1 mM DTT, 2 mM ATP, 5 mM $MgCl_2$, pH 6.0), and an elution buffer (20 mM Bis-Tris, 100 mM NaCl, 1 mM DTT, 50 mM Biotin, pH 6.0) using Strep-Tactin XT 4 Flow (IBA Lifesciences, GmbH). Purification was performed according to the basic protocol attached to the manufacturing product.

The resulting sample was purified with an equilibration buffer (20 mM Bis-Tris, 1 mM DTT, pH 6.0) and an elution buffer (20 mM Bis-Tris, 1M NaCl, 1 mM DTT, pH 6.0) using HiTrapQ HP (Cytiva).

The resulting sample was purified by subjecting to size exclusion chromatography using HiLoad 16/600 Superdex 200 pg (GE) with a running buffer (20 mM Bis-Tris, 50 mM NaCl, 1 mM DTT, 0.1% CHAPS, pH 7.5).

The resulting sample was further purified with an equilibration buffer (20 mM Bis-Tris, 100 mM NaCl, 1 mM DTT, pH 6.0) and an elution buffer (20 mM Bis-Tris, 100 mM NaCl, 1 mM DTT, 50 mM Biotin, pH 6.0) using Strep-Tactin XT 4 Flow high capacity resin (IBA Lifesciences, GmbH). Purification was performed according to the basic protocol attached to the manufacturing product.

Finally, the resulting sample was purified by subjecting to size exclusion chromatography with a running buffer (20 mM Tris-HCl pH 7.5, 50 mM NaCl, 1 mM DTT, 0.1% CHAPS) using HiLoad 16/600 Superdex 200 pg (GE). The sample containing the fraction of interest was concentrated with Amicon Ultra-4 30K (Merck) to obtain the protein of interest His-CnB-CnA-CyA-FLAG-TEV-Avi-bio. The sample was stored at -80°C.

[Preparation Example 3: preparation of calcineurin-FKBP fusion protein]

<Preparation Example 3-1: preparation of GL-FKBP-FLAG-TEV-Avi-bio>

As a target protein for the study, GL-FKBP-FLAG-TEV-Avi-bio, an FKBP added with a OaAEP1 recognition sequence at the N-terminus and a purification tag, a TEV protease cleavage tag and a biotinylation enzyme recognition tag at the C-terminus, was expressed in E. coli and purified. "K*" represents biotinylated Lys.

Amino acid sequence of GL-FKBP-FLAG-TEV-Avi-bio (SEQ ID NO: 46)

GLGGGGSGGGGSGVQVETISPGDGRTFPKRGQTCVVHYTGMLEDGKKFDSSRDRNKPF
KFMLGKQEVIRGWEEGVAQMSVGQRAKLTISPDYAYGATGHPGIIPPHATLVFDVELLK
LEGGGSSDYKDDDDKSSGENLYFQSSGGGLNDIFEAQK*IEWHE

Specifically, an expression vector prepared by cloning genes of His-WELQ-GL-FKBP-FLAG-TEV-Avi-bio and biotin ligase (BirA) in pCDFDuet-1 was used to transform E. coli BL21 (DE3) to obtain an expression strain. The expression strain was cultured in LB medium until $OD_{600}$ reached 0.5. IPTG and biotin were then added to be the final concentrations of 1 mM and 100 μM, respectively to cause induction, and after cultured at 18°C for 18 hours, the sample was centrifuged at 8000 × g, 15 minutes, at 4°C and the cultured bacteria cells were recovered.

The recovered cultured bacteria cells were suspended with a disruption buffer (50 mM Tris-HCl pH 7.0, 200 mM NaCl, 20 mM Imidazole, 2 mM DTT, 0.1% CHAPS, 0.1 mg/mL lysozyme, 1/1000 (v/v) Benzonase, Protease inhibitor cocktail). The suspension was stirred at room temperature for 20 minutes, then the cells were ultrasonically disrupted. This sample was centrifuged at 15000 × g, 30 minutes, at 4°C, and the supernatant was recovered. The supernatant was then further centrifuged at 15000 × g, 10 minutes, at 4°C, and then filtered through a 0.22 μm filter to obtain a lysate sample.

The lysate sample was purified using Strep Tactin XT 4flow HC (IBA, #2-5030-010) 5 mL resin. For purification, an equilibration buffer (50 mM Tris-HCl pH 7.5, 100 mM NaCl, 1 mM TCEP) and an elution buffer (50 mM Tris-HCl pH 7.5, 100 mM NaCl, 1 mM TCEP, 50 mM D-biotin) were used. A fraction containing the protein of interest was recovered and concentrated with Amicon Ultra-15 10K (MERCK, #UFC901024).

A portion of the concentrated sample was dialyzed with a dialysis buffer (50 mM Tris-HCl pH 7.5, 300 mM NaCl, 20 mM imidazole, 1 mM TCEP). Then, WELQut protease (Thermo Fisher Scientific, #EO0861), which recognizes and cleaves a WELQut protease recognition sequence (WEQL), was added at the rate of 20 U/100 μg sample and the sample was processed at 4°C overnight to remove the purification tag at the N-terminus of the raw material protein.

The sample treated with the WELQut protease processing was purified using His Trap FF 5 mL (GE, #17525501). For purification, Buffer A (50 mM Tris-HCl pH 7.5, 300 mM NaCl, 20 mM imidazole, 1 mM TCEP) and Buffer B (50 mM Tris-HCl pH 7.5, 300 mM NaCl, 400 mM imidazole, 1 mM TCEP) were used. Fractions containing the protein of interest were recovered.

The recovered sample was purified using Strep Tactin XT 4flow HC (IBA, #2-5030-010) 3 mL resin. For purification, an equilibration buffer (50 mM Tris-HCl pH 7.5, 100 mM NaCl, 1 mM TCEP) and an elution buffer (50 mM Tris-HCl pH 7.5, 100 mM NaCl, 1 mM TCEP, 50 mM D-biotin) were used. Fractions containing the protein of interest were recovered.

The recovered sample was purified by gel filtration using HiLoad 16/600 Superdex 75 pg (Cytiva, #28-9893-33). For purification, an equilibration buffer (50 mM Tris-HCl pH 7.5, 300 mM NaCl, 1 mM TCEP) was used. A fraction containing the protein of interest was recovered and concentrated with Amicon Ultra-15 10K (MERCK, #UFC901024) to obtain the protein of interest GL-FKBP-FLAG-TEV-Avi-bio. The sample was stored at -80°C.

<Preparation Example 3-2: preparation of OaAEP1_C247A>

For the preparation of the fusion protein in vitro, OaAEP1, one of the aspartic endopeptidases (derived from plants) that catalyze the linking reaction of the peptide, was expressed in E. coli and purified according to the method of Non Patent Literature (J. Am. Chem. Soc. 2017, 139, 5351-5358.). It should be noted that the OaAEP1 synthesized here is not a wild-type, but a more active C247A variant (OaAEP1_C247A).

Amino acid sequence of OaAEP1_C247A (SEQ ID NO: 47)

ARDGDYLHLPSEVSRFFRPQETNDDHGEDSVGTRWAVLIAGSKGYANYRHQAGVCHA
YQILKRGGLKDENIVVFMYDDIAYNESNPRPGVIINSPHGSDVYAGVPKDYTGEEVNAK
NFLAAILGNKSAITGGSGKVVDSGPNDHIFIYYTDHGAAGVIGMPSKPYLYADELNDAL
KKKHASGTYKSLVFYLEACESGSMFEGILPEDLNIYALTSTNTTESSWAYYCPAQENPPP
PEYNVCLGDLFSVAWLEDSDVQNSWYETLNQQYHHVDKRISHASHATQYGNLKLGEE
GLFVYMGSNPANDNYTSLDGNALTPSSIVVNQRDAD

Specifically, an expression vector prepared by cloning a gene sequence of OaAEPl_C247A in pET-28b (+) was used to transform E. coli BL21 (DE3) to obtain an expression strain and prepare a glycerol stock.

The glycerol stock was suspended in a LBK medium, and then shaking-cultured at 37°C for 18 hours at 220 rpm until $OD_{600}$ was about 6.7 to 6.8. Then, the culture solution was added to a new LBK medium so that $OD_{600}$ was 0.1, and then shaking-cultured at 37°C for 140 minutes at 120 rpm. $OD_{600}$ was confirmed to reach greater than 0.5, and then the culture temperature was lowered to 16°C and the culture was cooled for 30 minutes. 1 mM IPTG was added for induction, and induction-culture was performed at 16°C for 20 hours at 120 rpm, and then the sample was centrifuged at 4000 × g, 15 minutes, at 4°C and the cultured bacteria cells were recovered. The recovered bacteria cells were resuspended in D-PBS (-) (NACALAI TESQUE, INC., #14249-95) and centrifuged again at 4000 × g, 30 minutes, at 4°C, and then the supernatant was removed to obtain a bacteria cell sample.

The recovered bacteria cell sample was suspended with a disruption buffer (50 mM Tris-HCl pH 8.0, 150 mM NaCl, 0.05% (w/v) CHAPS, 10% (v/v) Glycerol, 1/10000 (25 U/mL) Benzonase, 1 tablet/50 mL Protease inhibitor cocktail), and then ultrasonically disrupted on ice. This sample was centrifuged at 100000 × g, 30 minutes, at 4°C, and then the precipitates were removed and the supernatant was taken as a lysate sample.

The lysate sample was purified with cOmplete His-tag Purification Column (5 mL) (Roche, 06781535001). For purification, an equilibration buffer (50 mM Tris-HCl pH 8.0, 150 mM NaCl, 0.05% (w/v) CHAPS, 10% (v/v) Glycerol) and an elution buffer (50 mM Tris-HCl pH 8.0, 150 mM NaCl, 0.05% (w/v) CHAPS, 10% (v/v) Glycerol, 1M Imidazole) were used. The sample was analyzed by SDS-PAGE and fractions containing proteins of interest were recovered.

The recovered sample was dialyzed overnight using buffer A (20 mM Bis-Tris propane pH 7.0). However, the external liquid was replaced once in 2 hours. The sample was then purified with HiTrap Q HP 5 mL (Cytiva, #1-7115-401). For purification, buffer A and buffer B (20 mM Bis-Tris propane pH 7.0, 2M NaCl) were used. For the preparation of buffer A and buffer B, 1M Bis-Tris propane pH 6.5 (Molecular Dimensions, MD2-005-PH) was used. A fraction containing the protein of interest was recovered by SDS-PAGE analysis and concentrated with Amicon Ultra-4 10K (MERCK, #UFC901024).

The concentrated sample was purified by gel filtration using Superdex 200 Increase 10/300 GL (Cytiva, #28-9909-44). As a running buffer, D-PBS(-) (NACALAI TESQUE, INC., #14249-95) was used. A fraction containing the protein of interest was recovered by SDS-PAGE analysis to obtain the protein of interest OaAEP1_C247A.

The gel filtration purified sample was diluted to 1.5 μM with an activation buffer (50 mM sodium acetate pH 4.0, 50 mM NaCl, 1 mM EDTA, 0.5 mM TCEP), and then the diluted sample was dialyzed with the same buffer for 3 hours. It was then dialyzed overnight with a dialysis buffer (50 mM HEPES pH 7.5, 150 mM NaCl). However, the external liquid was replaced once in 2 hours. The protein of interest OaAEP1_C247A was thus activated. The sample was stored at -80°C.

<Preparation Example 3-3: preparation of Sortase A pentamutant (eSrtA)>

For the preparation of the fusion protein in vitro, eSrtA, one of the ligases that catalyze the linking reaction of the peptide, was expressed in E. coli and purified according to the method of Non Patent Literature (Proc. Natl. Acad. Sci. USA. 2011 Jul 12; 108(28): 11399-11404). The Sortase A synthesized here is not the wild type, but the more active P94R/D160N/D165A/K190E/K196T pentamutant (Sortase A pentamutant (eSrtA)).

Amino acid sequence of Sortase A (SEQ ID NO: 48)

MQAKPQIPKDKSKVAGYIEIPDADIKEPVYPGPATREQLNRGVSFAEENESLDDQNISIAG
HTFIDRPNYQFTNLKAAKKGSMVYFKVGNETRKYKMTSIRNVKPTAVEVLDEQKGKD
KQLTLITCDDYNEETGVWETRKIFVATEVKLEHHHHHH

<Preparation Example 3-4: preparation of CnB-CnA-FLAG-FKBP-FLAG-TEV-Avi-bio>

As a target protein for the study, CnB-CnA-FLAG-FKBP-FLAG-TEV-Avi-bio, a fusion protein linking calcineurin and FKBP by a click reaction, was used. Specifically, a linker with a transcyclooctene (TCO) functional group was added to GL-FKBP-FLAG-TEV-Avi-bio containing an OaAEP1 recognition sequence at the N-terminus prepared in Preparation Example 3-1 using OaAEP1 prepared in Preparation Example 3-2. At the same time, to CnB-CnA-FLAG-Sor-His containing a sortase recognition sequence at the C-terminus prepared in Preparation Example 2-4, a linker with a methyltetrazine (MeTz) functional group was added at the C-terminus, respectively, using sortase prepared in Preparation Example 3-3. Subsequently, FKBP to which TCO was added and CnB-CnA to which MeTz was added were linked by a click reaction to prepare a fusion protein in which the C-terminus of calcineurin and the N-terminus of FKBP were linked with a peptide linker. "K*" represents biotinylated Lys, "K(*1)" represents Lys in which a methyltetrazine functional group was added to the side chain, and "(*2)G" represents Gly in which a transcyclooctene functional group was added to the N-terminus. "K(*1)" and "(*2)G" are linked by a click reaction as in "(*3)" or "(*4)". The structure formulas of "K(*1)", "(*2)G", "(*3)" and "(*4)" are shown in Table 3.

[Table 3]

| Name | Structure formula |
|---|---|
| **K(*1)** | 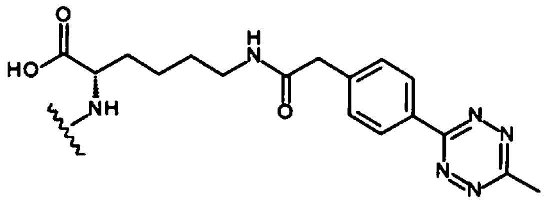 |
| **(*2) G** | 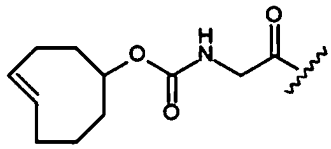 |
| **(*3)** | 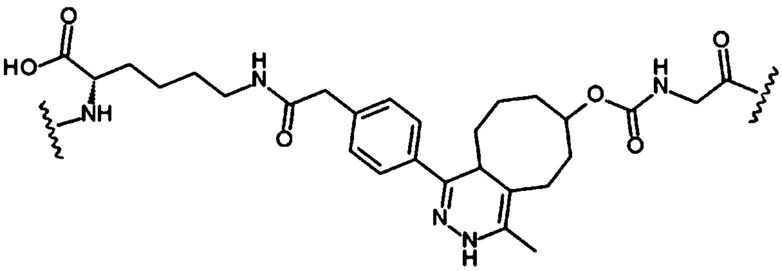 |
| **(*4)** | 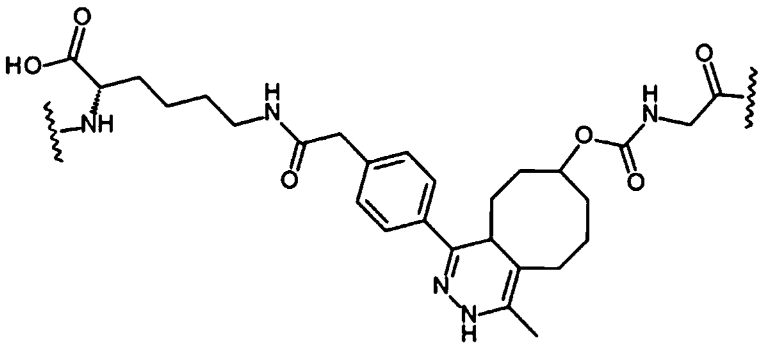 |

Amino acid sequence of CnB-CnA-FLAG-FKBP-FLAG-TEV-Avi-bio (SEQ ID NO: 49 and SEQ ID NO: 50)

MGNEASYPLEMCSHFDADEIKRLGKRFKKLDLDNSGSLSVEEFMSLPELQQNPLVQRVI DIFDTDGNGEVDFKEFIEGVSQFSVKGDKEQKLRFAFRIYDMDKDGYISNGELFQVLKM MVGNNLKDTQLQQIVDKTIINADKDGDGRISFEEFCAVVGGLDIHKKMVVDVGGGGGG MSEPKAIDPKLSTTDRVVKAVPFPPSHRLTAKEVFDNDGKPRVDILKAHLMKEGRLEES VALRIITEGASILRQEKNLLDIDAPVTVCGDIHGQFFDLMKLFEVGGSPANTRYLFLGDY VDRGYFSIECVLYLWALKILYPKTLFLLRGNHECRHLTEYFTFKQECKIKYSERVYDAC MDAFDCLPLAALMNQQFLCVHGGLSPEINTLDDIRKLDRFKEPPAYGPMCDILWSDPLE DFGNEKTQEHFTHNTVRGCSYFYSYPAVCEFLQHNNLLSILRAHEAQDAGYRMYRKSQ TTGFPSLITIFSAPNYLDVYNNKAAVLKYENNVMNIRQFNCSPHPYWLPNFMDVFTWSL

PFVGEKVTEMLVNVLNICSDDELGSEEDGFDGATAAARKEVIRNKIRAIGKMARVFSVL REESESVLTLKGLTPTGMLPSGVLSGGKQTLQSATVEAIEADEAIKGFSPQHKITSFEEAK GLDRINERMPPRRDAMPSDANLNSINKALTSETNGTDSNGSNSSNIQGSSDYKDDDDKG GSSLPMTGGGGSGGGGSK(*1)(*2)GGSGGGGSGGGGSNGLGGGGSGGGGSGVQVETISP GDGRTFPKRGQTCVVHYTGMLEDGKKFDSSRDRNKPFKFMLGKQEVIRGWEEGVAQM SVGQRAKLTISPDYAYGATGHPGIIPPHATLVFDVELLKLEGGGSSDYKDDDDKSSGENL YFQSSGGGLNDIFEAQK*IEWHE

<Synthesis Example 3-1: synthesis of TCO-GGS-(G4S)2-NGLH>

[Formula 66]

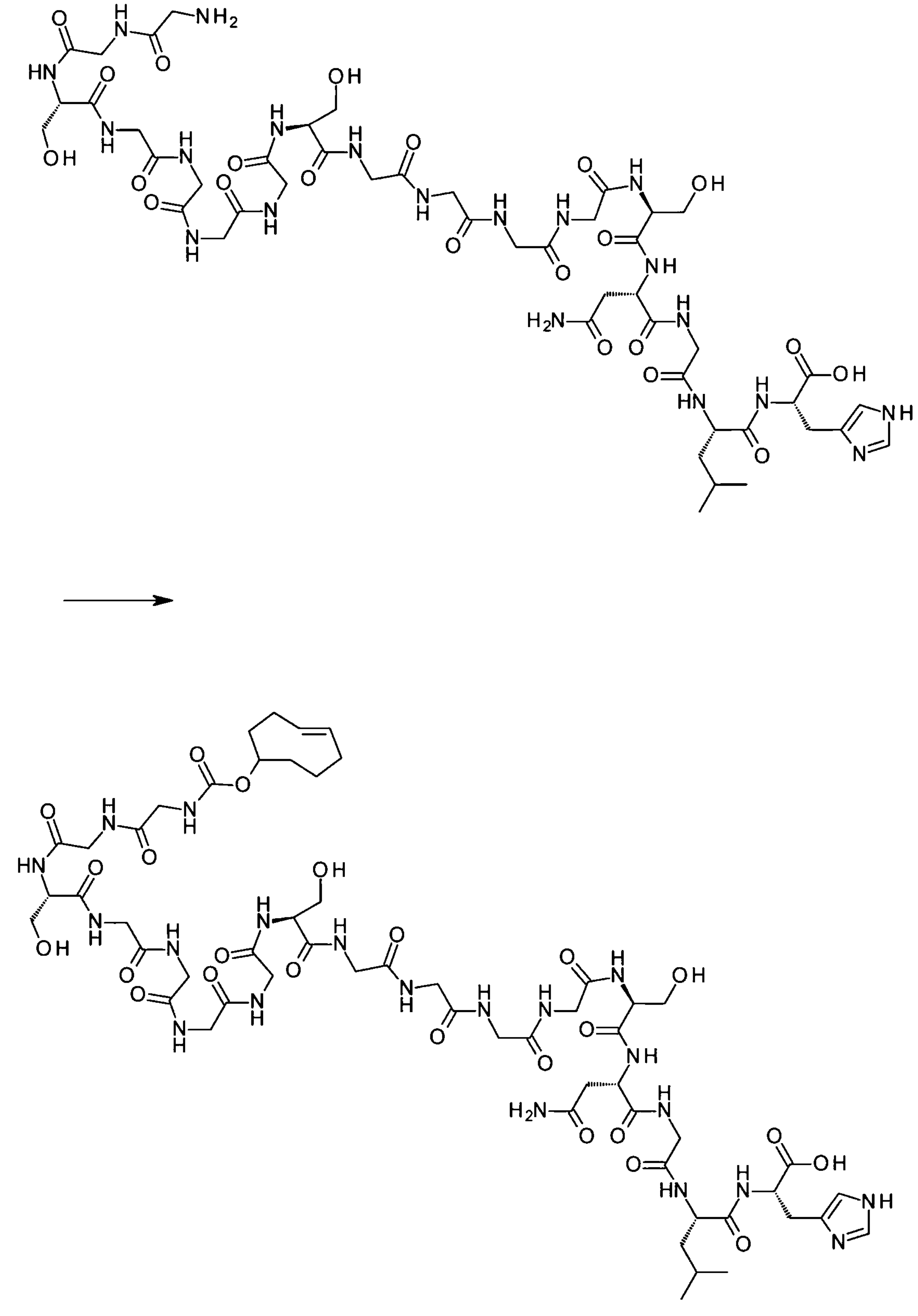

Glycylglycyl-L-serylglycylglycylglycyl-L-serylglycylglycylglycyl-L-seryl-L-aspartylglycyl-L-leucyl-L-histidine (40.6 mg, 0.032 mmol) and (E)-cyclooct-4-en-1-yl(2,5-dioxopyrrolidin-1-yl)carbonate were dissolved in dimethyl sulfoxide (1.3 mL) and N-ethyl diisopropylamine (28.3 μL, 0.16 mmol) was added dropwise. The reaction solution was stirred at 25°C for 60 minutes. The reaction solution was purified by chromatography to obtain (((E)-cyclooct-4-en-1-yl)oxy)carbonyl)glycylglycyl-L-serylglycylglycylglycyl-L-serylglycylglycylglycyl-L-seryl-L-aspartylglycyl-L-leucyl-L-histidine (40

mg, 88% yield).

Retention time: 0.42 min
m/z: 1422 [M-H]-

(Preparation Example 3-4-1: addition of peptide linker to FKBP by OaAEP1 reaction)

Specifically, 46 μM GL-FKBP-FLAG-TEV-Avi-bio, 1 mM TCO-GGS-(G4S)2-NGLH, 400 μM $NiCl_2$, and 0.6 μM OaAEP1 were mixed on ice and incubated at 4°C overnight.

Samples before and after the OaAEP1 reaction were analyzed by SDS-PAGE to confirm that the reaction proceeded properly by checking the gel shift corresponding to the theoretical molecular weight of the protein of interest.

The crude sample after the reaction was purified using Strep-Tactin XT Sepharose chromatography resin (Cytiva, 29401324), a wash buffer (50 mM HEPES pH 7.5, 150 mM NaCl, 0.5 mM TCEP), and an elution buffer (50 mM HEPES pH 7.5, 150 mM NaCl, 0.5 mM TCEP, 50 mM biotin). The purification was performed according to the basic protocol attached to the manufacturing product. The sample after purification was recovered and concentrated with Amicon Ultra-0.5 10K (MERCK, #UFC501096) to obtain TCO-GGS-(G4S)2-FKBP-FLAG-TEV-Avi-bio in which TCO was added to the N-terminus of FKBP.

(Preparation Example 3-4-2: addition of peptide linker to CnB-CnA by sortase reaction)

In a reaction buffer (50 mM Tris-HCl, pH 7.5, 150 mM NaCl, 10 mM $CaCl_2$), 40 μM CnB-CnA-FLAG-Sor-His, 1 mM (G4S)2-K(MeTz) (synthesized at SCRUM Inc.) and 1 μM eSrtA were mixed on ice and incubated at 4°C overnight.

Samples before and after the sortase reaction were analyzed by SDS-PAGE to confirm that the reaction proceeded properly by checking the gel shift corresponding to the theoretical molecular weight of the protein of interest.

The structure formula of (G4S)2-K(MeTz) used in the above reaction is as follows.

[Formula 67]

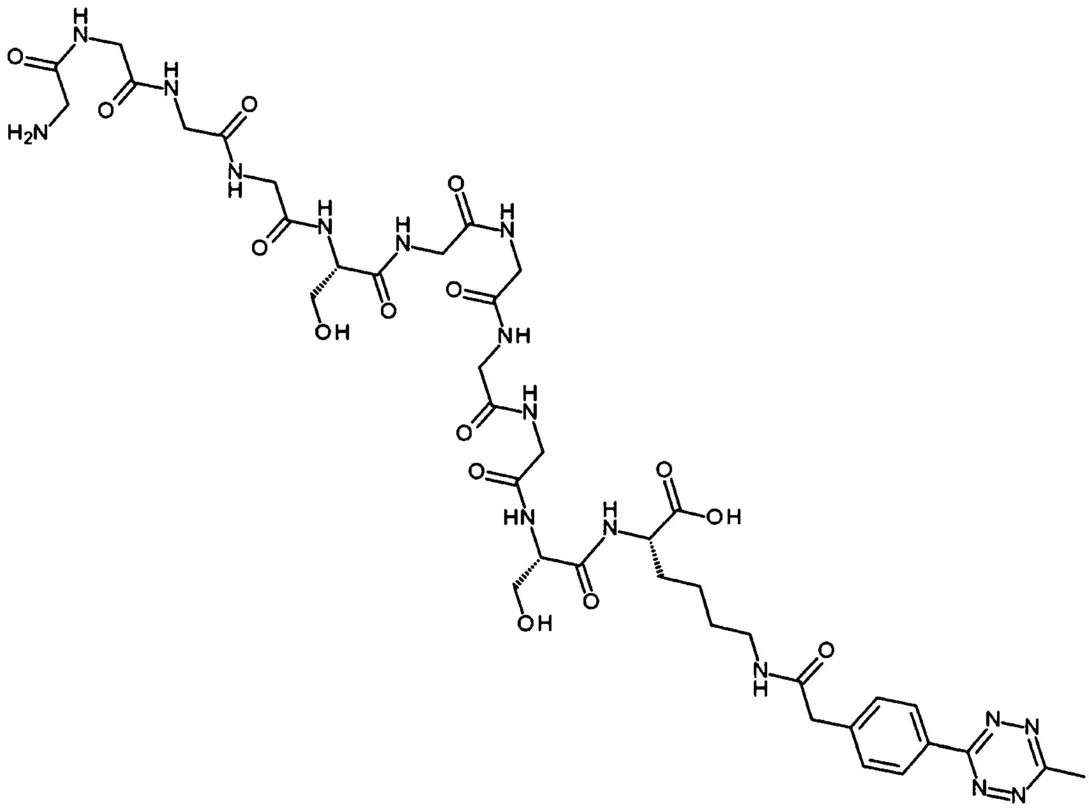

The crude sample after the reaction was mixed with Dynabeads His-Tag Isolation and Pulldown (Invitrogen, 10103D), and the mixture was mixed by inversion at 4°C for 10 minutes, and then the supernatant was recovered to obtain a crude purified sample. The crude purified sample was then purified using ANTI-FLAG(R) M2 Affinity Gel (Sigma,

#A2220), a wash buffer (50 mM HEPES pH 7.5, 150 mM NaCl, 0.5 mM TCEP), and an elution buffer (50 mM HEPES pH 7.5, 150 mM NaCl, 0.5 mM TCEP, 500 μg/mL FLAG peptide). The purification was performed according to the basic protocol attached to the manufacturing product. The sample after purification was recovered and concentrated with Amicon Ultra-0.5 10K (MERCK, #UFC501096) to obtain CnB-CnA-FLAG-(G4S)2-K(MeTz) in which MeTz was added at the C-terminus of calcineurin.

(Preparation Example 3-4-3: synthesis of calcineurin-FKBP fusion protein by click reaction)

TCO-GGS-(G4S)2-FKBP-FLAG-TEV-Avi-bio in which TCO was added to the N-terminus of FKBP and CnB-CnA-FLAG-(G4S)2-K(MeTz) in which MeTz was added to the C-terminus of CnB-CnA were mixed and incubated at 4°C for 2 hours. Samples before and after mixing were analyzed by SDS-PAGE to confirm that the reaction proceeded properly by checking the gel shift corresponding to the theoretical molecular weight of the protein of interest.

The concentrated sample was purified by gel filtration using Superdex 75 Increase 10/300 GL(Cytiva, #29148721). For purification, a running buffer (50 mM HEPES pH 7.5, 300 mM NaCl, 1 mM DTT) was used. A fraction containing the protein of interest was recovered to obtain the protein of interest CnB-CnA-FLAG-FKBP-FLAG-TEV-Avi-bio in which the C-terminus of calcineurin and the N-terminus of FKBP were linked with a peptide linker.

[Preparation Example 4: preparation of mRNA display library]

<Preparation Example 4-1: synthesis of acylated tRNA>

Acylated tRNA used for translation was prepared according to the method described in WO2018/143145 and WO2018/225864. 19 kinds of amino acids consisting of Pro(4-pip-4-F2), Asp(SMe), Hph(3-Cl), cisPro(4-pip-4-F2), MeHnl(7-F2), Ser(3-F-5-Me-Pyr), MeSer(nPr), MeAla(3-Pyr), Phe(3-Cl), Pic(2), MeGly, Nle, MeSer(tBuOH), Ser(Nt-Bu-Aca), Ser(iPen), nBuGly, MeHph, Ser(Ph-2-Cl), and D-MeSer were used to prepare an elongator aminoacylated tRNA mixture. The final concentration of each acylated tRNA in the translation solution was 10 μM to 20 μM. F-Pnaz-protected pCpA amino acids were subjected to a phenol extraction process or thereafter without deprotection. The initiator aminoacylated tRNA, which is the same compound as compound AAtR-3 (Acbz-MeCys(StBu)-tRNAtMetCAU) described in WO2017/150732, was used by adding to the translation solution to be the final concentration of 25 μM.

<Preparation Example 4-2: preparation of randomized double-stranded DNA Library>

The DNA library was constructed using the method described in WO2013/100132. In the prepared DNA library, 26 triplets of TTT, TTG, CTT, CTG, ATT, ATG, GTT, GTG, TCT, TCG, CCG, ACT, GCT, TAC, CAT, CAG, AAC, GAT, GAG, TGC, TGG, CGT, CGG, AGT, AGG, GGT appeared randomly 8 or 9 times (SEQ ID NO: 11, SEQ ID NO: 12).

Nucleotide sequence of DNA library (SEQ ID NO: 11)

GTAATACGACTCACTATAGGGTTAACTTTAATAAGGAGATATAAATATG(Random_triplet)$_9$TAGCCGACCGGCACCGGCACCGGCAAAAAAA

Nucleotide sequence of DNA library (SEQ ID NO: 12)

GTAATACGACTCACTATAGGGTTAACTTTAATAAGGAGATATAAATATG(Random_triplet)$_8$TAGCCGACCGGCACCGGCACCGGCAAAAAAA

<Preparation Example 4-3: translation of mRNA display library>

For the translation system, PURE system, a reconstituted cell-free protein synthesis system from a prokaryotic organism, was used.

In the panning of Examples 1-1 and 1-2 described below, translation was performed by adding the following components to the translation reaction mixture to achieve their respective final concentrations: 1 mM GTP, 1 mM ATP, 20 mM phosphocreatine, 50 mM HEPES-KOH pH 7.6, 100 mM potassium acetate, 4 mM magnesium acetate, 2 mM spermidine, 1 mM dithiothreitol, 1 mg/ml E. coli MRE 600 (RNase-negative) derived tRNA (F. Hoffmann-La Roche, Ltd.) (some tRNAs were removed by the method described in Non Patent Literature: Yokogawa T, et al. 2010), 3 μM in vitro transcriptional E. coli tRNA Ala1B, 4 μg/ml creatine kinase, 6.72 U/mL myokinase, 2 units/ml inorganic pyrophosphatase, 1.1 μg/ml nucleoside diphosphate kinase, 0.4 Unit/μL RNasein (R) Plus Ribonuclease Inhibitor (Promega Corporation),

0.26 μM EF-G, 2.7 μM IF1, 0.4 μM IF2, 1.5 μM IF3, 40 μM EF-Tu, 59 μM EF-Ts, 1 μM EF-P-Lys, 1.2 μM modified ribosome (WO2021/117848), 1.37 μM AlaRS, 1 μM GlyRS, 0.04 μM IleRS, 0.11 μM LysRS, 0.16 μM ProRS, 0.09 μM ThrRS, 0.5 μM variant PheRS (WO2016/148044), 1 μM variant SerRS (WO2016/148044), 1 μM variant ValRS (WO2016/148044), 250 μM glycine, 10 μM isoleucine, 250 μM lysine, 250 μM proline, 250 μM threonine, 2.5 mM N-methylalanine, 5 mM N-methylvaline, 5 mM N-methylserine, 5 mM N-methylphenylalanine, the elongator aminoacylated tRNA mixture prepared in Preparation Example 4-1, 25 μM Initiator aminoacylated tRNA (WO2017/150732) prepared in Preparation Example 4-1, 0.5 μM Penicillin G Amidase (PGA), and 1 μM mRNA-puromycin linked construct prepared according to WO2013/100132 from the double-stranded DNA library prepared in Preparation Example 4-2, followed by incubation at 37°C for 1 hour to perform translation. It is noted that axPM-27 described in Non Patent Literature (J. Am. Chem. Soc., 2023, 145, pp. 24035-24051) was used as a puromycin linker.

In the panning of Example 1-3, the translation was performed in the same manner as in the panning of Examples 1-1 and 1-2 except that the concentration of magnesium acetate was 6 mM, and the concentration of EF-Ts was 60 μM.

[Example 1: panning]

Panning was performed 4 rounds for the fusion protein and a single protein for comparison, using the translated mRNA display library and in accordance with WO2013/100132. However, the cyclization reaction and desulfurization reaction of the peptide were performed in accordance with WO2017/150732.

<Example 1-1: panning using FKBP, FRB and their fusion proteins as targets>

In Round 1, to a buffer containing 1 × TBS, 2 mg/mL BSA (Invitrogen) and 2 mM DTT, the display library was added to be 0.4 μM, and bio-Avi-TEV-FLAG-FKBP-FRB-His, FKBP-FLAG-TEV-Avi-bio or FRB-FLAG-TEV-Avi-bio as a target protein was added to be 0.4 μM, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.03-0.04 mM, and the mixture was mixed by inversion at 4°C for another 10 minutes.

Streptavidin-coated magnetic beads with immobilized target proteins were collected with a magnet and the supernatant was removed. The collected beads were washed several times with a wash buffer (1 × TBS, 2 mM DTT, 0.1% Tween 20 (Sigma Aldrich), 500 mM arginine), then treated with a TEV protease that recognizes and cleaves the TEV protease recognition sequence to elute the target protein and the peptide-nucleic acid conjugate that binds to the target protein.

To the streptavidin-coated magnetic beads after washing, 1 × TEV buffer, 1 mM DTT, and 0.1 U/μL AcTEV protease (Thermo Fisher Scientific Inc., #12575015) were added as a specific TEV eluent to react with. After the reaction, the supernatant was recovered, PCR was performed with library recognition primers (Fw-Primer and Rv-Primer), and the resultant was subjected to the next round.

In addition, qPCR was performed using a portion of the TEV elution product, and the recovery rate of the library of Round 1 was evaluated. For the qPCR primer, the aforementioned primers (Fw-Primer and Rv-Primer) were used. For the qPCR reaction solution, 1 × Ex Taq buffer, 0.2 mM dNTP, 0.5 μM Fw-Primer, 0.5 μM Rv-Primer, 50000-fold diluted SYBR Green I (LONZA, #50513), and 0.025 U/μL Ex Taq polymerase (Takara Bio Inc.) were mixed. The mixture was heated at 95°C for 2 minutes, and then subjected to 40 cycles of 95°C for 10 seconds, 57°C for 20 seconds, and 72°C for 30 seconds.

Nucleotide sequence of Fw-Primer (SEQ ID NO: 13) GTAATACGACTCACTATAGGGTTAACTTTAATAAGGAG
Nucleotide sequence of Rv-Primer (SEQ ID NO: 14) TTTTTTTGCCGGTGCCGGTGCCGGTCGGCTA

To draw a calibration curve to estimate the recovery rate of the library, a sample prepared by reverse transcription of the mRNA-puromycin linked construct prepared in Preparation Example 4-3 of Experimental Example 1 was diluted to be 1E + 8/μL, 1E + 6/μL, and 1E + 4/μL, and subjected to qPCR under the same conditions.

In Rounds 2 to 4, to a buffer containing 1 × TBS, 2 mg/mL BSA (Invitrogen) and 2 mM DTT, the display library was added to be 0.2-0.3 μM, and bio-Avi-TEV-FLAG-FKBP-FRB-His, FKBP-FLAG-TEV-Avi-bio or FRB-FLAG-TEV-Avi-bio as a target protein was added to be 0.4 μM, then the mixture was incubated at 4°C for 1 hour. Then, the resultant was mixed with streptavidin-coated magnetic beads or neutravidin-coated magnetic beads, and the mixture was mixed by inversion at 4°C for 10 minutes to immobilize the target protein. Biotin was then added to be 0.04-0.05 mM, and the mixture was mixed by inversion at 4°C for another 10 minutes. Thereafter, the same process as in Round 1 was performed.

At the second round or thereafter, the input library was divided into two, and selection was made with one in the presence of the target protein and the other in the absence of the target protein, and the nucleic acid recovery rates of both were evaluated from the results of qPCR. The recovery rate (%) of each round of panning was calculated by the expression below. The results are shown in the table below.

Recovery rate of library (%) = amount (number of molecules) of output nucleic acids of library/(amount (number of molecules) of input nucleic acids of library) × 100

[Table 4]

| Change in recovery rate when using bio-Avi-TEV-FLAG-FKBP-FRB-His (SEQ ID NO: 5) as target protein | | | | | | |
|---|---|---|---|---|---|---|
| Round | Amount of input nucleic acids of library (molecules) | In presence of target | | In absence of target | | Ratio of recovery rate in presence to absence of target |
| | | Molecules of output nucleic acids (molecules) | Recovery rate (%) | Molecules of output nucleic acids (molecules) | Recovery rate (%) | |
| **1** | **2.93E+13** | **1.37E+08** | **4.67E-04** | **-** | **-** | **-** |
| **2** | **8.26E+11** | **3.88E+06** | **4.69E-04** | **8.70E+05** | **1.05E-04** | **4.5** |
| **3** | **3.03E+11** | **1.51E+08** | **4.98E-02** | **2.47E+04** | **8.16E-06** | **6108.4** |
| **4** | **1.63E+11** | **4.67E+08** | **2.86E-01** | **1.13E+05** | **6.88E-05** | **4153.8** |

[Table 5]

| Change in recovery rate when using FKBP-FLAG-TEV-Avi-bio (SEQ ID NO: 1) as target protein | | | | | | |
|---|---|---|---|---|---|---|
| Round | Amount of input nucleic acids of library (molecules) | In presence of target | | In absence of target | | Ratio of recovery rate in presence to absence of target |
| | | Molecules of output nucleic acids (molecules) | Recovery rate (%) | Molecules of output nucleic acids (molecules) | Recovery rate (%) | |
| **1** | **3.09E+13** | **3.40E+06** | **1.10E-05** | **-** | **-** | **-** |
| **2** | **7.83E+11** | **7.87E+06** | **1.00E-03** | **4.78E+05** | **6.10E-05** | **16.5** |
| **3** | **5.82E+11** | **2.17E+09** | **3.72E-01** | **1.32E+04** | **2.27E-06** | **163794.4** |
| **4** | **4.54E+11** | **2.71E+09** | **5.96E-01** | **1.09E+05** | **2.39E-05** | **24908.1** |
| **5** | **5.52E+11** | **3.31E+08** | **5.98E-02** | **8.01E+03** | **1.45E-06** | **41268.3** |

[Table 6]

| Change in recovery rate when using FRB-FLAG-TEV-Avi-bio (SEQ ID NO: 2) as target protein | | | | | | |
|---|---|---|---|---|---|---|
| Round | Amount of input nucleic acids of library (molecules) | In presence of target | | In absence of target | | Ratio of recovery rate in presence to absence of target |
| | | Molecules of output nucleic acids (molecules) | Recovery rate (%) | Molecules of output nucleic acids (molecules) | Recovery rate (%) | |
| **1** | **2.49E+13** | **1.01E+07** | **4.04E-05** | **-** | **-** | **-** |
| **2** | **6.62E+11** | **3.11E+05** | **4.69E-05** | **4.53E+05** | **6.84E-05** | **0.7** |
| **3** | **4.34E+11** | **9.19E+06** | **2.11E-03** | **3.36E+04** | **7.73E-06** | **273.8** |
| **4** | **3.63E+11** | **4.60E+08** | **1.27E-01** | **7.07E+05** | **1.95E-04** | **651.1** |

As a result, in the second half of the rounds, as shown in Tables 4 to 6, there was a difference in the recovery rate between the selections in the presence of the target protein and in the absence of the target protein. It was thus suggested that molecules that were recovered only in the presence of the target protein were present in the library of each round, and they were concentrated each time of the repeated rounds. In particular, this phenomenon can be seen in panning against

not only fusion proteins but also single proteins. It is thus strongly suggested that not only a binder that can form a ternary complex with FKBP and FRB, but also a binder that binds to FKBP or FRB alone are present in the library.

DNA pools of an each round obtained by panning was subjected to nucleotide sequence analysis with next-generation sequencers by methods known to those skilled in the art.

<Example 1-2: panning using calcineurin, cyclophilin A and fusion protein thereof as targets>

In Round 1, to a buffer containing 1 × TBS, 2 mg/mL BSA (Invitrogen), 2 mM DTT, and 5 mM $CaCl_2$, the display library was added to be 0.4 μM, and His-CnB-CnA-CyA-FLAG-TEV-Avi-bio, CyA-FLAG-TEV-Avi-bio, or CnB-CnA-FLAG-TEV-Avi-bio as a target protein was added to be 0.4 μM. In the case where the target protein was His-CnB-CnA-CyA-FLAG-TEV-Avi-bio or CnB-CnA-FLAG-TEV-Avi-bio, Calmodulin (FUJIFILM Wako Pure Chemical Corporation) was further added to be 2 μM, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.03-0.04 mM, and the mixture was mixed by inversion at 4°C for another 10 minutes.

Streptavidin-coated magnetic beads with immobilized target proteins were collected with a magnet and the supernatant was removed. The collected beads were washed several times with a wash buffer (1 × TBS, 2 mM DTT, 0.1% Tween 20 (Sigma Aldrich), 500 mM arginine), then treated with a TEV protease that recognizes and cleaves the TEV protease recognition sequence to elute the target protein and the peptide-nucleic acid conjugate that binds to the target protein.

To the streptavidin-coated magnetic beads after washing, 1 × TEV buffer, 1 mM DTT, and 0.1 U/μL AcTEV protease (Thermo Fisher Scientific Inc., #12575015) were added as a specific TEV eluent to react with. After the reaction, the supernatant was recovered, PCR was performed with library recognition primers (Fw-Primer and Rv-Primer), and the resultant was subjected to the next round.

In addition, qPCR was performed using a portion of the TEV elution product, and the recovery rate of the library of Round 1 was evaluated. For the qPCR primer, the aforementioned primers (Fw-Primer and Rv-Primer) were used. For the qPCR reaction solution, 1 × Ex Taq buffer, 0.2 mM dNTP, 0.5 μM Fw-Primer, 0.5 μM Rv-Primer, 50000-fold diluted SYBR Green I (LONZA, #50513), and 0.025 U/μL Ex Taq polymerase (Takara Bio Inc.) were mixed. The mixture was heated at 95°C for 2 minutes, and then subjected to 40 cycles of 95°C for 10 seconds, 57°C for 20 seconds, and 72°C for 30 seconds.

To draw a calibration curve to estimate the recovery rate of the library, a sample prepared by reverse transcription of the mRNA-puromycin linked construct prepared in Preparation Example 4-3 of Experimental Example 1 was diluted to be 1E + 8/μL, 1E + 6/μL, and 1E + 4/μL, and subjected to qPCR under the same conditions.

In Rounds 2 to 4, to a buffer containing 1 × TBS, 2 mg/mL BSA (Invitrogen), 2 mM DTT, and 5 mM $CaCl_2$, the display library was added to be 0.2-0.3 μM, and His-CnB-CnA-CyA-FLAG-TEV-Avi-bio, CyA-FLAG-TEV-Avi-bio, or CnB-CnA-FLAG-TEV-Avi-bio as a target protein was added to be 0.4 μM. In the case where the target protein was His-CnB-CnA-CyA-FLAG-TEV-Avi-bio or CnB-CnA-FLAG-TEV-Avi-bio, Calmodulin (FUJIFILM Wako Pure Chemical Corporation) was further added to be 2 μM, then the mixture was incubated at 4°C for 1 hour. Then, the resultant was mixed with streptavidin-coated magnetic beads or neutravidin-coated magnetic beads, and the mixture was mixed by inversion at 4°C for 10 minutes to immobilize the target protein. Biotin was then added to be 0.04-0.05 mM, and the mixture was mixed by inversion at 4°C for another 10 minutes. Thereafter, the same process as in Round 1 was performed.

At the second round or thereafter, the input library was divided into two, and selection was made with one in the presence of the target protein and the other in the absence of the target protein, and the nucleic acid recovery rates of both were evaluated from the results of qPCR. The recovery rate (%) of each round of panning was calculated by the expression below. The results are shown in the table below.

Recovery rate of library (%) = amount (number of molecules) of output nucleic acids of library/(amount (number of molecules) of input nucleic acids of library) × 100

[Table 7]

| Change in recovery rate when using His-CnB-CnA-CyA-FLAG-TEV-Avi-bio (SEQ ID NO: 10) as target protein | | | | | | |
|---|---|---|---|---|---|---|
| Round | Amount of input nucleic acids of library (molecules) | In presence of target | | In absence of target | | Ratio of recovery rate in presence to absence of target |
| | | Molecules of output nucleic acids (molecules) | Recovery rate (%) | Molecules of output nucleic acids (molecules) | Recovery rate (%) | |
| **1** | **3.25E+13** | **1.62E+08** | **4.98E-04** | **-** | **-** | **-** |
| **2** | **6.62E+11** | **4.59E+07** | **6.94E-03** | **4.81E+05** | **7.27E-05** | **95.4** |
| **3** | **2.40E+11** | **8.94E+06** | **3.72E-03** | **5.47E+04** | **2.28E-05** | **163.4** |
| **4** | **2.92E+11** | **9.89E+08** | **3.38E-01** | **2.70E+05** | **9.25E-05** | **3660.9** |
| **5** | **5.79E+11** | **4.15E+08** | **7.16E-02** | **3.01E+04** | **5.20E-06** | **13761.6** |

[Table 8]

| Change in recovery rate when using CyA-FLAG-TEV-Avi-bio (SEQ ID NO: 6) as target protein | | | | | | |
|---|---|---|---|---|---|---|
| Round | Amount of input nucleic acids of library (molecules) | In presence of target | | In absence of target | | Ratio of recovery rate in presence to absence of target |
| | | Molecules of output nucleic acids (molecules) | Recovery rate (%) | Molecules of output nucleic acids (molecules) | Recovery rate (%) | |
| **1** | **3.22E+13** | **2.50E+07** | **7.76E-05** | **-** | **-** | **-** |
| **2** | **1.15E+12** | **4.15E+07** | **3.61E-03** | **8.88E+05** | **7.73E-05** | **46.7** |
| **3** | **4.00E+11** | **7.97E+07** | **1.99E-02** | **5.18E+04** | **1.29E-05** | **1538.3** |
| **4** | **3.88E+11** | **1.70E+09** | **4.38E-01** | **1.88E+05** | **4.85E-05** | **9031.9** |
| **5** | **6.97E+11** | **1.04E+09** | **1.49E-01** | **2.43E+04** | **3.49E-06** | **42645.9** |

[Table 9]

| Change in recovery rate when using CnB-CnA-FLAG-TEV-Avi-bio (SEQ ID NO: 7) as target protein | | | | | | |
|---|---|---|---|---|---|---|
| Round | Amount of input nucleic acids of library (molecules) | In presence of target | | In absence of target | | Ratio of recovery rate in presence to absence of target |
| | | Molecules of output nucleic acids (molecules) | Recovery rate (%) | Molecules of output nucleic acids (molecules) | Recovery rate (%) | |
| **1** | **3.30E+13** | **7.25E+08** | **2.20E-03** | **-** | **-** | **-** |
| **2** | **8.90E+11** | **1.98E+08** | **2.22E-02** | **6.34E+05** | **7.13E-05** | **311.9** |
| **3** | **4.68E+11** | **8.95E+07** | **1.91E-02** | **7.90E+04** | **1.69E-05** | **1133.1** |
| **4** | **1.02E+11** | **8.45E+07** | **8.29E-02** | **9.52E+04** | **9.35E-05** | **887.1** |
| **5** | **2.38E+11** | **1.25E+08** | **5.26E-02** | **8.67E+04** | **3.64E-05** | **1444.7** |

As a result, in the second half of the rounds, as shown in Tables 7 to 9, there was a difference in the recovery rate between the selections in the presence of the target protein and in the absence of the target protein. It was thus suggested that molecules that were recovered only in the presence of the target protein were present in the library of each round, and they were concentrated each time of the repeated rounds. In particular, this phenomenon was seen in panning against not only fusion proteins but also single protein. It is thus strongly suggested that not only a binder that can form a ternary

complex with cyclophilin A and calcineurin, but also a binder that binds to cyclophilin A or calcineurin alone are present in the library.

DNA pools of an each round obtained by panning was subjected to nucleotide sequence analysis with next-generation sequencers by methods known to those skilled in the art.

<Example 1-3: panning using fusion protein of calcineurin-FKBP as targets>

In Round 1, CnB-CnA-FLAG-FKBP-FLAG-TEV-Avi-bio as a target protein was added to a buffer containing 1 × TBS, 2 mg/mL BSA (Invitrogen), 2 mM DTT, and 5 mM $CaCl_2$ to be 0.4 μM, and then mixed with streptavidin-coated magnetic beads, and the mixture was mixed by inversion at 4°C for 10 minutes to immobilize the target protein. Biotin was then added to be 0.5 mM, and the mixture was mixed by inversion at 4°C for another 10 minutes. Then, the streptavidin-coated magnetic beads with immobilized target proteins were collected with a magnet and the supernatant was removed, and the beads were once washed with a wash buffer (1 × TBS, 2 mM DTT, 0.1% Tween 20 (Sigma Aldrich), 500 mM arginine), and then the display library was added to be 0.6 μM and Calmodulin (FUJIFILM Wako Pure Chemical Corporation) was added to be 2 μM, then the mixture was mixed by inversion at 4°C for 1 hour.

Streptavidin-coated magnetic beads mixed with the display library were collected with a magnet and the supernatant was removed. The collected beads were washed several times with the wash buffer described above, then treated with a TEV protease that recognizes and cleaves the TEV protease recognition sequence to elute the target protein and the peptide-nucleic acid conjugate that binds to the target protein.

To the streptavidin-coated magnetic beads after washing, 1 × TEV buffer, 1 mM DTT, and 0.1 U/μL AcTEV protease (Thermo Fisher Scientific Inc., #12575015) were added as a specific TEV eluent to react with. After the reaction, the supernatant was recovered, PCR was performed with library recognition primers (Fw-Primer and Rv-Primer), and the resultant was subjected to the next round.

In addition, qPCR was performed using a portion of the TEV elution product, and the recovery rate (%) of the library of Round 1 was evaluated. For the qPCR primer, the aforementioned primers (Fw-Primer and Rv-Primer) were used. For the qPCR reaction solution, 1 × Ex Taq buffer, 0.2 mM dNTP, 0.5 μM Fw-Primer, 0.5 μM Rv-Primer, 50000-fold diluted SYBR Green I (LONZA, #50513), and 0.025 U/μL Ex Taq polymerase (Takara Bio Inc.) were mixed. The mixture was heated at 95°C for 2 minutes, and then subjected to 40 cycles of 95°C for 10 seconds, 57°C for 20 seconds, and 72°C for 30 seconds.

To draw a calibration curve to estimate the recovery rate of the library, a sample prepared by reverse transcription of the mRNA-puromycin linked construct prepared in Preparation Example 4-3 of Experimental Example 1 was diluted to be 1E + 8/μL, 1E + 6/μL, and 1E + 4/μL, and subjected to qPCR under the same conditions.

In Rounds 2 to 4, CnB-CnA-FLAG-FKBP-FLAG-TEV-Avi-bio as a target protein was added to a buffer containing 1 × TBS, 2 mg/mL BSA (Invitrogen), 2 mM DTT, and 5 mM $CaCl_2$ to be 0.4 μM, and then mixed with streptavidin-coated magnetic beads or neutravidin-coated magnetic beads, and the mixture was mixed by inversion at 4°C for 10 minutes to immobilize the target protein. Biotin was then added to be 0.5 mM, and the mixture was mixed by inversion at 4°C for another 10 minutes. Then, the streptavidin-coated magnetic beads with immobilized target proteins or neutravidin-coated magnetic beads were collected with a magnet and the supernatant was removed, and the beads were once washed the wash buffer described above, and then the display library was added to be 0.2-0.3 μM and Calmodulin (Fujifilm Wako Pure Chemical) was added to be 2 μM, then the mixture was mixed by inversion at 4°C for 1 hour. Thereafter, the same process as in Round 1 was performed.

At the second round or thereafter, the input library was divided into two, and selection was made with one in the presence of the target protein and the other in the absence of the target protein, and the nucleic acid recovery rates of both were evaluated from the results of qPCR. The recovery rate (%) of each round of panning was calculated by the expression below. The results are shown in the table below.

Recovery rate of library (%) = amount (number of molecules) of output nucleic acids of library/(amount (number of molecules) of input nucleic acids of library) × 100

[Table 10]

| Change in recovery rate when using CnB-CnA-FLAG-FKBP-FLAG-TEV-Avi-bio (SEQ ID NO: 49 and SEQ ID NO: 50) as target protein | | | | | | |
|---|---|---|---|---|---|---|
| Round | Amount of input nucleic acids of library (molecules) | In presence of target | | In absence of target | | Ratio of recovery rate in presence to absence of target |
| | | Molecules of output nucleic acids (molecules) | Recovery rate (%) | Molecules of output nucleic acids (molecules) | Recovery rate (%) | |
| **1** | **1.68E+13** | **4.31E+08** | **2.57E-03** | **-** | **-** | **-** |
| **2** | **6.22E+11** | **1.83E+08** | **2.94E-02** | **3.62E+06** | **5.82E-04** | **50.6** |
| **3** | **4.11E+11** | **2.95E+07** | **7.18E-03** | **1.37E+05** | **3.34E-05** | **215.0** |
| **4** | **3.16E+11** | **2.85E+09** | **9.02E-01** | **3.42E+06** | **1.08E-03** | **833.3** |

As a result, in the second half of the rounds, as shown in Table 10, there was a difference in the recovery rate between the selections in the presence of the target protein and in the absence of the target protein. It was thus suggested that molecules that were recovered only in the presence of the target protein were present in the library of each round, and they were concentrated each time of the repeated rounds.

DNA pools of an each round obtained by panning was subjected to nucleotide sequence analysis with next-generation sequencers by methods known to those skilled in the art.

[Example 2: identification of peptide candidate capable of forming complex with plurality of kinds of targets by counter-assay]

As described above, it is expected that the concentrated sequence in panning using a fusion protein includes not only a peptide capable of forming a complex with a plurality of kinds of targets, but also a peptide that binds only to a single target. A counter-assay was performed to identify, from these, a molecule of interest that is a peptide capable of forming a complex with a plurality of kinds of targets.

<Example 2-1: identification of peptide capable of forming ternary complex with FKBP and FRB>

(Example 2-1-1: preparation of mRNA display library consisting of sequences concentrated in panning)

An mRNA-puromycin linked construct was prepared from a DNA pool after 3 rounds of panning using a fusion protein of FKBP and FRB in accordance with the method of WO2013/100132. It is noted that axPM-27 described above was used as a puromycin linker. This construct was translated in a translation system prepared in Preparation Example 4-3 of Experimental Example 1, and subjected to a cyclization reaction, a desulfurization reaction and a reverse transcription in accordance with the method described in WO2017/150732 to prepare an mRNA-display library consisting of peptide sequences concentrated by panning using the fusion protein.

(Example 2-1-2: affinity selection of mRNA display library)

To a buffer containing 1 × TBS, 2 mg/mL BSA (Invitrogen), and 2 mM DTT, the display library prepared in Example 2-1-1 was added to be 0.2-0.3 μM and the target protein was added to be as shown in Table 11, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.04-0.05 mM, and the mixture was mixed by inversion at 4°C for another 10 minutes. The proteins prepared in Preparation Examples 1-1 to 1-5 were used as the target proteins.

[Table 11]

| Condition | Target protein |
|---|---|
| **1** | **0.4 μM bio-Avi-TEV-FLAG-FKBP-FRB-His** |
| **2** | **0.4 μM FKBP-FLAG-TEV-Avi-bio** |

(continued)

| Condition | Target protein |
|---|---|
| **3** | **0.4 μM FKBP-FLAG-TEV-Avi-bio** and **0.4 μM FRB-FLAG-Sor-His** |
| **4** | **0.4 μM FRB-FLAG-TEV-Avi-bio** |
| **5** | **0.4 μM FRB-FLAG-TEV-Avi-bio** and **0.4 μM FKBP-FLAG-Sor-His** |
| **6** | No target protein |

Condition 1 is a condition in which a fusion protein is used, and the two kinds of target proteins are in proximity. Conditions 2 and 4 are conditions in which only FKBP or only FRB is present in the system. Conditions 3 and 5 are conditions in which FKBP and FRB are present, but the target protein pulled down with streptavidin-coated magnetic beads is FKBP or FRB, respectively, and the two kinds of target proteins are not in proximity. Condition 6 is a condition under the absence of the target protein.

Display molecules pulled down in the same manner as in Examples 1-1 of Experimental Example 1 were recovered. Some of them were quantified by qPCR, and the number of input molecules, the number of molecules recovered under each condition, and the recovery rate were calculated and summarized in Table 12.

[Table 12]

| Number of input molecules (molecules) | Condition | Number of recovered molecules (molecules) | Recovery rate (%) |
|---|---|---|---|
| **1.86E+11** | **1** | **1.01E+09** | **5.43E-01** |
| | **2** | **5.06E+08** | **2.71E-01** |
| | **3** | **3.99E+08** | **2.14E-01** |
| | **4** | **4.77E+05** | **2.56E-04** |
| | **5** | **1.99E+05** | **1.07E-04** |
| | **6** | **8.88E+04** | **4.76E-05** |

PCR was performed on the remaining eluate, which was not subjected to qPCR, with the same primer as in qPCR to amplify the DNA recovered by pull-down. The nucleotide sequences of these and the library of inputs were analyzed with a next-generation sequencer by methods known to those skilled in the art. Based on this result, the proportion of each sequence in the total DNA pool was calculated.

The recovery rate (%) of each mRNA display molecule encoding each peptide in each selection condition was calculated according to the following expression (1).

[Expression 3]

$$Recovery\ rate\ (\%) = \frac{Number\ of\ output\ molecules\ Xa \times Particular\ display\ molecule\ proportion\ Ra}{Number\ of\ input\ molecules\ Xi \times Particular\ display\ molecule\ proportion\ Ri} \times 100\ \ldots$$

Expression (1)

In the above expression (1), the number of output molecules Xa represents the number of all mRNA display molecules recovered by the pull-down operation; the particular display molecule proportion Ra represents a proportion of the number of mRNA display molecules encoding a particular peptide in the number of output molecules Xa; the number of input molecules Xi represents the number of all mRNA display molecules contained in the library prior to the pull-down operation; and the particular display molecule proportion Ri represents a proportion of the number of mRNA display molecules encoding the specific peptide in the number of input molecules Xi.

Here, the molecule of interest, which is a peptide capable of forming a ternary complex with FKBP and FRB, was assumed to be most efficiently recovered in condition 1 above, and a peptide encoded by mRNA display molecules that meet the following conditions was identified as the molecule of interest.

(Recovery rate in condition 1 of mRNA-display molecule encoding particular peptide)/(Recovery rate in condition 2 of mRNA-display molecule encoding the particular peptide) ≥ 5 and (Recovery rate in condition 1 of mRNA-display molecule encoding the particular peptide)/Recovery rate in condition 4 of mRNA-display molecule encoding the particular peptide) ≥ 5

Some of the peptides meeting these conditions were selected and synthesized in Synthesis Example 1.

<Example 2-2: identification of peptide capable of forming ternary complex with cyclophilin A and calcineurin>

(Example 2-2-1: preparation of mRNA display library consisting of sequences concentrated in panning)

An mRNA-puromycin linked construct was prepared from a DNA pool after 3 rounds of panning using a fusion protein of cyclophilin A and calcineurin in accordance with the method of WO2013/100132. It is noted that axPM-27 described above was used as a puromycin linker. This construct was translated in a translation system prepared in Preparation Example 4-3 of Experimental Example 1, and subjected to a cyclization reaction, a desulfurization reaction and a reverse transcription in accordance with the method described in WO2017/150732 to prepare an mRNA display library consisting of peptide sequences concentrated by panning using the fusion protein.

(Example 2-2-2: affinity selection of mRNA display library)

To a buffer containing 1 × TBS, 2 mg/mL BSA (Invitrogen), 2 mM DTT, and 5 mM $CaCl_2$, the display library prepared in Example 2-2-1 of Experimental Example 1 was added to be 0.2-0.3 μM, Calmodulin (FUJIFILM Wako Pure Chemical Corporation) to be 2 μM and the target protein to be shown in Table 13, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.04 to 0.05 mM, and the mixture was mixed by inversion at 4°C for another 10 minutes. The proteins prepared in Preparation Examples 2-1 to 2-5 were used as the target proteins.

[Table 13]

| Condition | Target protein |
|---|---|
| **1** | **0.4 μM His-CnB-CnA-CyA-FLAG-TEV-Avi-bio** |
| **2** | **0.4 μM CyA-FLAG-TEV-Avi-bio** |
| **3** | **0.4 μM CyA-FLAG-TEV-Avi-bio** and **0.4 μM CnB-CnA-FLAG-Sor-His** |
| **4** | **0.4 μM CnB-CnA-FLAG-TEV-Avi-bio** |
| **5** | **0.4 μM CnB-CnA-FLAG-TEV-Avi-bio** and **0.4 μM CyA-FLAG-Sor-His** |
| **6** | No target protein |

Condition 1 is a condition in which a fusion protein is used, and the two kinds of target proteins are in proximity. Conditions 2 and 4 are conditions in which only cyclophilin A or only calcineurin is present in the system. Conditions 3 and 5 are conditions in which cyclophilin A and calcineurin are present, but the target protein pulled down with streptavidin-coated magnetic beads is cyclophilin A or calcineurin, respectively, and the two kinds of target proteins are not in proximity. Condition 6 is a condition under the absence of the target protein.

Display molecules pulled down in the same manner as in Examples 1-2 of Experimental Example 1 were recovered. Some of them were quantified by qPCR, and the number of input molecules, the number of molecules recovered under each condition, and the recovery rate were calculated and summarized in Table 14.

[Table 14]

| Number of input molecules (molecules) | Condition | Number of recovered molecules (molecules) | Recovery rate (%) |
|---|---|---|---|
| **6.84E+11** | **1** | **8.13E+09** | **1.19E+00** |
| | **2** | **1.19E+09** | **1.74E-01** |
| | **3** | **1.36E+09** | **1.98E-01** |
| | **4** | **3.01E+09** | **4.40E-01** |
| | **5** | **2.99E+09** | **4.37E-01** |
| | **6** | **3.50E+05** | **5.12E-05** |

PCR was performed on the remaining eluate, which was not subjected to qPCR, with the same primer as in qPCR to amplify the DNA recovered by pull-down. The nucleotide sequences of these and the library of inputs were analyzed with a next-generation sequencer by methods known to those skilled in the art. Based on this result, the proportion of each sequence in the total DNA pool was calculated.

The recovery rate of mRNA display molecule encoding each peptide in each selection condition was calculated according to the above expression (1).

Here, the molecule of interest, which is a peptide capable of forming a ternary complex with cyclophilin A and calcineurin, was assumed to be most efficiently recovered in condition 1 above, and a peptide encoded by mRNA display molecules that meet the following conditions was identified as the molecule of interest.

(Recovery rate in condition 1 of mRNA-display molecule encoding particular peptide)/(Recovery rate in condition 2 of mRNA-display molecule encoding the particular peptide) $\geq 5$ and (Recovery rate in condition 1 of mRNA-display molecule encoding the particular peptide)/(Recovery rate in condition 4 of mRNA-display molecule encoding the particular peptide) $\geq 5$

Some of the peptides meeting these conditions were selected and synthesized in Synthesis Example 1.

<Example 2-3: identification of peptide capable of forming ternary complex with FKBP and calcineurin>

(Example 2-3-1: preparation of mRNA display library consisting of sequences concentrated in panning)

An mRNA-puromycin linked construct was prepared from a DNA pool after 3 rounds of panning using a fusion protein of FKBP and calcineurin in accordance with the method of WO2013/100132. It is noted that axPM-27 described above was used as a puromycin linker. This construct was translated in a translation system prepared in Preparation Example 4-3 of Experimental Example 1, and subjected to a cyclization reaction, a desulfurization reaction and a reverse transcription in accordance with the method described in WO2017/150732 to prepare an mRNA-display library consisting of peptide sequences concentrated by panning using the fusion protein. As the translation system and the aminoacylated tRNA required for translation, those used in Example 1-3 of Experimental Example 1 were used.

(Example 2-3-2: affinity selection of mRNA display library)

A biotinylated target protein was added to a buffer containing 1 × TBS, 2 mg/mL BSA (Invitrogen), 2 mM DTT, and 5 mM $CaCl_2$ as shown in Table 15, and then mixed with neutravidin-coated magnetic beads, and the mixture was mixed by inversion at 4°C for 10 minutes to immobilize the target protein. Biotin was then added to be 0.5 mM, and the mixture was mixed by inversion at 4°C for another 10 minutes. Then, the neutravidin-coated magnetic beads with immobilized target proteins were collected with a magnet and the supernatant was removed, and the beads were once washed with a wash buffer (1 × TBS, 2 mM DTT, 0.1% Tween 20 (Sigma Aldrich), 500 mM arginine), and then the display library prepared in Examples 2-3-1 of Experimental Example 1 was added to be 0.2-0.3 μM, Calmodulin (Fujifilm Wako Pure Chemical) to be 2 μM, and non-biotinylated protein, when added, to be the concentrations as shown in Table 15, then the mixture was mixed by inversion at 4°C for 1 hour. The proteins prepared in Preparation Examples 1-1, 1-3, 2-2, 2-4, 3-4 were used as the target proteins.

[Table 15]

| Condition | Target protein |
|---|---|
| **1** | **0.4 μM CnB-CnA-FLAG-FKBP-FLAG-TEV-Avi-bio** |
| **2** | **0.4 μM FKBP-FLAG-TEV-Avi-bio** |
| **3** | **0.4 μM FKBP-FLAG-TEV-Avi-bio** and **0.4 μM CnB-CnA-FLAG-Sor-His** |
| **4** | **0.4 μM CnB-CnA-FLAG-TEV-Avi-bio** |
| **5** | **0.4 μM CnB-CnA-FLAG-TEV-Avi-bio** and **0.4 μM FKBP-FLAG-Sor-His** |
| **6** | No target protein |

Condition 1 is a condition in which a fusion protein is used, and the two kinds of target proteins are in proximity. Conditions 2 and 4 are conditions in which only FKBP or only calcineurin is present in the system. Conditions 3 and 5 are conditions in which FKBP and calcineurin are present, but the target proteins pulled down with neutravidin-coated magnetic beads are FKBP or calcineurin, respectively, and the two kinds of target proteins are not in proximity. Condition 6 is a condition under the absence of the target protein.

Display molecules pulled down in the same manner as in Examples 1-3 of Experimental Example 1 were recovered. Some of them were quantified by qPCR, and the number of input molecules, the number of molecules recovered under each condition, and the recovery rate were calculated and summarized in Table 16.

[Table 16]

| Number of input molecules (molecules) | Condition | Number of recovered molecules (molecules) | Recovery rate (%) |
|---|---|---|---|
| **1.99E+11** | **1** | **3.25E+09** | **1.63E+00** |
| | **2** | **1.76E+09** | **8.84E-01** |
| | **3** | **1.68E+09** | **8.44E-01** |
| | **4** | **2.73E+09** | **1.37E+00** |
| | **5** | **2.23E+09** | **1.12E+00** |
| | **6** | **2.97E+06** | **1.49E-03** |

PCR was performed on the remaining eluate, which was not subjected to qPCR, with the same primer as in qPCR to amplify the DNA recovered by pull-down. The nucleotide sequences of these and the library of inputs were analyzed with a next-generation sequencer by methods known to those skilled in the art. Based on this result, the proportion of each sequence in the total DNA pool was calculated.

The recovery rate of mRNA display molecule encoding each peptide in each selection condition was calculated according to the above expression (1).

Here, the molecule of interest, which is a peptide capable of forming a ternary complex with FKBP and calcineurin, was assumed to be most efficiently recovered in condition 1 above, and a peptide encoded by mRNA display molecules that meet the following conditions was identified as the molecule of interest.

(Recovery rate in condition 1 of mRNA-display molecule encoding particular peptide)/(Recovery rate in condition 2 of mRNA-display molecule encoding the particular peptide) ≥ 5 and (Recovery rate in condition 1 of mRNA-display molecule encoding the particular peptide)/(Recovery rate in condition 4 of mRNA-display molecule encoding the particular peptide) ≥ 5

Some of the peptides meeting these conditions were selected and synthesized in Synthesis Example 1.

[Synthesis Example 1: synthesis of peptides obtained by panning and subsequent counter assay]

The analysis conditions of LC-MS are as described below.

[Table 17]

| Preparative condition | Device | Column (I.D. × length (mm)) | Mobile phase | Gradient (A/B) | Flow rate (ml/min) | Column temperature (°C) | Wavelength |
|---|---|---|---|---|---|---|---|
| **SQD FA06** | **Acquity UPLC/SQD2** | **Aldrich Ascentis Express C18**<br>**(2.1 x 50)** | **A) 0.1% FA. in H2O**<br>**B) 0.1% FA in CH3CN** | **95/5 => 0/100** (1.0 min) =><br>**0/100**(0.4 min) | **0.9** | **35** | **210-400nm**<br>**PDA total** |
| **SQD AA05** | **Acquity UPLC/SQD2** | **Aldrich Ascentis Express C18**<br>**(2.1 x 50)** | **A) 10mM AcONH4, H2O**<br>**B) metha-nol** | **95/5 => 0/100** (1.0 min)**=>**<br>**0/100** (0.4 min) | **0.9** | **35** | **210-400nm**<br>**PDA total** |

All starting materials and reagents were obtained from commercial suppliers, or synthesized using known methods.

[Synthesis Example 1: solid phase synthesis of peptide]

The peptide was extended to synthesize a compound according to the synthesis methods using the Fmoc method described in WO2013/100132, WO2018/225864, and WO2022/234852.

The method included the following 5 steps:

1) peptide extension reaction from the N-terminus of an amino acid by the Fmoc method using one in which a carboxylic acid of the Asp side chain was carried on a 2-chlorotrityl resin;
2) cleavage process of the peptide from the 2-chlorotrityl resin;
3) amide cyclization by condensation of the carboxylic acid of the Asp side chain derived from the 2-chlorotrityl resin and the amino group at the N-terminus of the peptide chain;
4) optionally, deprotection of the protecting group of the side chain functional group contained in the peptide chain; and
5) purification of the compound by preparative HPLC. Unless otherwise stated, the peptides in Examples were synthesized based on this synthesis method.

According to the above synthesis conditions, cyclic peptides were synthesized. In Tables 18 and 19, sequences of amino acids were shown from the N-terminus in order in columns 1 to 11, and the exact mass, LC-MS analysis conditions, observed mass spectrum (obs. mass; m/z), ion form, and retention time (min) of each compound were described. All peptides are cyclic peptides in which an amide bond between an amino group at the N-terminus and a carboxyl group of the Asp side chain at the C-terminus is formed. The analysis conditions of LC-MS are shown in Table 17.

[Table 18]

| ID | 1 | 2 | 3 | 4 | 5 | 6 | 7 | 8 | 9 | 10 | 11 | Exact mass | Analysis condition | obs. Mass (m/z) | Ion form | Retention time (min) |
|---|---|---|---|---|---|---|---|---|---|---|---|---|---|---|---|---|
| FF-01 | MeAla | Hph(3-Cl) | MeGly | Gly | D-MeSer | Pic(2) | Hph(3-Cl) | Thr | MeSer | Ser(NtBu-Aca) | Asp2-pyrro | 1385.6 | FA05 | 1384.9 | [M-H]- | 0.74 |
| FF-02 | MeAla | MePhe | MeGly | D-MeSer | Pic(2) | Thr | Nle | MeSer(tBuOH) | Pro | Ser(Ph-2-Cl) | Asp2-pyrro | 1378.7 | FA05 | 1380.0 | [M+H]+ | 0.66 |
| FF-03 | MeAla | Ser(NtBu-Aca) | Gly | MeAla(3-Pyr) | Hph(3-Cl) | nBuGly | Pic(2) | Hph(3-Cl) | Ile | Ser(3-F-5-Me-Pyr) | Asp2-pyrro | 1595.8 | FA05 | 1595.0 | [M-H]- | 0.82 |
| FF-04 | MeAla | Phe(3-Cl) | MeVal | Ser(Ph-2-Cl) | nBuGly | Pic(2) | Ser(NtBu-Aca) | Thr | Gly | Gly | Asp2-pyrro | 1383.6 | FA05 | 1382.9 | [M-H]- | 0.92 |
| FF-05 | MeAla | MeVal | Pic(2) | Ser(NtBu-Aca) | Ser(NtBu-Aca) | Ser(3-F-5-Me-Pyr) | Ser(NtBu-Aca) | nBuGly | Nle | MeSer(nPr) | Asp2-pyrro | 1643.0 | FA05 | 1642.3 | [M-H]- | 0.94 |
| FF-06 | MeAla | Thr | nBuGly | Nle | D-MeSer | Pic(2) | Gly | Hph(3-Cl) | Pic(2) | Hph(3-Cl) | Asp2-pyrro | 1350.7 | FA05 | 1351.8 | [M+H]+ | 0.84 |
| FF-07 | MeAla | MeSer(tBuOH) | Ser(Ph-2-Cl) | D-MeSer | Phe(3-Cl) | MePhe | MeVal | Phe(3-Cl) | Pro | Thr | Asp2-pyrro | 1558.6 | FA05 | 1559.9 | [M+H]+ | 0.93 |
| CC-01 | MeAla | MeAla(3-Pyr) | MeSer(tBuOH) | nBuGly | Ile | MeSer(nPr) | Phe(3-Cl) | MeVal | MeHnl(7-F2) | Thr | Asp2-pyrro | 1529.8 | FA05 | 1529.2 | [M-H]- | 0.82 |
| CC-02 | MeAla | Phe(3-Cl) | MePhe | cisPro(4-pip-4-F2) | Nle | MeGly | Nle | MeHnl(7-F2) | MeHnl(7-F2) | Thr | Asp2-pyrro | 1563.8 | FA05 | 1563.2 | [M-H]- | 0.83 |
| CC-03 | MeAla | Ser(iPen) | Pro | Ser(3-F-5-Me-Pyr) | Nle | MeVal | MeHph | Thr | Thr | MeSer(tBuOH) | Asp2-pyrro | 1479.8 | FA05 | 1479.2 | [M-H]- | 0.81 |
| CC-04 | MeAla | MePhe | MeSer | Gly | Ile | Pro | Phe(3-Cl) | MeVal | MeHnl(7-F2) | Ile | Asp2-pyrro | 1366.7 | FA05 | 1366.1 | [M-H]- | 0.93 |
| CC-05 | MeAla | Ser(NtBu-Aca) | Thr | MeAla | MeHnl(7-F2) | Thr | Ser(3-F-5-Me-Pyr) | Ser(Ph-2-Cl) | Nle | MeSer | Asp2-pyrro | 1524.7 | FA05 | 1526.0 | [M+H]+ | 0.72 |
| CC-06 | MeAla | cisPro(4-pip-4-F2) | Phe(3-Cl) | Pro | Gly | Ser(Ph-2-Cl) | MeSer(nPr) | Thr | Ile | Gly | Asp2-pyrro | 1415.6 | AA05 | 1416.6 | [M+H]+ | 1.10 |
| CC-07 | MeAla | Ile | Phe(3-Cl) | MeVal | MeHph | Thr | Nle | Thr | MeVal | MeVal | Asp2-pyrro | 1376.8 | FA05 | 1377.9 | [M+H]+ | 0.88 |

[Table 19]

| ID | 1 | 2 | 3 | 4 | 5 | 6 | 7 | 8 | 9 | 10 | 11 | Exact mass | Analysis condition | obs. Mass (m/z) | ion form | Retention time (min) |
|---|---|---|---|---|---|---|---|---|---|---|---|---|---|---|---|---|
| CF-01 | MeAla | Pic(2) | Thr | Ser(NtBu-Aca) | Thr | Gly | Pro(4-pip-4- | Phe(3-Cl) | MeHph | Hph(3-Cl) | Asp2-pyrro | 1590.7 | FA05 | 1590.0 | [M-H]- | 0.81 |
| CF-02 | MeAla | Ile | Nle | MeHnl(7-F2) | MePhe | MeGly | Gly | Pic(2) | Pro(4-pip-4- | Pro | Asp2-pyrro | 1369.8 | FA05 | 1369.0 | [M-H]- | 0.70 |

The structure formulas of the synthesized compounds FF-01 to FF-07, CC-01 to CC-07, and CF-01 to CF-02 are

shown in Tables 20 to 26.

[Table 20]

| Compound name | Structure formula |
|---|---|
| FF-01 | 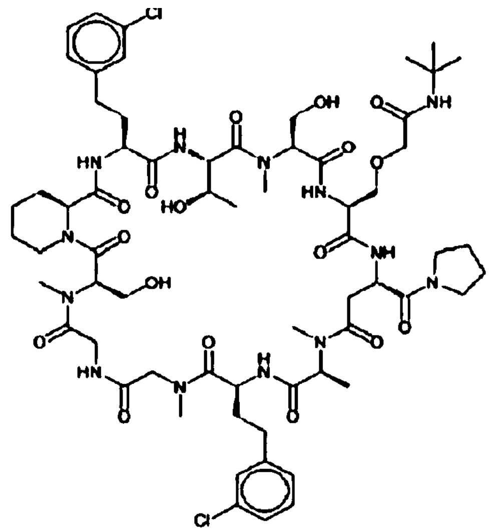 |
| FF-02 | 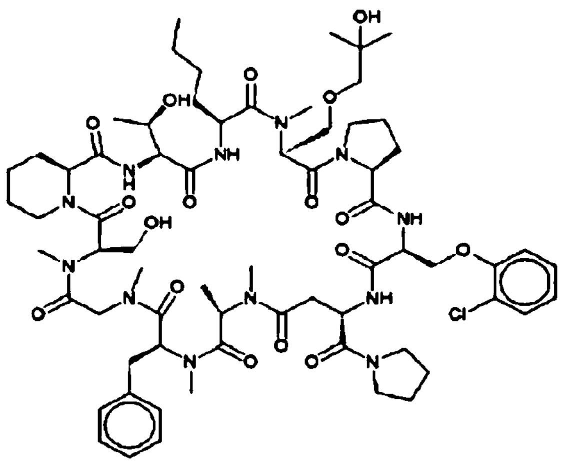 |
| FF-03 | 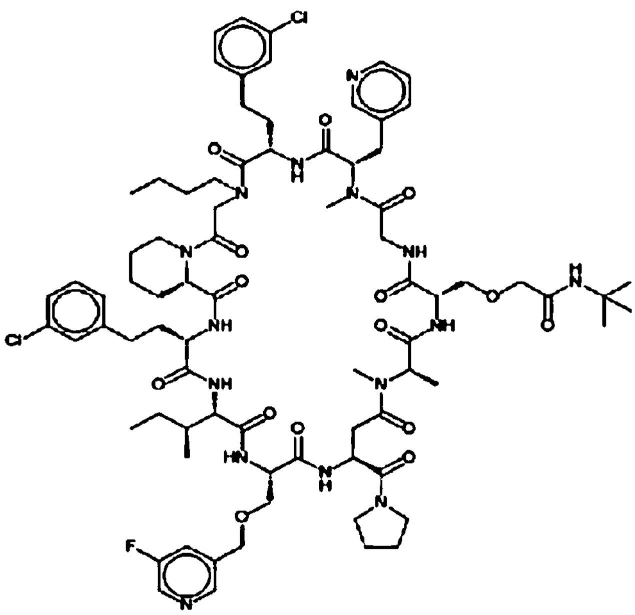 |

[Table 21]

| Compound name | Structure formula |
|---|---|
| FF-04 | 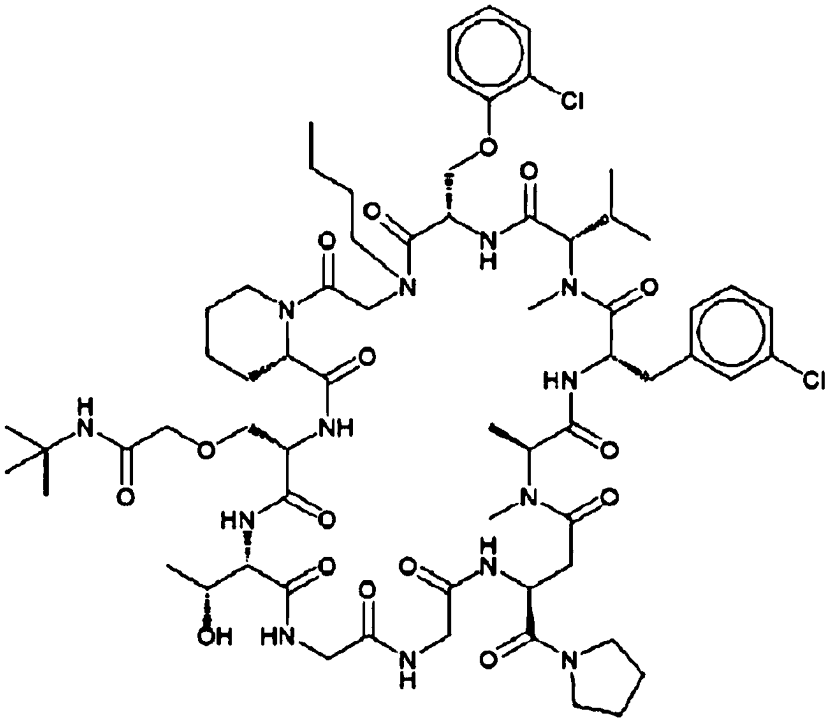 |
| FF-05 | 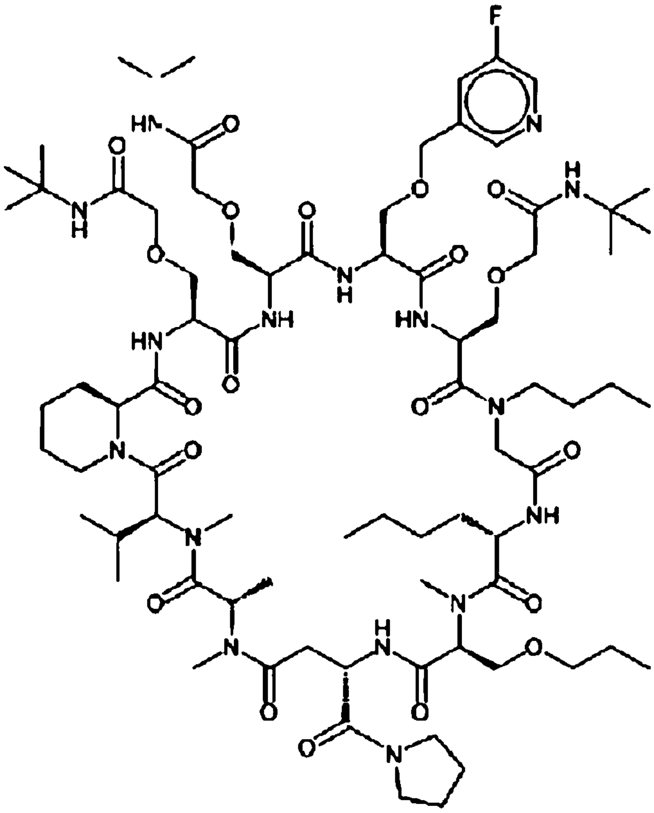 |

[Table 22]

| Compound name | Structure formula |
|---|---|
| FF-06 | 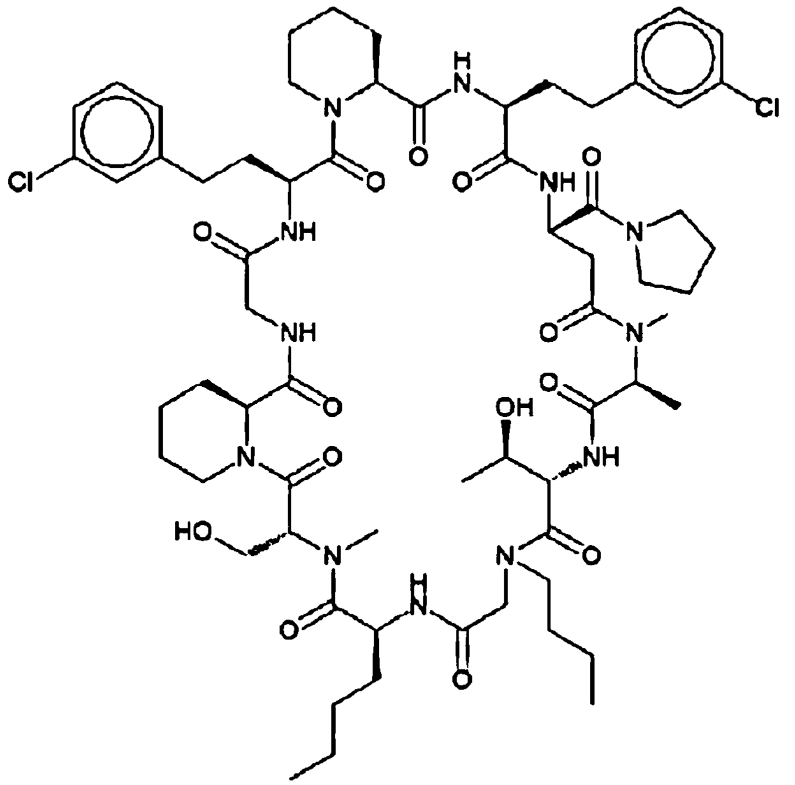 |
| FF-07 | 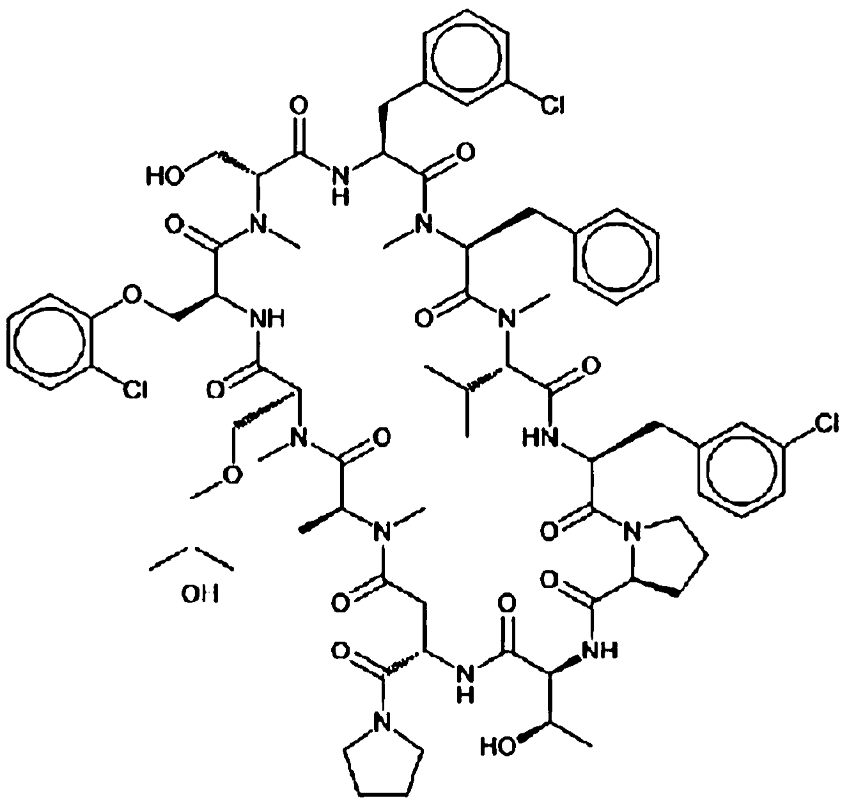 |

[Table 23]

| Compound name | Structure formula |
|---|---|
| CC-01 | 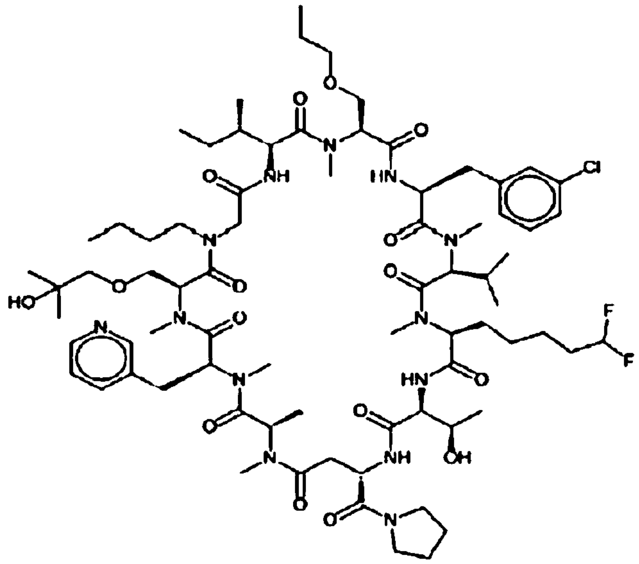 |
| CC-02 | 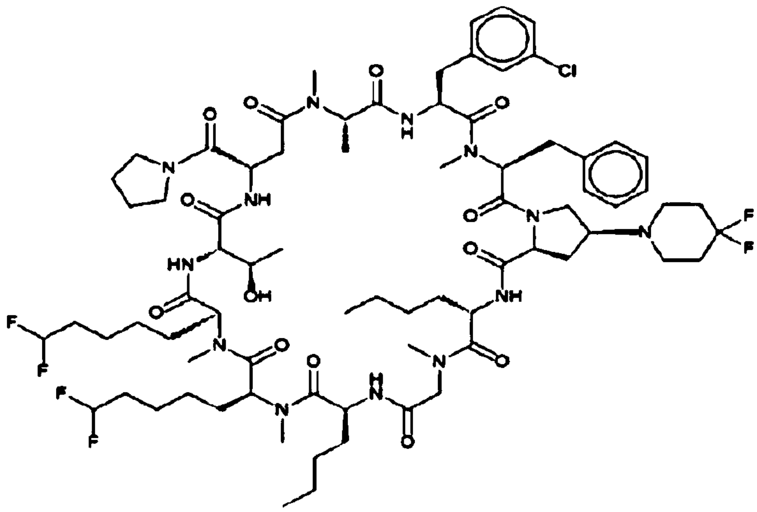 |
| CC-03 | 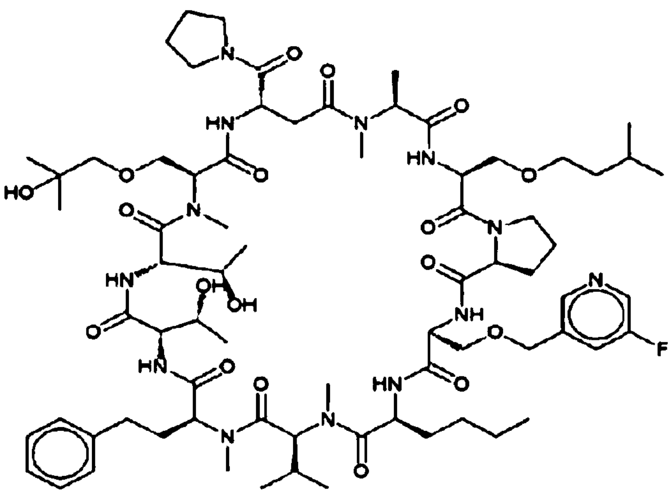 |

[Table 24]

| Compound name | Structure formula |
|---|---|
| CC-04 | 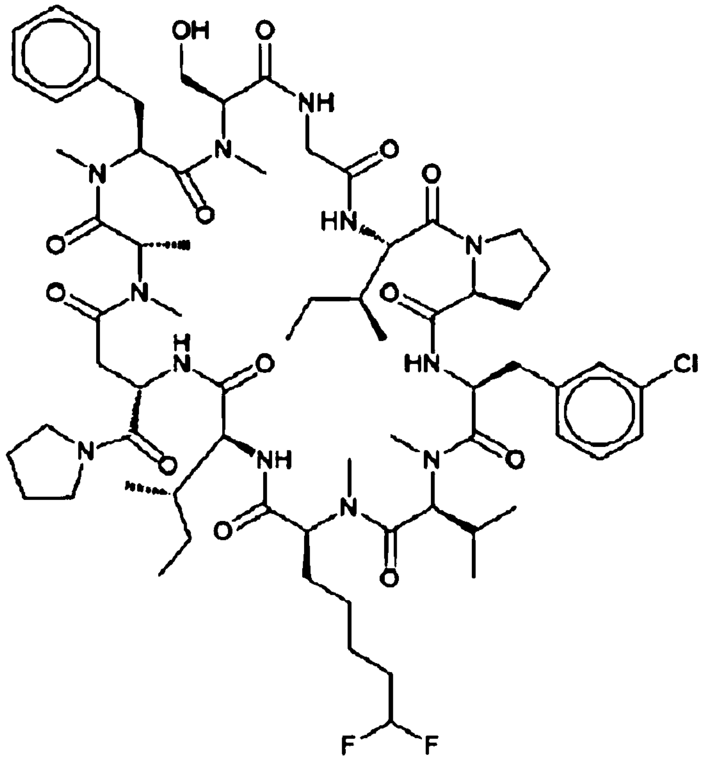 |
| CC-05 | 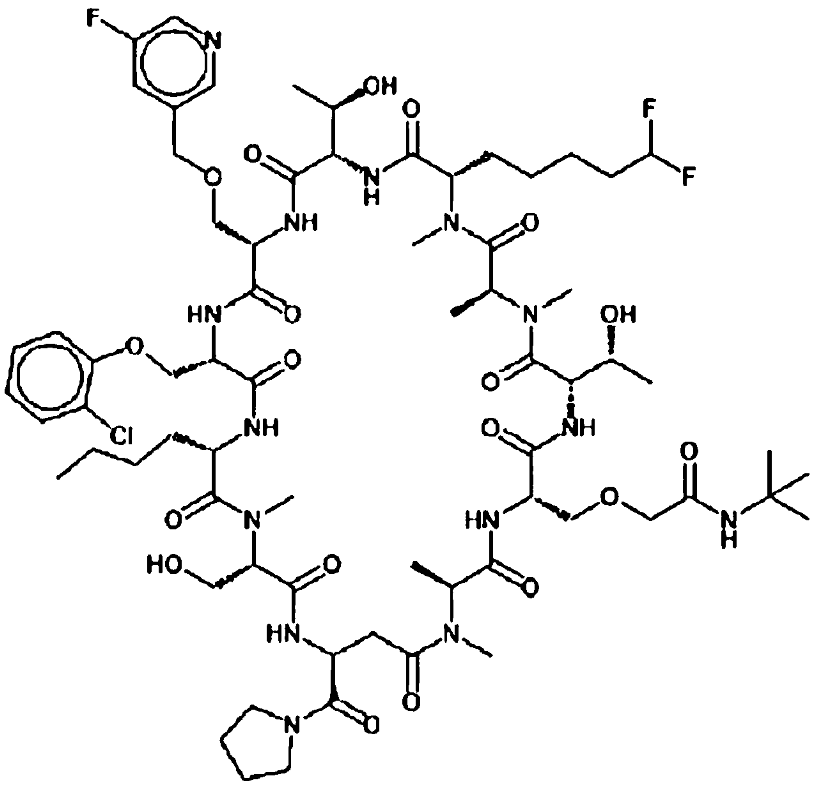 |

[Table 25]

| Compound name | Structure formula |
|---|---|
| CC-06 | 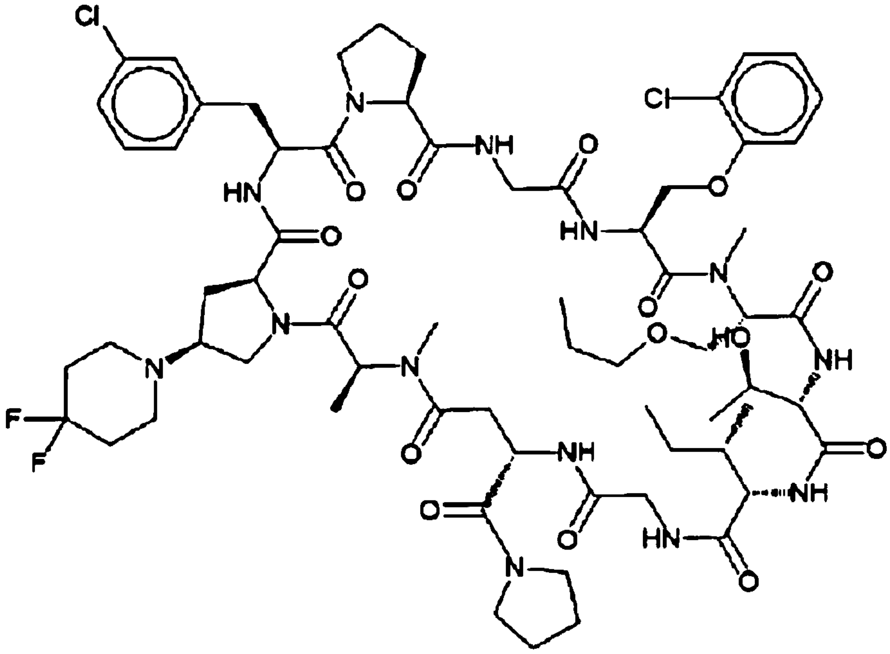 |
| CC-07 | 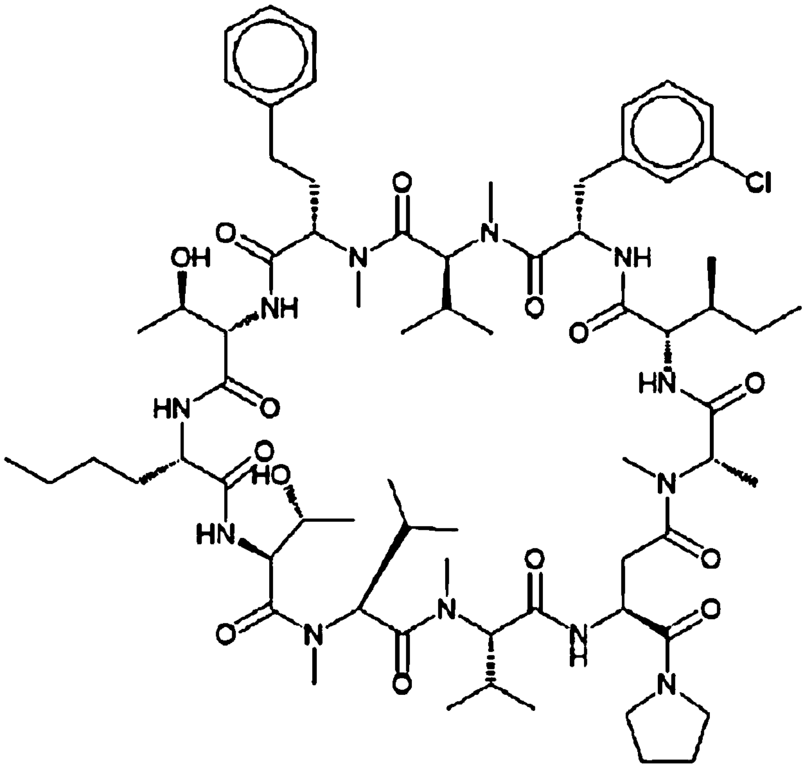 |

[Table 26]

| Compound name | Structure formula |
|---|---|
| CF-01 | 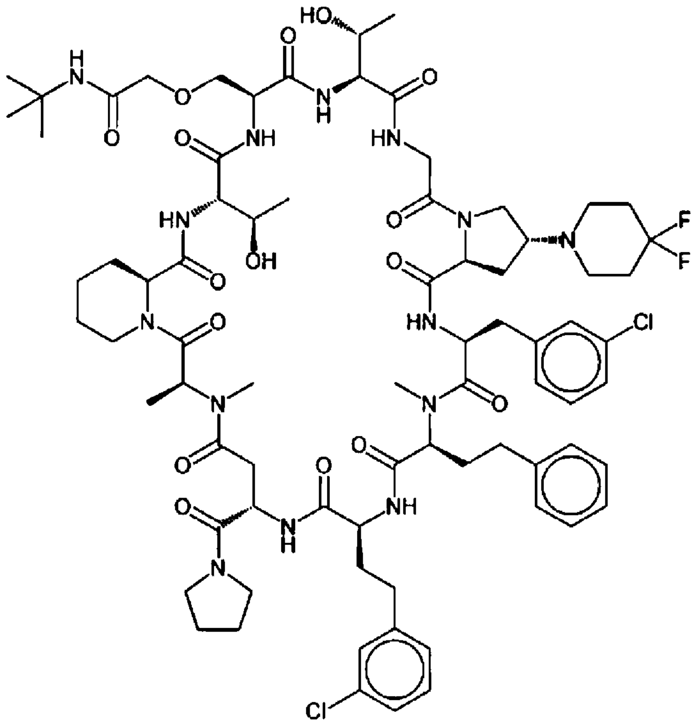 |
| CF-02 | 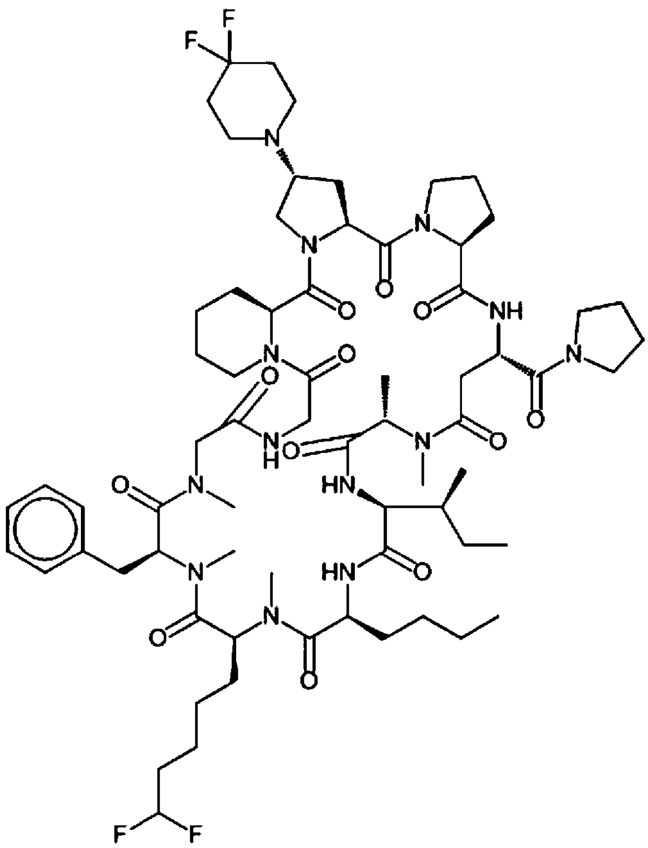 |

[Experimental Example 2: effect verification of method using fusion protein fused with plurality of kinds of target proteins for panning]

To improve the efficiency of obtaining a peptide forming a complex with a plurality of kinds of proteins, the effect of forming a fusion protein fused with the plurality of kinds of target proteins and making the plurality of kinds of target proteins be in proximity was verified.

Specifically, it was verified whether an improvement effect of a recovery rate of a peptide forming a complex with two kinds of target proteins can be obtained when a fusion protein in which the two kinds of target proteins are fused is used to make the both target proteins be in proximity.

[Evaluation Example 1: evaluation of synthetic peptide utilizing surface plasmon resonance (SPR)]

<Evaluation Example 1-1: evaluation of peptide forming complex with FKBP and FRB>

Whether or not the peptide obtained by panning is capable of forming a complex with FKBP and FRB was evaluated by SPR. The measurement was performed at 20°C with BiacoreT200 (Cytiva).

Specifically, Biotin CAPture reagent was immobilized in all flow cells on Biacore sensor chip Series S sensor chip CAP (Cytiva), and then FRB-FLAG-TEV-Avi-Bio was immobilized in some flow cells. As an analyte, a mixed solution of a dilution series of FKBP-FLAG-Sor-His at a maximum concentration of 10 μM and a synthetic peptide at 10 μM was prepared, and added to all flow cells to obtain a sensorgram of the binding. As a running buffer, a solution in which dimethylsulfoxide (DMSO) was added at the final concentration of 4% to HBS (NACALAI TESQUE, INC.), 1 mM Dithiothreitol, and 0.01% Tween 20 was used.

Analysis of the obtained sensorgram was performed with T200 evaluation software (Cytiva). The sensorgram obtained in flow cells where FRB-FLAG-TEV-Avi-Bio was not immobilized and the sensorgram obtained when the running buffer was added were subtracted as a double reference, and DMSO solvent correction was performed. A binding rate constant ($k_a$), a dissociation rate constant ($k_d$), and a dissociation constant $K_D$ were determined from curve fitting using a 1 : 1 binding model for the sensorgram that was processed as described above. In some peptides, $K_D$ was determined by equilibrium value analysis. The $K_D$ obtained by the above, the apparent dissociation constant of FRB and FKBP binding in the presence of 10 μM peptide, was considered as an indicator that the peptide induces complex formation with the two proteins. Note that no binding response was observed when 10 μM FKBP-FLAG-Sor-His solution was added as an analyte.

The obtained $K_D$ is shown below.

[Table 27]

| Compound name | $K_D$ [mol/L] |
|---|---|
| FF-01 | 1.3E-06 |
| FF-02 | 5.5E-07 |
| FF-03 | 5.2E-07 |
| FF-04 | 7.1E-07 |
| FF-05 | 3.0E-06 |
| FF-06 | 1.2E-06 |
| FF-07 | 6.1E-06 |

As a result of the above, it was confirmed that a plurality of peptides that induce complex formation with FKBP and FRB were obtained by panning.

<Evaluation Example 1-2: evaluation of peptides forming complex with cyclophilin A and calcineurin>

Whether or not the peptide obtained by panning is capable of forming a complex with cyclophilin A and calcineurin was evaluated by SPR. The measurement was performed at 20°C with Biacore T200 (Cytiva).

Specifically, Biotin CAPture reagent was immobilized in all flow cells on Biacore sensor chip Series S sensor chip CAP (Cytiva), and then CnB-CnA-FLAG-TEV-Avi-bio was immobilized in some flow cells. As an analyte, a mixed solution of a dilution series of CyA-FLAG-Sor-His at a maximum concentration of 10 μM and a synthetic peptide at 10 μM was prepared, and added to flow cells after addition of 1 μM Calmodulin to obtain a sensorgram of the binding. As a running

buffer, a solution in which DMSO was added at the final concentration of 4% to HBS (NACALAI TESQUE, INC.), 1 mM Dithiothreitol, 5 mM $CaCl_2$, and 0.01% Tween 20 was used.

Analysis of the obtained sensorgram was performed with T200 evaluation software (Cytiva). The sensorgram obtained in flow cells where CnB-CnA-FLAG-TEV-Avi-bio was not immobilized and the sensorgram obtained when the running buffer was added were subtracted as a double reference, and DMSO solvent correction was performed. A binding rate constant ($k_a$), a dissociation rate constant ($k_d$), and a dissociation constant $K_D$ were determined from curve fitting using a 1 : 1 binding model for the sensorgram that was processed as described above. In some peptides, $K_D$ was determined by equilibrium value analysis. The $K_D$ obtained by the above, the apparent dissociation constant of cyclophilin A and calcineurin binding in the presence of 10 μM peptide, was considered as an indicator that the peptide induces complex formation with the two proteins. It should be noted that the binding of cyclophilin A and calcineurin in the presence of 10 μM peptide was also confirmed under the condition in which 1 μM Calmodulin was not added. However, no binding response was observed when 10 μM CyA-FLAG-Sor-His solution was added as an analyte.

The obtained $K_D$ is shown below.

[Table 28]

| Compound name | $K_D$ [mol/L] |
|---|---|
| CC-01 | 3.7E-07 |
| CC-02 | 5.7E-07 |
| CC-03 | 4.0E-06 |
| CC-04 | 2.1E-06 |
| CC-05 | 6.4E-07 |
| CC-06 | 5.4E-06 |
| CC-07 | 1.9E-06 |

As a result of the above, it was confirmed that a plurality of peptides that induce complex formation with cyclophilin A and calcineurin were obtained by panning.

<Evaluation Example 1-3: evaluation of peptides forming complex with FKBP and calcineurin>

Whether or not the peptide obtained by panning is capable of forming a complex with FKBP and calcineurin was evaluated by SPR. The measurement was performed at 20°C with Biacore T200 (Cytiva).

Specifically, CnB-CnA-FLAG-TEV-Avi-bio was immobilized in some flow cells on Biacore sensor chip Series S sensor chip SA (Cytiva), and then 10 μM of biotin solution was poured into all flow cells. As an analyte, a dilution series FKBP-FLAG-Sor-His at a maximum concentration of 20 μM, containing a synthetic peptide at 10 μM at a constant concentration was prepared, and added to flow cells after addition of 1 μM Calmodulin to obtain a sensorgram of the binding. As a running buffer, a solution in which DMSO was added at the final concentration of 4% to HBS (NACALAI TESQUE, INC.), 1 mM Dithiothreitol, 5 mM $CaCl_2$, and 0.01% Tween 20 was used.

Analysis of the obtained sensorgram was performed with Biacore T200 evaluation software (Cytiva). The sensorgram obtained in flow cells where CnB-CnA-FLAG-TEV-Avi-bio was not immobilized and the sensorgram obtained when the running buffer was added were subtracted as a double reference, and DMSO solvent correction was performed. A binding rate constant ($k_a$), a dissociation rate constant ($k_d$), and a dissociation constant $K_D$ were determined from curve fitting using a 1 : 1 binding model for the sensorgram that was processed as described above. The $K_D$ obtained by the above, the apparent dissociation constant of FKBP and calcineurin in the presence of 10 μM peptide, was considered as an indicator that the peptide induces complex formation with the two proteins. It should be noted that the binding of FKBP and calcineurin in the presence of 10 μM peptide was also confirmed under the condition in which 1 μM Calmodulin was not added. However, no binding response was observed when 20 μM FKBP-FLAG-Sor-His solution was added as an analyte.

The obtained $K_D$ is shown below.

[Table 29]

| Compound name | $K_D$ [mol/L] |
|---|---|
| CF-01 | 1.2E-06 |
| CF-02 | 1.8E-06 |

[Evaluation Example 2: verification using fusion protein]

<Evaluation Example 2-1: verification with peptide with FKBP and FRB as target proteins>

To consider whether or not the peptide forming a ternary complex with FKBP and FRB obtained by the panning experiment of Example 1-1 could be obtained by using a fusion protein, verification was performed by comparing the pull-down efficiency of the mRNA display molecule of each peptide synthesized in Synthesis Example 1. Specifically, a display molecule in which mRNA and a peptide were linked was prepared using peptides FF-01, FF-02, FF-03, and FF-04, and the recovery rate of the display molecule of each peptide when pulled down under various conditions was verified.

(Preparation Example 2-1-1: preparation of mRNA (FF-01 to FF-04))

From template DNA ((D-1) to (D-4)), mRNA ((mR-1) to (mR-4)) was synthesized by in vitro transcription reaction using T7 RNA polymerase and purified by RNAClean XP (Beckman Coulter, Inc.).

Nucleotide sequence of template DNA (D-1) (SEQ ID NO: 15) (DNA sequence: FF-01)

GGCGTAATACGACTCACTATAGGGTTAACTTTAATAAGGAGATATAAATATGCATTC
GGGTGAGTGCCATACTAGTTCTTAGCCGACCGGCACCGGCACCGGCAAAAAAA

Nucleotide sequence of template DNA (D-2) (SEQ ID NO: 16) (DNA sequence: FF-02)

GGCGTAATACGACTCACTATAGGGTTAACTTTAATAAGGAGATATAAATATGTTTTC
GGAGTGCACTAACCAGCCGTTGTAGCCGACCGGCACCGGCACCGGCAAAAAAA

Nucleotide sequence of template DNA (D-3) (SEQ ID NO: 17) (DNA sequence: FF-03)

GGCGTAATACGACTCACTATAGGGTTAACTTTAATAAGGAGATATAAATATGTCTGG
TCGTCATCTGTGCCATATTAGGTAGCCGACCGGCACCGGCACCGGCAAAAAAA

Nucleotide sequence of template DNA (D-4) (SEQ ID NO: 18) (DNA sequence: FF-04)

GGCGTAATACGACTCACTATAGGGTTAACTTTAATAAGGAGATATAAATATGTGGGT
TTTGCTGTGCTCTACTGGTGGTTAGCCGACCGGCACCGGCACCGGCAAAAAAA

Nucleotide sequence of mRNA (mR-1) (SEQ ID NO: 19) (RNA sequence: FF-01)

GGGUUAACUUUAAUAAGGAGAUAUAAAUAUGCAUUCGGGUGAGUGCCAUACUAG
UUCUUAGCCGACCGGCACCGGCACCGGCAAAAAAA

Nucleotide sequence of mRNA (mR-2) (SEQ ID NO: 20) (RNA sequence: FF-02)

GGGUUAACUUUAAUAAGGAGAUAUAAAUAUGUUUUCGGAGUGCACUAACCAGCC
GUUGUAGCCGACCGGCACCGGCACCGGCAAAAAAA

Nucleotide sequence of mRNA (mR-3) (SEQ ID NO: 21) (RNA sequence: FF-03)

GGGUUAACUUUAAUAAGGAGAUAUAAAUAUGUCUGGUCGUCAUCUGUGCCAUAU
UAGGUAGCCGACCGGCACCGGCACCGGCAAAAAAA

Nucleotide sequence of mRNA (mR-4) (SEQ ID NO: 22) (RNA sequence: FF-04)

GGGUUAACUUUAAUAAGGAGAUAUAAAUAUGUGGGUUUUGCUGUGCUCUACUGG
UGGUUAGCCGACCGGCACCGGCACCGGCAAAAAAA

(Preparation Example 2-1-2: preparation of mRNA-peptide display molecules (FF-01 to FF-04))

Puromycin was linked via a linker to the 3' end of mRNA purified in Preparation Example 2-1-1 according to the method described in WO2013/100132. It is noted that axPM-27 described above was used as a puromycin linker. This was translated, and subjected to a cyclization reaction, a desulfurization reaction, and a reverse transcription according to the method described in WO2017/150732 to prepare a display molecule of mRNA-peptide.

<Preparation Example 2-1-2-1: preparation of aminoacylated tRNA (FF-01 to FF-04)>

The aminoacylated tRNA used for translation was prepared in the same way as in Preparation Example 4-1 of Experimental Example 1.

<Preparation Example 2-1-2-2: translation of mRNA-peptide display molecules (FF-01 to FF-04)>

For the translation system, PURE system, a reconstituted cell-free protein synthesis system from a prokaryotic organism, was used. Specifically, the following components were added to the translation reaction mixture to achieve their respective final concentrations: 1 mM GTP, 1 mM ATP, 20 mM phosphocreatine, 50 mM HEPES-KOH pH 7.6, 100 mM potassium acetate, 5 mM magnesium acetate, 2 mM spermidine, 1 mM dithiothreitol, 1 mg/ml E. coli MRE 600 (RNase-negative) derived tRNA (F. Hoffmann-La Roche, Ltd.) (some tRNAs were removed by the method described in Non Patent Literature: Yokogawa T, et al. 2010), 3 μM in vitro transcriptional E. coli tRNA AlaIB, 4 μg/ml creatine kinase, 6.72 U/mL myokinase, 2 units/ml inorganic pyrophosphatase, 1.1 μg/ml nucleoside diphosphate kinase, 0.4 Unit/μL RNasein (R) Plus Ribonuclease Inhibitor (Promega Corporation), 0.26 μM EF-G, 2.7 μM IF1, 0.4 μM IF2, 1.5 μM IF3, 40 μM EF-Tu, 60 μM EF-Ts, 1 μM EF-P-Lys, 1.2 μM modified ribosome (WO2021/117848), 1.37 μM AlaRS, 1 μM GlyRS, 0.04 μM IleRS, 0.11 μM LysRS, 0.16 μM ProRS, 0.09 μM ThrRS, 0.5 μM variant PheRS (WO2016/148044), 1 μM variant SerRS (WO2016/148044), 1 μM variant ValRS (WO2016/148044), 250 μM glycine, 10 μM isoleucine, 250 μM lysine, 250 μM proline, 250 μM threonine, 2.5 mM N-methylalanine, 5 mM N-methylvaline, 5 mM N-methylserine, 5 mM N-methylphenylalanine, Elongator aminoacylated tRNA mixture prepared in Preparation Example 2-1-2-1, 25 μM Initiator aminoacylated tRNA (WO2017/150732) prepared in Preparation Example 2-1-2, 0.5 μM Penicillin G Amidase (PGA), and 1 μM mRNA-puromycin linked construct prepared in Preparation Example 2-1-2, followed by incubation at 37°C for 1 hour to perform translation.

(Evaluation Example 2-1-1: pull-down assay of mRNA-peptide display molecule (FF-01 to FF-04))

To a buffer containing 1 × TBS, 2 mg/mL BSA (Invitrogen), and 2 mM DTT, the display molecules prepared in Preparation Example 2-1-2 was added to be 0.3 μM, and the target protein was added to be as shown in Table 30, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.08 mM, and the mixture was mixed by inversion at 4°C for another 10 minutes. The proteins prepared in Preparation Examples 1-1 to 1-5 were used as the target proteins, and those in six conditions shown in the following table were compared.

[Table 30]

| Condition | Target protein |
|---|---|
| **1** | **0.4 μM bio-Avi-TEV-FLAG-FKBP-FRB-His** |
| **2** | **0.4 μM FKBP-FLAG-TEV-Avi-bio** |
| **3** | **0.4 μM FKBP-FLAG-TEV-Avi-bio and 0.4 μM FRB-FLAG-Sor-His** |
| **4** | **0.4 μM FRB-FLAG-TEV-Avi-bio** |
| **5** | **0.4 μM FRB-FLAG-TEV-Avi-bio and 0.4 μM FKBP-FLAG-Sor-His** |
| **6** | No target protein |

Condition 1 is a condition in which a fusion protein is used, and the two kinds of target proteins are in proximity. Conditions 2 and 4 are conditions in which only FKBP or only FRB is present in the system. Conditions 3 and 5 are

conditions in which FKBP and FRB are present, but the target protein pulled down with streptavidin-coated magnetic beads is FKBP or FRB, respectively, and the two kinds of target proteins are not in proximity. Condition 6 is a condition under the absence of the target protein.

Streptavidin-coated magnetic beads with immobilized target proteins were collected with a magnet and the supernatant was removed. The collected beads were washed several times with a wash buffer (1 × TBS, 2 mM DTT, 0.1% Tween 20 (Sigma Aldrich), 500 mM arginine), then treated with a TEV protease that recognizes and cleaves the TEV protease recognition sequence to elute the target protein and the peptide-nucleic acid conjugate that binds to the target protein.

To the streptavidin-coated magnetic beads after washing, 1 × TEV buffer, 1 mM DTT, and 0.1 U/μL AcTEV protease (Thermo Fisher Scientific Inc., #12575015) were added as a specific TEV eluent to react with. After the reaction, the supernatant was recovered and qPCR was performed. For the qPCR primer, the aforementioned primers (Fw-Primer and Rv-Primer) were used. For the qPCR reaction solution, 1 × Ex Taq buffer, 0.2 mM dNTP, 0.5 μM Fw-Primer, 0.5 μM Rv-Primer, 50000-fold diluted SYBR Green I (LONZA, #50513), and 0.025 U/μL Ex Taq polymerase (Takara Bio Inc.) were mixed. The mixture was heated at 95°C for 2 minutes, and then subjected to 40 cycles of 95°C for 10 seconds, 57°C for 20 seconds, and 72°C for 30 seconds. In this case, each display molecule before mixing with the target protein was also subjected to qPCR in the same way.

To draw a calibration curve to estimate the recovery rate of the library, a sample prepared by reverse transcription of the mRNA-puromycin linked construct prepared in Preparation Example 4-3 of Experimental Example 1 was diluted to be 1E + 8/μL, 1E + 6/μL, and 1E + 4/μL, and subjected to qPCR under the same conditions.

The recovery rate was calculated by dividing the amount of nucleic acid of each display molecule recovered by binding to a target protein by the amount of nucleic acid of each display molecule that was present before mixing with the target protein (input value).

Recovery rate (%) = amount (number of molecules) of recovered nucleic acid /amount (number of molecules) of input nucleic acid × 100

The recovery rates of the display molecules FF-01, FF-02, FF-03, and FF-04 and the ratio obtained by dividing the recovery rate in condition 1 by the respective recovery rates in conditions 2 to 6 were summarized in Table 31, Table 32, Table 33, and Table 34, respectively.

[Table 31]

| Peptide: FF-01 | | |
|---|---|---|
| Condition | Recovery rate (%) | Recovery rate in condition 1/recovery rate |
| **1** | **1.15E+01** | **-** |
| **2** | **1.19E-02** | **963** |
| **3** | **1.02E-02** | **1120** |
| **4** | **4.46E-04** | **25691** |
| **5** | **3.77E-04** | **30450** |
| **6** | **7.11E-05** | **161292** |

[Table 32]

| Peptide: FF-02 | | |
|---|---|---|
| Condition | Recovery rate (%) | Recovery rate in condition 1/recovery rate |
| **1** | **1.79E+00** | **-** |
| **2** | **1.06E-04** | **16961** |
| **3** | **4.87E-05** | **36771** |
| **4** | **6.51E-05** | **27532** |
| **5** | **9.80E-05** | **18290** |
| **6** | **3.96E-05** | **45250** |

[Table 33]

| Peptide: FF-03 | | |
|---|---|---|
| Condition | Recovery rate (%) | Recovery rate in condition 1/recovery rate |
| **1** | **9.32E+00** | **-** |
| **2** | **1.49E-04** | **62390** |
| **3** | **2.68E-04** | **34754** |
| **4** | **7.71E-04** | **12078** |
| **5** | **7.06E-04** | **13191** |
| **6** | **8.16E-05** | **114154** |

[Table 34]

| Peptide: FF-04 | | |
|---|---|---|
| Condition | Recovery rate (%) | Recovery rate in condition 1/recovery rate |
| **1** | **7.11E+00** | **-** |
| **2** | **1.23E-04** | **57854** |
| **3** | **1.38E-04** | **51333** |
| **4** | **2.89E-04** | **24591** |
| **5** | **4.75E-04** | **14961** |
| **6** | **4.38E-05** | **162319** |

As a result, it was revealed that in these four peptides (FF-01 to FF-04) the recovery rate in condition 1, where two kinds of target proteins, FKBP and FRB, were in proximity to each other by the fusion protein prepared in Preparation Examples 1-5, was at least 1100-fold higher than the recovery rates in conditions 3 and 5 where two respective target proteins were added separately. This suggests that the peptides forming a ternary complex with FKBP and FRB can be pulled down efficiently by making the two kinds of target proteins be in proximity, which strongly suggests that these peptides could be obtained by panning using the fusion protein.

In addition, the results show a molecule of interest capable of forming a complex with a plurality of kinds of target molecules can be identified by the method of counter-assay shown in Example 2. That is, it shows that a peptide capable of forming a complex with a plurality of kinds of target proteins can be screened based on the difference in pull-down efficiency between when two kinds of target proteins are present in the system in proximity and when only one kind of target protein is present in the system as an indicator. For example, in this example, a method of comparing the recovery rate of the display molecule in condition 1, which is a condition in which a plurality of kinds of target molecules are in proximity, with the recovery rate of the display molecule in conditions 2 and 4, which are conditions in which only a single target molecule is present, has been shown to be superior in sensitivity to identify a peptide that is a molecule of interest, compared to an existing method of comparing the recovery rate of the display molecule in condition 3 with the recovery rate of the display molecule in condition 2, or the recovery rate of the display molecule in condition 5 with the recovery rate of the display molecule in condition 4.

In this example, a counter-assay was performed on a display library prepared after concentration by panning to efficiently identify the molecule of interest from a highly diverse library. However, since the recovery rate of each display molecule under each condition is individually confirmed and compared in the counter assay, it is possible to identify the molecule of interest without panning by performing a counter assay on the display library in the case where a less diverse library is used.

<Evaluation Example 2-2: verification with peptide with cyclophilin A and calcineurin as target proteins>

To consider whether or not the peptides forming a ternary complex with cyclophilin A and calcineurin obtained by the panning experiment of Example 1-2 could be obtained by using a fusion protein, verification was performed by comparing the pull-down efficiency (hereinafter referred to as a recovery rate) of the mRNA display molecule of each peptide synthesized in Synthesis Example 1. Specifically, a display molecule in which mRNA and a peptide were linked was prepared using peptides CC-01, CC-02, CC-03, and CC-04, and the recovery rate of each peptide under various

conditions was verified.

(Preparation Example 2-2-1: preparation of mRNA (CC-01 to CC-04))

From template DNA ((D-5) to (D-8)), mRNA ((mR-5) to (mR-8)) was synthesized by in vitro transcription reaction using T7 RNA polymerase and purified by RNAClean XP (Beckman Coulter, Inc.).

Nucleotide sequence of template DNA (D-5) (SEQ ID NO: 23) (DNA sequence: CC-01)

GGCGTAATACGACTCACTATAGGGTTAACTTTAATAAGGAGATATAAATATGCGTCA
GCTGATTCGGTGGGTTGTGACTTAGCCGACCGGCACCGGCACCGGCAAAAAAA

Nucleotide sequence of template DNA (D-6) (SEQ ID NO: 24) (DNA sequence: CC-02)

GGCGTAATACGACTCACTATAGGGTTAACTTTAATAAGGAGATATAAATATGTGGTT
TGATAACTCGAACGTGGTGACTTAGCCGACCGGCACCGGCACCGGCAAAAAAA

Nucleotide sequence of template DNA (D-7) (SEQ ID NO: 25) (DNA sequence: CC-03)

GGCGTAATACGACTCACTATAGGGTTAACTTTAATAAGGAGATATAAATATGATGCC
GAGGAACGTTCTTACTACTCAGTAGCCGACCGGCACCGGCACCGGCAAAAAAA

Nucleotide sequence of template DNA (D-8) (SEQ ID NO: 26) (DNA sequence: CC-04)

GGCGTAATACGACTCACTATAGGGTTAACTTTAATAAGGAGATATAAATATGTTTAG
TGGTATTCCGTGGGTTGTGATTTAGCCGACCGGCACCGGCACCGGCAAAAAAA

Nucleotide sequence of mRNA (mR-5) (SEQ ID NO: 27) (RNA sequence: CC-01)

GGGUUAACUUUAAUAAGGAGAUAUAAAUAUGCGUCAGCUGAUUCGGUGGGUUGU
GACUUAGCCGACCGGCACCGGCACCGGCAAAAAAA

Nucleotide sequence of mRNA (mR-6) (SEQ ID NO: 28) (RNA sequence: CC-02)

GGGUUAACUUUAAUAAGGAGAUAUAAAUAUGUGGUUUGAUAACUCGAACGUGGU
GACUUAGCCGACCGGCACCGGCACCGGCAAAAAAA

Nucleotide sequence of mRNA (mR-7) (SEQ ID NO: 29) (RNA sequence: CC-03)

GGGUUAACUUUAAUAAGGAGAUAUAAAUAUGAUGCCGAGGAACGUUCUUACUAC
UCAGUAGCCGACCGGCACCGGCACCGGCAAAAAAA

Nucleotide sequence of mRNA (mR-8) (SEQ ID NO: 30) (RNA sequence: CC-04)

GGGUUAACUUUAAUAAGGAGAUAUAAAUAUGUUUAGUGGUAUUCCGUGGGUUGU
GAUUUAGCCGACCGGCACCGGCACCGGCAAAAAAA

(Preparation Example 2-2-2: preparation of mRNA-peptide display molecules (CC-01 to CC-04))

Puromycin was linked via a linker to the 3' end of mRNA purified in Preparation Example 2-2-1 according to the method described in WO2013/100132. It is noted that axPM-27 described above was used as a puromycin linker. This was translated, and subjected to a cyclization reaction, a desulfurization reaction, and a reverse transcription according to the method described in WO2017/150732 to prepare a display molecule of mRNA-peptide. As the translation system and the

aminoacylated tRNA required for translation, those used in Preparation Example 2-1-2 of Experimental Example 2 were used.

(Evaluation Example 2-2-1: pull-down assay of mRNA display molecule (CC-01 to CC-04))

To a buffer containing 1 × TBS, 2 mg/mL BSA (Invitrogen), 2 mM DTT, and 5 mM $CaCl_2$, the display molecule prepared in Preparation Example 2-2-2 was added to be 0.3 μM, Calmodulin (FUJIFILM Wako Pure Chemical Corporation) was added to be 2 μM and the target protein was added to be as shown in Table 35, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.08 mM, and the mixture was mixed by inversion at 4°C for another 10 minutes. The target proteins prepared in Preparation Examples 2-1 to 2-5 were used as the target proteins, and those in six conditions shown in the following table were compared.

[Table 35]

| Condition | Target protein |
|---|---|
| **1** | **0.4 μM His-CnB-CnA-CyA-FLAG-TEV-Avi-bio** |
| **2** | **0.4 μM CyA-FLAG-TEV-Avi-bio** |
| **3** | **0.4 μM CyA-FLAG-TEV-Avi-bio and 0.4 μM CnB-CnA-FLAG-Sor-His** |
| **4** | **0.4 μM CnB-CnA-FLAG-TEV-Avi-bio** |
| **5** | **0.4 μM CnB-CnA-FLAG-TEV-Avi-bio and 0.4 μM CyA-FLAG-Sor-His** |
| **6** | No target protein |

Condition 1 is a condition in which a fusion protein is used, and the two kinds of target proteins are in proximity. Conditions 2 and 4 are conditions in which only cyclophilin A or only calcineurin is present in the system. Conditions 3 and 5 are conditions in which cyclophilin A and calcineurin are present, but the target protein pulled down with streptavidin-coated magnetic beads is cyclophilin A or calcineurin, respectively, and the two kinds of target proteins are not in proximity. Condition 6 is a condition under the absence of the target protein.

Streptavidin-coated magnetic beads with immobilized target proteins were collected with a magnet and the supernatant was removed. The collected beads were washed several times with a wash buffer (1 × TBS, 2 mM DTT, 0.1% Tween 20 (Sigma Aldrich), 500 mM arginine), then treated with a TEV protease that recognizes and cleaves the TEV protease recognition sequence to elute the target protein and the peptide-nucleic acid conjugate that binds to the target protein.

To the streptavidin-coated magnetic beads after washing, 1 × TEV buffer, 1 mM DTT, and 0.1 U/μL AcTEV protease (Thermo Fisher Scientific Inc., #12575015) were added as a specific TEV eluent to react with. After the reaction, the supernatant was recovered and qPCR was performed. For the qPCR primer, the aforementioned primers (Fw-Primer and Rv-Primer) were used. For the qPCR reaction solution, 1 × Ex Taq buffer, 0.2 mM dNTP, 0.5 μM Fw-Primer, 0.5 μM Rv-Primer, 50000-fold diluted SYBR Green I (LONZA, #50513), and 0.025 U/μL Ex Taq polymerase (Takara Bio Inc.) were mixed. The mixture was heated at 95°C for 2 minutes, and then subjected to 40 cycles of 95°C for 10 seconds, 57°C for 20 seconds, and 72°C for 30 seconds. In this case, each display molecule before mixing with the target protein was also subjected to qPCR in the same way.

To draw a calibration curve to estimate the recovery rate of the library, a sample prepared by reverse transcription of the mRNA-puromycin linked construct prepared in Preparation Example 4-3 of Experimental Example 1 was diluted to be 1E + 8/μL, 1E + 6/μL, and 1E + 4/μL, and subjected to qPCR under the same conditions.

The recovery rate was calculated by dividing the amount of nucleic acid of each display molecule recovered by binding to a target protein by the amount of nucleic acid of each display molecule that was present before mixing with the target protein (input value).

Recovery rate (%) = amount (number of molecules) of recovered nucleic acid /amount (number of molecules) of input nucleic acid × 100

The recovery rates of the display molecules CC-01, CC-02, CC-03, and CC-04 and the ratio obtained by dividing the recovery rate in condition 1 by the respective recovery rates in conditions 2 to 6 were summarized in Table 36, Table 37, Table 38, and Table 39, respectively.

[Table 36]

| Peptide: CC-01 | | |
|---|---|---|
| Condition | Recovery rate (%) | Recovery rate in condition 1/ recovery rate |
| 1 | 1.09E+00 | - |
| 2 | 2.91E-04 | 3745 |
| 3 | 1.99E-02 | 55 |
| 4 | 8.14E-04 | 1338 |
| 5 | 2.26E-02 | 48 |
| 6 | 5.72E-05 | 19031 |

[Table 37]

| Peptide: CC-02 | | |
|---|---|---|
| Condition | Recovery rate (%) | Recovery rate in condition 1/ recovery rate |
| 1 | 2.73E+00 | - |
| 2 | 5.53E-04 | 4944 |
| 3 | 1.07E-02 | 255 |
| 4 | 2.16E-03 | 1267 |
| 5 | 1.61E-02 | 170 |
| 6 | 3.54E-04 | 7730 |

[Table 38]

| Peptide: CC-03 | | |
|---|---|---|
| Condition | Recovery rate (%) | Recovery rate in condition 1/ recovery rate |
| 1 | 1.22E+00 | - |
| 2 | 3.47E-04 | 3525 |
| 3 | 1.36E-03 | 897 |
| 4 | 1.07E-03 | 1145 |
| 5 | 1.28E-03 | 954 |
| 6 | 2.61E-05 | 46858 |

[Table 39]

| Peptide: CC-04 | | |
|---|---|---|
| Condition | Recovery rate (%) | Recovery rate in condition 1/ recovery rate |
| 1 | 1.92E+00 | - |
| 2 | 2.40E-03 | 800 |
| 3 | 2.06E-03 | 935 |
| 4 | 1.11E-03 | 1731 |
| 5 | 1.02E-03 | 1890 |
| 6 | 3.49E-04 | 5510 |

As a result, it was revealed that in these four peptides (CC-01 to CC-04) the recovery rate in condition 1, where two kinds of target proteins, cyclophilin A and calcineurin, were in proximity to each other by the fusion protein prepared in

Preparation Examples 2-5, was at least 40-fold higher than the recovery rates in conditions 3 and 5, where two respective target proteins were added separately. This suggests that the peptides forming a ternary complex with cyclophilin A and calcineurin can be pulled down efficiently by making the two kinds of target proteins be in proximity, which strongly suggests that these peptides could be first obtained by panning using the fusion protein.

In addition, the results show a molecule of interest capable of forming a complex with a plurality of kinds of target molecules can be identified by the method of counter-assay shown in Example 2. That is, it shows that a peptide capable of forming a complex with a plurality of kinds of target proteins can be screened based on the difference in pull-down efficiency between when two kinds of target proteins are present in the system in proximity and when only one kind of target protein is present in the system as an indicator. For example, in this example, a method of comparing the recovery rate of the display molecule in condition 1, which is a condition in which a plurality of kinds of target molecules are in proximity, with the recovery rate of the display molecule in conditions 2 and 4, which are conditions in which only a single target molecule is present, has been shown to be superior in sensitivity to identify a peptide that is a molecule of interest, compared to an existing method of comparing the recovery rate of the display molecule in condition 3 with the recovery rate of the display molecule in condition 2, or the recovery rate of the display molecule in condition 5 with the recovery rate of the display molecule in condition 4.

In this example, a counter-assay was performed on a display library prepared after concentration by panning to efficiently identify the molecule of interest from a highly diverse library. However, since the recovery rate of each display molecule under each condition is individually confirmed and compared in the counter assay, it is possible to identify the molecule of interest without panning by performing a counter assay on the display library in the case where a less diverse library is used.

<Evaluation Example 2-3: verification with peptide with FKBP and calcineurin as target proteins>

To consider whether or not the peptides forming a ternary complex with FKBP and calcineurin obtained by the panning experiment of Example 1-3 could be obtained by using a fusion protein, verification was performed by comparing the pull-down efficiency of the mRNA display molecule of each peptide synthesized in Synthesis Example 1. Specifically, a display molecule in which mRNA and a peptide were linked was prepared using peptides CF-01 and CF-02, and the recovery rate of the display molecule of each peptide when pulled down under various conditions was verified.

(Preparation Example 2-3-1: preparation of mRNA (CF-01, 02))

From template DNA ((D-9), (D-10)), mRNA ((mR-9), (mR-10)) was synthesized by in vitro transcription reaction using T7 RNA polymerase and purified by RNAClean XP (Beckman Coulter, Inc.).

Nucleotide sequence of template DNA (D-9) (SEQ ID NO: 51) (DNA sequence: CF-01)

GTAATACGACTCACTATAGGGTTAACTTTAATAAGGAGATATAAATATGTGCACTTC
TACTGGTTACTGGCTTCATTAGCCGACCGGCACCGGCACCGGCAAAAAAA

Nucleotide sequence of template DNA (D-10) (SEQ ID NO: 52) (DNA sequence: CF-02)

GTAATACGACTCACTATAGGGTTAACTTTAATAAGGAGATATAAATATGATTAACGT
GTTTTCGGGTTGCTACCCGTAGCCGACCGGCACCGGCACCGGCAAAAAAA

Nucleotide sequence of mRNA (mR-9) (SEQ ID NO: 53) (RNA sequence: CF-01)

GGGUUAACUUUAAUAAGGAGAUAUAAAUAUGUGCACUUCUACUGGUUACUGGCU
UCAUUAGCCGACCGGCACCGGCACCGGCAAAAAAA

Nucleotide sequence of mRNA (mR-10) (SEQ ID NO: 54) (RNA sequence: CF-02)

GGGUUAACUUUAAUAAGGAGAUAUAAAUAUGAUUAACGUGUUUUCGGGUUGCUA
CCCGUAGCCGACCGGCACCGGCACCGGCAAAAAAA

(Preparation Example 2-3-2: preparation of mRNA-peptide display molecules (CF-01, 02))

Puromycin was linked via a linker to the 3' end of mRNA purified in Preparation Example 2-3-1 according to the method described in WO2013/100132. It is noted that axPM-27 described above was used as a puromycin linker. This was translated, and subjected to a cyclization reaction, a desulfurization reaction, and a reverse transcription according to the method described in WO2017/150732 to prepare a display molecule of mRNA-peptide. As the translation system and the aminoacylated tRNA required for translation, those used in Example 1-3 of Experimental Example 1 were used.

(Evaluation Example 2-3-1: pull-down assay of mRNA display molecule (CF-01, 02))

Biotinylated target proteins were added to a buffer containing 1 × TBS, 2 mg/mL BSA (Invitrogen), 2 mM DTT, and 5 mM $CaCl_2$ as shown in Table 40, and then mixed with streptavidin-coated magnetic beads, and the mixture was mixed by inversion at 4°C for 10 minutes to immobilize the target proteins. Biotin was then added to be 0.5 mM, and the mixture was mixed by inversion at 4°C for another 10 minutes. The target proteins prepared in Preparation Examples 1-1, 1-3, 2-2, 2-4, and 3-4 were used as the target proteins, and those in six conditions shown in the following table were compared.

[Table 40]

| Condition | Target protein |
|---|---|
| **1** | **0.4 μM CnB-CnA-FLAG-FKBP-FLAG-TEV-Avi-bio** |
| **2** | **0.4 μM FKBP-FLAG-TEV-Avi-bio** |
| **3** | **0.4 μM FKBP-FLAG-TEV-Avi-bio and 0.4 μM CnB-CnA-FLAG-Sor-His** |
| **4** | **0.4 μM CnB-CnA-FLAG-TEV-Avi-bio** |
| **5** | **0.4 μM CnB-CnA-FLAG-TEV-Avi-bio and 0.4 μM FKBP-FLAG-Sor-His** |
| **6** | No target protein |

Condition 1 is a condition in which a fusion protein is used, and the two kinds of target proteins are in proximity. Conditions 2 and 4 are conditions in which only FKBP or only calcineurin is present in the system. Conditions 3 and 5 are conditions in which FKBP and calcineurin are present, but the target protein pulled down with streptavidin-coated magnetic beads is FKBP or calcineurin, respectively, and the two kinds of target proteins are not in proximity. Condition 6 is a condition under the absence of the target protein.

The streptavidin-coated magnetic beads with immobilized target proteins were collected with a magnet and the supernatant was removed, and the beads were once washed with a wash buffer (1 × TBS, 2 mM DTT, 0.1% Tween 20 (Sigma Aldrich), 500 mM arginine), and then the display library prepared in Preparation Example 2-3-2 was added to be 0.2-0.3 μM, Calmodulin (Fujifilm Wako Pure Chemical) to be 2 μM, and non-biotinylated proteins, when added, to be the concentrations as shown in Table 40, then the mixture was mixed by inversion at 4°C for 1 hour.

The streptavidin-coated magnetic beads mixed with the display library were collected with a magnet and the supernatant was removed, and the beads were washed several times with the wash buffer described above, then treated with a TEV protease that recognizes and cleaves the TEV protease recognition sequence to elute the target protein and the peptide-nucleic acid conjugate that binds to the target protein.

To the streptavidin-coated magnetic beads after washing, 1 × TEV buffer, 1 mM DTT, and 0.1 U/μL AcTEV protease (Thermo Fisher Scientific Inc., #12575015) were added as a specific TEV eluent to react with. After the reaction, the supernatant was recovered and qPCR was performed. For the qPCR primer, the aforementioned primers (Fw-Primer and Rv-Primer) were used. For the qPCR reaction solution, 1 × Ex Taq buffer, 0.2 mM dNTP, 0.5 μM Fw-Primer, 0.5 μM Rv-Primer, 50000-fold diluted SYBR Green I (LONZA, #50513), and 0.025 U/μL Ex Taq polymerase (Takara Bio Inc.) were mixed. The mixture was heated at 95°C for 2 minutes, and then subjected to 40 cycles of 95°C for 10 seconds, 57°C for 20 seconds, and 72°C for 30 seconds. In this case, each display molecule before mixing with the target protein was also subjected to qPCR in the same way.

To draw a calibration curve to estimate the recovery rate of the library, a sample prepared by reverse transcription of the mRNA-puromycin linked construct prepared in Preparation Example 4-3 of Experimental Example 1 was diluted to be 1E + 8/μL, 1E + 6/μL, and 1E + 4/μL, and subjected to qPCR under the same conditions.

The recovery rate was calculated by dividing the amount of nucleic acid of each display molecule recovered by binding to a target protein by the amount of nucleic acid of each display molecule that was present before mixing with the target protein (input value).

Recovery rate (%) = amount (number of molecules) of recovered nucleic acid /amount (number of molecules) of input nucleic acid × 100

The recovery rates of each display molecule of CF-01 and CF-02, and the ratios obtained by dividing the recovery rate in condition 1 by the respective recovery rates in conditions 2 to 6 were summarized in Table 41 and Table 42, respectively.

[Table 41]

| Peptide: CF-01 | | |
|---|---|---|
| Condition | Recovery rate (%) | Recovery rate in condition 1/recovery rate |
| **1** | **4.58E-01** | **-** |
| **2** | **2.95E-06** | **155142** |
| **3** | **4.17E-03** | **110** |
| **4** | **1.11E-02** | **41** |
| **5** | **1.51E-02** | **30** |
| **6** | **1.14E-05** | **40313** |

[Table 42]

| Peptide: CF-02 | | |
|---|---|---|
| Condition | Recovery rate (%) | Recovery rate in condition 1/recovery rate |
| **1** | **1.09E+00** | **-** |
| **2** | **1.27E-05** | **85434** |
| **3** | **7.83E-03** | **139** |
| **4** | **4.25E-03** | **256** |
| **5** | **2.14E-02** | **51** |
| **6** | **2.23E-06** | **488308** |

As a result, it was revealed that in these two peptides (CF-01, 02) the recovery rate in condition 1, where two kinds of target proteins, FKBP and calcineurin, were in proximity to each other by the fusion protein prepared in Preparation Examples 3-4, was at least 30-fold higher than the recovery rates in conditions 3 and 5, where two respective target proteins were added separately. This suggests that the peptides forming a ternary complex with FKBP and calcineurin can be pulled down efficiently by making the two kinds of target proteins be in proximity, which strongly suggests that these peptides could be first obtained by panning using the fusion protein.

In addition, the results show a molecule of interest capable of forming a complex with a plurality of kinds of target molecules can be identified by the method of counter-assay shown in Example 2. That is, it shows that a peptide capable of forming a complex with a plurality of kinds of target proteins can be screened based on the difference in pull-down efficiency between when two kinds of target proteins are present in the system in proximity and when only one kind of target protein is present in the system as an indicator. For example, in this example, a method of comparing the recovery rate of the display molecule in condition 1, which is a condition in which a plurality of kinds of target molecules are in proximity, with the recovery rate of the display molecule in conditions 2 and 4, which are conditions in which only a single target molecule is present, has been shown to be superior in sensitivity to identify a peptide that is a molecule of interest, compared to an existing method of comparing the recovery rate of the display molecule in condition 3 with the recovery rate of the display molecule in condition 2, or the recovery rate of the display molecule in condition 5 with the recovery rate of the display molecule in condition 4.

In this example, a counter-assay was performed on a display library prepared after concentration by panning to efficiently identify the molecule of interest from a highly diverse library. However, since the recovery rate of each display molecule under each condition is individually confirmed and compared in the counter assay, it is possible to identify the molecule of interest without panning by performing a counter assay on the display library in the case where a less diverse library is used.

[Evaluation Example 2-4: analysis of DNA pool obtained by panning using fusion protein]

Evaluation Examples 2-1, 2-2 and 2-3 show that the difference between the recovery rate of the display molecule in the conditions in which the two kinds of target proteins are in proximity and the recovery rate in the conditions in which the respective target protein is present in the system alone is a very useful indicator for identifying the molecule of interest, which is a peptide capable of forming a complex with a plurality of kinds of target proteins, and shows the validity of the method of selecting a peptide that meets the conditions as the molecule of interest performed in Example 2 of Experimental Example 1.

Based on this, the proportion of peptides that meet the conditions as the molecule of interest defined in the counter assay of Example 2 in the high-frequency top 100 sequences in each round of DNA pool concentrated by panning using fusion proteins was analyzed and summarized in Tables 43 and 44.

[Table 43]

| Analysis of DNA pool obtained by panning using fusion protein of FKBP and FRB | |
|---|---|
| Number of rounds performing panning | Number of candidate molecules included in high-frequency top 100 sequences |
| **3** | **80** |
| **4** | **82** |

[Table 44]

| Analysis of DNA pool obtained by panning using fusion protein of cyclophilin A and calcineurin | |
|---|---|
| Number of rounds performing panning | Number of candidate molecules included in high-frequency top 100 sequences |
| **3** | **89** |
| **4** | **87** |

As a result, it has been revealed that, although it is strongly suggested that there were peptides in the library used in this example that bind to each single target protein, as shown in Example 1 of Experimental Example 1, the molecule of interest that is a peptide capable of forming a complex with a plurality of kinds of target proteins, accounts for 80% or more of the high-frequency top 100 sequences and is preferentially obtained. This strongly suggests that performing panning using a fusion protein is extremely useful for obtaining a molecule of interest that is a peptide capable of forming a complex with a plurality of kinds of target molecules.

From the results of Experimental Example 2 composed of Evaluation Examples 1 and 2, it was shown that the peptide (molecule of interest) that forms a complex with the plurality of kinds of target proteins can be efficiently obtained from among molecules including other peptides (candidate molecules) present in the library by performing panning using a target fusion protein (target fusion molecule) composed of a plurality of kinds of target proteins (target molecules).

Although not intended to be bound by any particular theory, it is believed that this is possible because a plurality of kinds of target proteins are in proximity to each other by making the plurality of kinds of target proteins into a fusion protein, and a complex of the plurality of kinds of target proteins and a peptide becomes more stable.

[Evaluation Example 2-5: verification for linker length of fusion protein]

Evaluation Examples 2-1, 2-2 and 2-3 have shown that peptides that form complexes with a plurality of targets can be efficiently pulled down by using fusion proteins. Thus, the effect of the linker length of the fusion protein on the pull-down efficiency was then verified using a protein pair of FKBP and FRB.

[Preparation Example 2-5-1: preparation of FKBP-FRB fusion protein group with different linker lengths]

<Preparation Example 2-5-1-1: preparation of His-FKBP-(54)-FRB-FLAG-TEV-Avi-bio>

As a target protein for the study, a fusion protein His-FKBP-(54)-FRB-FLAG-TEV-Avi-bio in which FKBP and FRB were linked with a linker, a purification tag was added at the N-terminus, and a purification tag, a TEV protease cleavage tag and a biotinylation enzyme recognition tag were added at the C-terminus, was expressed in E. coli and purified. "K*"

represents biotinylated Lys.

Amino acid sequence of His-FKBP-(54)-FRB-FLAG-TEV-Avi-bio (SEQ ID NO: 55)

MHHHHHHSGGVQVETISPGDGRTFPKRGQTCVVHYTGMLEDGKKFDSSRDRNKPFKF
MLGKQEVIRGWEEGVAQMSVGQRAKLTISPDYAYGATGHPGIIPPHATLVFDVELLKLE
GSGGGSGGGGSGGGGSGGGGSGGGGSGGGGSGGGGSGGGGSGGGGSGGGGSGGTELI
RVAILWHEMWHEGLEEASRLYFGERNVKGMFEVLEPLHAMMERGPQTLKETSFNQAY
GRDLMEAQEWCRKYMKSGNVKDLTQAWDLYYHVFRRISKQGGGSSDYKDDDDKSSG
ENLYFQSSGGGLNDIFEAQK*IEWHE

Specifically, an expression vector prepared by cloning gene sequences of His-FKBP-(54)-FRB-FLAG-TEV-Avi-bio and biotin ligase (BirA) in pCDFDuet-1 was used to transform E. coli BL21 (DE3) to obtain an expression strain. The expression strain was cultured in LB medium until $OD_{600}$ was about 0.4 to 0.6. 1 mM IPTG and 100 μM biotin were then added to cause induction, and after cultured at 18°C for 18 hours, the sample was centrifuged at 8000 × g, 15 minutes, at 4°C and the cultured bacteria cells were recovered.

The recovered cultured bacteria cells were suspended with a disruption buffer (50 mM Tris-HCl pH 7.0, 200 mM NaCl, 2 mM DTT, 0.1% CHAPS, 0.1 mg/mL lysozyme, Protease inhibitor cocktail). The suspension was stirred at room temperature for 20 minutes, then the cells were ultrasonically disrupted. This sample was then centrifuged at 15000 × g, 10 minutes, at 4°C, and the supernatant was recovered and then filtered through a 0.22 μm filter to obtain a lysate sample.

The lysate sample was purified using Streptactin XT 4flow HC (IBA, #2-5030-010) 5 mL resin. For purification, an equilibration buffer (50 mM Tris-HCl pH 7.5, 300 mM NaCl, 1 mM TCEP) and an elution buffer (50 mM Tris-HCl pH 7.5, 300 mM NaCl, 1 mM TCEP, 50 mM D-biotin) were used. A fraction containing the protein of interest was recovered and concentrated with Amicon Ultra-15 10K (MERCK, #UFC901024).

The concentrated sample was purified by gel filtration using HiLoad 16/600 Superdex 75 pg (Cytiva, #28-9893-33). For purification, a running buffer (50 mM Tris-HCl pH 7.5, 300 mM NaCl, 1 mM TCEP) was used. A fraction containing the protein of interest was recovered and then concentrated with Amicon Ultra-15 10K (MERCK, #UFC901024) to obtain the protein of interest His-FKBP-(54)-FRB-FLAG-TEV-Avi-bio. The sample was stored at -80°C.

<Preparation Example 2-5-1-2: preparation of bio-Avi-TEV-FLAG-FKBP-(23)-FRB-His>

As a target protein for the study, a fusion protein bio-Avi-TEV-FLAG-FKBP-(23)-FRB-His in which FKBP and FRB were linked with a linker, a purification tag, a TEV protease cleavage tag and a biotinylation enzyme recognition tag were added at the N-terminus, and a purification tag was added at the C-terminus, was expressed in E. coli and purified. "K*" represents biotinylated Lys.

Amino acid sequence of bio-Avi-TEV-FLAG-FKBP-(23)-FRB-His (SEQ ID NO: 56)

MGLNDIFEAQK*IEWHESSGENLYFQSGGGSSDYKDDDDKSGGVQVETISPGDGRTFPK
RGQTCVVHYTGMLEDGKKFDSSRDRNKPFKFMLGKQEVIRGWEEGVAQMSVGQRAK
LTISPDYAYGATGHPGIIPPHATLVFDVELLKLEGSGSSGSSGLEVLFQGPSSGSSGSSGSS
GTELIRVAILWHEMWHEGLEEASRLYFGERNVKGMFEVLEPLHAMMERGPQTLKETSF
NQAYGRDLMEAQEWCRKYMKSGNVKDLTQAWDLYYHVFRRISKQGGGSSHHHHHH

Specifically, an expression vector prepared by cloning gene sequences of bio-Avi-TEV-FLAG-FKBP-(23)-FRB-His and biotin ligase (BirA) in pCDFDuet-1 was used to transform E. coli BL21 (DE3) to obtain an expression strain. The expression strain was cultured in LB medium until $OD_{600}$ was about 0.4 to 0.6. 1 mM IPTG and 100 μM biotin were then added to cause induction, and after cultured at 23°C for 18 hours, the sample was centrifuged at 8000 × g, 15 minutes, at 4°C and the cultured bacteria cells were recovered.

The recovered cultured bacteria cells were suspended with a disruption buffer (50 mM Tris-HCl pH 7.0, 200 mM NaCl, 2 mM DTT, 0.1% CHAPS, 0.1 mg/mL lysozyme, 1/1000 (v/v) Benzonase, Protease inhibitor cocktail). The suspension was stirred at room temperature for 20 minutes, then the cells were ultrasonically disrupted. This sample was centrifuged at 15000 × g, 30 minutes, at 4°C, and the supernatant was recovered. The supernatant was then further centrifuged at 15000 × g for 10 minutes or more at 4°C, and then filtered through a 0.22 μm filter to obtain a lysate sample.

The lysate sample was purified using Streptactin XT Superflow HC (IBA, #2-4030-010) 5 mL resin. For purification, a binding buffer (50 mM Tris-HCl pH 7.5, 200 mM NaCl, 1 mM TCEP) and an elution buffer (50 mM Tris-HCl pH 7.5, 200 mM NaCl, 1 mM TCEP, 50 mM D-biotin) were used. A fraction containing the protein of interest was recovered and concentrated with Amicon Ultra-15 10K (MERCK, #UFC901024).

The concentrated sample was purified by gel filtration using HiLoad 16/600 Superdex 75 pg (Cytiva, #28-9893-343). For purification, a running buffer (50 mM Tris-HCl pH 7.5, 300 mM NaCl, 1 mM TCEP) was used. A fraction containing the protein of interest was recovered and diluted with a running buffer, then filtered with a 0.22 μm filter to obtain the protein of interest bio-Avi-TEV-FLAG-FKBP-(23)-FRB-His. The sample was stored at -80°C.

<Preparation Example 2-5-1-3: preparation of FLAG-Avi-bio-TEV-FKBP-Sor-His>

As a target protein for the study, FLAG-Avi-bio-TEV-FKBP-Sor-His, an FKBP in which a purification tag, a TEV protease cleavage tag and a biotinylation enzyme recognition tag were added at the N-terminus, and a purification tag and a sortase recognition sequence were added at the C-terminus, was expressed in E. coli and purified. "K*" represents biotinylated Lys.

Amino acid sequence of FLAG-Avi-bio-TEV-FKBP-Sor-His (SEQ ID NO: 57)

MDYKDDDDKGGSSGLNDIFEAQK*IEWHESSGENLYFQGGGGSGVQVETISPGDGRTFP
KRGQTCVVHYTGMLEDGKKFDSSRDRNKPFKFMLGKQEVIRGWEEGVAQMSVGQRA
KLTISPDYAYGATGHPGIIPPHATLVFDVELLKLEGGGSSLPMTGGGGSSHHHHHH

Specifically, an expression vector prepared by cloning genes of FLAG-Avi-bio-TEV-FKBP-Sor-His and biotin ligase (BirA) in pCDFDuet-1 was used to transform E. coli BL21 (DE3) to obtain an expression strain. The expression strain was cultured in LB medium until $OD_{600}$ reached 0.6. IPTG and biotin were then added to be the final concentrations of 0.1 mM and 100 μM, respectively to cause induction, and after cultured at 18°C for 18 hours, the sample was centrifuged at 8000 × g, 15 minutes, at 4°C and the cultured bacteria cells were recovered.

The recovered cultured bacteria cells were suspended with a disruption buffer (50 mM Tris-HCl pH 7.0, 200 mM NaCl, 2 mM DTT, 0.1% CHAPS, 0.1 mg/mL lysozyme, 1/1000 (v/v) Benzonase, Protease inhibitor cocktail). The suspension was stirred at room temperature for 20 minutes, then the cells were homogenized. This sample was then centrifuged at 30000 × g, 60 minutes, at 4°C, and then the supernatant was filtered through a 0.22 μm filter to obtain a lysate sample.

The lysate sample was purified using Strep Tactin XT 4flow HC (IBA, #2-5030-010) 5 mL resin. For purification, an equilibration buffer (50 mM Tris-HCl pH 7.5, 200 mM NaCl, 1 mM TCEP) and an elution buffer (50 mM Tris-HCl pH 7.5, 200 mM NaCl, 1 mM TCEP, 50 mM D-biotin) were used. A fraction containing the protein of interest was recovered and concentrated with Amicon Ultra-15 10K (MERCK, #UFC901024).

The concentrated sample was purified by gel filtration using HiLoad 16/600 Superdex 75 pg (Cytiva, #28-9893-33). For purification, an equilibration buffer (50 mM Tris-HCl pH 7.5, 300 mM NaCl, 1 mM TCEP) was used. A fraction containing the protein of interest was recovered and concentrated with Amicon Ultra-15 10K (MERCK, #UFC901024) to obtain the protein of interest FLAG-Avi-bio-TEV-FKBP-Sor-His. The sample was stored at -80°C.

<Preparation Example 2-5-1-4: preparation of bio-Avi-TEV-FRB-FLAG>

As a target protein for the study, bio-Avi-TEV-FRB-FLAG, an FRB in which a TEV protease cleavage tag and a biotinylation enzyme recognition tag were added at the N-terminus and a purification tag was added at the C-terminus, was expressed in E. coli and purified. "K*" represents biotinylated Lys.

Amino acid sequence of bio-Avi-TEV-FRB-FLAG (SEQ ID NO: 58)

MGLNDIFEAQK*IEWHESSGENLYFQGGGGSELIRVAILWHEMWHEGLEEASRLYFGER
NVKGMFEVLEPLHAMMERGPQTLKETSFNQAYGRDLMEAQEWCRKYMKSGNVKDLT
QAWDLYYHVFRRISKQGGGSSDYKDDDDK

Specifically, an expression vector prepared by cloning genes of bio-Avi-TEV-FRB-FLAG and biotin ligase (BirA) in pCDFDuet-1 was used to transform E. coli BL21 (DE3) to obtain an expression strain. The expression strain was cultured in LB medium until $OD_{600}$ was about 0.4 to 0.6. IPTG and biotin were then added to be the final concentrations of 1 mM and 100 μM, respectively to cause induction, and after cultured at 18°C for 16 hours, the sample was centrifuged at

8000 × g, 15 minutes, at 4°C and the cultured bacteria cells were recovered.

The recovered cultured bacteria cells were suspended with a disruption buffer (50 mM Tris-HCl pH 7.0, 200 mM NaCl, 2 mM DTT, 0.1% CHAPS, 0.1 mg/mL lysozyme, 1/1000 (v/v) Benzonase, Protease inhibitor cocktail). The suspension was stirred at room temperature for 20 minutes, then the cells were ultrasonically disrupted. This sample was then centrifuged at 15000 × g, 30 minutes, at 4°C, and then the supernatant was filtered through a 0.22 μm filter to obtain a lysate sample.

The lysate sample was purified using Strep Tactin XT 4flow HC (IBA, #2-5030-010) 5 mL resin. For purification, an equilibration buffer (50 mM Tris-HCl pH 7.5, 200 mM NaCl, 1 mM TCEP) and an elution buffer (50 mM Tris-HCl pH 7.5, 200 mM NaCl, 1 mM TCEP, 50 mM D-biotin) were used. Fractions containing the protein of interest were recovered.

The portion of the recovered sample was purified by gel filtration using HiLoad 16/600 Superdex 75 pg (Cytiva, #28-9893-33). For purification, a running buffer (50 mM Tris-HCl pH 7.5, 300 mM NaCl, 1 mM TCEP) was used. A fraction containing the protein of interest was recovered to obtain bio-Avi-TEV-FRB-FLAG. The sample was stored at -80°C.

<Preparation Example 2-5-1-5: preparation of FLAG-Avi-bio-TEV-FKBP-(14)-FRB-FLAG>

As a target protein for the study, a fusion protein in which the N-terminus of FRB and the C-terminus of FKBP were linked with a peptide linker was prepared by performing a peptide linking reaction using a sortase prepared in Preparation Example 3-3 for two kinds of proteins that are FLAG-Avi-bio-TEV-FKBP-Sor-His containing a sortase recognition sequence at the C-terminus prepared in Preparation Example 2-5-1-3 and GGG-FRB-FLAG exposing a sortase recognition sequence at the N-terminus by treating bio-Avi-TEV-FRB-FLAG prepared in Preparation Example 2-5-1-4 with a TEV protease. "K*" represents biotinylated Lys.

Amino acid sequence of FLAG-Avi-bio-TEV-FKBP-(14)-FRB-FLAG (SEQ ID NO: 59)

MDYKDDDDKGGSSGLNDIFEAQK*IEWHESSGENLYFQGGGGSGVQVETISPGDGRTFP
KRGQTCVVHYTGMLEDGKKFDSSRDRNKPFKFMLGKQEVIRGWEEGVAQMSVGQRA
KLTISPDYAYGATGHPGIIPPHATLVFDVELLKLEGGGSSLPMTGGGGSELIRVAILWHE
MWHEGLEEASRLYFGERNVKGMFEVLEPLHAMMERGPQTLKETSFNQAYGRDLMEAQ
EWCRKYMKSGNVKDLTQAWDLYYHVFRRISKQGGGSSDYKDDDDK

(Preparation Example 2-5-1-5-1: removal of N-terminal tag of FRB by TEV protease reaction)

The bio-Avi-TEV-FRB-FLAG prepared in Preparation Example 2-5-1-4 was treated with a TEV protease that recognizes and cleaves a TEV protease recognition sequence and cleaved the tag sequence at the N-terminus.

Specifically, 42.5 μL of bio-Avi-TEV-FRB-FLAG and a TEV eluent of 1 × TEV buffer, 1 mM DTT, 0.1 U/μL and AcTEV protease (Thermo Fisher Scientific Inc., #12575015) were mixed on ice and incubated at 4°C overnight.

Samples before and after the TEV protease reaction were analyzed by SDS-PAGE to confirm that the reaction proceeded properly by checking the gel shift corresponding to the theoretical molecular weight of the protein of interest.

The crude sample after the reaction was mixed with Dynabeads His-Tag Isolation and Pulldown (Invitrogen, 10103D), and mixed by inversion at 4°C for 30 minutes, and then mixed with streptavidin-coated magnetic beads, and mixed by inversion at 4°C for 30 minutes to obtain a purified sample. A sample after purification was recovered and concentrated with Amicon Ultra-0.5 10K (MERCK, #UFC501096). Then, a buffer exchange (50 mM Tris pH 7.5,300 mM NaCl, 1 mM DTT) was performed using Zeba Spin Desalting Columns, 7K MWCO, 0.5 mL (Thermo Fisher Scientific, Inc., #89883) according to the basic protocol attached to the product to obtain GGG-FRB-FLAG in which the N-terminal tag sequence was cleaved and the sortase recognition sequence was exposed.

(Preparation Example 2-5-1-5-2: addition of FRB to FKBP by sortase reaction)

In a reaction buffer (50 mM Tris-HCl, pH 7.5, 150 mM NaCl, 10 mM CaCl2), 40 μM FLAG-Avi-bio-TEV-FKBP-Sor-His, 40 μM GGG-FRB-FLAG and 1 μM eSrtA were mixed on ice and incubated at 4°C overnight.

Samples before and after the sortase reaction were analyzed by SDS-PAGE to confirm that the reaction proceeded properly by checking the gel shift corresponding to the theoretical molecular weight of the protein of interest.

The crude sample after the reaction was mixed with Dynabeads His-Tag Isolation and Pulldown (Invitrogen, 10103D), and the mixture was mixed by inversion at 4°C for 10 minutes, and then the supernatant was recovered. Then, crude purification was performed using Strep-Tactin XT Sepharose chromatography resin (Cytiva, 29401324), a wash buffer (50 mM HEPES pH 7.5, 150 mM NaCl, 0.5 mM TCEP), and an elution buffer (50 mM HEPES pH 7.5, 150 mM NaCl,

0.5 mM TCEP, 50 mM biotin). The crude purification was performed according to the basic protocol attached to the manufacturing product.

The sample after the crude purification was purified by gel filtration using Superdex 75 Increase 10/300 GL (Cytiva, #29148721). For purification, a running buffer (50 mM HEPES pH 7.5, 300 mM NaCl, 1 mM DTT) was used. A fraction containing the protein of interest was recovered and then concentrated to obtain the protein of interest FLAG-Avi-bio-TEV-FKBP-(14)-FRB-FLAG in which the N-terminus of FRB and the C-terminus of FKBP were linked with a peptide linker.

<Evaluation Example 2-5-1: pull-down assay of mRNA-peptide display molecules (FF-01 to FF-04) for fusion proteins with different linker lengths>

To a buffer containing 1 × TBS, 2 mg/mL BSA (Invitrogen), and 2 mM DTT, the display molecules prepared in a method according to Preparation Example 2-1-2 was added to be 0.2-0.3 μM, and the target protein was added to be as shown in Table 45, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.04 mM, and the mixture was mixed by inversion at 4°C for another 10 minutes. The target proteins prepared in Preparation Examples 2-5-1-1, 2-5-1-2, and 2-5-1-5 were used as the target proteins, and those in four conditions shown in the following table were compared.

[Table 45]

| Condition | Target protein | Linker length (AA) |
|---|---|---|
| **1** | **0.4 μM His-FKBP-(54)-FRB-FLAG-TEV-Avi-bio** | **54** |
| **2** | **0.4 μM bio-Avi-TEV-FLAG-FKBP-(23)-FRB-His** | **23** |
| **3** | **0.4 μM FLAG-Avi-bio-TEV-FKBP-(14)-FRB-FLAG** | **14** |
| **4** | No target protein | **-** |

Conditions 1 to 3 are conditions in which a fusion protein of FKBP and FRB was used, and the length of the peptide linker, which was the fusion moiety of the fusion protein, was 54 amino acid residues, 23 amino acid residues, and 14 amino acid residues, respectively. Condition 4 is a condition under the absence of the target protein.

Streptavidin-coated magnetic beads with immobilized target proteins were collected with a magnet and the supernatant was removed. The collected beads were washed several times with a wash buffer (1 × TBS, 2 mM DTT, 0.1% Tween 20 (Sigma Aldrich), 500 mM arginine), then treated with a TEV protease that recognizes and cleaves the TEV protease recognition sequence to elute the target protein and the peptide-nucleic acid conjugate that binds to the target protein.

To the streptavidin-coated magnetic beads after washing, 1 × TEV buffer, 1 mM DTT, and 0.1 U/μL AcTEV protease (Thermo Fisher Scientific Inc., #12575015) were added as a specific TEV eluent to react with. After the reaction, the supernatant was recovered and qPCR was performed. For the qPCR primer, the aforementioned primers (Fw-Primer and Rv-Primer) were used. For the qPCR reaction solution, 1 × Ex Taq buffer, 0.2 mM dNTP, 0.5 μM Fw-Primer, 0.5 μM Rv-Primer, 50000-fold diluted SYBR Green I (LONZA, #50513), and 0.025 U/μL Ex Taq polymerase (Takara Bio Inc.) were mixed. The mixture was heated at 95°C for 2 minutes, and then subjected to 40 cycles of 95°C for 10 seconds, 57°C for 20 seconds, and 72°C for 30 seconds. In this case, each display molecule before mixing with the target protein was also subjected to qPCR in the same way.

To draw a calibration curve to estimate the recovery rate of the library, a sample prepared by reverse transcription of the mRNA-puromycin linked construct prepared in Preparation Example 4-3 of Experimental Example 1 was diluted to be 1E + 8/μL, 1E + 6/μL, and 1E + 4/μL, and subjected to qPCR under the same conditions.

The recovery rate was calculated by dividing the amount of nucleic acid of each display molecule recovered by binding to a target protein by the amount of nucleic acid of each display molecule that was present before mixing with the target protein (input value).

Recovery rate (%) = amount (number of molecules) of recovered nucleic acid /amount (number of molecules) of input nucleic acid × 100

The recovery rates of each display molecule of FF-01, FF-02, FF-03, and FF-04 in conditions 1 to 4 are summarized in Table 46.

[Table 46]

| Condition | Recovery rate (%) | | | |
|---|---|---|---|---|
| | FF-01 | FF-02 | FF-03 | FF-04 |
| 1 | 8.09E+00 | 1.88E+00 | 1.38E+01 | 6.87E+00 |
| 2 | 8.81E+00 | 2.09E+00 | 1.31E+01 | 6.65E+00 |
| 3 | 6.36E+00 | 1.26E+00 | 8.14E+00 | 5.30E+00 |
| 4 | 4.11E-05 | 4.11E-05 | 2.25E-04 | 3.12E-04 |

As a result, it was revealed that in these four peptides (FF-01 to FF-04) the recovery rates in conditions 1 to 3, where two kinds of target proteins, FKBP and FRB, were in proximity to each other by the fusion protein prepared in Preparation Examples 2-5-1-1, 2-5-1-2, and 2-5-1-5, were at least 16000-fold higher than the recovery rate in condition 4 where the target proteins were absent.

Based on the recovery rates under these conditions 1 to 3 and the recovery rate in conditions where FKBP and FRB were not in proximity in Evaluation Example 2-1-1, it was strongly suggested that, with any length of the peptide linker, which is the fusion moiety of the fusion protein, of 54 amino acid residues, 23 amino acid residues, and 14 amino acid residues, respectively, the effect of the invention can be obtained in this system.

[Evaluation Example 2-6: verification for linking of N- and C-termini of each protein in fusion protein]

Evaluation Examples 2-1, 2-2 and 2-3 have shown that peptides that form complexes with a plurality of targets can be efficiently pulled down by using fusion proteins. Thus, the effect of the linking between the termini of two kinds of proteins in fusion protein on the pull-down efficiency was then verified using a protein pair of FKBP and FRB.

[Preparation Example 2-6-1: preparation of a group of FKBP-FRB fusion proteins with different linkings of N- and C-termini of FKBP and FRB]

<Preparation Example 2-6-1-1: preparation of GL-FRB-FLAG>

As a target protein for the study, GL-FRB-FLAG, an FRB in which a OaAEP1 recognition sequence was added at the N-terminus and a purification tag was added at the C-terminus, was expressed in E. coli and purified.

Amino acid sequence of GL-FRB-FLAG (SEQ ID NO: 60)

GLGGGGSGGGGSELIRVAILWHEMWHEGLEEASRLYFGERNVKGMFEVLEPLHAMME
RGPQTLKETSFNQAYGRDLMEAQEWCRKYMKSGNVKDLTQAWDLYYHVFRRISKQG
GGSSDYKDDDDK

Specifically, an expression vector prepared by cloning a gene of His-TEV-FRB-FLAG in pET11a was used to transform E. coli BL21 (DE3) to obtain an expression strain. The expression strain was cultured in LB medium until $OD_{600}$ reached 0.6. IPTG was then added to be the final concentrations of 1 mM to cause induction, and after cultured at 18°C for 18 hours, the sample was centrifuged at 8000 × g, 15 minutes, at 4°C and the cultured bacteria cells were recovered.

The recovered cultured bacteria cells were suspended with a disruption buffer (50 mM Tris-HCl pH 7.0, 200 mM NaCl, 1 mM TCEP, 0.1% CHAPS, 0.1 mg/mL lysozyme, 1/1000 (v/v) Benzonase). The suspension was stirred at room temperature for 20 minutes, then the cells were homogenized. This sample was then centrifuged at 40000 rpm, 60 minutes, at 4°C, and then the supernatant was recovered and filtered through a 0.22 μm filter to obtain a lysate sample.

The lysate sample was purified with His Trap HP 5 mL (Cytiva, 17524801). For purification, an equilibration buffer (50 mM Tris-HCl pH 7.5, 300 mM NaCl, 20 mM imidazole, 1 mM TCEP) and an elution buffer (50 mM Tris-HCl pH 7.5, 300 mM NaCl, 250 mM imidazole, 1 mM TCEP) were used. Fractions containing the protein of interest were recovered.

The recovered samples were treated at 4°C overnight with addition of 5 μg of TEV protease that recognizes and cleaves a TEV protease recognition sequence per 100 μg of the protein of interest to remove the purification tag at the N-terminus of the raw protein. Simultaneously, the sample was dialyzed with a dialysis buffer (50 mM Tris-HCl pH 7.5, 300 mM NaCl, 20 mM imidazole, 1 mM TCEP).

The sample treated with the TEV protease was purified using His Trap HP 5 mL (Cytiva, 17524801). For purification, an equilibration buffer (50 mM Tris-HCl pH 7.5, 300 mM NaCl, 20 mM imidazole, 1 mM TCEP) and an elution

buffer (50 mM Tris-HCl pH 7.5, 300 mM NaCl, 250 mM imidazole, 1 mM TCEP) were used. A fraction containing the protein of interest was recovered and concentrated with Amicon Ultra-15 10K (MERCK, #UFC901024).

The concentrated sample was purified by gel filtration using HiLoad 16/600 Superdex 75 pg (Cytiva, #28-9893-33). For purification, an equilibration buffer (50 mM Tris-HCl pH 7.5, 300 mM NaCl, 1 mM TCEP) was used. A fraction containing the protein of interest was recovered and concentrated with Amicon Ultra-15 10K (MERCK, #UFC901024) to obtain the protein of interest GL-FRB-FLAG. The sample was stored at -80°C.

<Preparation Example 2-6-1-2: preparation of FLAG-Avi-bio-TEV-FKBP-FRB-FLAG (N-to-C) using enzymatic peptide linking reaction>

A fusion protein in which the N-terminus of FRB and the C-terminus of FKBP were linked with a peptide linker was prepared by a two-step peptide linking reaction using a sortase prepared in Preparation Example 3-3 and OaAEP1 prepared in Preparation Examples 3-2 for FLAG-Avi-bio-TEV-FKBP-Sor-His containing a sortase recognition sequence at the C-terminus prepared in Preparation Example 2-5-1-3 and GL-FRB-FLAG containing an OaAEP1 ligase recognition sequence at the N-terminus prepared in Preparation Example 2-6-1-1. "K*" represents biotinylated Lys.

Amino acid sequence of FLAG-Avi-bio-TEV-FKBP-FRB-FLAG (N-to-C) (SEQ ID NO: 61)

MDYKDDDDKGGSSGLNDIFEAQK*IEWHESSGENLYFQGGGGSGVQVETISPGDGRTFP
KRGQTCVVHYTGMLEDGKKFDSSRDRNKPFKFMLGKQEVIRGWEEGVAQMSVGQRA
KLTISPDYAYGATGHPGIIPPHATLVFDVELLKLEGGGSSLPMTGGGGGGGSGGGGSGG
GGSGGGGSNGLGGGGSGGGGSELIRVAILWHEMWHEGLEEASRLYFGERNVKGMFEV
LEPLHAMMERGPQTLKETSFNQAYGRDLMEAQEWCRKYMKSGNVKDLTQAWDLYYH
VFRRISKQGGGSSDYKDDDDK

Amino acid sequence of peptide linker GGG-(G4S)4-NGLH (SEQ ID NO: 62)
GGGGGGGSGGGGSGGGGSGGGGSNGLH

(Preparation Example 2-6-1-2-1: addition of peptide linker to FKBP by sortase reaction)

In a reaction buffer (50 mM Tris-HCl, pH 7.5, 150 mM NaCl, 10 mM CaCl2), 100 μM FLAG-Avi-bio-TEV-FKBP-Sor-His, 1 mM GGG-(G4S)4-NGLH (synthesized at SCRUM Inc.) and 1 μM eSrtA were mixed on ice and incubated at 4°C overnight.

Samples before and after the sortase reaction were analyzed by SDS-PAGE to confirm that the reaction proceeded properly by checking the gel shift corresponding to the theoretical molecular weight of the protein of interest.

The crude sample after the reaction was mixed with Dynabeads His-Tag Isolation and Pulldown (Invitrogen, 10103D), and the mixture was mixed by inversion at 4°C for 10 minutes, and then the supernatant was recovered to obtain a crude purified sample. Then, the crude purified sample was purified using Strep-Tactin XT Sepharose chromatography resin (Cytiva, 29401324), a wash buffer (50 mM HEPES pH 7.5, 150 mM NaCl, 0.5 mM TCEP), and an elution buffer (50 mM HEPES pH 7.5, 150 mM NaCl, 0.5 mM TCEP, 50 mM biotin). The purification was performed according to the basic protocol attached to the manufacturing product. The sample after purification was recovered and concentrated with Amicon Ultra-0.5 10K (MERCK, #UFC501096) to obtain a complex FLAG-Avi-bio-TEV-FKBP-GGG-(G4S)4-NGLH in which the linker was added at the C-terminus of FKBP.

(Preparation Example 2-6-1-2-2: addition of FRB to FKBP-peptide linker complex by OaAEP1 reaction)

Specifically, 29.3 μM FLAG-Avi-bio-TEV-FKBP-GGG-(G4S)4-NGLH, 29.3 μM FRB-FLAG, 293 μM $NiCl_2$, and 0.44 μM OaAEP1 were mixed on ice and incubated at 4°C overnight.

Samples before and after the OaAEP1 reaction were analyzed by SDS-PAGE to confirm that the reaction proceeded properly by checking the gel shift corresponding to the theoretical molecular weight of the protein of interest.

The concentrated sample was purified by gel filtration using Superdex 75 Increase 10/300 GL(Cytiva, #29148721). For purification, a running buffer (50 mM HEPES pH 7.5, 300 mM NaCl, 1 mM DTT) was used. A fraction containing the protein of interest was recovered to obtain the protein of interest FLAG-Avi-bio-TEV-FKBP-FRB-FLAG (N-to-C) in which the N-terminus of FRB and the C-terminus of FKBP were linked with a peptide linker.

<Preparation Example 2-6-1-3: preparation of FRB-FLAG-FKBP-FLAG-TEV-Avi-bio(C-to-N) using enzymatic peptide linking reaction>

A fusion protein in which the C-terminus of FRB and the N-terminus of FKBP were linked with a peptide linker was prepared by adding a linker by a two-step peptide linking reaction using a sortase prepared in Preparation Example 3-3 and OaAEP1 prepared in Preparation Examples 3-2 for FRB-FLAG-Sor-His containing a sortase recognition sequence at the C-terminus prepared in Preparation Example 1-4 and GL-FKBP-FLAG-TEV-Avi-bio containing an OaAEP1 recognition sequence at the N-terminus prepared in Preparation Example 3-1. "K*" represents biotinylated Lys.

Amino acid sequence of FRB-FLAG-FKBP-FLAG-TEV-Avi-bio (C-to-N) (SEQ ID NO: 63)

MELIRVAILWHEMWHEGLEEASRLYFGERNVKGMFEVLEPLHAMMERGPQTLKETSFN
QAYGRDLMEAQEWCRKYMKSGNVKDLTQAWDLYYHVFRRISKQGGGSSDYKDDDD
KGGSSLPMTGGGGGGGSGGGGSGGGGSGGGGSNGLGGGGSGGGGSGVQVETISPGDG
RTFPKRGQTCVVHYTGMLEDGKKFDSSRDRNKPFKFMLGKQEVIRGWEEGVAQMSVG
QRAKLTISPDYAYGATGHPGIIPPHATLVFDVELLKLEGGGSSDYKDDDDKSSGENLYFQ
SSGGGLNDIFEAQK*IEWHE

(Preparation Example 2-6-1-3-1: addition of peptide linker to FRB by sortase reaction)

In a reaction buffer (50 mM Tris-HCl, pH 7.5, 150 mM NaCl, 10 mM CaCl2), 100 μM FRB-FLAG-Sor-His, 1 mM GGG-(G4S)4-NGLH (synthesized at SCRUM Inc.) and 1 μM eSrtA were mixed on ice and incubated at 4°C for 5 hours.

Samples before and after the sortase reaction were analyzed by SDS-PAGE to confirm that the reaction proceeded properly by checking the gel shift corresponding to the theoretical molecular weight of the protein of interest.

The crude sample after the reaction was mixed with Dynabeads His-Tag Isolation and Pulldown (Invitrogen, 10103D), and the mixture was mixed by inversion at 4°C for 10 minutes, and then the supernatant was recovered to obtain a crude purified sample. The crude purified sample was then purified using ANTI-FLAG(R) M2 Affinity Gel (Sigma, #A2220), a wash buffer (50 mM HEPES pH 7.5, 150 mM NaCl, 0.5 mM TCEP), and an elution buffer (50 mM HEPES pH 7.5, 150 mM NaCl, 0.5 mM TCEP, 500 μg/mL FLAG peptide). The purification was performed according to the basic protocol attached to the manufacturing product. The sample after purification was recovered and concentrated with Amicon Ultra-0.5 10K (MERCK, #UFC501096) to obtain a complex FRB-FLAG-GGG-(G4S)4-NGLH in which a linker was added at the C-terminus of FRB.

(Preparation Example 2-6-1-3-2: addition of FKBP to FRB-peptide linker complex by OaAEP1 reaction)

Specifically, 30 μM FRB-FLAG-GGG-(G4S)4-NGLH, 30 μM GL-FKBP-FLAG-TEV-Avi-bio, 300 μM $NiCl_2$, and 0.45 μM OaAEP1 were mixed on ice and incubated at 4°C overnight.

Samples before and after the OaAEP1 reaction were analyzed by SDS-PAGE to confirm that the reaction proceeded properly by checking the gel shift corresponding to the theoretical molecular weight of the protein of interest.

The concentrated sample was purified by gel filtration using Superdex 75 Increase 10/300 GL(Cytiva, #29148721). For purification, a running buffer (50 mM HEPES pH 7.5, 300 mM NaCl, 1 mM DTT) was used. A fraction containing the protein of interest was recovered to obtain the protein of interest FRB-FLAG-FKBP-FLAG-TEV-Avi-bio (C-to-N) in which the C-terminus of FRB and the N-terminus of FKBP were linked with a peptide linker.

<Preparation Example 2-6-1-4: preparation of FLAG-Avi-bio-TEV-FKBP-FLAG-FRB (C-to-C)>

To FLAG-Avi-bio-TEV-FKBP-Sor-His containing a sortase recognition sequence at the C-terminus prepared in Preparation Example 2-5-1-3 and FRB-FLAG-Sor-His containing a sortase recognition sequence at the C-terminus prepared in Preparation Example 1-4, a linker with a methyltetrazine (MeTz) functional group and a transcyclooctene (TCO) functional group were added at the C-terminus using sortase prepared in Preparation Example 3-3, respectively. The obtained proteins were then linked by a click reaction to prepare a fusion protein in which the C-terminus of FRB and the C-terminus of FKBP were linked with a peptide linker. "K*" represents biotinylated Lys, "K(*1)" represents Lys in which a methyltetrazine functional group was added to the side chain, and "K(*5)" represents Lys in which a transcyclooctene functional group was added to the side chain. "K(*1)" and "K(*5)" are linked by a click reaction as in "(*6)" or "(*7)". The structure formulas of "K(*5)", "(*6)" and "(*7)" are shown in Table 47.

[Table 47]

| Name | Structure formula |
|---|---|
| **K (* 5)** | 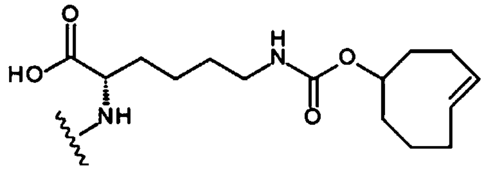 |
| **(* 6)** | 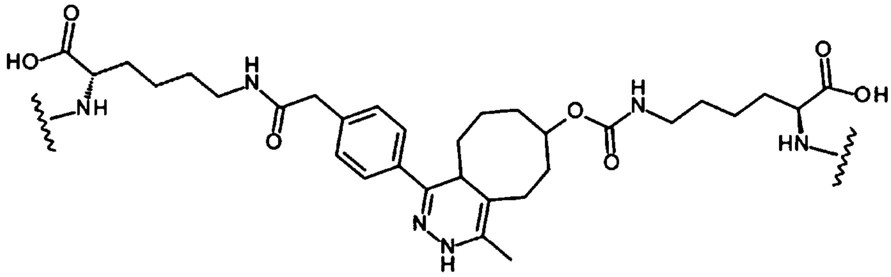 |
| **(* 7)** | 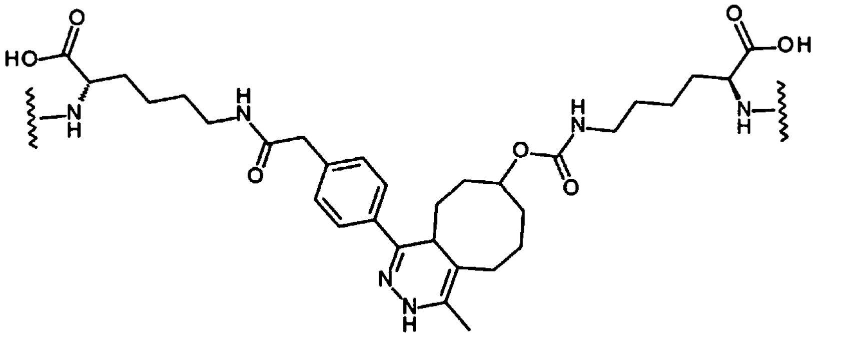 |

Amino acid sequence of FLAG-Avi-bio-TEV-FKBP-FLAG-FRB (C-to-C) (SEQ ID NO: 64 and SEQ ID NO: 65)

MDYKDDDDKGGSSGLNDIFEAQK*IEWHESSGENLYFQGGGGSGVQVETISPGDGRTFP
KRGQTCVVHYTGMLEDGKKFDSSRDRNKPFKFMLGKQEVIRGWEEGVAQMSVGQRA
KLTISPDYAYGATGHPGIIPPHATLVFDVELLKLEGGGSSLPMTGGGGSGGGGSK(*1)
MELIRVAILWHEMWHEGLEEASRLYFGERNVKGMFEVLEPLHAMMERGPQTLKETSFN
QAYGRDLMEAQEWCRKYMKSGNVKDLTQAWDLYYHVFRRISKQGGGSSDYKDDDD
KGGSSLPMTGGGGSGGGGSGGGGSK(*5)

<Synthesis Example 2-6-1: synthesis of (G4S)3-K (TCO)>

[Formula 68]

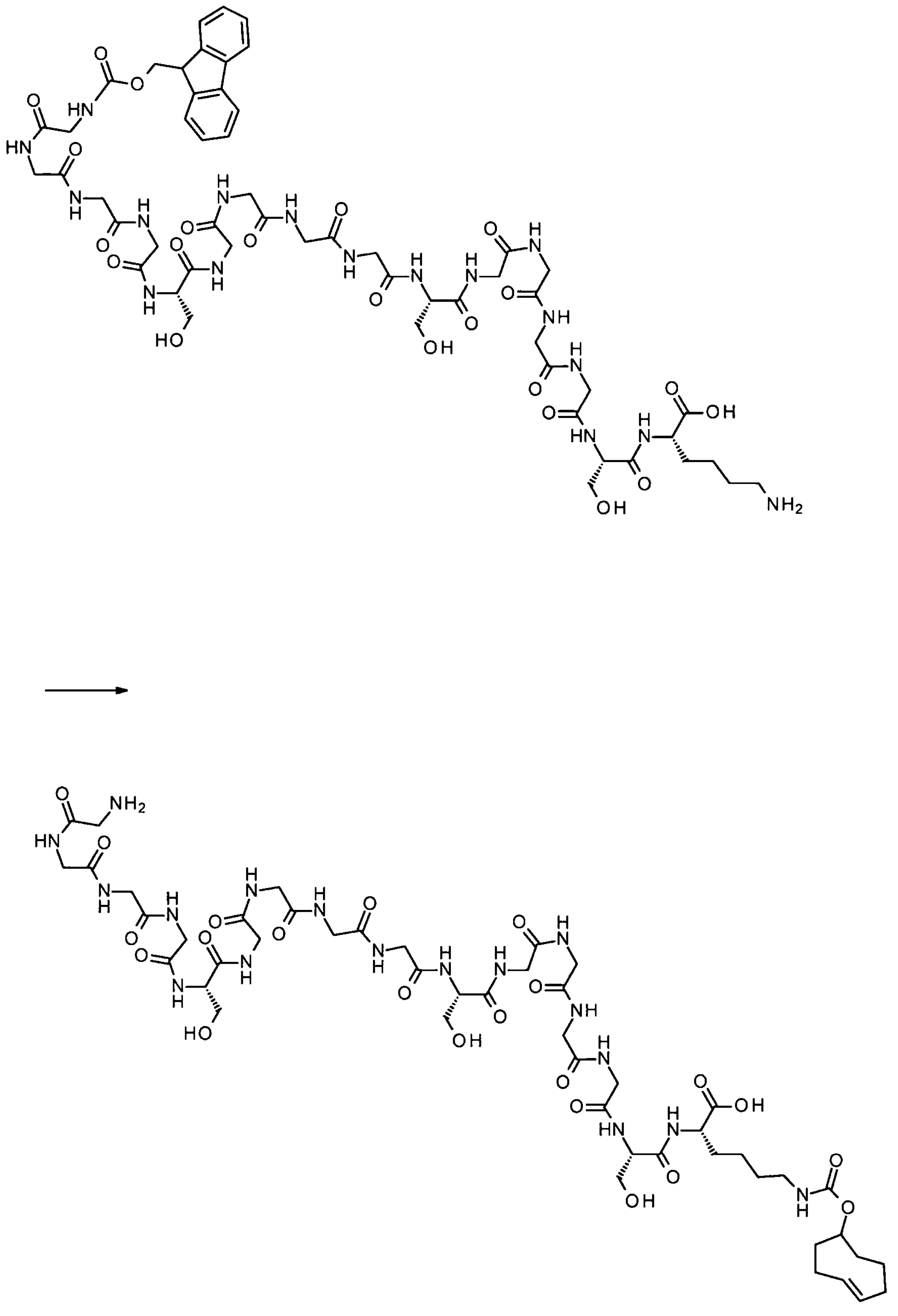

(((9H-fluoren-9-yl)methoxy)carbonyl)glycylglycylglycylglycylglycylglycyl-L-serylglycylglycylglycylglycyl-L-serylglycylglycylglycylglycyl-L-seryl-L-lysine (50.5 mg, 0.038 mmol) and (E)-cyclooct-4-en-1-yl(2,5-dioxopyrrolidin-1-yl)carbonate were dissolved in dimethyl sulfoxide (1281 μL) and N-ethyl diisopropylamine (34.0 μL, 0.16 mmol) was added dropwise. The reaction solution was stirred at 25°C for 60 minutes. The reaction solution was purified by reverse phase column to obtain the crude product (90 mg) of $N^2$-(((9H-fluoren-9-yl)methoxy)carbonyl)glycylglycylglycylglycylglycyl-L-serylglycylglycylglycylglycyl-L-seryl-$N^6$-(((E)-cyclooct-4-en-1-yl)oxy)carbonyl)-L-lysine.

$N^2$-(((9H-fluoren-9-yl)methoxy)carbonyl)glycylglycylglycylglycylglycyl-L-serylglycylglycylglycylglycyl-L-seryl-$N^6$-(((E)-cyclooct-4-en-1-yl)oxy)carbonyl)-L-lysine (90 mg, 0.061 mmol) was dissolved in dimethyl sulfoxide (3.1 mL), and 1,8-diazabicyclo[5.4.0]-7-undecene (93 mg, 0.614 mmol) was added dropwise. The reaction solution was stirred at 25°C for 25 minutes. The reaction solution was purified by chromatography to obtain $N^6$-(((E)-cyclooct-4-en-1-yl)oxy)carbonyl)-$N^2$-glycylglycylglycylglycyl-L-serylglycylglycylglycylglycyl-L-seryllysine (14 mg).

Retention time: 0.43 min
m/z: 1243 [M-H]-

(Preparation Example 2-6-1-4-1: addition of peptide linker to FKBP by sortase reaction)

In a reaction buffer (50 mM Tris-HCl (pH 7.5), 150 mM NaCl, 10 mM CaCl2), 100 μM FLAG-Avi-bio-TEV-FKBP-Sor-His, 1 mM (G4S)2-K(MeTz) (synthesized at SCRUM Inc.) and 1 μM eSrtA were mixed on ice and incubated at 4°C overnight.

Samples before and after the sortase reaction were analyzed by SDS-PAGE to confirm that the reaction proceeded properly by checking the gel shift corresponding to the theoretical molecular weight of the protein of interest.

The crude sample after the reaction was mixed with Dynabeads His-Tag Isolation and Pulldown (Invitrogen, 10103D), and the mixture was mixed by inversion at 4°C for 10 minutes, and then the supernatant was recovered to obtain a crude purified sample. Then, the crude purified sample was purified using Strep-Tactin XT Sepharose chromatography resin (Cytiva, 29401324), a wash buffer (50 mM HEPES pH 7.5, 150 mM NaCl, 0.5 mM TCEP), and an elution buffer (50 mM HEPES pH 7.5, 150 mM NaCl, 0.5 mM TCEP, 50 mM biotin). The purification was performed according to the basic protocol attached to the manufacturing product. The sample after purification was recovered and concentrated with Amicon Ultra-0.5 10K (MERCK, #UFC501096) to obtain FLAG-Avi-bio-TEV-FKBP-(G4S)2-K(MeTz) in which the linker was added at the C-terminus of FKBP.

(Preparation Example 2-6-1-4-2: addition of peptide linker to FRB by sortase reaction)

In a reaction buffer (50 mM Tris-HCl, pH 7.5, 150 mM NaCl, 10 mM CaCl2), 100 μM FRB-FLAG-Sor-His, 1 mM (G4S)3-K(TCO) and 1 μM eSrtA were mixed on ice and incubated at 4°C overnight.

Samples before and after the sortase reaction were analyzed by SDS-PAGE to confirm that the reaction proceeded properly by checking the gel shift corresponding to the theoretical molecular weight of the protein of interest.

The crude sample after the reaction was mixed with Dynabeads His-Tag Isolation and Pulldown (Invitrogen, 10103D), and the mixture was mixed by inversion at 4°C for 10 minutes, and then the supernatant was recovered to obtain a crude purified sample. The crude purified sample was then purified using ANTI-FLAG(R) M2 Affinity Gel (Sigma, #A2220), a wash buffer (50 mM HEPES pH 7.5, 150 mM NaCl, 0.5 mM TCEP), and an elution buffer (50 mM HEPES pH 7.5, 150 mM NaCl, 0.5 mM TCEP, 500 μg/mL FLAG peptide). The purification was performed according to the basic protocol attached to the manufacturing product. The sample after purification was recovered and concentrated with Amicon Ultra-0.5 10K (MERCK, #UFC501096) to obtain FRB-FLAG-(G4S)3-K(TCO) in which a linker was added at the C-terminus of FRB.

(Preparation Example 2-6-1-4-3: synthesis of FKBP-FRB fusion protein by click reaction)

A complex FLAG-Avi-bio-TEV-FKBP-(G4S)2-K(MeTz) in which a linker was added at the C-terminus of FKBP and a complex FRB-FLAG-(G4S)3-K(TCO) in which a linker was added at the C-terminus of FRB were mixed and incubated at 4°C for 2 hours. Samples before and after mixing were analyzed by SDS-PAGE to confirm that the reaction proceeded properly by checking the gel shift corresponding to the theoretical molecular weight of the protein of interest.

The concentrated sample was purified by gel filtration using Superdex 75 Increase 10/300 GL(Cytiva, #29148721). For purification, a running buffer (50 mM HEPES pH 7.5, 300 mM NaCl, 1 mM DTT) was used. A fraction containing the protein of interest was recovered to obtain the protein of interest FLAG-Avi-bio-TEV-FKBP-FLAG-FRB (C-to-C) in which the C-terminus of FRB and the N-terminus of FKBP were linked with a peptide linker.

<Preparation Example 2-6-1-5: preparation of bio-Avi-TEV-FLAG-FKBP-FRB-FLAG (N-toN)>

To GL-FKBP-FLAG-TEV-Avi-bio containing an OaAEP1 recognition sequence at the N-terminus prepared in

Preparation Example 3-1 and GL-FRB-FLAG containing an OaAEP1 recognition sequence at the N-terminus prepared in Preparation Example 2-6-1-1, linkers with a methyltetrazine (MeTz) functional group and a transcyclooctene (TCO) functional group were added at the N-terminus, respectively, using OaAEP1 prepared in Preparation Example 3-2. The obtained proteins were linked by a click reaction to prepare a fusion protein in which the N-terminus of FRB and the N-terminus of FKBP were linked with a peptide linker. "K*" represents biotinylated Lys, "(*8)G" represents Gly in which a methyltetrazine functional group was added to the N-terminus, and "(*2)G" represents Gly in which a transcyclooctene functional group was added to the N-terminus. "(*8)G" and "(*2)G" are linked by a click reaction as in "(*9)" or "(*10)". The structure formulas of "(*8)G", "(*9)", and "(*10)" are shown in Table 48.

[Table 48]

| Name | Structure formula |
|---|---|
| (* 8) G | 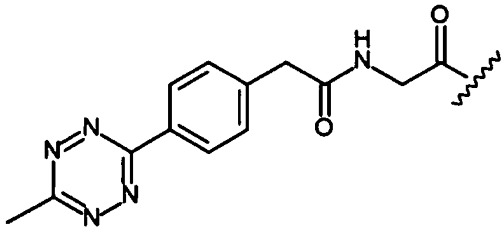 |
| (* 9) | 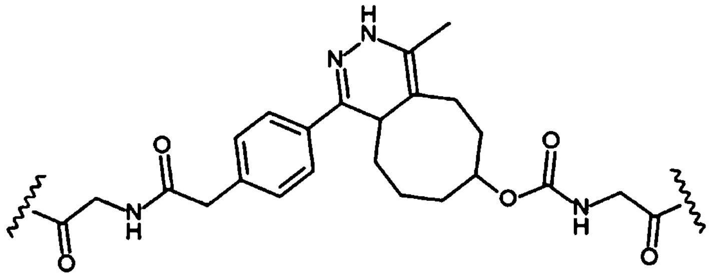 |
| (* 10) | 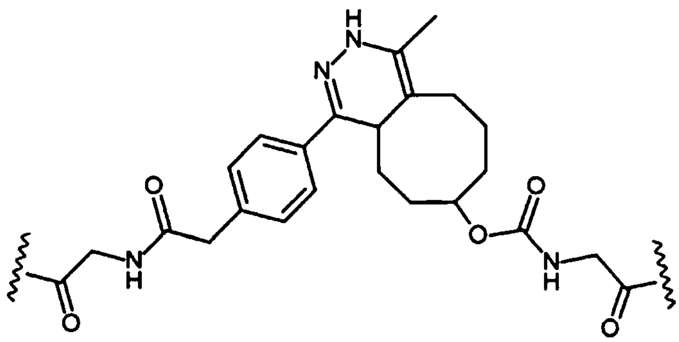 |

Amino acid sequence of bio-Avi-TEV-FLAG-FKBP-FRB-FLAG (N-to-N) (SEQ ID NO: 66 and SEQ ID NO: 67)

(*8)GGSGGGGSGGGGSNGLGGGGSGGGGSGVQVETISPGDGRTFPKRGQTCVVHYTGMLEDGKKFDSSRDRNKPFKFMLGKQEVIRGWEEGVAQMSVGQRAKLTISPDYAYGATGHPGIIPPHATLVFDVELLKLEGGGSSDYKDDDDKSSGENLYFQSSGGGLNDIFEAQK*IEWHE

(*2)GGSGGGGSGGGGSNGLGGGGSGGGGSELIRVAILWHEMWHEGLEEASRLYFGERNVKGMFEVLEPLHAMMERGPQTLKETSFNQAYGRDLMEAQEWCRKYMKSGNVKDLTQAWDLYYHVFRRISKQGGGSSDYKDDDDK

<Synthesis Example 2-6-2: synthesis of (MeTz)-GGS-(G4S)2-NGLH>

[Formula 69]

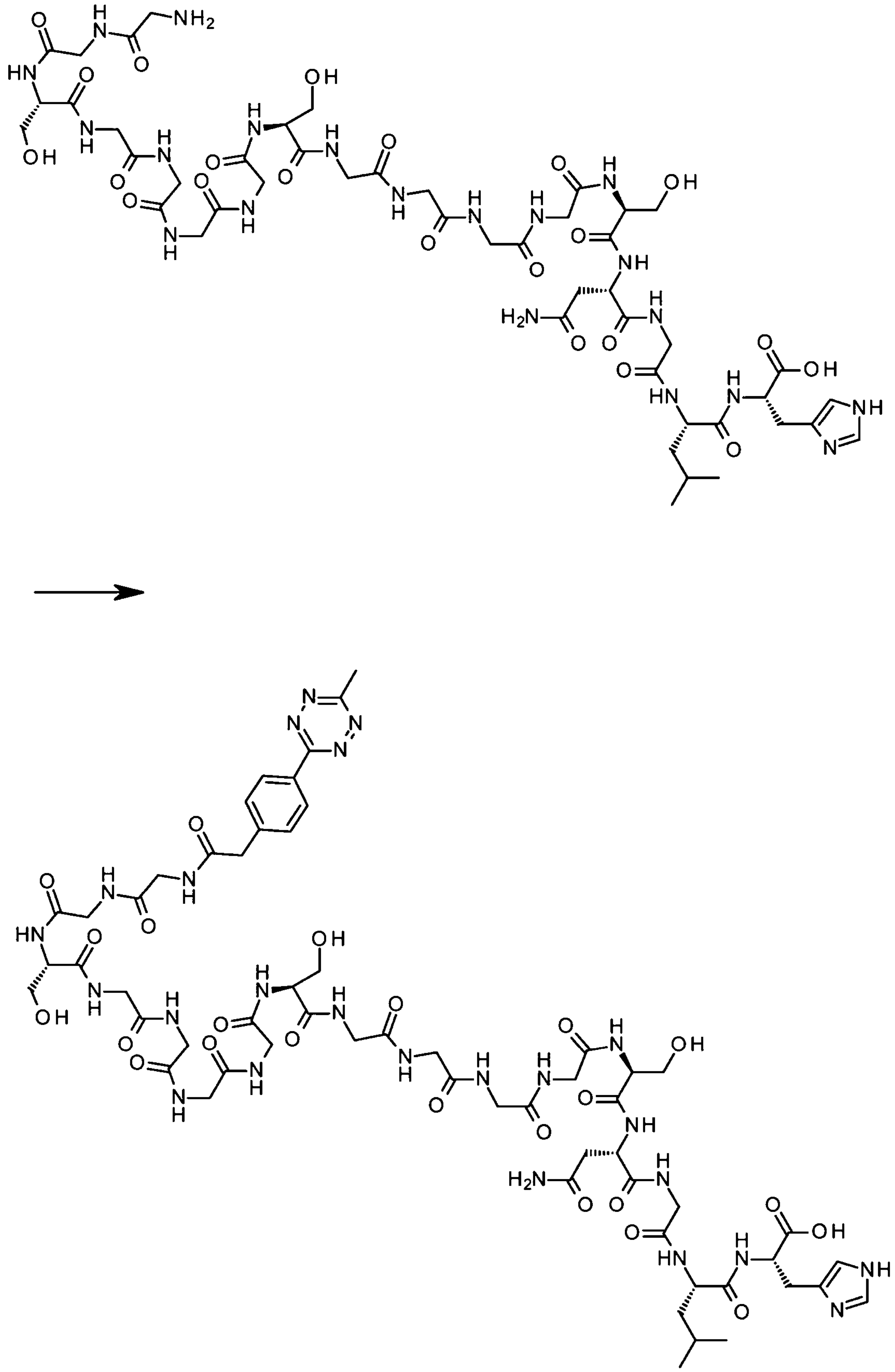

Serylglycylglycylglycyl-L-serylglycylglycylglycyl-L-seryl-L-aspartylglycyl-L-leucyl-L-histidine (31.4 mg, 0.025

mmol) and 2,5-dioxo-1-pyrrolidinyl-4-(6-methyl-1,2,4,5-tetrazin-3-yl)benzene-acetate (24.25 mg, 0.074 mmol) were dissolved in dimethyl sulfoxide (494 μL) and N-ethyl diisopropylamine (21.9 μL, 0.124 mmol) was added dropwise. The reaction solution was stirred at 25°C for 60 minutes. The reaction solution was purified by chromatography to obtain (2-(4-(6-methyl-1,2,4,5-tetrazin-3-yl)phenyl)acetyl)glycylglycyl-L-serylglycylglycylglycyl-L-serylglycylglycylglycyl-L-seryl-L-aspartylglycyl-L-leucyl-L-histidine (26 mg, 71% yield).

Retention time: 0.37 min
m/z: 1482 [M-H]-

(Preparation Example 2-6-1-5-1: addition of peptide linker to FKBP by OaAEP1 reaction)

Specifically, 48 μM GL-FKBP-FLAG-TEV-Avi-bio, 1 mM (MeTz)-GGS-(G4S)2-NGLH, 400 μM $NiCl_2$, and 0.6 μM OaAEP1 were mixed on ice and incubated at 4°C overnight.

Samples before and after the OaAEP1 reaction were analyzed by SDS-PAGE to confirm that the reaction proceeded properly by checking the gel shift corresponding to the theoretical molecular weight of the protein of interest.

The crude sample after the reaction was purified using Strep-Tactin XT Sepharose chromatography resin (Cytiva, 29401324), a wash buffer (50 mM HEPES pH 7.5, 150 mM NaCl, 0.5 mM TCEP), and an elution buffer (50 mM HEPES pH 7.5, 150 mM NaCl, 0.5 mM TCEP, 50 mM biotin). The purification was performed according to the basic protocol attached to the manufacturing product. The sample after purification was recovered and concentrated with Amicon Ultra-0.5 10K (MERCK, #UFC501096) to obtain (MeTz)-GGS-(G4S)2-FKBP-FLAG-TEV-Avi-bio in which the linker was added at the N-terminus of FKBP.

(Preparation Example 2-6-1-5-2: addition of peptide linker to FRB by OaAEP1 reaction)

76 μM GL-FRB-FLAG, 1 mM (TCO)-GGS-(G4S)2-NGLH, 400 μM $NiCl_2$, and 0.6 μM OaAEP1 were mixed on ice and incubated at 4°C overnight.

Samples before and after the OaAEP1 reaction were analyzed by SDS-PAGE to confirm that the reaction proceeded properly by checking the gel shift corresponding to the theoretical molecular weight of the protein of interest.

The crude sample after reaction was then purified using ANTI-FLAG(R) M2 Affinity Gel (Sigma, #A2220), a wash buffer (50 mM HEPES pH 7.5, 150 mM NaCl, 0.5 mM TCEP), and an elution buffer (50 mM HEPES pH 7.5, 150 mM NaCl, 0.5 mM TCEP, 500 μg/mL FLAG peptide). The purification was performed according to the basic protocol attached to the manufacturing product. The sample after purification was recovered and concentrated with Amicon Ultra-0.5 10K (MERCK, #UFC501096) to obtain (TCO)-GGS-(G4S)2-FRB-FLAG in which a linker was added at the N-terminus of FRB.

(Preparation Example 2-6-1-5-3: synthesis of FKBP-FRB fusion protein by click reaction)

(MeTz)-GGS-(G4S)2-FKBP-FLAG-TEV-Avi-bio in which a linker was added at the N-terminus of FKBP and (TCO)-GGS-(G4S)2-FRB-FLAG in which a linker was added at the N-terminus of FRB were mixed and incubated at 4°C for 2 hours. Samples before and after mixing were analyzed by SDS-PAGE to confirm that the reaction proceeded properly by checking the gel shift corresponding to the theoretical molecular weight of the protein of interest.

The concentrated sample was purified by gel filtration using Superdex 75 Increase 10/300 GL(Cytiva, #29148721). For purification, a running buffer (50 mM HEPES pH 7.5, 300 mM NaCl, 1 mM DTT) was used. A fraction containing the protein of interest was recovered to obtain the protein of interest bio-Avi-TEV-FLAG-FKBP-FRB-FLAG (N-to-N) in which the N-terminus of FRB and the N-terminus of FKBP were linked with a peptide linker.

<Evaluation Example 2-6-1: pull-down assay of mRNA-peptide display molecules (FF-01 to FF-04) for fusion proteins with different linkings of N- and C-termini of proteins to each other>

To a buffer containing 1 × TBS, 2 mg/mL BSA (Invitrogen), and 2 mM DTT, the display molecules prepared in a method according to Preparation Example 2-1-2 was added to be 0.2-0.3 μM, and the target protein was added to be as shown in Table 49, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.04 mM, and the mixture was mixed by inversion at 4°C for another 10 minutes. The target proteins prepared in Preparation Examples 2-6-1-2 to 2-6-1-5 were used as the target proteins, and those in five conditions shown in the following table were compared.

[Table 49]

| Condition | Target protein | Linking |
|---|---|---|
| **1** | **0.4 μM FLAG-Avi-bio-TEV-FKBP-FRB-FLAG (N-to-C)** | **N-to-C** |
| **2** | **0.4 μM FRB-FLAG-FKBP-FLAG-TEV-Avi-bio (C-to-N)** | **C-to-N** |
| **3** | **0.4 μM FLAG-Avi-bio-TEV-FKBP-FLAG-FRB (C-to-C)** | **C-to-C** |
| **4** | **0.4 μM bio-Avi-TEV-FLAG-FKBP-FRB-FLAG (N-to-N)** | **N-to-N** |
| **5** | No target protein | - |

Conditions 1 to 4 are conditions in which fusion proteins of FKBP and FRB are used. They are states in which the N-terminus of the FRB and the C-terminus of the FKBP are linked (N-to-C), the C-terminus of the FRB and the N-terminus of the FKBP are linked (C-to-N) , the C-termini of the FRB and the FKBP are linked (C-to-C), and the N-termini of the FRB and the FKBP are linked (N-to-N), respectively, and their linkings between the termini of the two kinds of target proteins are different but the two kinds of target proteins are in proximity. Condition 5 is a condition under the absence of the target protein.

The recovery rates of display molecules of FF-01 to FF-04 in conditions 1 to 5 were calculated by the method according to Evaluation Example 2-5-1 and summarized in Table 50.

[Table 50]

| Condition | Recovery rate (%) | | | |
|---|---|---|---|---|
| | **FF-01** | **FF-02** | **FF-03** | **FF-04** |
| **1** | **2.08E+00** | **7.11E-01** | **4.62E+00** | **3.45E+00** |
| **2** | **7.86E+00** | **1.06E+00** | **7.47E+00** | **4.68E+00** |
| **3** | **7.00E+00** | **7.44E-01** | **5.87E+00** | **2.78E+00** |
| **4** | **5.33E+00** | **1.27E+00** | **9.68E+00** | **5.39E+00** |
| **5** | **1.25E-06** | **2.48E-06** | **1.01E-05** | **4.83E-06** |

As a result, it was revealed that in FF-01 to FF-04 the recovery rates in conditions 1 to 4, where two kinds of target proteins, FKBP and FRB, were in proximity to each other by the fusion protein prepared in Preparation Examples 2-6-1-2 to 2-6-1-5, were at least 287000-fold higher than the recovery rate in condition 5 where the target proteins were absent.

Thus, it was found that the effect of the invention can be obtained by adopting any of the linkings between the termini of the proteins, that are the N-terminus and the C-terminus, the C-terminus and the N-terminus, between the C-termini, or the N-termini of the proteins to make the two target proteins be in proximity in this system. This can be said to suggest that the effect of the invention can be obtained in all four kinds of the linkings in this system.

<Evaluation Example 2-7: verification for conditions for coexistence of fusion protein and non-biotinylated single protein>

Evaluation Examples 2-1, 2-2 and 2-3 have shown that peptides that form complexes with a plurality of targets can be efficiently pulled down by using fusion proteins. To further efficiently concentrate the peptide that forms a complex with a plurality of targets, the present inventors have studied here whether it is possible to more efficiently concentrate molecules that form complexes with a plurality of targets while inhibiting concentration of molecules that cannot form complexes with a plurality of targets by performing panning the biotinylated fusion proteins in the coexistence of a non-biotinylated single protein.

<Evaluation Example 2-7-1: verification with peptide with FKBP and FRB as target proteins>

Whether or not peptides that form complexes with a plurality of targets can be more selectively pulled down was evaluated by performing selection in the state of coexistence of biotinylated fusion protein and non-biotinylated single protein. Specifically, it was verified by comparing the pull-down efficiency of the mRNA display molecule of the peptide (FF-01, FF-02, FF-03, FF-04) forming a ternary complex with FKBP and FRB obtained by Evaluation Example 1-1 between in the presence and the absence of non-biotinylated single protein.

(Preparation Example 2-7-1-1: preparation of mRNA-peptide display molecules (FF-01 to FF-04))

Puromycin was linked via a linker to the 3' end of mRNA purified in Preparation Example 2-1-1 according to the method described in WO2013/100132. It is noted that axPM-27 described above was used as a puromycin linker. This was translated, and subjected to a cyclization reaction, a desulfurization reaction, and a reverse transcription according to the method described in WO2017/150732 to prepare a display molecule of mRNA-peptide. The aminoacylated tRNA and the translation system used in the translation reaction were equivalent to those in Preparation Example 2-1-2 except that the concentration of magnesium acetate in the translation system was 6 mM.

(Evaluation Example 2-7-1-2: pull-down assay of mRNA-peptide display molecule (FF-01 to FF-04))

To a buffer containing 1 × TBS, 2 mg/mL BSA (Invitrogen), and 2 mM DTT, the display molecules prepared in Preparation Example 2-7-1-1 was added to be 0.2 μM, and the target protein was added to be as shown in Table 51, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.08 mM, and the mixture was mixed by inversion at 4°C for another 10 minutes. The proteins prepared in Preparation Examples 1-3 and 1-5 of Experimental Example 1 were used as the target proteins, and those in two conditions shown in the following table were compared.

[Table 51]

| Condition | Target protein |
|---|---|
| 1 | 0.4 μM bio-Avi-TEV-FLAG-FKBP-FRB-His |
| 2 | 0.4 μM bio-Avi-TEV-FLAG-FKBP-FRB-His and 4 μM FKBP-FLAG-Sor-His |

Condition 1 is a condition in which only biotinylated fusion protein is present, and Condition 2 is a condition in which biotinylated fusion protein coexist with non-biotinylated FKBP.

Streptavidin-coated magnetic beads with immobilized target proteins were collected with a magnet and the supernatant was removed. The collected beads were washed several times with a wash buffer (1 × TBS, 2 mM DTT, 0.1% Tween 20 (Sigma Aldrich), 500 mM arginine), then treated with a TEV protease that recognizes and cleaves the TEV protease recognition sequence to elute the target protein and the peptide-nucleic acid conjugate that binds to the target protein.

To the streptavidin-coated magnetic beads after washing, 1 × TEV buffer, 1 mM DTT, and 0.1 U/μL AcTEV protease (Thermo Fisher Scientific Inc., #12575015) were added as a specific TEV eluent to react with. After the reaction, the supernatant was recovered and qPCR was performed. For the qPCR primer, the aforementioned primers (Fw-Primer and Rv-Primer) were used. For the qPCR reaction solution, 1 × Ex Taq buffer, 0.2 mM dNTP, 0.5 μM Fw-Primer, 0.5 μM Rv-Primer, 50000-fold diluted SYBR Green I (LONZA, #50513), and 0.025 U/μL Ex Taq polymerase (Takara Bio Inc.) were mixed. The mixture was heated at 95°C for 2 minutes, and then subjected to 40 cycles of 95°C for 10 seconds, 57°C for 20 seconds, and 72°C for 30 seconds. In this case, each display molecule before mixing with the target protein was also subjected to qPCR in the same way.

To draw a calibration curve to estimate the recovery rate of the library, a sample prepared by reverse transcription of the mRNA-puromycin linked construct prepared in Preparation Example 4-3 of Experimental Example 1 was diluted to be 1E + 8/μL, 1E + 6/μL, and 1E + 4/μL, and subjected to qPCR under the same conditions.

The recovery rate was calculated by dividing the amount of nucleic acid of each display molecule recovered by binding to a target protein by the amount of nucleic acid of each display molecule that was present before mixing with the target protein (input value).

Recovery rate (%) = amount (number of molecules) of recovered nucleic acid /amount (number of molecules) of input nucleic acid × 100

The recovery rates of each display molecule of FF-01 to FF-04, and the ratios obtained by dividing the recovery rate in condition 1 by the recovery rate in condition 2 were summarized in Table 52.

[Table 52]

| Peptide | Recovery rate (%) in condition 1: (1) | Recovery rate (%) in condition 2: (2) | Ratio of recovery rate: (1)/(2) |
|---|---|---|---|
| **FF-01** | **8.52E+00** | **8.86E+00** | **1.0** |
| **FF-02** | **3.30E+00** | **2.56E+00** | **1.3** |
| **FF-03** | **1.14E+01** | **1.22E+01** | **0.9** |
| **FF-04** | **9.97E+00** | **1.02E+01** | **1.0** |

As a result, it was revealed that the change of recovery rates of the four peptides (FF-01 to FF-04) that can form a complex with FKBP and FRB was only reduction of 1.3-fold at most, even in the presence of non-biotinylated FKBP. It is believed that this is because the four peptides preferentially bind to the fusion proteins compared to the non-biotinylated FKBP. On the other hand, in the case of peptides that cannot form a complex with FKBP and FRB but can bind to FKBP, the existence of non-biotinylated FKBP should cause not only binding to the FKBP contained in the biotinylated fusion protein, but also binding to the non-biotinylated FKBP in an equal extent, so that the recovery efficiency thereof is expected to be reduced. It is thus expected that when performing panning with fusion proteins, it is possible to more selectively concentrate peptides that forms a complex with a plurality of kinds of target molecules by coexisting the fusion proteins with a non-biotinylated single protein.

<Evaluation Example 2-7-2: verification with peptide with cyclophilin A and calcineurin as target proteins>

Whether or not peptides that form complexes with a plurality of targets can be more selectively pulled down was evaluated by performing selection in the state of coexistence of biotinylated fusion protein and non-biotinylated single protein. Specifically, it was verified by comparing the pull-down efficiency of the mRNA display molecule of the peptide (CC-01, CC-02, CC-03, CC-04) forming a ternary complex with cyclophilin A and calcineurin obtained by Evaluation Example 1-2 between in the presence and the absence of non-biotinylated single protein.

(Preparation Example 2-7-2-1: preparation of mRNA-peptide display molecules (CC-01 to CC-04))

Puromycin was linked via a linker to the 3' end of mRNA purified in Preparation Example 2-2-2 according to the method described in WO2013/100132. It is noted that axPM-27 described above was used as a puromycin linker. This was translated, and subjected to a cyclization reaction, a desulfurization reaction, and a reverse transcription according to the method described in WO2017/150732 to prepare a display molecule of mRNA-peptide. The aminoacylated tRNA and the translation system used in the translation reaction were equivalent to those in Preparation Example 2-1-2 except that the concentration of magnesium acetate in the translation system was 6 mM.

(Evaluation Example 2-7-2-2: pull-down assay of mRNA-peptide display molecule (CC-01 to CC-04))

To a buffer containing 1 × TBS, 2 mg/mL BSA (Invitrogen), 2 mM DTT, and 5 mM $CaCl_2$, the display molecule prepared in Preparation Example 2-7-2-1 was added to be 0.2 μM, Calmodulin (FUJIFILM Wako Pure Chemical Corporation) was added to be 2 μM and the target protein was added to be as shown in Table 53, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.08 mM, and the mixture was mixed by inversion at 4°C for another 10 minutes. The proteins prepared in Preparation Examples 2-3 and 2-5 of Experimental Example 1 were used as the target proteins, and those in two conditions shown in the following table were compared.

[Table 53]

| Condition | Target protein |
|---|---|
| **1** | 0.4 μM His-CnB-CnA-CyA-FLAG-TEV-Avi-bio |
| **2** | 0.4 μM His-CnB-CnA-CyA-FLAG-TEV-Avi-bio and 4 μM CyA-FLAG-Sor-His |

Condition 1 is a condition in which only biotinylated fusion protein is present, and Condition 2 is a condition in which biotinylated fusion protein coexist with non-biotinylated cyclophilin A.

Streptavidin-coated magnetic beads with immobilized target proteins were collected with a magnet and the supernatant was removed. The collected beads were washed several times with a wash buffer (1 × TBS, 2 mM DTT, 0.1% Tween 20 (Sigma Aldrich), 500 mM arginine), then treated with a TEV protease that recognizes and cleaves the TEV protease recognition sequence to elute the target protein and the peptide-nucleic acid conjugate that binds to the target protein.

To the streptavidin-coated magnetic beads after washing, 1 × TEV buffer, 1 mM DTT, and 0.1 U/μL AcTEV protease (Thermo Fisher Scientific Inc., #12575015) were added as a specific TEV eluent to react with. After the reaction, the supernatant was recovered and qPCR was performed. For the qPCR primer, the aforementioned primers (Fw-Primer and Rv-Primer) were used. For the qPCR reaction solution, 1 × Ex Taq buffer, 0.2 mM dNTP, 0.5 μM Fw-Primer, 0.5 μM Rv-Primer, 50000-fold diluted SYBR Green I (LONZA, #50513), and 0.025 U/μL Ex Taq polymerase (Takara Bio Inc.) were mixed. The mixture was heated at 95°C for 2 minutes, and then subjected to 40 cycles of 95°C for 10 seconds, 57°C for 20 seconds, and 72°C for 30 seconds. In this case, each display molecule before mixing with the target protein was also subjected to qPCR in the same way.

To draw a calibration curve to estimate the recovery rate of the library, a sample prepared by reverse transcription of the mRNA-puromycin linked construct prepared in Preparation Example 4-3 of Experimental Example 1 was diluted to be 1E + 8/μL, 1E + 6/μL, and 1E + 4/μL, and subjected to qPCR under the same conditions.

The recovery rate was calculated by dividing the amount of nucleic acid of each display molecule recovered by binding to a target protein by the amount of nucleic acid of each display molecule that was present before mixing with the target protein (input value).

Recovery rate (%) = amount (number of molecules) of recovered nucleic acid /amount (number of molecules) of input nucleic acid × 100

The recovery rates of each display molecule of CC-01 to CC-04, and the ratios obtained by dividing the recovery rate in condition 1 by the recovery rate in condition 2 were summarized in Table 54.

[Table 54]

| Peptide | Recovery rate (%) in condition 1: (1) | Recovery rate (%) in condition 2: (2) | Ratio of recovery rate: (1)/(2) |
|---|---|---|---|
| **CC-01** | **9.29E-01** | **8.04E-01** | **1.2** |
| **CC-02** | **5.27E+00** | **4.21E+00** | **1.3** |
| **CC-03** | **2.10E+00** | **2.04E+00** | **1.0** |
| **CC-04** | **3.22E+00** | **1.43E+00** | **2.2** |

As a result, it was revealed that the change of recovery rates of the four peptides (CC-01 to CC-04) that can form a complex with cyclophilin A and calcineurin was only reduction of 2.2-fold at most, even in the presence of non-biotinylated cyclophilin A. It is believed that this is because the four peptides preferentially bind to the fusion proteins compared to the non-biotinylated cyclophilin A. On the other hand, in the case of peptides that cannot form a complex with cyclophilin A and calcineurin but can bind to cyclophilin A, the existence of non-biotinylated cyclophilin A should cause not only binding to the cyclophilin A contained in the biotinylated fusion protein, but also binding to the non-biotinylated cyclophilin A in an equal extent, so that the recovery efficiency thereof is expected to be reduced. It is thus expected that when performing panning with fusion proteins, it is possible to more selectively concentrate peptides that forms a complex with a plurality of kinds of target molecules by coexisting the fusion proteins with a non-biotinylated single protein.

From the results of Evaluation Examples 2-7-1 and 2-7-2, it is expected that the peptide (molecule of interest) that forms a complex with the plurality of kinds of target proteins can be efficiently obtained from among molecules including other peptides (candidate molecules) present in the library by performing panning the target fusion protein (target fusion molecule) in the coexistence of a non-biotinylated single protein.

[Experimental Example 3: effect verification of method using plurality of kinds of target proteins immobilized on streptavidin-coated magnetic beads for panning]

To improve the efficiency of obtaining peptides forming a complex with a plurality of kinds of target proteins, it was examined whether or not the same effect could be obtained by making the plurality of kinds of target proteins in proximity by a method other than the formation of fusion proteins.

Specifically, it was verified whether an improvement effect of a recovery rate of a peptide forming a complex with

two kinds of target proteins can be obtained when the two kinds of biotinylated target proteins were immobilized on streptavidin-coated magnetic beads to make the both target proteins be in proximity on the streptavidin-coated magnetic beads.

[Evaluation Example 3: verification using target protein immobilized on streptavidin-coated magnetic beads]

<Evaluation Example 3-1: verification using FKBP and FRB immobilized on streptavidin-coated magnetic beads as targets>

FKBP and FRB were selected as target proteins, and FF-01, FF-02, FF-03, and FF-04 synthesized in Synthesis Example 1 were selected as peptides forming a ternary complex with these two kinds of target proteins, then the recovery rate of the mRNA display molecule of each peptide was measured. The mRNA-peptide display molecule was prepared in the same manner as in Preparation Example 2-1-2.

To a buffer containing 1 × TBS, 2 mg/mL BSA (Invitrogen) and 2 mM DTT, the display molecules prepared in Preparation Example 2-1-2 were added to be 0.3 μM, and FKBP-FLAG-TEV-Avi-bio prepared in Preparation Example 1-1 and FRB-FLAG-TEV-Avi-bio prepared in Preparation Example 1-2 were added to be 0.4 μM, respectively, as target proteins, then the mixture was incubated at 4°C for 1 hour. This reaction solution was prepared to be total of 5 μL. Then, the resultant was mixed with streptavidin-coated magnetic beads such that the two kinds of target proteins in part are in proximity, and the mixture was mixed by inversion at 4°C for 10 minutes to immobilize the target protein. Biotin was then added to be 0.08 mM, and the mixture was mixed by inversion at 4°C for another 10 minutes. In this experiment, 3 μL of Dynabeads M-270 Streptavidin (Thermo Fisher Scientific Inc., https://www.veritastk.co.jp/products/PGN-DB0115.html) was used as streptavidin-coated magnetic beads.

Since the bead diameter of Dynabeads M-270 Streptavidin is 2.8 μm, its surface area is calculated to be $2.46 \times 10^{-11}$ $m^2$ per particle. In addition, since the concentration of Dynabeads M-270 Streptavidin is 10 mg/mL ($6 \times 10^8$ to $7 \times 10^8$ beads/mL), the number of beads used in this experiment is $1.80 \times 10^6$ to $2.10 \times 10^6$. Thus, the total surface area of the beads used in this experiment is calculated to be $4.43 \times 10^{-5}$ $m^2$ to $5.17 \times 10^{-5}$ $m^2$.

In addition, the amount of FKBP-FLAG-TEV-Avi-bio prepared in Preparation Example 1-1 and the amount of FRB-FLAG-TEV-Avi-bio prepared in Preparation Example 1-2 used in this experiment is calculated to be 2 pmol, respectively, and the total number of molecules is calculated to be $2.41 \times 10^{12}$.

From the above, in this experiment, it is calculated that target molecules of $4.66 \times 10^{16}$ to $5.43 \times 10^{16}$ are immobilized per 1 $m^2$ of the surface of the bead.

Display molecules pulled down in the same manner as in Evaluation Example 2-1 of Experimental Example 2 were recovered and quantified by qPCR, and the recovery rate thereof was calculated.

The recovery rate obtained by this method and the recovery rate obtained by Evaluation Example 2-1 of Experimental Example 2 were compared, and summarized in the following table.

Condition 1 is a condition in which a fusion protein is used, and the two kinds of target proteins are in proximity. Condition 2 is a condition in which FKBP and FRB are present, but only FKBP is immobilized on the streptavidin-coated magnetic beads, and a protein pulled down by the streptavidin-coated magnetic bead is also FKBP. Condition 3 is a condition in which FKBP and FRB are present, but only FRB is immobilized on the streptavidin-coated magnetic beads, and a protein pulled down by the streptavidin-coated magnetic bead is also FRB. In conditions 2 and 3, the two kinds of target proteins are not in proximity. Condition 4 is a condition in which both FKBP and FRB are biotinylated and it is presumed that two proteins are in proximity on the streptavidin-coated magnetic bead.

[Table 55]

| Peptide: FF-01 | | | |
|---|---|---|---|
| Condition | Target protein | Recovery rate (%) | Recovery rate in condition 4/ recovery rate |
| **1** | **0.4 μM bio-Avi-TEV-FLAG-FKBP-FRB-His** | **1.15E+01** | **0.8** |
| **2** | **0.4 μM FKBP-FLAG-TEV-Avi-bio** and **0.4 μM FRB-FLAG-Sor-His** | **1.02E-02** | **893** |
| **3** | **0.4 μM FRB-FLAG-TEV-Avi-bio** and **0.4 μM FKBP-FLAG-Sor-His** | **3.77E-04** | **24278** |
| **4** | **0.4 μM FRB-FLAG-TEV-Avi-bio** and **0.4 μM FKBP-FLAG-TEV-Avi-bio** | **9.14E+00** | **-** |

[Table 56]

| Peptide: FF-02 | | | |
|---|---|---|---|
| Condition | Target protein | Recovery rate (%) | Recovery rate in condition 4/ recovery rate |
| **1** | **0.4 μM bio-Avi-TEV-FLAG-FKBP-FRB-His** | **1.79E+00** | **0.3** |
| **2** | **0.4 μM FKBP-FLAG-TEV-Avi-bio** and **0.4 μM FRB-FLAG-Sor-His** | **4.87E-05** | **12860** |
| **3** | **0.4 μM FRB-FLAG-TEV-Avi-bio** and **0.4 μM FKBP-FLAG-Sor-His** | **9.80E-05** | **6397** |
| **4** | **0.4 μM FRB-FLAG-TEV-Avi-bio** and **0.4 μM FKBP-FLAG-TEV-Avi-bio** | **6.27E-01** | **-** |

[Table 57]

| Peptide: FF-03 | | | |
|---|---|---|---|
| Condition | Target protein | Recovery rate (%) | Recovery rate in condition 4/ |
| **1** | **0.4 μM bio-Avi-TEV-FLAG-FKBP-FRB-His** | **9.32E+00** | **0.6** |
| **2** | **0.4 μM FKBP-FLAG-TEV-Avi-bio** and **0.4 μM FRB-FLAG-Sor-His** | **2.68E-04** | **21137** |
| **3** | **0.4 μM FRB-FLAG-TEV-Avi-bio** and **0.4 μM FKBP-FLAG-Sor-His** | **7.06E-04** | **8023** |
| **4** | **0.4 μM FRB-FLAG-TEV-Avi-bio** and **0.4 μM FKBP-FLAG-TEV-Avi-bio** | **5.67E+00** | **-** |

[Table 58]

| Peptide: FF-04 | | | |
|---|---|---|---|
| Condition | Target protein | Recovery rate (%) | Recovery rate in condition 4/ recovery rate |
| **1** | **0.4 μM bio-Avi-TEV-FLAG-FKBP-FRB-His** | **7.11E+00** | **0.4** |
| **2** | **0.4 μM FKBP-FLAG-TEV-Avi-bio** and **0.4 μM FRB-FLAG-Sor-His** | **1.38E-04** | **18760** |
| **3** | **0.4 μM FRB-FLAG-TEV-Avi-bio** and **0.4 μM FKBP-FLAG-Sor-His** | **4.75E-04** | **5468** |
| **4** | **0.4 μM FRB-FLAG-TEV-Avi-bio** and **0.4 μM FKBP-FLAG-TEV-Avi-bio** | **2.60E+00** | **-** |

From the results of Tables 55 to 58, it was revealed that in these four peptides (FF-01 to FF-04) the recovery rate in condition 4, where two kinds of target proteins are presumed to be in proximity via streptavidin-coated magnetic beads, was at least 800-fold higher compared to the recovery rates in conditions 2 and 3, where two respective target proteins were added separately. It was also revealed that the recovery rate is equivalent to the recovery rate in condition 1 in which two kinds of target proteins were in proximity by a fusion protein. This strongly suggests that the peptide forming a ternary complex with FKBP and FRB can also be pulled down efficiently by making the two kinds of target proteins be in a state where two kinds of target proteins are presumed to be in proximity on the streptavidin-coated magnetic beads.

<Evaluation Example 3-2: verification using cyclophilin A and calcineurin immobilized on streptavidin-coated magnetic beads as targets>

Cyclophilin A and calcineurin were selected as target proteins, and CC-01, CC-02, CC-03, and CC-04

synthesized in Synthesis Example 1 were selected as peptides forming a ternary complex with these two kinds of target proteins, then the recovery rate of the mRNA display molecule of each peptide was measured. The mRNA-peptide display molecule was prepared in the same manner as in Preparation Example 2-2-1.

To a buffer containing 1 × TBS, 2 mg/mL BSA (Invitrogen), 2 mM DTT, and 5 mM $CaCl_2$, the display molecules prepared in Preparation Example 2-2-2 was added to be 0.3 μM, CyA-FLAG-TEV-Avi-bio prepared in Preparation Example 2-1 and CnB-CnA-FLAG-TEV-Avi-bio prepared in Preparation Example 2-2 were added to be 0.4 μM, respectively, as target proteins, and Calmodulin (FUJIFILM Wako Pure Chemical Corporation) was added to be 2 μM, then the mixture was incubated at 4°C for 1 hour. This reaction solution was prepared to be total of 5 μL. Then, the resultant was mixed with streptavidin-coated magnetic beads such that the two kinds of target proteins in part are in proximity, and the mixture was mixed by inversion at 4°C for 10 minutes to immobilize the target protein. Biotin was then added to be 0.08 mM, and the mixture was mixed by inversion at 4°C for another 10 minutes. In this experiment, 3 μL of Dynabeads M-270 Streptavidin (Thermo Fisher Scientific Inc.) was used as streptavidin-coated magnetic beads.

Here, the number of target molecules immobilized per 1 $m^2$ of the surface of the bead in this experiment is 4.66 × $10^{16}$ to 5.43 × $10^{16}$ according to the same calculation as in Evaluation Example 3-1.

Display molecules pulled down in the same manner as in Evaluation Example 2-2 of Experimental Example 2 were recovered and quantified by qPCR, and the recovery rate thereof was calculated.

The recovery rate obtained by this method and the recovery rate obtained by Evaluation Example 2-2 of Experimental Example 2 were compared, and summarized in the following table.

Condition 1 is a condition in which a fusion protein is used, and the two kinds of target proteins are in proximity. Condition 2 is a condition in which cyclophilin A and calcineurin are present, but only cyclophilin A is immobilized on the streptavidin-coated magnetic beads, and a protein pulled down by the streptavidin-coated magnetic bead is also cyclophilin A. Condition 3 is a condition in which cyclophilin A and calcineurin are present, but only calcineurin is immobilized on the streptavidin-coated magnetic beads, and a protein pulled down by the streptavidin-coated magnetic bead is also calcineurin. In conditions 2 and 3, the two kinds of target proteins are not in proximity. Condition 4 is a condition in which both cyclophilin A and calcineurin are biotinylated and it is presumed that two proteins are in proximity on the streptavidin-coated magnetic bead.

[Table 59]

Peptide: CC-01

| Condition | Target protein | Recovery rate (%) | Recovery rate in condition 4/ recovery rate |
|---|---|---|---|
| 1 | 0.4 μM His-CnB-CnA-CyA-FLAG-TEV-Avi-bio | 1.09E+00 | 0.8 |
| 2 | 0.4 μM CyA-FLAG-TEV-Avi-bio and 0.4 μM CnB-CnA-FLAG-Sor-His | 1.99E-02 | 41.5 |
| 3 | 0.4 μM CnB-CnA-FLAG-TEV-Avi-bio and 0.4 μM CyA-FLAG-Sor-His | 2.26E-02 | 36.5 |
| 4 | 0.4 μM CyA-FLAG-TEV-Avi-bio and 0.4 μM CnB-CnA-FLAG-TEV-Avi-bio | 8.25E-01 | - |

[Table 60]

Peptide: CC-02

| Condition | Target protein | Recovery rate (%) | Recovery rate in condition 4/ recovery rate |
|---|---|---|---|
| 1 | 0.4 μM His-CnB-CnA-CyA-FLAG-TEV-Avi-bio | 2.73E+00 | 0.9 |
| 2 | 0.4 μM CyA-FLAG-TEV-Avi-bio and 0.4 μM CnB-CnA-FLAG-Sor-His | 1.07E-02 | 219.4 |
| 3 | 0.4 μM CnB-CnA-FLAG-TEV-Avi-bio and 0.4 μM CyA-FLAG-Sor-His | 1.61E-02 | 146.1 |
| 4 | 0.4 μM CyA-FLAG-TEV-Avi-bio and 0.4 μM CnB-CnA-FLAG-TEV-Avi-bio | 2.36E+00 | - |

[Table 61]

Peptide: CC-03

| Condition | Target protein | Recovery rate (%) | Recovery rate in condition 4/ recovery rate |
|---|---|---|---|
| 1 | 0.4 μM His-CnB-CnA-CyA-FLAG-TEV-Avi-bio | 1.22E+00 | 2.0 |
| 2 | 0.4 μM CyA-FLAG-TEV-Avi-bio and 0.4 μM CnB-CnA-FLAG-Sor-His | 1.36E-03 | 1801.7 |
| 3 | 0.4 μM CnB-CnA-FLAG-TEV-Avi-bio and 0.4 μM CyA-FLAG-Sor-His | 1.28E-03 | 1915.1 |
| 4 | 0.4 μM CyA-FLAG-TEV-Avi-bio and 0.4 μM CnB-CnA-FLAG-TEV-Avi-bio | 2.45E+00 | - |

[Table 62]

Peptide: CC-04

| Condition | Target protein | Recovery rate (%) | Recovery rate in condition 4/ recovery rate |
|---|---|---|---|
| 1 | 0.4 μM His-CnB-CnA-CyA-FLAG-TEV-Avi-bio | 1.92E+00 | 0.7 |
| 2 | 0.4 μM CyA-FLAG-TEV-Avi-bio and 0.4 μM CnB-CnA-FLAG-Sor-His | 2.06E-03 | 689.1 |
| 3 | 0.4 μM CnB-CnA-FLAG-TEV-Avi-bio and 0.4 μM CyA-FLAG-Sor-His | 1.02E-03 | 1393.5 |
| 4 | 0.4 μM CyA-FLAG-TEV-Avi-bio and 0.4 μM CnB-CnA-FLAG-TEV-Avi-bio | 1.42E+00 | - |

From the results of Tables 59 to 62, it was revealed that in these four peptides (CC-01 to CC-04) the recovery rate in condition 4, where two kinds of target proteins are presumed to be in proximity via streptavidin-coated magnetic beads, was at least 35-fold higher compared to the recovery rates in conditions 2 and 3, where two respective target proteins were added separately. It was also revealed that the recovery rate is equivalent to the recovery rate in condition 1 in which two kinds of target proteins were in proximity by a fusion protein. This strongly suggests that the peptide forming a ternary complex with cyclophilin A and calcineurin can also be pulled down efficiently by making the two kinds of target proteins be in proximity on the streptavidin-coated magnetic beads.

<Evaluation Example 3-3: verification using FKBP and calcineurin immobilized on streptavidin-coated magnetic beads as targets>

FKBP and calcineurin were selected as target proteins, and CF-01 and CF-02 synthesized in Synthesis Example 1 were selected as peptides forming a ternary complex with these two kinds of target proteins, then the recovery rate of the mRNA display molecule of each peptide was measured. The mRNA-peptide display molecule was prepared in the same manner as in Preparation Example 2-3-1.

FKBP-FLAG-TEV-Avi-bio prepared in Preparation Example 1-1 and CnB-CnA-FLAG-TEV-Avi-bio prepared in Preparation Example 2-2 were added to a buffer containing 1 × TBS, 2 mg/mL BSA (Invitrogen), 2 mM DTT, and 5 mM $CaCl_2$ to be 0.4 μM, respectively, and then mixed with streptavidin-coated magnetic beads, and the mixture was mixed by inversion at 4°C for 10 minutes to immobilize the target protein such that the two kinds of target proteins were in proximity in part. This reaction solution was prepared to be total of 5 μL. Biotin was then added to be 0.5 mM, and the mixture was mixed by inversion at 4°C for another 10 minutes. In this experiment, 3 μL of Dynabeads M-270 Streptavidin (Thermo Fisher Scientific Inc.) was used as streptavidin-coated magnetic beads.

Here, the number of target molecules immobilized per 1 mof the surface of the bead in this experiment is $4.66 \times 10^{16}$ to $5.43 \times 10^{16}$ according to the same calculation as in Evaluation Example 3-1.

The target proteins immobilized streptavidin-coated magnetic beads were collected with a magnet and the supernatant was removed, and the beads were once washed with a wash buffer (1 × TBS, 2 mM DTT, 0.1% Tween 20 (Sigma Aldrich), 500 mM arginine), and then the mRNA-peptide display molecule was added to be 0.2-0.3 μM and Calmodulin (FUJIFILM Wako Pure Chemical Corporation) was added to be 2 μM, then the mixture was mixed by inversion at 4°C for 1 hour.

Display molecules pulled down in the same manner as in Evaluation Example 2-3 of Experimental Example 2 were recovered and quantified by qPCR, and the recovery rate thereof was calculated.

The recovery rate obtained by this method and the recovery rate obtained by Evaluation Example 2-3 of Experimental Example 2 were compared, and summarized in the following table.

Condition 1 is a condition in which a fusion protein is used, and the two kinds of target proteins are in proximity. Condition 2 is a condition in which FKBP and calcineurin are present, but only FKBP is immobilized on the streptavidin-coated magnetic beads, and a protein pulled down by the streptavidin-coated magnetic bead is also FKBP. Condition 3 is a condition in which FKBP and calcineurin exist, but only calcineurin is immobilized on the streptavidin-coated magnetic beads, and a protein pulled down by the streptavidin-coated magnetic bead is also calcineurin. In conditions 2 and 3, the two kinds of target proteins are not in proximity. Condition 4 is a condition in which both FKBP and calcineurin are biotinylated and it is presumed that two proteins are in proximity on the streptavidin-coated magnetic bead.

[Table 63]

Peptide: CF-01

| Condition | Target protein | Recovery rate (%) | Recovery rate in condition 4/ recovery rate |
|---|---|---|---|
| 1 | 0.4 μM CnB-CnA-FLAG-FKBP-FLAG-TEV-Avi-bio | 4.58E-01 | 4.3 |
| 2 | 0.4 μM FKBP-FLAG-TEV-Avi-bio and 0.4 μM CnB-CnA-FLAG-Sor-His | 4.17E-03 | 476.7 |
| 3 | 0.4 μM CnB-CnA-FLAG-TEV-Avi-bio and 0.4 μM FKBP-FLAG-Sor-His | 1.51E-02 | 131.6 |
| 4 | 0.4 μM CnB-CnA-FLAG-TEV-Avi-bio and 0.4 μM FKBP-FLAG-TEV-Avi-bio | 1.99E+00 | - |

[Table 64]

Peptide: CF-02

| Condition | Target protein | Recovery rate (%) | Recovery rate in condition 4/ recovery rate |
|---|---|---|---|
| 1 | 0.4 μM CnB-CnA-FLAG-FKBP-FLAG-TEV-Avi-bio | 1.09E+00 | 3.4 |
| 2 | 0.4 μM FKBP-FLAG-TEV-Avi-bio and 0.4 μM CnB-CnA-FLAG-Sor-His | 7.83E-03 | 477.0 |
| 3 | 0.4 μM CnB-CnA-FLAG-TEV-Avi-bio and 0.4 μM FKBP-FLAG-Sor-His | 2.14E-02 | 174.5 |
| 4 | 0.4 μM CnB-CnA-FLAG-TEV-Avi-bio and 0.4 μM FKBP-FLAG-TEV-Avi-bio | 3.73E+00 | - |

From the results of Tables 63 to 64, it was revealed that in these two peptides (CF-01, 02) the recovery rate in condition 4, where two kinds of target proteins are presumed to be in proximity via streptavidin-coated magnetic beads, was at least 130-fold higher compared to the recovery rates in conditions 2 and 3, where two respective target proteins were added separately. It was also revealed that the recovery rate is equivalent to the recovery rate in condition 1 in which two kinds of target proteins were in proximity by a fusion protein. This strongly suggests that the peptide forming a ternary complex with FKBP and calcineurin can also be pulled down efficiently by making the two kinds of target proteins be in proximity on the streptavidin-coated magnetic beads.

The results of Experimental Example 3 composed of Evaluation Example 3 show that the effect of the present invention seen in panning with fusion protein is also achieved in panning using target proteins immobilized on the same streptavidin-coated magnetic beads (solid phase support).

Although not intended to be bound by any particular theory, it is believed that this is possible because a plurality of kinds of target proteins in part are in proximity to each other by immobilizing the plurality of kinds of target proteins on streptavidin-coated magnetic beads (solid phase support), and a complex of the plurality of kinds of target proteins and a peptide becomes more stable.

The results of Experimental Example 2 and Experimental Example 3 show that making a plurality of kinds of target proteins be in proximity is extremely effective when obtaining a peptide that forms a complex with the plurality of kinds of target proteins by selection by affinity.

In addition, the results of Experimental Example 2 and Experimental Example 3 show that the use of a pull-down efficiency under the condition where a plurality of kinds of target proteins are in proximity, that is the recovery rate of a display molecule, as an indicator is extremely useful to screen for a molecule of interest that is a peptide capable of forming a complex with a plurality of kinds of target proteins.

[Experimental Example 4: effect verification of method using linked construct of display molecule and target protein in panning]

To improve the efficiency of obtaining a peptide that forms a complex with a plurality of kinds of proteins, the mRNA-display molecule and the target protein were focused as elements that form a complex, and the effect of making them be in proximity was verified.

Specifically, a linked construct in which an mRNA-display molecule and one target protein that was not biotinylated are linked was formed by utilizing an interaction between Tris-Ni-NTA and a polyhistidine tag (hereinafter also referred to as His tag) to make the mRNA-display molecule and the one target protein be in proximity, then whether the recovery rate of the peptide forming a complex with the one target protein and another target protein increases or not was

verified. It has been reported that the dissociation constant $K_D$ of Tris-Ni-NTA and polyhistidine tag is about 0.1-40 nM (J. Am. Chem. Soc., 2005, 127, pp.10205-10215).

[Preparation Example 4: preparation of reverse transcription primer having Ni-NTA]

<Synthesis Example 4-1: synthesis of DBCO-PEG(4)-NTA>

The abbreviation and structure of the synthesized compounds are as shown in Table 65 below.

[Table 65]

| Abbreviation | Structure | Name |
|---|---|---|
| NTA | 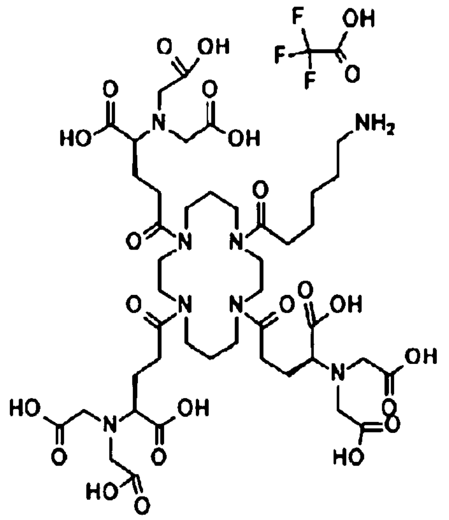 | (2S)-5-[11-(6-Aminohexanoyl)-4,8-bis[(4S)-4-[bis(carboxymethyl)amino]-4-carboxybutanoyl]-1,4,8,11-tetrazacyclotetradec-1-yl]-2-[bis(carboxymethyl)amino-5-oxopentanoic acid: 2,2,2-trifluoroacetic acid |
| DBCO-PEG(4) - NTA | 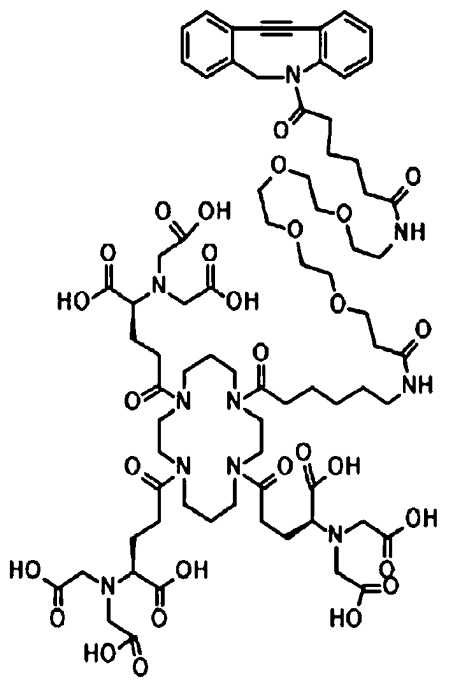 | (2S)-5-[11-[6-[3-[2-[2-[2-[2-[[6-(2-azatricyclo [10.4.0.04.9]hexad eca-1(12),4,6,8,13,15-hexaen-10-yn-2-yl)-6-oxohexanoyl]amino]ethoxy]ethoxy]ethoxy]ethoxy]propanoyl amino]hexanoyl]-4,8-bis[(4S)-4-[bis(carboxymethyl)amino]-4-carboxybutanoyl]-1,4,8,11-tetrazaclotetradec-1-yl]-2-[bis(carboxymethyl)amino-5-oxopentanoic acid |

The analysis conditions of LC-MS are as described below.

[Table 66]

| Preparative condition | Device | Column (I.D. × length (mm)) | Mobile phase | Gradient (A/B) | Flow rate (ml/min) | Column temperature (°C) | Wavelength |
|---|---|---|---|---|---|---|---|
| SQD FA05 | Acquity UPLC/SQD2 | Aldrich Ascentis Express C18 (2.1 × 50) | A)0.1% FA, $H_2O$ B) 0.1% FA, $CH_3CN$ | 95/5 ⇒0/100(1.0 min)⇒0/100(0.4 min) | 0.9 | 35 | 210-400nm PDA total |

Synthesis of NTA (ST01)

[Formula 70]

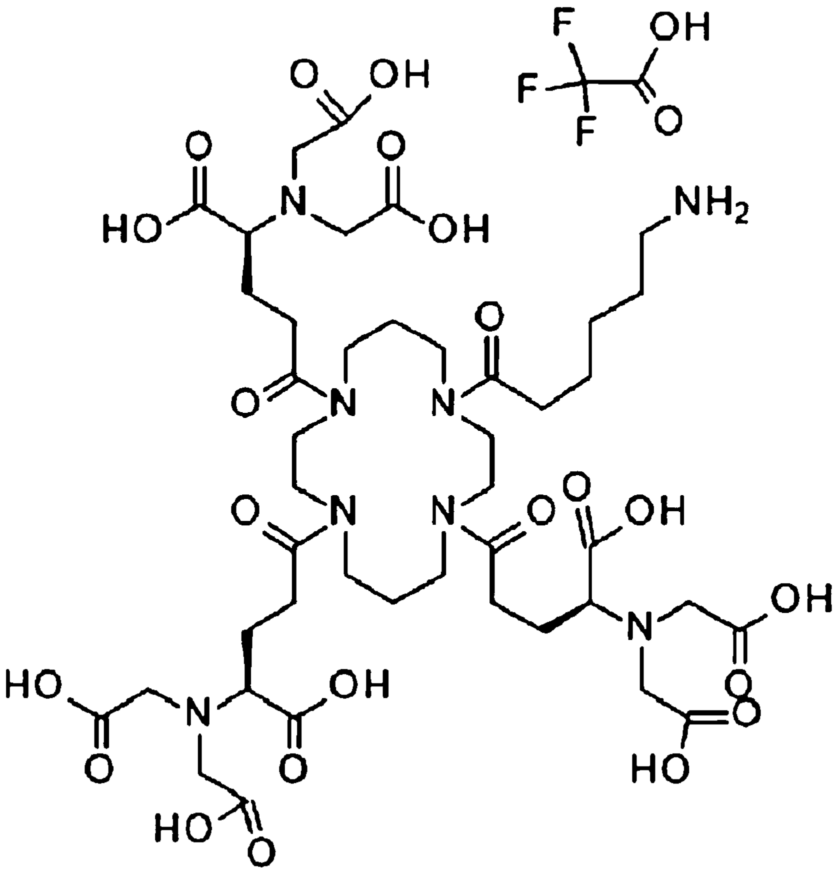

It was synthesized according to the method described in Non Patent Literature (J. Am. Chem. Soc., 2006, vol. 128, No. 7, pp. 2365-2372).

LCMS (ESI) m/z = 1049.9 (M + H)+
Retention time: 0.26 min (analysis condition SQD FA05)

Synthesis of DBCO-PEG(4)-NTA (ST02)

[Formula 71]

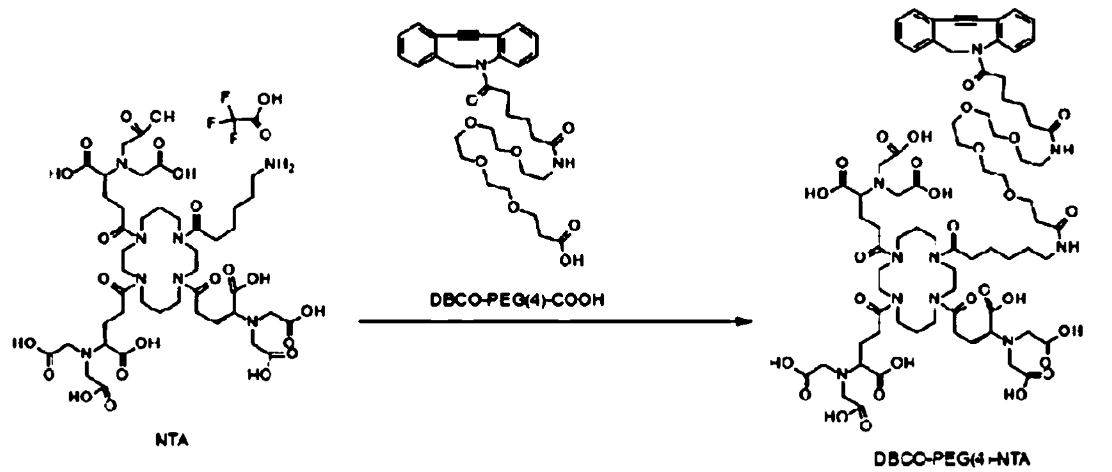


Under the nitrogen atmosphere, DBCO-PEG(4)-COOH (5.8 mg, 0.01 mmol) and HATU (34.9 mg, 0.03 mmol, 1.1 eq) were dissolved in DMF (50 μL), and then DIEPA (3.8 μL, 0.02 mmol, 2.0 eq) was added thereto, and the mixture was stirred at room temperature for 30 minutes. To this, a DMF solution (100 μL) in which NTA (35 mg, 3.0 eq) and DIEPA (22.8 μL, 0.12 mmol, 6.0 eq) were dissolved was added, and the mixture was stirred at room temperature for another 30 minutes.
The reaction solution was directly purified by reverse-phase silica gel chromatography (0.1% aqueous formic acid solution/0.1% formic acid in acetonitrile) to obtain the title compound (DBCO-PEG(4)-NTA, 6.7 mg, 42%).

LCMS (ESI) m/z = 1612.3 (M + H)+
Retention time: 0.57 min (analysis condition SQD FA05)

<Preparation Example 4-1: preparation of Tris-Ni-NTA added-reverse transcription primer]

0.7 mM reverse transcription primer (synthesized at Fasmac Co., Ltd.) having an azide group at the 5' side and 3.3 mM DBCO-PEG(4)-NTA were reacted in 67% DMSO solution containing 3.3 mM aqueous sodium acetate at 37°C for 24 hours. The reaction solution was purified by ethanol precipitation to prepare a reverse transcription primer modified with Tris-NTA at the 5' end. The prepared Tris-NTA reverse transcription primer (final concentration 90 μM) and aqueous nickel chloride solution (final concentration 100 mM) were mixed and reacted at room temperature for 1 hour. The reaction solution was purified by ethanol precipitation to prepare a Tris-Ni-NTA added-reverse transcription primer.
Nucleotide sequence of reverse transcription prime having an azide group at the 5' side (SEQ ID NO: 31)

[Formula 72]

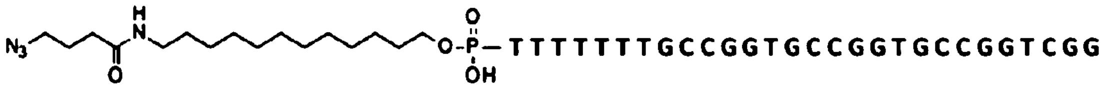


[Evaluation Example 4: verification using linked construct of display molecule and target protein]

<Evaluation Example 4-1: verification using display molecule-FKBP linked construct or display molecule-FRB linked construct>

FKBP and FRB were selected as target proteins, and FF-05, FF-06, and FF-07 synthesized in Synthesis Example 1 were selected as peptides forming a ternary complex with these two kinds of target proteins, then the recovery rate of the mRNA-display molecule of each peptide was measured.

(Preparation Example 4-1-1: synthesis of mRNA (FF-05 to FF-07))

From template DNA ((D-9) to (D-11)), mRNA ((mR-9) to (mR-11)) was synthesized by in vitro transcription reaction using T7 RNA polymerase and purified by RNAClean XP (Beckman Coulter, Inc.) or RNeasy MinElute Cleanup Kit (QIAGEN).

Nucleotide sequence of template DNA (D-9) (SEQ ID NO: 32) (DNA sequence: FF-05)

GGCGTAATACGACTCACTATAGGGTTAACTTTAATAAGGAGATATAAATATGGTTTG
CTCTTCTAGGTCTCTGAACCGGTAGCCGACCGGCACCGGCACCGGCAAAAAAA

Nucleotide sequence of template DNA (D-10) (SEQ ID NO: 33) (DNA sequence: FF-06)

GGCGTAATACGACTCACTATAGGGTTAACTTTAATAAGGAGATATAAATATGACTCT
GAACGAGTGCGGTCATTGCCATTAGCCGACCGGCACCGGCACCGGCAAAAAAA

Nucleotide sequence of template DNA (D-11) (SEQ ID NO: 34) (DNA sequence: FF-07)

GGCGTAATACGACTCACTATAGGGTTAACTTTAATAAGGAGATATAAATATGCAGTT
GGAGTGGTTTGTTTGGCCGACTTAGCCGACCGGCACCGGCACCGGCAAAAAAA

Nucleotide sequence of mRNA (mR-9) (SEQ ID NO: 35) (RNA sequence: FF-05)

GGGUUAACUUUAAUAAGGAGAUAUAAAUAUGGUUUGCUCUUCUAGGUCUCUGAA
CCGGUAGCCGACCGGCACCGGCACCGGCAAAAAAA

Nucleotide sequence of mRNA (mR-10) (SEQ ID NO: 36) (RNA sequence: FF-06)

GGGUUAACUUUAAUAAGGAGAUAUAAAUAUGACUCUGAACGAGUGCGGUCAUUG
CCAUUAGCCGACCGGCACCGGCACCGGCAAAAAAA

Nucleotide sequence of mRNA (mR-11) (SEQ ID NO: 37) (RNA sequence: FF-07)

GGGUUAACUUUAAUAAGGAGAUAUAAAUAUGCAGUUGGAGUGGUUUGUUUGGCC
GACUUAGCCGACCGGCACCGGCACCGGCAAAAAAA

(Preparation Example 4-1-2: preparation of mRNA-peptide display molecules (FF-05 to FF-07))

Puromycin was linked via a linker to the 3' end of mRNA purified in Preparation Example 4-1-1 according to the method described in WO2013/100132. It is noted that axPM-27 described above was used as a puromycin linker. This was translated, and subjected to a cyclization reaction, a desulfurization reaction, and a reverse transcription according to the method described in WO2017/150732 to prepare a display molecule of mRNA-peptide. The translation system and the aminoacylated tRNA required for translation were the same as in Preparation Example 2-1-2 of Experimental Example 2.

However, in the reverse transcription reaction, 1.1 equal amount of the following reverse transcription primer (reverse transcription primer without Tris-Ni-NTA) or the Tris-Ni-NTA-added reverse transcription primer prepared in Preparation Example 4-1 was added to the prepared sample, and the mixture was incubated at 70°C for 5 minutes, then incubated on ice for 5 minutes. The reagents of 1 × M-MLV RT Reaction buffer (Promega Corporation), 0.5 mM dNTP mixture, 8 U/μL M-MLV RT (H-) (Promega Corporation) were then added, and the mixture was reacted at 42°C for 1 hour.

Nucleotide sequence of reverse transcription primer (SEQ ID NO: 38)
TTTTTTTGCCGGTGCCGGTGCCGGTCGG

(Evaluation Example 4-1-1: pull-down assay of mRNA display molecule (FF-05 to FF-07))

To a buffer containing 1 × TBS, 2 mg/mL BSA (Invitrogen), and 2 mM DTT, the display library prepared in Preparation Example 4-1-2 was added to be 0.3 μM and the target protein was added to be as shown in Table 67, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.08 mM, and the mixture was mixed by inversion at 4°C for another 10 minutes. The proteins prepared in Preparation Examples 1-1 to 1-4 were used as the target proteins.

[Table 67]

| Condition | Target protein |
|---|---|
| **1** | **0.4 μM FKBP-FLAG-TEV-Avi-bio** and **0.4 μM FRB-FLAG-Sor-His** |
| **2** | **0.4 μM FRB-FLAG-TEV-Avi-bio** and **0.4 μM FKBP-FLAG-Sor-His** |
| **3** | **0.4 μM FKBP-FLAG-TEV-Avi-bio** |
| **4** | **0.4 μM FRB-FLAG-TEV-Avi-bio** |

Condition 1 is a condition in which FKBP is biotinylated, while only FRB is added with a His tag. Condition 2 is a condition in which FRB is biotinylated, while only FKBP is added with a His tag. Thus, the mRNA-display molecules which were reverse-transcribed with the Tris-Ni-NTA-added reverse transcription primer were to be incubated in the system in a state that the mRNA-display molecule and the non-biotinylated protein are in proximity. Conditions 3 and 4 are conditions in which only FKBP and only FRB, which have been biotinylated but do not have a His tag, are present, respectively.

Streptavidin-coated magnetic beads with immobilized target proteins were collected with a magnet and the supernatant was removed. This was washed once with a buffer containing 1 × TBS, 2 mg/mL BSA, and 2 mM DTT when using a reverse transcription primer without Tris-Ni-NTA, and once with a buffer containing 1 × TBS, 2 mg/mL BSA, 2 mM DTT, and 500 mM imidazole when using a Tris-Ni-NTA added reverse transcription primer. The washed streptavidin-coated magnetic beads were washed several times with a wash buffer (1 × TBS, 2 mM DTT, 0.1% Tween 20 (Sigma Aldrich), 500 mM arginine), then treated with a TEV protease that recognizes and cleaves the TEV protease recognition sequence to elute the target protein and the peptide-nucleic acid conjugate that binds to the target protein.

Display molecules pulled down in the same manner as in Evaluation Example 2-1 of Experimental Example 2 were recovered and quantified by qPCR, and the recovery rate thereof was calculated and summarized in the following table. Further, in conditions 1 to 4, the cases in the presence or absence of Tris-Ni-NTA were also compared.

[Table 68]

| Peptide: FF-05 | | |
|---|---|---|
| Condition | **Tris-Ni-NTA** | |
| | (-) | (+) |
| | Recovery rate (%) | Recovery rate (%) |
| **1** | **6.58E-04** | **7.31E-02** |
| **2** | **7.76E-04** | **2.76E-02** |
| **3** | **2.22E-05** | **2.61E-05** |
| **4** | **2.29E-05** | **4.14E-05** |

[Table 69]

| Peptide: FF-06 | | |
|---|---|---|
| Condition | **Tris-Ni-NTA** | |
| | (-) | (+) |
| | Recovery rate (%) | Recovery rate (%) |
| **1** | **1.00E-02** | **1.86E-01** |
| **2** | **6.84E-03** | **1.49E-01** |
| **3** | **2.87E-05** | **2.03E-05** |
| **4** | **4.88E-05** | **4.17E-05** |

[Table 70]

| Peptide: FF-07 | | |
|---|---|---|
| Condition | **Tris-Ni-NTA** | |
| | **(-)** | **(+)** |
| | Recovery rate (%) | Recovery rate (%) |
| **1** | **1.04E-04** | **3.21E-03** |
| **2** | **1.75E-04** | **6.57E-03** |
| **3** | **1.16E-05** | **1.29E-05** |
| **4** | **2.08E-05** | **2.56E-05** |

As a result, it has been revealed that, in the conditions where the mRNA-display molecule and the non-biotinylated protein are in proximity by the Tris-Ni-NTA reverse transcription primer in these three kinds of peptides (FF-05 to FF-07), the recovery rate of the peptide forming a ternary complex with FKBP and FRB is increased by up to 100-fold or more compared to the conditions where the mRNA-display molecule and the non-biotinylated protein are not in proximity.

[Evaluation Example 4-2: verification using display molecule-cyclophilin A linked construct or display molecule-calcineurin linked construct]

Cyclophilin A and calcineurin were selected as target proteins, and CC-05, CC-06, and CC-07 synthesized in Synthesis Example 1 were selected as peptides forming a ternary complex with these two kinds of target proteins, then the recovery rate of the mRNA-display molecule of each peptide was measured.

(Preparation Example 4-2-1: synthesis of mRNA (CC-05 to CC-07))

From template DNA ((D-12) to (D-14)), mRNA ((mR-12) to (mR-14)) was synthesized by in vitro transcription reaction using T7 RNA polymerase and purified by RNAClean XP (Beckman Coulter, Inc.) or RNeasy MinElute Cleanup Kit (QIAGEN).

Nucleotide sequence of template DNA (D-12) (SEQ ID NO: 39) (DNA sequence: CC-05)

GGCGTAATACGACTCACTATAGGGTTAACTTTAATAAGGAGATATAAATATGTCTAC
TGCTGTGACTAGGTTGAACAGTTAGCCGACCGGCACCGGCACCGGCAAAAAAA

Nucleotide sequence of template DNA (D-13) (SEQ ID NO: 40) (DNA sequence: CC-06)

GGCGTAATACGACTCACTATAGGGTTAACTTTAATAAGGAGATATAAATATGGATTG
GCCGGGTTTGCGGACTATTGGTTAGCCGACCGGCACCGGCACCGGCAAAAAAA

Nucleotide sequence of template DNA (D-14) (SEQ ID NO: 41) (DNA sequence: CC-07)

GGCGTAATACGACTCACTATAGGGTTAACTTTAATAAGGAGATATAAATATGATTTG
GGTTCTTACTAACACTGTTGTTTAGCCGACCGGCACCGGCACCGGCAAAAAAA

Nucleotide sequence of mRNA (mR-12) (SEQ ID NO: 42) (RNA sequence: CC-05)

GGGUUAACUUUAAUAAGGAGAUAUAAAUAUGUCUACUGCUGUGACUAGGUUGAA
CAGUUAGCCGACCGGCACCGGCACCGGCAAAAAAA

Nucleotide sequence of mRNA (mR-13) (SEQ ID NO: 43) (RNA sequence: CC-06)

GGGUUAACUUUAAUAAGGAGAUAUAAAUAUGGAUUGGCCGGGUUUGCGGACUAU
UGGUUAGCCGACCGGCACCGGCACCGGCAAAAAAA

Nucleotide sequence of mRNA (mR-14) (SEQ ID NO: 44) (RNA sequence: CC-07)

GGGUUAACUUUAAUAAGGAGAUAUAAAUAUGAUUUGGGUUCUUACUAACACUGU
UGUUUAGCCGACCGGCACCGGCACCGGCAAAAAAA

(Preparation Example 4-2-2: preparation of mRNA display molecules (CC-05 to CC-07))

The mRNA display molecule was prepared in the same manner as in Preparation Example 4-1-2 of Experimental Example 4.

(Evaluation Example 4-2-1: pull-down assay of mRNA display molecule (CC-05 to CC-07))

To a buffer containing 1 × TBS, 2 mg/mL BSA (Invitrogen), 2 mM DTT, and 5 mM $CaCl_2$, the display library prepared in Preparation Example 4-2-2 was added to be 0.3 μM, Calmodulin (FUJIFILM Wako Pure Chemical Corporation) was added to be 2 μM, and the target protein was added to be as shown in Table 71, then the mixture was incubated at 4°C for 1 hour. Then, the resultant was mixed with streptavidin-coated magnetic beads, and the mixture was mixed by inversion at 4°C for 10 minutes to immobilize the target protein. Biotin was then added to be 0.08 mM, and the mixture was mixed by inversion at 4°C for another 10 minutes. The proteins prepared in Preparation Examples 2-1 to 2-4 were used as the target proteins.

[Table 71]

| Condition | Target protein |
|---|---|
| **1** | **0.4 μM CyA-FLAG-TEV-Avi-bio and 0.4 μM CnB-CnA-FLAG-Sor-His** |
| **2** | **0.4 μM CnB-CnA-FLAG-TEV-Avi-bio and 0.4 μM CyA-FLAG-Sor-His** |
| **3** | **0.4 μM CyA-FLAG-TEV-Avi-bio** |
| **4** | **0.4 μM CnB-CnA-FLAG-TEV-Avi-bio** |

Condition 1 is a condition in which cyclophilin A is biotinylated, while only calcineurin is added with a His tag. Condition 2 is a condition in which calcineurin is biotinylated, while only cyclophilin A is added with a His tag. Thus, the mRNA-display molecules which were reverse-transcribed with the Tris-Ni-NTA-added reverse transcription primer were to be incubated in the system in a state that the mRNA-display molecule and the non-biotinylated protein are in proximity. Conditions 3 and 4 are conditions in which only cyclophilin A and only calcineurin, which have been biotinylated but do not have a His tag, are present, respectively.

Streptavidin-coated magnetic beads with immobilized target proteins were collected with a magnet and the supernatant was removed. This was washed once with a buffer containing 1 × TBS, 2 mg/mL BSA, 2 mM DTT, and 5 mM $CaCl_2$ when using a reverse transcription primer without Tris-Ni-NTA, and once with a buffer containing 1 × TBS, 2 mg/mL BSA, 2 mM DTT, and 5 mM $CaCl_2$, and 500 mM imidazole when using a Tris-Ni-NTA added reverse transcription primer. The washed streptavidin-coated magnetic beads were washed several times with a wash buffer (1 × TBS, 2 mM DTT, 0.1% Tween 20 (Sigma Aldrich), 500 mM arginine), then treated with a TEV protease that recognizes and cleaves the TEV protease recognition sequence to elute the target protein and the peptide-nucleic acid conjugate that binds to the target protein.

Display molecules pulled down in the same manner as in Evaluation Example 2-2-1 of Experimental Example 2 were recovered and quantified by qPCR, and the recovery rate thereof was calculated and summarized in the following table. Further, in conditions 1 to 4, the cases in the presence or absence of Tris-Ni-NTA were also compared.

[Table 72]

| Peptide: CC-05 | | |
|---|---|---|
| Condition | Tris-Ni-NTA | |
| | (-) | (+) |
| | Recovery rate (%) | Recovery rate (%) |
| 1 | 1.63E-02 | 4.71E-02 |
| 2 | 1.41E-02 | 5.22E-01 |
| 3 | 1.80E-04 | 4.05E-04 |
| 4 | 1.58E-03 | 5.42E-03 |

[Table 73]

| Peptide: CC-06 | | |
|---|---|---|
| Condition | Tris-Ni-NTA | |
| | (-) | (+) |
| | Recovery rate (%) | Recovery rate (%) |
| 1 | 4.38E-02 | 2.64E-01 |
| 2 | 1.61E-01 | 8.07E-01 |
| 3 | 1.40E-04 | 7.82E-04 |
| 4 | 3.95E-03 | 1.13E-02 |

[Table 74]

| Peptide: CC-07 | | |
|---|---|---|
| Condition | Tris-Ni-NTA | |
| | (-) | (+) |
| | Recovery rate (%) | Recovery rate (%) |
| 1 | 3.13E-02 | 1.10E-01 |
| 2 | 5.46E-02 | 4.00E-01 |
| 3 | 7.66E-04 | 9.75E-04 |
| 4 | 2.55E-02 | 3.68E-02 |

As a result, it has been revealed that, in the conditions where the mRNA-display molecule and the non-biotinylated protein are in proximity by the Tris-Ni-NTA reverse transcription primer in these three kinds of peptides (CC-05 to CC-07), the recovery rate of the peptide forming a ternary complex with cyclophilin A and calcineurin is increased by up to 30-fold or more compared to the conditions where the mRNA-display molecule and the non-biotinylated protein are not in proximity.

The results of Experimental Example 4 composed of Preparation Example 4 and Evaluation Example 4 show that the effect of improving the efficiency of obtaining a peptide forming a complex with a plurality of kinds of proteins is also achieved with a linked construct in which a display molecule and a target protein are linked by a linker (a linkage moiety) such as a Tris-Ni-NTA reverse transcription primer.

Although not intended to be bound by any particular theory, it is believed that this is possible because a complex of the plurality of kinds of target proteins and a peptide becomes more stable by using a linked construct in which a display molecule and one target protein are linked.

The results of Experimental Example 4 show that making a display molecule and one target protein be in proximity is extremely effective when obtaining a peptide that forms a complex with the plurality of kinds of target proteins by selection by affinity.

**Claims**

1. A method of screening for a molecule of interest, comprising: (1) a step of preparing a linked construct by linking a plurality of kinds of target molecules to each other, or linking one or more kinds of target molecules and a candidate molecule-nucleic acid conjugate (linking step); and (2) a step of contacting the linked construct in which the plurality of kinds of target molecules are linked to each other with a candidate molecule-nucleic acid conjugate, or contacting the linked construct in which the one or more kinds of target molecules and the candidate molecule-nucleic acid conjugate are linked with a target molecule that is not used in the linking step (contacting step).

2. A method of screening for a molecule of interest, comprising: (1) a step of preparing a linked construct by linking a plurality of kinds of target molecules to each other (linking step); and (2) a step of contacting the linked construct prepared in the linking step with a candidate molecule-nucleic acid conjugate (contacting step).

3. A method of screening for a molecule of interest, comprising: (1) a step of preparing a linked construct by linking one or more kinds of target molecules to a candidate molecule-nucleic acid conjugate (linking step); and (2) a step of contacting the linked construct prepared in the linking step with a target molecule that is not used in the linking step (contacting step).

4. A method of screening for a molecule of interest, comprising a step of contacting a linked construct in which a plurality of kinds of target molecules are linked to each other with a candidate molecule-nucleic acid conjugate, or contacting a linked construct in which one or more kinds of target molecules and a candidate molecule-nucleic acid conjugate are linked with a target molecule that is not contained in the linked construct (contacting step).

5. A method of screening for a molecule of interest, comprising a step of contacting a linked construct in which a plurality of kinds of target molecules are linked to each other with a candidate molecule-nucleic acid conjugate.

6. A method of screening for a molecule of interest, comprising a step of contacting a linked construct in which one or more kinds of target molecules and a candidate molecule-nucleic acid conjugate are linked with a target molecule that is not contained in the linked construct.

7. The method according to claim 1, 3, 4, or 6, wherein the one or more kinds of target molecules linked to the candidate molecule-nucleic acid conjugate are one kind of target molecule.

8. A method of screening for a molecule of interest, comprising a step of contacting a plurality of kinds of target molecules in proximity to each other with a candidate molecule-nucleic acid conjugate library (contacting step).

9. The method according to claim 1, 2, 4, 5 or 8, wherein the plurality of kinds of target molecules are fused to each other to form a target fusion molecule.

10. A method of screening for a molecule of interest, comprising a step of contacting a target fusion molecule in which a plurality of kinds of target molecules are fused to each other with a candidate molecule-nucleic acid conjugate library (contacting step).

11. The method according to claim 8, wherein the plurality of kinds of target molecules in proximity to each other are immobilized on a solid phase support.

12. A method of screening for a molecule of interest, comprising a step of contacting a plurality of kinds of target molecules immobilized on the same solid phase support with a candidate molecule-nucleic acid conjugate library (contacting step).

13. The method according to claim 8, wherein the contacting step is a step of contacting a first target molecule, which is one kind selected from the plurality of kinds of target molecules, with a linked construct library including a plurality of kinds of linked constructs in which each candidate molecule-nucleic acid conjugate and a target molecule different from the first target molecule are linked.

14. A method of screening for a molecule of interest, comprising a step of contacting a first target molecule, which is one kind selected from a plurality of kinds of target molecules, with a linked construct library including a plurality of kinds of linked constructs in which each candidate molecule-nucleic acid conjugate and a target molecule different from the

first target molecule are linked.

**15.** The method according to any one of claims 1 to 14, further comprising a step of recovering a complex of at least one kind of target molecule selected from the plurality of kinds of target molecules and the candidate molecule-nucleic acid conjugate (recovery step).

**16.** The method according to claim 15, further comprising a step of eluting a nucleic acid in the candidate molecule-nucleic acid conjugate recovered by the recovery step (elution step).

**17.** The method according to claim 15 or 16, further comprising a step of amplifying a nucleic acid in a candidate molecule-nucleic acid conjugate forming the complex (amplification step).

**18.** The method according to claim 17, wherein a candidate molecule-nucleic acid conjugate library is newly generated from the nucleic acid amplified in the amplification step, and the newly generated candidate molecule-nucleic acid conjugate library is used in multiple repeating of the contacting step, the recovery step, and the amplification step.

**19.** The method according to any one of claims 15 to 18, further comprising a step of identifying a molecule of interest from a candidate molecule-nucleic acid conjugate forming the complex (identification step).

**20.** The method according to any one of claims 1 to 19, wherein the candidate molecule is a peptide.

**21.** The method according to any one of claims 1 to 20, wherein the plurality of kinds of target molecules are two kinds or more and five kinds or less of target molecules.

**22.** The method according to any one of claims 1 to 21, wherein the molecule of interest is a molecule capable of forming a complex with the plurality of kinds of target molecules.

**23.** A method of producing a peptide, comprising the method according to any one of claims 1 to 22.

## INTERNATIONAL SEARCH REPORT

International application No.
**PCT/JP2024/014861**

**A. CLASSIFICATION OF SUBJECT MATTER**

***C07K 1/14***(2006.01)i; ***C07K 19/00***(2006.01)i; ***C12N 15/12***(2006.01)i; ***C12Q 1/6844***(2018.01)i; ***G01N 33/50***(2006.01)i; ***G01N 33/68***(2006.01)i
FI: C07K1/14 ZNA; C12N15/12; C12Q1/6844 Z; C07K19/00; G01N33/50 P; G01N33/68

According to International Patent Classification (IPC) or to both national classification and IPC

**B. FIELDS SEARCHED**

Minimum documentation searched (classification system followed by classification symbols)

C07K1/00-19/00; C12Q1/00-3/00; G01N33/50; G01N33/68; C12N15/00-15/90

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2022/138892 A1 (CHUGAI SEIYAKU KABUSHIKI KAISHA) 30 June 2022 (2022-06-30) claims 1, 2, 5, paragraphs [0001], [0012], [0025], [0033], [0046] | 1-23 |
| A | JP 2022-527351 A (ELI LILLY AND CO.) 01 June 2022 (2022-06-01) entire text | 1-23 |
| A | CHEN, Q. et al. Optimization of PROTAC Ternary Complex Using DNA Encoded Library Approach. ACS CHEMICAL BIOLOGY. 06 January 2023 entire text | 1-23 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 June 2024** | **25 June 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/014861**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**
**Information on patent family members**

International application No.
**PCT/JP2024/014861**

| Patent document cited in search report | Publication date (day/month/year) | Patent family member(s) | Publication date (day/month/year) |
|---|---|---|---|
| WO 2022/138892 A1 | 30 June 2022 | EP 4257978 A1<br>claims 1, 2, 5, paragraphs [0001], [0012], [0025], [0033], [0047]<br>US 2024/0069033 A1<br>CN 116670284 A<br>KR 10-2023-0124938 A | |
| JP 2022-527351 A | 01 June 2022 | US 2021/0403902 A1<br>entire text<br>WO 2020/205836 A1<br>EP 3947671 A1<br>CN 113661240 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021235509 A
- WO 2018143145 A
- WO 2018225864 A
- WO 2017150732 A
- WO 2013100132 A
- WO 2021117848 A
- WO 2016148044 A
- WO 2022234852 A


### Non-patent literature cited in the description


- *Scientific Reports*, vol. 7, 37716
- *Cancer Res.*, 15 August 2005, vol. 65 (16), 7413-7420
- *ACS Chem. Biol.*, 2023, vol. 18 (1), 25-33
- CLOGP Reference Manual Daylight Version 4.9. 01 August 2011
- **SMITH, R.** ; **HANSCH, C.** ; **AMES, M.** *J. Pharm. Sci.*, 1975, vol. 64, 599
- Quantitative Drug Design. **MARTIN, Y.** Medicinal Research Series. Marcel Dekker, 1978
- **MAGEE, P.** *Chemtech*, 1981, vol. 11, 378
- **SMYTH, R.** ; **PFEFFER, M.** ; **VAN HARKEN, D.** ; **COHEN, A.** ; **HOTTENDORF, G.** *Antimicrob. Agents Chemother.*, 1981, vol. 10, 1004
- **KOBLIN, D.** ; **EGER II, E.** ; **JOHNSON, B.** ; **COLLINS, P.** ; **TERRELL, R.** ; **SPEARS, L.** *Anesth. Analg.*, 1981, vol. 60, 464
- **BROWN, D.** ; **FLAGG, E.** *J. Environ. Qual.*, 1981, vol. 10, 382
- **ELLINGHAUSEN, H.** ; **GUTH, J.** ; **ESER, H.** *Ecotox. Environ. Safety*, 1980, vol. 4, 26
- **LEVITAN, H.** *Proc. Nat. Acad. Sci. USA*, 1977, vol. 74, 2914
- **NEELEY, W.** ; **BRANSON, D.** ; **BLAU, G.** *Environ. Sci. Technol.*, 1974, vol. 8, 1113
- **TANFORD, C.** The Hydrophobic Effect. Wiley, 1980
- **COATS, E.** ; **GENTHER, G.** ; **DIETRICH, S.** ; **GUO, Z.** ; **HANSCH, C.** *J. Med. Chem.*, 1981, vol. 24, 1422
- **SMITH, R.** ; **HANSCH, C.** ; **LANGRIDGE, R.** *Arch. Giochem. Biophys.*, 1982, vol. 215, 319
- **MARTINEK, K.** ; **SEMENOV, A.** *J. Appl. Biochem.*, 1981, vol. 3, 93
- **NEWCOMB, M.** ; **MOORE, S.** ; **CRAM, D.** *J. Am. Chem. Soc.*, 1977, vol. 99, 6405
- **HANSCH, C.** *J. Org. Chem.*, 1978, vol. 43, 4889
- **UNGER, S.** ; **FEUERMAN, T.** *J. Chromatog.*, 1979, vol. 176, 426
- **MIRRLEES, R.** ; **TAYLOR, P.** *J. Med. Chem.*, 1976, vol. 19, 615
- **TOMLINSON, E.** *Proceedings of A.Ph.A., Symposium on Partition Coefficients, San Diego, CA*, 1982
- **TEWARI, Y.** ; **MILLER, M.** ; **WASIK, S.** ; **MARTIRE, D.** *J. Chem. Eng. Data*, 1982, vol. 27, 451
- **FUJITA, T.** ; **IWASA, J.** ; **HANSCH, C.** *J. Am. Chem. Soc.*, 1964, vol. 86, 5157
- **REKKER, R.** The Hydrophobic Fragmental Constant. Elsevier, 1977
- **HANSCH, C.** ; **LEO, A.** Substituent Constants for Correlation Analysis in Chemistry and Biology. Wiley Interscience, 1979
- **LEO., A.** ; **HOEKMAN, D.** *Perspect. in Drug Discov. & Design*, 2000, vol. 18, 19
- **CHOU, J.** ; **JURS, P.** *J. Chem. Inf. Comput. Sci.*, 1979, vol. 19, 172
- **HAMMETT, L.** Physical Organic Chemistry: Reaction Rates, Equilibria and Mechanism. McGrawHill, 1970
- **LEO, A.** *J. Chem. Soc., Perkin Trans.*, 1983, vol. II, 825
- **OGINO, A.** ; **MATSUMURA, S.** ; **FUJITA, T.** *J. Med. Chem.*, 1980, vol. 23, 437
- **LEO, A.** ; **HANSCH, C.** *J. Org. Chem.*, 1971, vol. 36, 1539
- *Advanced Drug Delivery Reviews*, 2013, vol. 65, 1357-1369
- *PNAS*, 2018, vol. 115 (51), 12961-12966
- *J. Am. Chem. Soc.*, 2005, vol. 127, 10205-10215
- *J. Am. Chem. Soc.*, 2017, vol. 139, 5351-5358
- *Proc. Natl. Acad. Sci. USA.*, 12 July 2011, vol. 108 (28), 11399-11404

- *J. Am. Chem. Soc.*, 2023, vol. 145, 24035-24051
- *J. Am. Chem. Soc.*, 2006, vol. 128 (7), 2365-2372